# EUROPÄISCHE PATENTSCHRIFT

(11) **EP 3 051 946 B1**
(45) Veröffentlichungstag und Bekanntmachung des Hinweises auf die Patenterteilung: **29.11.2017**
(21) Anmeldenummer: 14783789.2
(22) Anmeldetag: 02.10.2014
(51) Int. Cl.: A01N 43/38, C07D 209/30, C07D 209/34, A01P 15/00, A01N 43/90, A01N 47/16, C07D 471/10, C07D 491/107, C07D 209/96

(54) **VERWENDUNG SUBSTITUIERTER DIHYDROOXINDOLYLSULFONAMIDE ODER DEREN SALZE ZUR STEIGERUNG DER STRESSTOLERANZ IN PFLANZEN**
USE OF SUBSTITUTED DIHYDROOXINDOLYLSULFONAMIDES OR THEIR SALTS FOR INCREASING STRESS TOLERANCE IN PLANTS
UTILISATION DE DIHYDROOXINDOLYLSULFONAMIDES SUBSTITUÉS OU DE LEURS SELS POUR AUGMENTER LA TOLÉRANCE AU STRESS DANS DES PLANTES

(30) Priorität: 04.10.2013 EP 13187361
(43) Veröffentlichungstag der Anmeldung: 10.08.2016
(73) Patentinhaber: Bayer CropScience Aktiengesellschaft, 40789 Monheim am Rhein (DE)
(72) Erfinder: FRACKENPOHL, Jens, 60322 Frankfurt (DE); BOJACK, Guido, 65207 Wiesbaden-Naurod (DE); HELMKE, Hendrik, 65835 Liederbach (DE); LEHR, Stefan, 65835 Liederbach (DE); MÜLLER, Thomas, 60323 Frankfurt (DE); WILLMS, Lothar, 65719 Hofheim (DE); DITTGEN, Jan, 60316 Frankfurt (DE); SCHMUTZLER, Dirk, 65795 Hattersheim (DE); BICKERS, Udo, 65779 Kelkheim (DE); STREK, Harry, 65191 Wiesbaden (DE); BALTZ, Rachel, 69660 Collonges au Mont D'Or (FR)
(74) Vertreter: BIP Patents
(86) Internationale Anmeldenummer: PCT/EP2014/071195
(87) Internationale Veröffentlichungsnummer: WO 2015/049351

(56) Entgegenhaltungen:
- WO-A1-2010/077839
- WO-A1-2012/089721

## Beschreibung

Die Erfindung betrifft die Verwendung substituierter Dihydrooxindolylsulfonamide oder deren Salze zur Steigerung der Stresstoleranz in Pflanzen gegenüber abiotischem Stress, sowie zur Steigerung des Pflanzenwachstums und/oder zur Erhöhung des Pflanzenertrags.

Es ist bekannt, dass bestimmte Arylsulfonamide wie beispielsweise 2-Cyanobenzolsulfonamide insektizide Eigenschaften besitzen (vgl. z. B. EP0033984 und WO2005035486, WO2006056433, WO2007060220). 2-Cyanobenzolsulfonamide mit besonderen heterocyclischen Substitutenten werden in EP2065370 beschrieben. Es ist weiter bekannt, daß bestimmte Aryl- und Heteroaryl-substituierte Sulfonamide als Wirkstoffe gegen abiotischen Pflanzenstress eingesetzt werden können (vgl. WO2011113861). Die Wirkung von bestimmten Aryl-, Heteroaryl- und Benzylsulfonamidocarbonsäuren, -carbonsäureestern, -carbonsäureamiden und - carbonitrilen gegen abiotischen Pflanzenstress wird in WO 2012089721 und WO 2012089722 beschrieben.

Die Herstellung von Sulfamidoalkancarbonsäuren und Sulfamidoalkancarbonsäurenitrilen wird in DE847006 beschrieben. Die Verwendung ausgewählter Arylsulfonamide mit Alkylcarboxyl-Substituenten als Wuchsregulatoren vor allem zur Beschränkung der Wuchslänge von Reis- und Weizenpflanzen mit dem Ziel der Minimierung des wetterbedingten Umknickens wird in DE2544859 beschrieben, während die fungizide Wirkung bestimmter N-Cyanoalkylsulfonamide in EP176327 beschrieben wird. Es ist außerdem bekannt, daß substituierte N-Sulfonylaminoacetonitrile zur Kontrolle von Parasiten in Warmblütern eingesetzt werden können (vg. WO2004000798).

Es ist außerdem bekannt, dass substituierte Arylsulfonamide (vgl. z. B. WO2009105774, WO2006124875, WO96/36595) und substitituierte Hetarylsulfonamide (vgl. WO2009113600, WO2007122219) als pharmazeutische Wirkstoffe verwendet werden können. WO2003007931 beschreibt ebenfalls die pharmazeutische Verwendung von substituierten Naphthylsulfonamiden, während in Eur. J. Med. Chem. 2010, 45, 1760 Naphthylsulfonyl-substituierte Glutaminsäureamide und ihre Antitumorwirkung beschrieben werden. Weiterhin ist bekannt, daß Pyrrolidinyl-substituierte Arylsulfonamide als Cathepsin C-Inhibitoren bei der Behandlung von Atemwegserkrankungen (WO2009026197) oder als Antiinfektiva bei der Behandlung von Hepatitis C (WO2007092588) eingesetzt werden können. Die pharmazeutische Verwendung von N-Arylsulfonylderivaten verschiedener weiterer Aminosäuren, z.B. als Urokinaseinhibitoren (vgl. WO200005214), als Wirkstoffe zur Behandlung von Diabetes (vgl. WO2003091211), als Analgetika (vgl. WO2008131947) und als γ-Sekretasemodulatoren (vgl. WO2010108067) ist ebenfalls beschrieben.

Die Herstellung von bestimmten N-Methyl-substituierten Dihydrooxindolylsulfonamiden wird z.B. in DE2159362 sowie J. Chem. Soc. C (1971), 952-955 beschrieben, während in ACS Combinatorial Science (2012), 14, 218 die Herstellung von Spiropyrrolidinonyl-substituierten Dihydrooxindolylsulfonamiden beschrieben wird. Es ist ebenfalls bekannt, dass bestimmte substituierte Oxindolylderivate, wie z. B. Pyrrolobenzimidazolone, als pharmazeutische Wirkstoffe verwendet werden können, beispielsweise als antiproliferative Substanzen (vgl. EP1598353), als CB2-Agonisten (vgl. WO2010077839) oder als antiarrhythmische und cardiotonische Wirkstoffe (vgl. EP0431943). In EP1598353 werden Synthesewege zur Herstellung von substituierten Amino-dihydrooxindolen aufgezeigt. Es ist weiterhin bekannt, daß Oxotetrahydrochinolinylsulfonamide als Rho-Kinaseinhibitoren eingesetzt werden können (vgl. Eur. J. Med. Chem. 2008, 43, 1730).

Es ist bekannt, dass Pflanzen auf natürliche Stressbedingungen, wie beispielsweise Kälte-, Hitze-, Trockenstress (Stress verursacht durch Trockenheit und/oder Wassermangel), Verwundung, Pathogenbefall (Viren, Bakterien, Pilze, Insekten) etc. aber auch auf Herbizide mit spezifischen oder unspezifischen Abwehrmechanismen reagieren können [Pflanzenbiochemie, S. 393-462 , Spektrum Akademischer Verlag, Heidelberg, Berlin, Oxford, Hans W. Heldt, 1996.; Biochemistry and Molecular Biology of Plants, S. 1102-1203, American Society of Plant Physiologists, Rockville, Maryland, eds. Buchanan, Gruissem, Jones, 2000].

In Pflanzen sind zahlreiche Proteine und die sie codierenden Gene bekannt, die an Abwehrreaktionen gegen abiotischen Stress (z.B. Kälte, Hitze, Trockenheit, Salz, Überflutung) beteiligt sind. Diese gehören teilweise zu Signaltransduktionsketten (z.B. Transkriptionsfaktoren, Kinasen, Phosphatasen) oder bewirken eine physiologische Antwort der Pflanzenzelle (z.B. Ionentransport, Entgiftung reaktiver Sauerstoff-Spezies). Zu den Signalkettengenen der abiotischen Stressreaktion gehören u.a. Transkriptionsfaktoren der Klassen DREB und CBF (Jaglo-Ottosen et al., 1998, Science 280: 104-106). An der Reaktion auf Salzstress sind Phosphatasen vom Typ ATPK und MP2C beteiligt. Ferner wird bei Salzstress häufig die Biosynthese von Osmolyten wie Prolin oder Sucrose aktiviert. Beteiligt sind hier z.B. die Sucrose-Synthase und Prolin-Transporter (Hasegawa et al., 2000, Annu Rev Plant Physiol Plant Mol Biol 51: 463-499). Die Stressabwehr der Pflanzen gegen Kälte und Trockenheit benutzt z.T. die gleichen molekularen Mechanismen. Bekannt ist die Akkumulation von sogenannten Late Embryogenesis Abundant Proteins (LEA-Proteine), zu denen als wichtige Klasse die Dehydrine gehören (Ingram and Bartels, 1996, Annu Rev Plant Physiol Plant Mol Biol 47: 277-403, Close, 1997, Physiol Plant 100: 291-296). Es handelt sich dabei um Chaperone, die Vesikel, Proteine und Membranstrukturen in gestressten Pflanzen stabilisieren (Bray, 1993, Plant Physiol 103: 1035-1040). Außerdem erfolgt häufig eine Induktion von Aldehyd-Deydrogenasen, welche die bei oxidativem Stress entstehenden reaktiven Sauerstoff-Spezies (ROS) entgiften (Kirch et al., 2005, Plant Mol Biol 57: 315-332).
Heat Shock Faktoren (HSF) und Heat Shock Proteine (HSP) werden bei Hitzestress aktiviert und spielen hier als Chaperone eine ähnliche Rolle wie die Dehydrine bei Kälte- und Trockenstress (Yu et al., 2005, Mol Cells 19: 328-333).

Eine Reihe von pflanzenendogenen Signalstoffen, die in die Stresstoleranz bzw. die Pathogenabwehr involviert sind, sind bereits bekannt. Zu nennen sind hier beispielsweise Salicylsäure, Benzoesäure, Jasmonsäure oder Ethylen [Biochemistry and Molecular Biology of Plants, S. 850-929, American Society of Plant Physiologists, Rockville, Maryland, eds. Buchanan, Gruissem, Jones, 2000]. Einige dieser Substanzen oder deren stabile synthetische Derivate und abgeleitete Strukturen sind auch bei externer Applikation auf Pflanzen oder Saatgutbeizung wirksam und aktivieren Abwehrreaktionen, die eine erhöhte Stress- bzw. Pathogentoleranz der Pflanze zur Folge haben [Sembdner, and Parthier, 1993, Ann. Rev. Plant Physiol. Plant Mol. Biol. 44: 569-589].

Es ist weiter bekannt, dass chemische Substanzen die Toleranz von Pflanzen gegen abiotischen Stress erhöhen können. Derartige Substanzen werden dabei entweder durch Saatgut-Beizung, durch Blattspritzung oder durch Bodenbehandung appliziert. So wird eine Erhöhung der abiotischen Stresstoleranz von Kulturpflanzen durch Behandlung mit Elicitoren der Systemic Acquired Resistance (SAR) oder Abscisinsäure-Derivaten beschrieben (Schading and Wei, WO200028055; Abrams and Gusta, US5201931; Abrams et al, WO97/23441, Churchill et al., 1998, Plant Growth Regul 25: 35-45). Desweiteren wurden Effekte von Wachstumsregulatoren auf die Stresstoleranz von Kulturpflanzen beschrieben (Morrison and Andrews, 1992, J Plant Growth Regul 11: 113-117, RD-259027). In diesem Zusammenhang ist ebenfalls bekannt, dass ein wachstumsregulierendes Naphthylsulfonamid (4-Brom-N-(pyridin-2-ylmethyl)naphthalin-1-sulfonamid) die Keimung von Pflanzensamen in dergleichen Weise wie Abscisinsäure beeinflusst (Park et al. Science 2009, 324, 1068-1071). Weiterhin zeigt eine Naphthylsulfamidocarbonsäure (N-[(4-Brom-1-naphthyl)sulfonyl]-5-methoxynorvalin) eine Wirkungsweise in biochemischen Rezeptortests, die mit 4-Brom-N-(pyridin-2-ylmethyl)naphthalin-1-sulfonamid vergleichbar ist (Melcher et al. Nature Structural & Molecular Biology 2010, 17, 1102-1108). Außerdem ist bekannt, dass ein weiteres Naphthylsulfonamid, N-(6-aminohexyl)-5-chlornaphthalin-1-sulfonamid, den Calcium-Spiegel in Pflanzen beeinflusst, die einem Kälteschock ausgesetzt wurden (Cholewa et al. Can. J. Botany 1997, 75, 375-382).

Auch bei Anwendung von Fungiziden, insbesondere aus der Gruppe der Strobilurine oder der Succinat Dehydrogenase Inhibitoren werden ähnliche Effekte beobachtet, die häufig auch mit einer Ertragssteigerung einhergehen (Draber et al., DE3534948, Bartlett et al., 2002, Pest Manag Sci 60: 309). Es ist ebenfalls bekannt, dass das Herbizid Glyphosat in niedriger Dosierung das Wachstum einiger Pflanzenarten stimuliert (Cedergreen, Env. Pollution 2008, 156, 1099).

Bei osmotischem Stress ist eine Schutzwirkung durch Applikation von Osmolyten wie z.B. Glycinbetain oder deren biochemischen Vorstufen, z.B. Cholin-Derivate beobachtet worden (Chen et al., 2000, Plant Cell Environ 23: 609-618, Bergmann et al., DE4103253). Auch die Wirkung von Antioxidantien wie z.B Naphthole und Xanthine zur Erhöhung der abiotischen Stresstoleranz in Pflanzen wurde bereits beschrieben (Bergmann et al., DD277832, Bergmann et al., DD277835). Die molekularen Ursachen der Anti-Stress-Wirkung dieser Substanzen sind jedoch weitgehend unbekannt.

Es ist weiter bekannt, dass die Toleranz von Pflanzen gegenüber abiotischem Stress durch eine Modifikation der Aktivität von endogenen Poly-ADP-ribose Polymerasen (PARP) oder Poly-(ADP-ribose) glycohydrolasen (PARG) erhöht werden kann (de Block et al., The Plant Journal, 2004, 41, 95; Levine et al., FEBS Lett. 1998, 440, 1; WO0004173; WO04090140).

Somit ist bekannt, dass Pflanzen über mehrere endogene Reaktionsmechanismen verfügen, die eine wirksame Abwehr gegenüber verschiedensten Schadorganismen und/oder natürlichem abiotischem Stress bewirken können. Da sich die ökologischen und ökonomischen Anforderungen an moderne Pflanzenbehandlungsmittel laufend erhöhen, beispielsweise was deren Toxizität, Selektivität, Aufwandmenge, Rückstandsbildung und günstige Herstellbarkeit angeht, besteht die ständige Aufgabe, neue Pflanzenbehandlungsmittel zu entwickeln, die zumindest in Teilbereichen Vorteile gegenüber den bekannten aufweisen.

Daher bestand die Aufgabe der vorliegenden Erfindung darin, Verbindungen bereitzustellen, die die Toleranz gegenüber abiotischem Stress in Pflanzen weiter erhöhen, eine Stärkung des Pflanzenwachstums bewirken und/oder zur Erhöhung des Pflanzenertrags beitragen. In diesem Zusammenhang wird unter Toleranz gegenüber abiotischem Stress beispielsweise die Toleranz gegenüber Kälte-, Hitze-, Trockenstress (Stress verursacht durch Trockenheit und/oder Wassermangel), Salzen und Überflutung, explizit aber nicht die erhöhte Widerstandskraft gegen das Umknicken der Pflanzen oder von Bestandteilen derselbigen wie beispielsweise bei oder nach schweren Regenfällen und Gewitterstürmen verstanden.

Überraschenderweise wurde nun gefunden, daß substituierte Dihydrooxindolylsulfonamide zur Steigerung der Stresstoleranz in Pflanzen gegenüber abiotischem Stress, sowie zur Steigerung des Pflanzenwachstums und/oder zur Erhöhung des Pflanzenertrags verwendet werden können.

Gegenstand der vorliegenden Erfindung ist demnach die Verwendung substituierter Dihydrooxindolylsulfonamide der allgemeinen Formel (I) oder deren Salzen 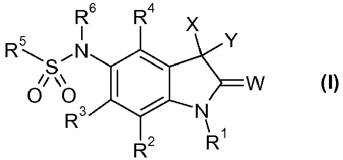 zur Toleranzerhöhung gegenüber abiotischem Stress in Pflanzen, worin
- R¹: für Wasserstoff, (C₁-C₁₀)-Alkyl, (C₃-C₈)-Cycloalkyl, (C₁-C₁₀)-Haloalkyl, (C₃-C₈)-Halocycloalkyl, (C₂-C₈)-Alkenyl, (C₂-C₈)-Haloalkenyl, (C₁-C₈)-Alkoxy-(C₁-C₈)-haloalkyl, (C₂-C₈)-Alkinyl, Aryl, Aryl-(C₁-C₈)-alkyl, Heteroaryl, Heteroaryl-(C₁-C₈)-alkyl, (C₃-C₈)-Cycloalkyl-(C₁-C₈)-alkyl, (C₂-C₈)-Haloalkinyl, Heterocyclyl, Heterocyclyl-(C₁-C₈)-alkyl, (C₁-C₈)-Alkoxy-(C₁-C₈)-alkyl, (C₁-C₈)-Alkylcarbonyl-(C₁-C₈)-alkyl, Hydroxycarbonyl-(C₁-C₈)-alkyl, (C₁-C₈)-Alkoxycarbonyl-(C₁-C₈)-alkyl, (C₂-C₈)-Alkenyloxycarbonyl-(C₁-C₈)-alkyl, (C₂-C₈)-Alkinyloxycarbonyl-(C₁-C₈)-alkyl, Aryl-(C₁-C₈)-alkoxycarbonyl-(C₁-C₈)-alkyl, (C₃-C₈)-Cycloalkoxycarbonyl-(C₁-C₈)-alkyl, (C₃-C₈)-Cycloalkyl-(C₁-C₈)-alkoxycarbonyl-(C₁-C₈)-alkyl, Aminocarbonyl-(C₁-C₈)-alkyl, (C₁-C₈)-Alkylaminocarbonyl-(C₁-C₈)-alkyl, (C₃-C₈)-Cycloalkylaminocarbonyl-(C₁-C₈)-alkyl, Aryl-(C₁-C₈)-alkylaminocarbonyl-(C₁-C₈)-alkyl, Heteroaryl-(C₁-C₈)-alkylaminocarbonyl-(C₁-C₈)-alkyl, (C₁-C₈)-Alkylthio-(C₁-C₈)-alkyl, (C₃-C₈)-Cycloalkylthio-(C₁-C₈)-alkyl, Arylthio-(C₁-C₈)-alkyl, Heterocyclylthio-(C₁-C₈)-alkyl, Heteroarylthio-(C₁-C₈)-alkyl, Aryl-(C₁-C₈)-alkylthio-(C₁-C₈)-alkyl, (C₁-C₈)-Alkylsulfinyl-(C₁-C₈)-alkyl, (C₁-C₈)-Alkylsulfonyl-(C₁-C₈)-alkyl, Arylsulfinyl-(C₁-C₈)-alkyl, Arylsulfonyl-(C₁-C₈)-alkyl, (C₃-C₈)-Cycloalkylsulfinyl-(C₁-C₈)-alkyl, (C₃-C₈)-Cycloalkylsulfonyl-(C₁-C₈)-alkyl, (C₁-C₈)-Alkoxy-(C₁-C₈)-alkoxy-(C₁-C₈)-alkyl, (C₁-C₈)-Alkylcarbonyl, (C₁-C₈)-Haloalkylcarbonyl, (C₃-C₈)-Cycloalkylcarbonyl, (C₁-C₈)-Alkoxycarbonyl, Aryl-(C₁-C₈)-alkoxycarbonyl, Arylcarbonyl, Heteroarylcarbonyl, Heterocyclylcarbonyl, Aryl-(C₁-C₈)-alkylcarbonyl, (C₁-C₈)-Alkylaminocarbonyl, (C₃-C₈)-Cycloalkylaminocarbonyl, Arylaminocarbonyl, Aryl-(C₁-C₈)-alkylaminocarbonylHeteroarylaminocarbonyl, Heterocyclylaminocarbonyl, Heteroaryl-(C₁-C₈)-alkylaminocarbonyl, Heterocyclyl-(C₁-C₈)-alkylaminocarbonyl, (C₁-C₈)-Alkylsulfonyl, (C₃-C₈)-Cycloalkylsulfonyl, Arylsulfonyl, Aryl-(C₁-C₈)-alkylsulfonyl, Heteroarylsulfonyl, Heterocyclylsulfonyl, Cyano-(C₁-C₈)-alkyl, (C₄-C₈)-Cycloalkenyl-(C₁-C₈)-alkyl, Nitro-(C₁-C₈)-alkyl, Halo-(C₁-C₈)-alkoxy-(C₁-C₈)-alkyl, Bis-[(C₁-C₈)-Alkyl]aminocarbonyl, (C₃-C₈)-Cycloalkyl-[(C₁-C₈)-Alkyl]aminocarbonyl, Aryl-[(C₁-C₈)-Alkyl]aminocarbonyl, Aryl-(C₁-C₈)-alkyl-[(C₁-C₈)-Alkyl]aminocarbonyl, (C₂-C₈)-Alkenylaminocarbonyl, (C₂-C₈)-Alkinylaminocarbonyl, (C₁-C₈)-Alkylaminosulfonyl, Bis-[(C₁-C₈)-Alkyl]aminosulfonyl, Heterocyclylsulfinyl-(C₁-C₈)-alkyl, Heteroarylsulfinyl-(C₁-C₈)-alkyl, Aryl-(C₁-C₈)-alkylsulfinyl-(C₁-C₈)-alkyl, Heterocyclylsulfonyl-(C₁-C₈)-alkyl, Heteroarylsulfonyl-(C₁-C₈)-alkyl, Aryl-(C₁-C₈)-alkylsulfonyl-(C₁-C₈)-alkyl, Bis-[(C₁-C₈)-Alkyl]aminocarbonyl-(C₁-C₈)-alkyl, (C₃-C₈)-Cycloalkyl-[(C₁-C₈)-Alkyl]aminocarbonyl-(C₁-C₈)-alkyl, Aryl-[(C₁-C₈)-Alkyl]aminocarbonyl-(C₁-C₈)-alkyl, Aryl-(C₁-C₈)-alkyl-[(C₁-C₈)-Alkyl]aminocarbonyl-(C₁-C₈)-alkyl, (C₂-C₈)-Alkenylaminocarbonyl-(C₁-C₈)-alkyl, (C₂-C₈)-Alkinylaminocarbonyl-(C₁-C₈)-alkyl, (C₁-C₈)-Alkylamino, Bis-[(C₁-C₈)-alkyl]amino, , (C₃-C₈)-Cycloalkyl[(C₁-C₈)-alkyl]amino steht,
- R², R³, R⁴: unabhängig voneinander für Wasserstoff, Halogen, (C₁-C₈)-Alkoxy, (C₁-C₈)-Alkyl, (C₁-C₈)-Haloalkyl, (C₁-C₈)-Haloalkoxy, (C₁-C₈)-Alkylthio, (C₁-C₈)-Haloalkylthio, Aryl, Aryl-(C₁-C₈)-alkyl, Heteroaryl, Heteroaryl-(C₁-C₈)-alkyl, Heterocyclyl, Heterocyclyl-(C₁-C₈)-alkyl, (C₃-C₈)-Cycloalkyl, Nitro, Amino, Hydroxy, (C₁-C₈)-Alkylamino, Bis-[(C₁-C₈)-alkyl]amino, Hydrothio, (C₁-C₈)-Alkylcarbonylamino, (C₃-C₈)-Cycloalkylcarbonylamino, Arylcarbonylamino, Heteroarylcarbonylamino, Heterocyclylcarbonylamino, Formyl, Hydroxyiminomethyl, (C₁-C₈)-Alkoxyiminomethyl, (C₃-C₈)-Cycloalkoxyiminomethyl, Aryloxyiminomethyl, (C₃-C₈)-Cycloalkyl-(C₁-C₈)-alkoxyiminomethyl, Thiocyanato, Isothiocyanato, Aryloxy, Heteroaryloxy, (C₃-C₈)-Cycloalkoxy, (C₃-C₈)-Cycloalkyl-(C₁-C₈)-alkoxy, Aryl-(C₁-C₈)-alkoxy, (C₂-C₈)-Alkinyl, (C₂-C₈)-Alkenyl, Aryl-(C₁-C₈)-alkinyl, Tris-[(C₁-C₈)-alkyl]silyl-(C₂-C₈)-alkinyl, Bis-[(C₁-C₈)-alkyl](aryl)silyl-(C₂-C₈)-alkinyl, Bis-aryl[(C₁-C₈)-alkyl]silyl-(C₂-C₈)-alkinyl, (C₃-C₈)-Cycloalkyl-(C₂-C₈)-alkinyl, Aryl-(C₂-C₈)-alkenyl, Heteroaryl-(C₂-C₈)-alkenyl, (C₃-C₈)-Cycloalkyl-(C₂-C₈)-alkenyl, (C₃-C₈)-Cycloalkyl-(C₂-C₈)-alkyl, (C₂-C₈)-Haloalkinyl, (C₂-C₈)-Haloalkenyl, (C₄-C₈)-Cycloalkenyl, (C₁-C₈)-Alkoxy-(C₁-C₈)-alkoxy-(C₁-C₈)-alkyl, (C₁-C₈)-Alkylsulfonyl, Arylsulfonyl, Heteroarylsulfonyl, (C₁-C₈)-Alkylsulfonylamino, Arylsulfonylamino, Aryl-(C₁-C₈)-alkylsulfonylamino, Heteroarylsulfonylamino, Heteroaryl-(C₁-C₈)-alkylsulfonylamino, Bis-[(C₁-C₈)-Alkyl]aminosulfonyl, (C₄-C₈)-Cycloalkenyl-(C₁-C₈)-alkyl, (C₁-C₈)-Alkylsulfinyl, Arylsulfinyl, Heteroarylsulfinyl stehen,
- R⁵: für Amino, (C₁-C₈)-Alkyl, (C₃-C₈)-Cycloalkyl, (C₃-C₈)-Cycloalkyl-(C₁-C₈)-alkyl, (C₁-C₈)-Haloalkyl, (C₃-C₈)-Halocycloalkyl, (C₄-C₈)-Cycloalkenyl, Aryl, Heteroaryl, Heterocyclyl, Aryl-(C₁-C₈)-alkyl, Heteroaryl-(C₁-C₈)-alkyl, Heterocyclyl-(C₁-C₈)-alkyl, (C₁-C₈)-Alkoxycarbonyl-(C₁-C₈)-alkyl, Aryl-(C₁-C₈)-alkoxycarbonyl-(C₁-C₈)-alkyl, (C₃-C₈)-Cycloalkoxycarbonyl-(C₁-C₈)-alkyl, (C₃-C₈)-Cycloalkyl-(C₁-C₈)-alkoxycarbonyl-(C₁-C₈)-alkyl, Heteroaryl-(C₁-C₈)-alkoxycarbonyl-(C₁-C₈)-alkyl, Aminocarbonyl-(C₁-C₈)-alkyl, (C₁-C₈)-Alkylaminocarbonyl-(C₁-C₈)-alkyl, (C₃-C₈)-Cycloalkylaminocarbonyl-(C₁-C₈)-alkyl, Aryl-(C₁-C₈)-alkylaminocarbonyl-(C₁-C₈)-alkyl, (C₁-C₈)-Alkylamino, Arylamino, (C₃-C₈)-Cycloalkylamino, Aryl-(C₁-C₈)-alkylamino, Heteroaryl-(C₁-C₈)-alkylamino, Heteroarylamino, Heterocyclylamino, Aryloxy-(C₁-C₈)-alkyl, (C₁-C₈)-Alkoxy-(C₁-C₈)-alkyl, Heteroaryloxy-(C₁-C₈)-alkyl, (C₂-C₈)-Alkenyl, (C₂-C₈)-Alkinyl, (C₂-C₈)-Alkenylamino, (C₂-C₈)-Alkinylamino, Bis-[(C₁-C₈)-alkyl]amino, Aryloxy, Bis-[(C₁-C₈)-Alkyl]amino, Aryl-(C₂-C₈)-alkenyl, Heteroaryl-(C₂-C₈)-alkenyl, Heterocyclyl-(C₂-C₈)-alkenyl, Aryloxycarbonyl-(C₁-C₈)-alkyl, Heteroaryloxycarbonyl-(C₁-C₈)-alkyl, Bis[(C₁-C₈)-alkyl]aminocarbonyl-(C₁-C₈)-alkyl, (C₁-C₈)-Alkylthio-(C₁-C₈)-alkyl, Cyano-(C₁-C₈)-alkyl, (C₁-C₈)-Alkoxy-(C₁-C₈)-alkoxy-(C₁-C₈)-alkyl steht,
- R⁶: für Wasserstoff, (C₁-C₈)-Alkyl, (C₃-C₈)-Cycloalkyl, Cyano-(C₁-C₈)-alkyl, (C₃-C₈)-Cycloalkyl-(C₁-C₈)-alkyl, (C₁-C₈)-Alkylsulfonyl, Arylsulfonyl, Heteroarylsulfonyl, (C₃-C₈)-Cycloalkylsulfonyl, Heterocyclylsulfonyl, Aryl-(C₁-C₈)-alkylsulfonyl, (C₁-C₈)-Alkylcarbonyl, Arylcarbonyl, Heteroarylcarbonyl, (C₃-C₈)-Cycloalkylcarbonyl, Heterocyclylcarbonyl, (C₁-C₈)-Alkoxycarbonyl, Aryl-(C₁-C₈)-alkoxycarbonyl, (C₁-C₈)-Haloalkylcarbonyl, (C₂-C₈)-Alkenyl, (C₂-C₈)-Alkinyl, (C₁-C₈)-Haloalkyl, Halo-(C₂-C₈)-alkinyl, Halo-(C₂-C₈)-alkenyl, (C₁-C₈)-Alkoxy-(C₁-C₈)-alkyl steht
- W: für Sauerstoff, Schwefel steht,
- X, Y: unabhängig voneinander für Wasserstoff, (C₁-C₈)-Alkyl, Halogen, (C₂-C₈)-Alkenyl, (C₂-C₈)-Alkinyl, (C₁-C₈)-Haloalkyl, Hydroxy-(C₁-C₈)-alkyl, Cyano-(C₁-C₈)-alkyl, Aryl, Heteroaryl, (C₃-C₈)-Cycloalkyl, (C₄-C₈)-Cycloalkenyl, Heterocyclyl, Cyano, Nitro, Hydroxy, (C₁-C₈)-Alkoxy, (C₁-C₈)-Alkylthio, (C₁-C₈)-Alkoxy-(C₁-C₈)-alkyl, (C₁-C₈)-Alkylthio-(C₁-C₈)-alkyl, Aryloxy, Aryl-(C₁-C₈)-alkoxy, (C₁-C₈)-Haloalkoxy, (C₁-C₈)-Haloalkylthio, (C₁-C₈)-Alkylamino, Bis-[(C₁-C₈)-Alkyl]amino, (C₁-C₈)-Alkoxy-(C₁-C₈)-alkoxy, Amino-(C₁-C₈)-alkyl, (C₁-C₈)-Alkylamino-(C₁-C₈)-alkyl, (C₃-C₈)-Cycloalkylamino-(C₁-C₈)-alkyl, Aryl-(C₁-C₈)-alkylamino-(C₁-C₈)-alkyl, Heteroaryl-(C₁-C₈)-alkylamino-(C₁-C₈)-alkyl, Heterocyclyl-(C₁-C₈)-alkylamino-(C₁-C₈)-alkyl, Heterocyclylamino-(C₁-C₈)-alkyl, Heteroarylamino-(C₁-C₈)-alkyl, (C₁-C₈)-Alkoxycarbonylamino-(C₁-C₈)-alkyl, Arylamino-(C₁-C₈)-alkyl, Aryl-(C₁-C₈)-alkoxycarbonylamino-(C₁-C₈)-alkyl, (C₃-C₈)-Cycloalkoxycarbonylamino-(C₁-C₈)-alkyl, (C₃-C₈)-Cycloalkyl-(C₁-C₈)-alkoxycarbonylamino-(C₁-C₈)-alkyl, Heteroaryl-(C₁-C₈)-alkoxycarbonylamino-(C₁-C₈)-alkyl, (C₁-C₈)-Alkylcarbonylamino-(C₁-C₈)-alkyl, (C₃-C₈)-Cycloalkylcarbonylamino-(C₁-C₈)-alkyl, Arylcarbonylamino-(C₁-C₈)-alkyl, Heteroarylcarbonylamino-(C₁-C₈)-alkyl, Heterocyclylcarbonylamino-(C₁-C₈)-alkyl, (C₂-C₈)-Alkenyloxycarbonylamino-(C₁-C₈)-alkyl, Aryl-(C₂-C₈)-Alkenylamino-(C₁-C₈)-alkyl, Arylsulfonyl-(C₁-C₈)-alkyl, Heteroarylsulfonyl-(C₁-C₈)-alkyl, (C₁-C₈)-Alkylsulfonyl-(C₁-C₈)-alkyl, (C₃-C₈)-Cycloalkylsulfonyl-(C₁-C₈)-alkyl, Arylsulfinyl-(C₁-C₈)-alkyl, Heteroarylsulfinyl-(C₁-C₈)-alkyl, (C₁-C₈)-Alkylsulfinyl-(C₁-C₈)-alkyl, (C₃-C₈)-Cycloalkylsulfinyl-(C₁-C₈)-alkyl, Bis[(C₁-C₈)-alkyl]amino-(C₁-C₈)-alkyl stehen oder
- X und Y: mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 7-gliedrigen monocyclischen oder bicyclischen Ring bilden.

Die Verbindungen der allgemeinen Formel (I) können durch Anlagerung einer geeigneten anorganischen oder organischen Säure, wie beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion. Geeignete Substituenten, die in deprotonierter Form, wie z.B. Sulfonsäuren, bestimmte Sulfonsäureamide oder Carbonsäuren, vorliegen, können innere Salze mit ihrerseits protonierbaren Gruppen, wie Aminogruppen bilden. Salzbildung kann auch durch Einwirkung einer Base auf Verbindungen der allgemeinen Formel (I) erfolgen. Geeignete Basen sind beispielsweise organische Amine, wie Trialkylamine, Morpholin, Piperidin und Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetall-salze oder Erdalkalimetallsalze, insbesondere Natrium- und Kaliumsalze, oder auch Ammoniumsalze, Salze mit organischen Aminen oder quartäre Ammoniumsalze, zum Beispiel mit Kationen der Formel [NR^{a}R^{b}R^{c}R^{d}]⁺, worin R^{a} bis R^{d} jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Im Folgenden werden die erfindungsgemäß verwendeten Verbindungen der Formel (I) und ihre Salze "Verbindungen der allgemeinen Formel (I)" bezeichnet.

Bevorzugt ist die erfindungsgemäße Verwendung von Verbindungen der allgemeinen Formel (I), worin
- R¹: für Wasserstoff, (C₁-C₁₀)-Alkyl, (C₃-C₇)-Cycloalkyl, (C₁-C₁₀)-Haloalkyl, (C₃-C₇)-Halocycloalkyl, (C₂-C₇)-Alkenyl, (C₂-C₇)-Haloalkenyl, (C₁-C₇)-Alkoxy-(C₁-C₇)-haloalkyl, (C₂-C₇)-Alkinyl, Aryl, Aryl-(C₁-C₇)-alkyl, Heteroaryl, Heteroaryl-(C₁-C₇)-alkyl, (C₃-C₇)-Cycloalkyl-(C₁-C₇)-alkyl, (C₂-C₇)-Haloalkinyl, Heterocyclyl, Heterocyclyl-(C₁-C₇)-alkyl, (C₁-C₇)-Alkoxy-(C₁-C₇)-alkyl, (C₁-C₇)-Alkylcarbonyl-(C₁-C₇)-alkyl, Hydroxycarbonyl-(C₁-C₇)-alkyl, (C₁-C₇)-Alkoxycarbonyl-(C₁-C₇)-alkyl, (C₂-C₇)-Alkenyloxycarbonyl-(C₁-C₇)-alkyl, (C₂-C₇)-Alkinyloxycarbonyl-(C₁-C₇)-alkyl, Aryl-(C₁-C₇)-alkoxycarbonyl-(C₁-C₇)-alkyl, (C₃-C₇)-Cycloalkoxycarbonyl-(C₁-C₇)-alkyl, (C₃-C₇)-Cycloalkyl-(C₁-C₇)-alkoxycarbonyl-(C₁-C₇)-alkyl, Aminocarbonyl-(C₁-C₇)-alkyl, (C₁-C₇)-Alkylaminocarbonyl-(C₁-C₇)-alkyl, (C₃-C₇)-Cycloalkylaminocarbonyl-(C₁-C₇)-alkyl, Aryl-(C₁-C₇)-alkylaminocarbonyl-(C₁-C₇)-alkyl, Heteroaryl-(C₁-C₇)-alkylaminocarbonyl-(C₁-C₇)-alkyl, (C₁-C₇)-Alkylthio-(C₁-C₇)-alkyl, (C₃-C₇)-Cycloalkylthio-(C₁-C₇)-alkyl, Arylthio-(C₁-C₇)-alkyl, Heterocyclylthio-(C₁-C₇)-alkyl, Heteroarylthio-(C₁-C₇)-alkyl, Aryl-(C₁-C₇)-alkylthio-(C₁-C₇)-alkyl, (C₁-C₇)-Alkylsulfinyl-(C₁-C₇)-alkyl, (C₁-C₇)-Alkylsulfonyl-(C₁-C₇)-alkyl, Arylsulfinyl-(C₁-C₇)-alkyl, Arylsulfonyl-(C₁-C₇)-alkyl, (C₃-C₇)-Cycloalkylsulfinyl-(C₁-C₇)-alkyl, (C₃-C₇)-Cycloalkylsulfonyl-(C₁-C₇)-alkyl, (C₁-C₇)-Alkoxy-(C₁-C₇)-alkoxy-(C₁-C₇)-alkyl, (C₁-C₇)-Alkylcarbonyl, (C₁-C₇)-Haloalkylcarbonyl, (C₃-C₇)-Cycloalkylcarbonyl, (C₁-C₇)-Alkoxycarbonyl, Aryl-(C₁-C₇)-alkoxycarbonyl, Arylcarbonyl, Heteroarylcarbonyl, Heterocyclylcarbonyl, Aryl-(C₁-C₇)-alkylcarbonyl, (C₁-C₇)-Alkylaminocarbonyl, (C₃-C₇)-Cycloalkylaminocarbonyl, Arylaminocarbonyl, Aryl-(C₁-C₇)-alkylaminocarbonyl, Heteroarylaminocarbonyl, Heterocyclylaminocarbonyl, Heteroaryl-(C₁-C₇)-alkylaminocarbonyl, Heterocyclyl-(C₁-C₇)-alkylaminocarbonyl, (C₁-C₇)-Alkylsulfonyl, (C₃-C₇)-Cycloalkylsulfonyl, Arylsulfonyl, Aryl-(C₁-C₇)-alkylsulfonyl, Heteroarylsulfonyl, Heterocyclylsulfonyl, Cyano-(C₁-C₇)-alkyl, (C₄-C₇)-Cycloalkenyl-(C₁-C₇)-alkyl, Nitro-(C₁-C₇)-alkyl, Halo-(C₁-C₇)-alkoxy-(C₁-C₇)-alkyl, Bis-[(C₁-C₇)-Alkyl]aminocarbonyl, (C₃-C₇)-Cycloalkyl-[(C₁-C₇)-Alkyl]aminocarbonyl, Aryl-[(C₁-C₇)-Alkyl]aminocarbonyl, Aryl-(C₁-C₇)-alkyl-[(C₁-C₇)-Alkyl]aminocarbonyl, (C₂-C₇)-Alkenylaminocarbonyl, (C₂-C₇)-Alkinylaminocarbonyl, (C₁-C₇)-Alkylaminosulfonyl, Bis-[(C₁-C₇)-Alkyl]aminosulfonyl, Heterocyclylsulfinyl-(C₁-C₇)-alkyl, Heteroarylsulfinyl-(C₁-C₇)-alkyl, Aryl-(C₁-C₇)-alkylsulfinyl-(C₁-C₇)-alkyl, Heterocyclylsulfonyl-(C₁-C₇)-alkyl, Heteroarylsulfonyl-(C₁-C₇)-alkyl, Aryl-(C₁-C₇)-alkylsulfonyl-(C₁-C₇)-alkyl, Bis-[(C₁-C₇)-Alkyl]aminocarbonyl-(C₁-C₇)-alkyl, (C₃-C₇)-Cycloalkyl-[(C₁-C₇)-Alkyl]aminocarbonyl-(C₁-C₇)-alkyl, Aryl-[(C₁-C₇)-Alkyl]aminocarbonyl-(C₁-C₇)-alkyl, Aryl-(C₁-C₇)-alkyl-[(C₁-C₇)-Alkyl]aminocarbonyl-(C₁-C₇)-alkyl, (C₂-C₇)-Alkenylaminocarbonyl-(C₁-C₇)-alkyl, (C₂-C₇)-Alkinylaminocarbonyl-(C₁-C₇)-alkyl, Bis-[(C₁-C₇)-alkyl]amino, (C₃-C₇)-Cycloalkyl[(C₁-C₇)-alkyl]amino steht,
- R², R³, R⁴: unabhängig voneinander für Wasserstoff, Halogen, (C₁-C₇)-Alkoxy, (C₁-C₇)-Alkyl, (C₁-C₇)-Haloalkyl, (C₁-C₇)-Haloalkoxy, (C₁-C₇)-Alkylthio, (C₁-C₇)-Haloalkylthio, Aryl, Aryl-(C₁-C₇)-alkyl, Heteroaryl, Heteroaryl-(C₁-C₇)-alkyl, Heterocyclyl, Heterocyclyl-(C₁-C₇)-alkyl, (C₃-C₇)-Cycloalkyl, Nitro, Amino, Hydroxy, (C₁-C₇)-Alkylamino, Bis-[(C₁-C₇)-alkyl]amino, Hydrothio, (C₁-C₇)-Alkylcarbonylamino, (C₃-C₇)-Cycloalkylcarbonylamino, Arylcarbonylamino, Heteroarylcarbonylamino, Heterocyclylcarbonylamino, Formyl, Hydroxyiminomethyl, (C₁-C₇)-Alkoxyiminomethyl, (C₃-C₇)-Cycloalkoxyiminomethyl, Aryloxyiminomethyl, (C₃-C₇)-Cycloalkyl-(C₁-C₇)-alkoxyiminomethyl, Thiocyanato, Isothiocyanato, Aryloxy, Heteroaryloxy, (C₃-C₇)-Cycloalkoxy, (C₃-C₇)-Cycloalkyl-(C₁-C₇)-alkoxy, Aryl-(C₁-C₇)-alkoxy, (C₂-C₇)-Alkinyl, (C₂-C₇)-Alkenyl, Aryl-(C₁-C₇)-alkinyl, Tris-[(C₁-C₇)-alkyl]silyl-(C₂-C₇)-alkinyl, Bis-[(C₁-C₇)-alkyl](aryl)silyl-(C₂-C₇)-alkinyl, Bis-aryl[(C₁-C₇)-alkyl]silyl-(C₂-C₇)-alkinyl, (C₃-C₇)-Cycloalkyl-(C₂-C₇)-alkinyl, Aryl-(C₂-C₇)-alkenyl, Heteroaryl-(C₂-C₇)-alkenyl, (C₃-C₇)-Cycloalkyl-(C₂-C₇)-alkenyl, (C₃-C₇)-Cycloalkyl-(C₂-C₇)-alkyl, (C₂-C₇)-Haloalkinyl, (C₂-C₇)-Haloalkenyl, (C₄-C₇)-Cycloalkenyl, (C₁-C₇)-Alkoxy-(C₁-C₇)-alkoxy-(C₁-C₇)-alkyl, (C₁-C₇)-Alkylsulfonyl, Arylsulfonyl, Heteroarylsulfonyl, (C₁-C₇)-Alkylsulfonylamino, Arylsulfonylamino, Aryl-(C₁-C₇)-alkylsulfonylamino, Heteroarylsulfonylamino, Heteroaryl-(C₁-C₇)-alkylsulfonylamino, Bis-[(C₁-C₇)-Alkyl]aminosulfonyl, (C₄-C₇)-Cycloalkenyl-(C₁-C₇)-alkyl, (C₁-C₇)-Alkylsulfinyl, Arylsulfinyl, Heteroarylsulfinyl stehen,
- R⁵: für Amino, (C₁-C₇)-Alkyl, (C₃-C₇)-Cycloalkyl, (C₃-C₇)-Cycloalkyl-(C₁-C₇)-alkyl, (C₁-C₇)-Haloalkyl, (C₃-C₇)-Halocycloalkyl, (C₄-C₇)-Cycloalkenyl, Aryl, Heteroaryl, Heterocyclyl, Aryl-(C₁-C₇)-alkyl, Heteroaryl-(C₁-C₇)-alkyl, Heterocyclyl-(C₁-C₇)-alkyl, (C₁-C₇)-Alkoxycarbonyl-(C₁-C₇)-alkyl, Aryl-(C₁-C₇)-alkoxycarbonyl-(C₁-C₇)-alkyl, (C₃-C₇)-Cycloalkoxycarbonyl-(C₁-C₇)-alkyl, (C₃-C₇)-Cycloalkyl-(C₁-C₇)-alkoxycarbonyl-(C₁-C₇)-alkyl, Heteroaryl-(C₁-C₇)-alkoxycarbonyl-(C₁-C₇)-alkyl, Aminocarbonyl-(C₁-C₇)-alkyl, (C₁-C₇)-Alkylaminocarbonyl-(C₁-C₇)-alkyl, (C₃-C₇)-Cycloalkylaminocarbonyl-(C₁-C₇)-alkyl, Aryl-(C₁-C₇)-alkylaminocarbonyl-(C₁-C₇)-alkyl, (C₁-C₇)-Alkylamino, Arylamino, (C₃-C₇)-Cycloalkylamino, Aryl-(C₁-C₇)-alkylamino, Heteroaryl-(C₁-C₇)-alkylamino, Heteroarylamino, Heterocyclylamino, Aryloxy-(C₁-C₇)-alkyl, (C₁-C₇)-Alkoxy-(C₁-C₇)-alkyl, Heteroaryloxy-(C₁-C₇)-alkyl, (C₂-C₇)-Alkenyl, (C₂-C₇)-Alkinyl, (C₂-C₇)-Alkenylamino, (C₂-C₇)-Alkinylamino, Bis-[(C₁-C₇)-alkyl]amino, Aryloxy, Bis-[(C₁-C₇)-Alkyl]amino, (C₁-C₇)-Alkyl-[(C₁-C₇)-Alkyl]amino, Aryl-(C₂-C₇)-alkenyl, Heteroaryl-(C₂-C₇)-alkenyl, Heterocyclyl-(C₂-C₇)-alkenyl, Aryloxycarbonyl-(C₁-C₇)-alkyl, Heteroaryloxycarbonyl-(C₁-C₇)-alkyl, Bis[(C₁-C₇)-alkyl]aminocarbonyl-(C₁-C₇)-alkyl, (C₁-C₇)-Alkylthio-(C₁-C₇)-alkyl, Cyano-(C₁-C₇)-alkyl, (C₁-C₇)-Alkoxy-(C₁-C₇)-alkoxy-(C₁-C₇)-alkyl steht,
- R⁶: für Wasserstoff, (C₁-C₇)-Alkyl, (C₃-C₇)-Cycloalkyl, Cyano-(C₁-C₇)-alkyl, (C₃-C₇)-Cycloalkyl-(C₁-C₇)-alkyl, (C₁-C₇)-Alkylsulfonyl, Arylsulfonyl, Heteroarylsulfonyl, (C₃-C₇)-Cycloalkylsulfonyl, Heterocyclylsulfonyl, Aryl-(C₁-C₇)-alkylsulfonyl, (C₁-C₇)-Alkylcarbonyl, Arylcarbonyl, Heteroarylcarbonyl, (C₃-C₇)-Cycloalkylcarbonyl, Heterocyclylcarbonyl, (C₁-C₇)-Alkoxycarbonyl, Aryl-(C₁-C₇)-alkoxycarbonyl, (C₁-C₇)-Haloalkylcarbonyl, (C₂-C₇)-Alkenyl, (C₂-C₇)-Alkinyl, (C₁-C₇)-Haloalkyl, Halo-(C₂-C₇)-alkinyl, Halo-(C₂-C₇)-alkenyl, (C₁-C₇)-Alkoxy-(C₁-C₇)-alkyl steht
- W: für Sauerstoff, Schwefel steht,
- X, Y: unabhängig voneinander für Wasserstoff, (C₁-C₇)-Alkyl, Halogen, (C₂-C₇)-Alkenyl, (C₂-C₇)-Alkinyl, (C₁-C₇)-Haloalkyl, Hydroxy-(C₁-C₇)-alkyl, Cyano-(C₁-C₇)-alkyl, Aryl, Heteroaryl, (C₃-C₇)-Cycloalkyl, (C₄-C₇)-Cycloalkenyl, Heterocyclyl, Cyano, Nitro, Hydroxy, (C₁-C₇)-Alkoxy, (C₁-C₇)-Alkylthio, (C₁-C₇)-Alkoxy-(C₁-C₇)-alkyl, (C₁-C₇)-Alkylthio-(C₁-C₇)-alkyl, Aryloxy, Aryl-(C₁-C₇)-alkoxy, (C₁-C₇)-Haloalkoxy, (C₁-C₇)-Haloalkylthio, (C₁-C₇)-Alkylamino, Bis-[(C₁-C₇)-Alkyl]amino, (C₁-C₇)-Alkoxy-(C₁-C₇)-alkoxy, Amino-(C₁-C₇)-alkyl, (C₁-C₇)-Alkylamino-(C₁-C₇)-alkyl, (C₃-C₇)-Cycloalkylamino-(C₁-C₇)-alkyl, Aryl-(C₁-C₇)-alkylamino-(C₁-C₇)-alkyl, Heteroaryl-(C₁-C₇)-alkylamino-(C₁-C₇)-alkyl, Heterocyclyl-(C₁-C₇)-alkylamino-(C₁-C₇)-alkyl, Heterocyclylamino-(C₁-C₇)-alkyl, Heteroarylamino-(C₁-C₇)-alkyl, (C₁-C₇)-Alkoxycarbonylamino-(C₁-C₇)-alkyl, Arylamino-(C₁-C₇)-alkyl, Aryl-(C₁-C₇)-alkoxycarbonylamino-(C₁-C₇)-alkyl, (C₃-C₇)-Cycloalkoxycarbonylamino-(C₁-C₇)-alkyl, (C₃-C₇)-Cycloalkyl-(C₁-C₇)-alkoxycarbonylamino-(C₁-C₇)-alkyl, Heteroaryl-(C₁-C₇)-alkoxycarbonylamino-(C₁-C₇)-alkyl, (C₁-C₇)-Alkylcarbonylamino-(C₁-C₇)-alkyl, (C₃-C₇)-Cycloalkylcarbonylamino-(C₁-C₇)-alkyl, Arylcarbonylamino-(C₁-C₇)-alkyl, Heteroarylcarbonylamino-(C₁-C₇)-alkyl, Heterocyclylcarbonylamino-(C₁-C₇)-alkyl, (C₂-C₇)-Alkenyloxycarbonylamino-(C₁-C₇)-alkyl, Aryl-(C₂-C₇)-Alkenylamino-(C₁-C₇)-alkyl, Arylsulfonyl-(C₁-C₇)-alkyl, Heteroarylsulfonyl-(C₁-C₇)-alkyl, (C₁-C₇)-Alkylsulfonyl-(C₁-C₇)-alkyl, (C₃-C₇)-Cycloalkylsulfonyl-(C₁-C₇)-alkyl, Arylsulfinyl-(C₁-C₇)-alkyl, Heteroarylsulfinyl-(C₁-C₇)-alkyl, (C₁-C₇)-Alkylsulfinyl-(C₁-C₇)-alkyl, (C₃-C₇)-Cycloalkylsulfinyl-(C₁-C₇)-alkyl, Bis[(C₁-C₇)-alkyl]amino-(C₁-C₇)-alkyl stehen oder
- X und Y: mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 7-gliedrigen monocyclischen oder bicyclischen Ring bilden.

Besonders bevorzugt ist die erfindungsgemäße Verwendung von Verbindungen der allgemeinen Formel (I), worin
- R¹: Wasserstoff, (C₁-C₈)-Alkyl, (C₃-C₆)-Cycloalkyl, (C₁-C₈)-Haloalkyl, (C₃-C₆)-Halocycloalkyl, (C₂-C₆)-Alkenyl, (C₂-C₆)-Haloalkenyl, (C₁-C₆)-Alkoxy-(C₁-C₆)-haloalkyl, (C₂-C₆)-Alkinyl, Aryl, Aryl-(C₁-C₆)-alkyl, Heteroaryl, Heteroaryl-(C₁-C₆)-alkyl, (C₃-C₆)-Cycloalkyl-(C₁-C₆)-alkyl, (C₂-C₆)-Haloalkinyl, Heterocyclyl, Heterocyclyl-(C₁-C₆)-alkyl, (C₁-C₆)-Alkoxy-(C₁-C₆)-alkyl, (C₁-C₆)-Alkylcarbonyl-(C₁-C₆)-alkyl, Hydroxycarbonyl-(C₁-C₆)-alkyl, (C₁-C₆)-Alkoxycarbonyl-(C₁-C₆)-alkyl, (C₂-C₆)-Alkenyloxycarbonyl-(C₁-C₆)-alkyl, (C₂-C₆)-Alkinyloxycarbonyl-(C₁-C₆)-alkyl, Aryl-(C₁-C₆)-alkoxycarbonyl-(C₁-C₆)-alkyl, (C₃-C₆)-Cycloalkoxycarbonyl-(C₁-C₆)-alkyl, (C₃-C₆)-Cycloalkyl-(C₁-C₆)-alkoxycarbonyl-(C₁-C₆)-alkyl, Aminocarbonyl-(C₁-C₆)-alkyl, (C₁-C₆)-Alkylaminocarbonyl-(C₁-C₆)-alkyl, (C₃-C₆)-Cycloalkylaminocarbonyl-(C₁-C₆)-alkyl, Aryl-(C₁-C₆)-alkylaminocarbonyl-(C₁-C₆)-alkyl, Heteroaryl-(C₁-C₆)-alkylaminocarbonyl-(C₁-C₆)-alkyl, (C₁-C₆)-Alkylthio-(C₁-C₆)-alkyl, (C₃-C₆)-Cycloalkylthio-(C₁-C₆)-alkyl, Arylthio-(C₁-C₆)-alkyl, Heterocyclylthio-(C₁-C₆)-alkyl, Heteroarylthio-(C₁-C₆)-alkyl, Aryl-(C₁-C₆)-alkylthio-(C₁-C₆)-alkyl, (C₁-C₆)-Alkylsulfinyl-(C₁-C₆)-alkyl, (C₁-C₆)-Alkylsulfonyl-(C₁-C₆)-alkyl, Arylsulfinyl-(C₁-C₆)-alkyl, Arylsulfonyl-(C₁-C₆)-alkyl, (C₃-C₆)-Cycloalkylsulfinyl-(C₁-C₆)-alkyl, (C₃-C₆)-Cycloalkylsulfonyl-(C₁-C₆)-alkyl, (C₁-C₆)-Alkoxy-(C₁-C₆)-alkoxy-(C₁-C₆)-alkyl, (C₁-C₆)-Alkylcarbonyl, (C₁-C₆)-Haloalkylcarbonyl, (C₃-C₆)-Cycloalkylcarbonyl, (C₁-C₆)-Alkoxycarbonyl, Aryl-(C₁-C₆)-alkoxycarbonyl, Arylcarbonyl, Heteroarylcarbonyl, Heterocyclylcarbonyl, Aryl-(C₁-C₆)-alkylcarbonyl, (C₁-C₆)-Alkylaminocarbonyl, (C₃-C₆)-Cycloalkylaminocarbonyl, Arylaminocarbonyl, Aryl-(C₁-C₆)-alkylaminocarbonyl, Heteroarylaminocarbonyl, Heterocyclylaminocarbonyl, Heteroaryl-(C₁-C₆)-alkylaminocarbonyl, Heterocyclyl-(C₁-C₆)-alkylaminocarbonyl, (C₁-C₆)-Alkylsulfonyl, (C₃-C₆)-Cycloalkylsulfonyl, Arylsulfonyl, Aryl-(C₁-C₆)-alkylsulfonyl, Heteroarylsulfonyl, Heterocyclylsulfonyl, Cyano-(C₁-C₆)-alkyl, (C₄-C₆)-Cycloalkenyl-(C₁-C₆)-alkyl, Nitro-(C₁-C₆)-alkyl, Halo-(C₁-C₆)-alkoxy-(C₁-C₆)-alkyl, Bis-[(C₁-C₆)-Alkyl]aminocarbonyl, (C₃-C₆)-Cycloalkyl-[(C₁-C₆)-Alkyl]aminocarbonyl, Aryl-[(C₁-C₆)-Alkyl]aminocarbonyl, Aryl-(C₁-C₆)-alkyl-[(C₁-C₆)-Alkyl]aminocarbonyl, (C₂-C₆)-Alkenylaminocarbonyl, (C₂-C₆)-Alkinylaminocarbonyl, (C₁-C₆)-Alkylaminosulfonyl, Bis-[(C₁-C₆)-Alkyl]aminosulfonyl, Heterocyclylsulfinyl-(C₁-C₆)-alkyl, Heteroarylsulfinyl-(C₁-C₆)-alkyl, Aryl-(C₁-C₆)-alkylsulfinyl-(C₁-C₆)-alkyl, Heterocyclylsulfonyl-(C₁-C₆)-alkyl, Heteroarylsulfonyl-(C₁-C₆)-alkyl, Aryl-(C₁-C₆)-alkylsulfonyl-(C₁-C₆)-alkyl, Bis-[(C₁-C₆)-Alkyl]aminocarbonyl-(C₁-C₆)-alkyl, (C₃-C₆)-Cycloalkyl-[(C₁-C₆)-Alkyl]aminocarbonyl-(C₁-C₆)-alkyl, Aryl-[(C₁-C₆)-Alkyl]aminocarbonyl-(C₁-C₆)-alkyl, Aryl-(C₁-C₆)-alkyl-[(C₁-C₆)-Alkyl]aminocarbonyl-(C₁-C₆)-alkyl, (C₂-C₆)-Alkenylaminocarbonyl-(C₁-C₆)-alkyl, (C₂-C₆)-Alkinylaminocarbonyl-(C₁-C₆)-alkyl, Bis-[(C₁-C₆)-alkyl]amino, (C₃-C₆)-Cycloalkyl[(C₁-C₆)-alkyl]amino steht,
- R², R³, R⁴: unabhängig voneinander für Wasserstoff, Halogen, (C₁-C₆)-Alkoxy, (C₁-C₆)-Alkyl, (C₁-C₆)-Haloalkyl, (C₁-C₆)-Haloalkoxy, (C₁-C₆)-Alkylthio, (C₁-C₆)-Haloalkylthio, Aryl, Aryl-(C₁-C₆)-alkyl, Heteroaryl, Heteroaryl-(C₁-C₆)-alkyl, Heterocyclyl, Heterocyclyl-(C₁-C₆)-alkyl, (C₃-C₆)-Cycloalkyl, Nitro, Amino, Hydroxy, (C₁-C₆)-Alkylamino, Bis-[(C₁-C₆)-alkyl]amino, Hydrothio, (C₁-C₆)-Alkylcarbonylamino, (C₃-C₆)-Cycloalkylcarbonylamino, Arylcarbonylamino, Heteroarylcarbonylamino, Heterocyclylcarbonylamino, Formyl, Hydroxyiminomethyl, (C₁-C₆)-Alkoxyiminomethyl, (C₃-C₆)-Cycloalkoxyiminomethyl, Aryloxyiminomethyl, (C₃-C₆)-Cycloalkyl-(C₁-C₆)-alkoxyiminomethyl, Thiocyanato, Isothiocyanato, Aryloxy, Heteroaryloxy, (C₃-C₆)-Cycloalkoxy, (C₃-C₆)-Cycloalkyl-(C₁-C₆)-alkoxy, Aryl-(C₁-C₆)-alkoxy, (C₂-C₆)-Alkinyl, (C₂-C₆)-Alkenyl, Aryl-(C₁-C₆)-alkinyl, Tris-[(C₁-C₆)-alkyl]silyl-(C₂-C₆)-alkinyl, Bis-[(C₁-C₆)-alkyl](aryl)silyl-(C₂-C₆)-alkinyl, Bis-aryl[(C₁-C₆)-alkyl]silyl-(C₂-C₆)-alkinyl, (C₃-C₆)-Cycloalkyl-(C₂-C₆)-alkinyl, Aryl-(C₂-C₆)-alkenyl, Heteroaryl-(C₂-C₆)-alkenyl, (C₃-C₆)-Cycloalkyl-(C₂-C₆)-alkenyl, (C₃-C₆)-Cycloalkyl-(C₂-C₆)-alkyl, (C₂-C₆)-Haloalkinyl, (C₂-C₆)-Haloalkenyl, (C₄-C₆)-Cycloalkenyl, (C₁-C₆)-Alkoxy-(C₁-C₆)-alkoxy-(C₁-C₆)-alkyl, (C₁-C₆)-Alkylsulfonyl, Arylsulfonyl, Heteroarylsulfonyl, (C₁-C₆)-Alkylsulfonylamino, Arylsulfonylamino, Aryl-(C₁-C₆)-alkylsulfonylamino, Heteroarylsulfonylamino, Heteroaryl-(C₁-C₆)-alkylsulfonylamino, Bis-[(C₁-C₆)-Alkyl]aminosulfonyl stehen,
- R⁵: für Amino, (C₁-C₆)-Alkyl, (C₃-C₆)-Cycloalkyl, (C₃-C₆)-Cycloalkyl-(C₁-C₆)-alkyl, (C₁-C₆)-Haloalkyl, (C₃-C₆)-Halocycloalkyl, (C₄-C₆)-Cycloalkenyl, Aryl, Heteroaryl, Heterocyclyl, Aryl-(C₁-C₆)-alkyl, Heteroaryl-(C₁-C₆)-alkyl, Heterocyclyl-(C₁-C₆)-alkyl, (C₁-C₆)-Alkoxycarbonyl-(C₁-C₆)-alkyl, Aryl-(C₁-C₆)-alkoxycarbonyl-(C₁-C₆)-alkyl, (C₃-C₆)-Cycloalkoxycarbonyl-(C₁-C₆)-alkyl, (C₃-C₆)-Cycloalkyl-(C₁-C₆)-alkoxycarbonyl-(C₁-C₆)-alkyl, Heteroaryl-(C₁-C₆)-alkoxycarbonyl-(C₁-C₆)-alkyl, Aminocarbonyl-(C₁-C₆)-alkyl, (C₁-C₆)-Alkylaminocarbonyl-(C₁-C₆)-alkyl, (C₃-C₆)-Cycloalkylaminocarbonyl-(C₁-C₆)-alkyl, Aryl-(C₁-C₆)-alkylaminocarbonyl-(C₁-C₆)-alkyl, (C₁-C₆)-Alkylamino, Arylamino, (C₃-C₆)-Cycloalkylamino, Aryl-(C₁-C₆)-alkylamino, Heteroaryl-(C₁-C₆)-alkylamino, Heteroarylamino, Heterocyclylamino, Aryloxy-(C₁-C₆)-alkyl, (C₁-C₆)-Alkoxy-(C₁-C₆)-alkyl, Heteroaryloxy-(C₁-C₆)-alkyl, (C₂-C₆)-Alkenyl, (C₂-C₆)-Alkinyl, (C₂-C₆)-Alkenylamino, (C₂-C₆)-Alkinylamino, Bis-[(C₁-C₆)-alkyl]amino, Aryloxy, Bis-[(C₁-C₇)-Alkyl]amino, Aryl-(C₂-C₇)-alkenyl, Heteroaryl-(C₂-C₇)-alkenyl, Heterocyclyl-(C₂-C₇)-alkenyl steht,
- R⁶: für Wasserstoff, (C₁-C₆)-Alkyl, (C₃-C₆)-Cycloalkyl, Cyano-(C₁-C₆)-alkyl, (C₃-C₆)-Cycloalkyl-(C₁-C₆)-alkyl, (C₁-C₆)-Alkylsulfonyl, Arylsulfonyl, Heteroarylsulfonyl, (C₃-C₆)-Cycloalkylsulfonyl, Heterocyclylsulfonyl, Aryl-(C₁-C₆)-alkylsulfonyl, (C₁-C₆)-Alkylcarbonyl, Arylcarbonyl, Heteroarylcarbonyl, (C₃-C₆)-Cycloalkylcarbonyl, Heterocyclylcarbonyl, (C₁-C₆)-Alkoxycarbonyl, Aryl-(C₁-C₆)-alkoxycarbonyl, (C₁-C₆)-Haloalkylcarbonyl, (C₂-C₆)-Alkenyl, (C₂-C₆)-Alkinyl, (C₁-C₆)-Haloalkyl, Halo-(C₂-C₆)-alkinyl, Halo-(C₂-C₆)-alkenyl, (C₁-C₆)-Alkoxy-(C₁-C₆)-alkyl steht
- W: für Sauerstoff, Schwefel steht,
- X, Y: unabhängig voneinander für Wasserstoff, (C₁-C₆)-Alkyl, Fluor, Chlor, (C₂-C₆)-Alkenyl, (C₁-C₆)-Haloalkyl, (C₃-C₆)-Cycloalkyl, (C₄-C₆)-Cycloalkenyl, Heterocyclyl, (C₁-C₆)-Alkoxy, (C₁-C₆)-Alkylthio, (C₁-C₆)-Alkoxy-(C₁-C₆)-alkyl, (C₁-C₆)-Alkylthio-(C₁-C₆)-alkyl, (C₁-C₆)-Haloalkoxy, (C₁-C₆)-Haloalkylthio, (C₁-C₆)-Alkoxy-(C₁-C₆)-alkoxy, Amino-(C₁-C₆)-alkyl, (C₁-C₆)-Alkylamino-(C₁-C₆)-alkyl, (C₃-C₆)-Cycloalkylamino-(C₁-C₆)-alkyl, Aryl-(C₁-C₆)-alkylamino-(C₁-C₆)-alkyl, Heteroaryl-(C₁-C₆)-alkylamino-(C₁-C₆)-alkyl, Heterocyclyl-(C₁-C₆)-alkylamino-(C₁-C₆)-alkyl, Heterocyclylamino-(C₁-C₆)-alkyl, Heteroarylamino-(C₁-C₆)-alkyl, (C₁-C₆)-Alkoxycarbonylamino-(C₁-C₆)-alkyl, Arylamino-(C₁-C₆)-alkyl, Aryl-(C₁-C₆)-alkoxycarbonylamino-(C₁-C₆)-alkyl, (C₃-C₆)-Cycloalkoxycarbonylamino-(C₁-C₆)-alkyl, (C₃-C₆)-Cycloalkyl-(C₁-C₆)-alkoxycarbonylamino-(C₁-C₆)-alkyl, Heteroaryl-(C₁-C₆)-alkoxycarbonylamino-(C₁-C₆)-alkyl, (C₁-C₆)-Alkylcarbonylamino-(C₁-C₆)-alkyl, (C₃-C₆)-Cycloalkylcarbonylamino-(C₁-C₆)-alkyl, Arylcarbonylamino-(C₁-C₆)-alkyl, Heteroarylcarbonylamino-(C₁-C₆)-alkyl, Heterocyclylcarbonylamino-(C₁-C₆)-alkyl, (C₂-C₆)-Alkenyloxycarbonylamino-(C₁-C₆)-alkyl, Aryl-(C₂-C₆)-Alkenylamino-(C₁-C₆)-alkyl, Arylsulfonyl-(C₁-C₆)-alkyl, Heteroarylsulfonyl-(C₁-C₆)-alkyl, (C₁-C₆)-Alkylsulfonyl-(C₁-C₆)-alkyl, (C₃-C₆)-Cycloalkylsulfonyl-(C₁-C₆)-alkyl, Arylsulfinyl-(C₁-C₆)-alkyl, Heteroarylsulfinyl-(C₁-C₆)-alkyl, (C₁-C₆)-Alkylsulfinyl-(C₁-C₆)-alkyl, (C₃-C₆)-Cycloalkylsulfinyl-(C₁-C₆)-alkyl, Bis[(C₁-C₆)-alkyl]amino-(C₁-C₆)-alkyl stehen oder
- X und Y: mit dem Kohlenstoffatom, an das sie gebunden sind, einen vollständig gesättigten oder teilgesättigten, gegebenenfalls durch O (Sauerstoff), S (Schwefel), N-H, (C₁-C₆)-Alkyl-N, (C₁-C₆)-Alkoxy-N, (C₁-C₆)-Alkoxycarbonyl-N, Aryl-(C₁-C₆)-alkoxycarbonyl-N unterbrochenen und gegebenenfalls weiter substituierten 3 bis 7-gliedrigen monocyclischen oder bicyclischen Ring bilden, wobei nicht mehr als zwei gleiche oder verschiedene Heteroatome aus der Gruppe O, S, N nebeneinander stehen.

Ganz besonders bevorzugt ist die erfindungsgemäße Verwendung von Verbindungen der allgemeinen Formel (I), die durch die Formeln (Ia) bis (Iz) sowie (Iab) beschrieben werden, 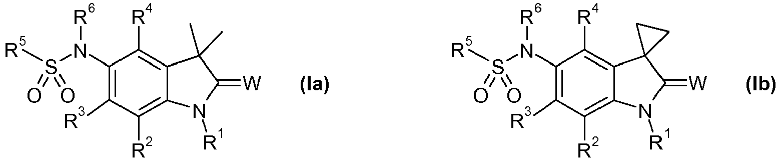 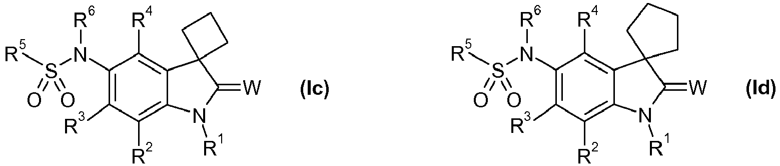 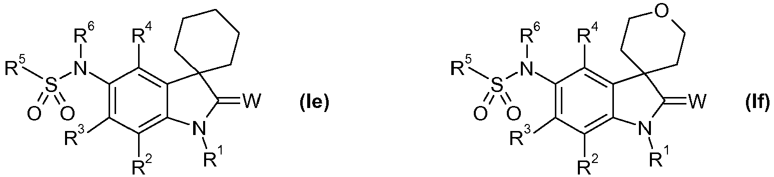 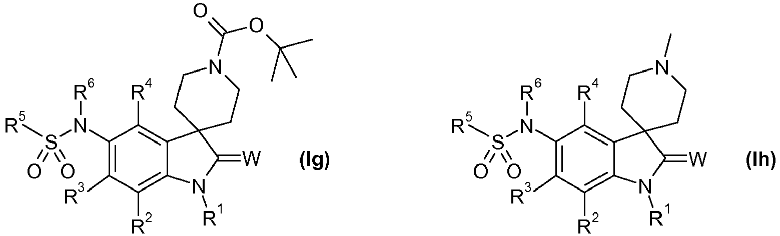 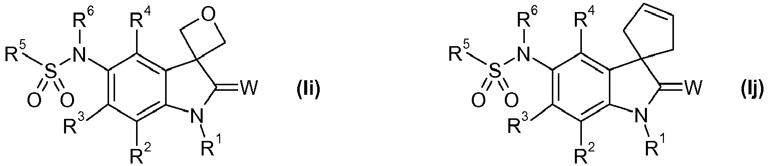 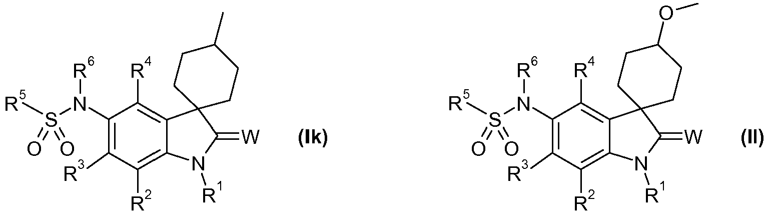 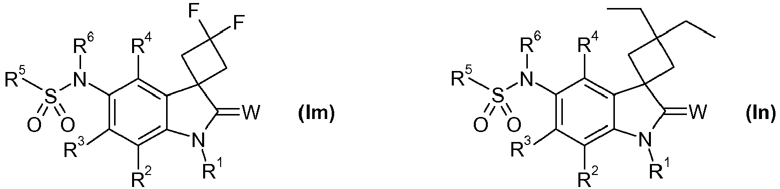 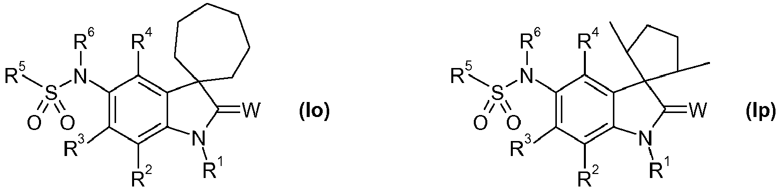 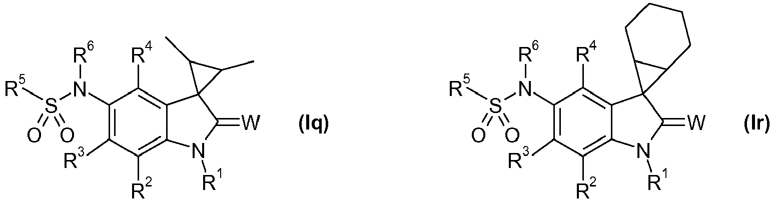 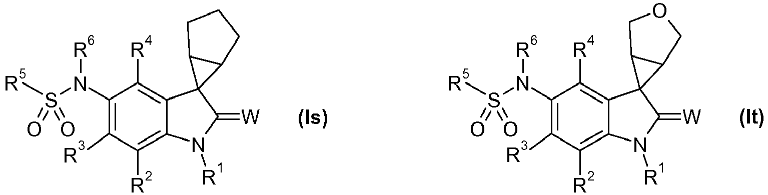 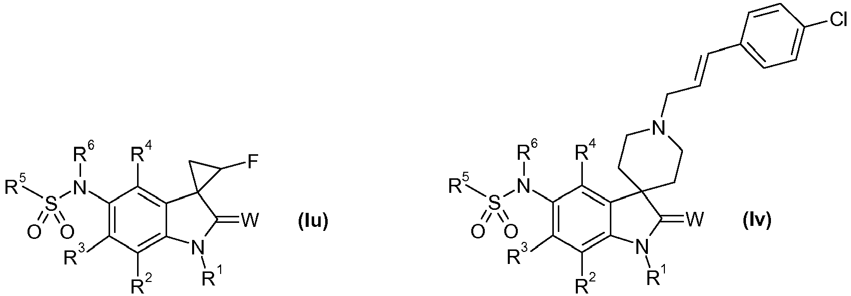 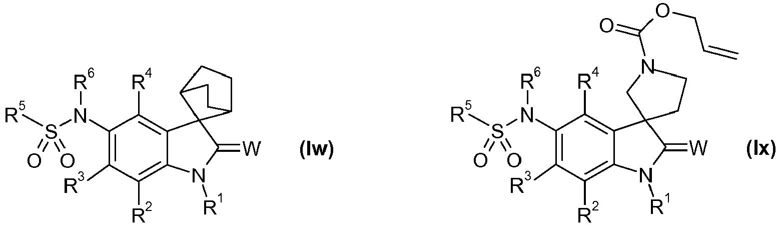 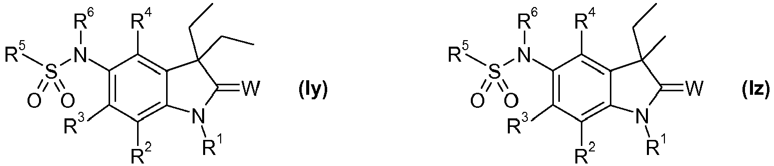 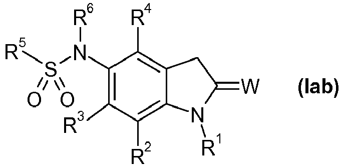 worin
- R¹: Wasserstoff, (C₁-C₆)-Alkyl, (C₃-C₆)-Cycloalkyl, (C₁-C₆)-Haloalkyl, (C₃-C₆)-Halocycloalkyl, (C₂-C₆)-Alkenyl, (C₂-C₆)-Haloalkenyl, (C₁-C₅)-Alkoxy-(C₁-C₅)-haloalkyl, (C₂-C₅)-Alkinyl, Aryl, Aryl-(C₁-C₅)-alkyl, Heteroaryl, Heteroaryl-(C₁-C₅)-alkyl, (C₃-C₆)-Cycloalkyl-(C₁-C₅)-alkyl, (C₂-C₅)-Haloalkinyl, Heterocyclyl, Heterocyclyl-(C₁-C₅)-alkyl, (C₁-C₅)-Alkoxy-(C₁-C₅)-alkyl, (C₁-C₅)-Alkylcarbonyl-(C₁-C₅)-alkyl, Hydroxycarbonyl-(C₁-C₅)-alkyl, (C₁-C₅)-Alkoxycarbonyl-(C₁-C₅)-alkyl, (C₂-C₅)-Alkenyloxycarbonyl-(C₁-C₅)-alkyl, (C₂-C₅)-Alkinyloxycarbonyl-(C₁-C₅)-alkyl, Aryl-(C₁-C₅)-alkoxycarbonyl-(C₁-C₅)-alkyl, (C₃-C₆)-Cycloalkoxycarbonyl-(C₁-C₅)-alkyl, (C₃-C₆)-Cycloalkyl-(C₁-C₅)-alkoxycarbonyl-(C₁-C₅)-alkyl, Aminocarbonyl-(C₁-C₅)-alkyl, (C₁-C₅)-Alkylaminocarbonyl-(C₁-C₅)-alkyl, (C₃-C₆)-Cycloalkylaminocarbonyl-(C₁-C₅)-alkyl, Aryl-(C₁-C₅)-alkylaminocarbonyl-(C₁-C₅)-alkyl, Heteroaryl-(C₁-C₅)-alkylaminocarbonyl-(C₁-C₅)-alkyl, (C₁-C₅)-Alkoxy-(C₁-C₅)-alkoxy-(C₁-C₅)-alkyl, (C₁-C₅)-Alkylcarbonyl, (C₁-C₅)-Haloalkylcarbonyl, (C₃-C₆)-Cycloalkylcarbonyl, (C₁-C₅)-Alkoxycarbonyl, Aryl-(C₁-C₅)-alkoxycarbonyl, Arylcarbonyl, Heteroarylcarbonyl, Heterocyclylcarbonyl, Aryl-(C₁-C₆)-alkylcarbonyl, (C₁-C₆)-Alkylaminocarbonyl, (C₃-C₆)-Cycloalkylaminocarbonyl, Arylaminocarbonyl, Aryl-(C₁-C₆)-alkylaminocarbonyl, Heteroarylaminocarbonyl, Heterocyclylaminocarbonyl, Heteroaryl-(C₁-C₆)-alkylaminocarbonyl, Heterocyclyl-(C₁-C₆)-alkylaminocarbonyl, (C₁-C₆)-Alkylsulfonyl, (C₃-C₆)-Cycloalkylsulfonyl, Arylsulfonyl, Aryl-(C₁-C₆)-alkylsulfonyl, Heteroarylsulfonyl, Heterocyclylsulfonyl, Cyano-(C₁-C₅)-alkyl, Bis-[(C₁-C₅)-alkyl]amino, (C₃-C₆)-Cycloalkyl[(C₁-C₅)-alkyl]amino steht,
- R², R³, R⁴: unabhängig voneinander für Wasserstoff, Halogen, (C₁-C₅)-Alkoxy, (C₁-C₅)-Alkyl, (C₁-C₅)-Haloalkyl, (C₁-C₅)-Haloalkoxy, (C₁-C₅)-Alkylthio, (C₁-C₅)-Haloalkylthio, Aryl, Aryl-(C₁-C₅)-alkyl, Heteroaryl, Heteroaryl-(C₁-C₅)-alkyl, Heterocyclyl, Heterocyclyl-(C₁-C₅)-alkyl, (C₃-C₆)-Cycloalkyl, Nitro, Amino, Hydroxy, (C₁-C₅)-Alkylamino, Bis-[(C₁-C₅)-alkyl]amino, Hydrothio, (C₁-C₅)-Alkylcarbonylamino, (C₃-C₆)-Cycloalkylcarbonylamino, Arylcarbonylamino, Heteroarylcarbonylamino, Heterocyclylcarbonylamino, Formyl, Hydroxyiminomethyl, (C₁-C₅)-Alkoxyiminomethyl, (C₃-C₆)-Cycloalkoxyiminomethyl, Aryloxyiminomethyl, (C₃-C₆)-Cycloalkyl-(C₁-C₅)-alkoxyiminomethyl, Thiocyanato, Isothiocyanato, Aryloxy, Heteroaryloxy, (C₃-C₆)-Cycloalkoxy, (C₃-C₆)-Cycloalkyl-(C₁-C₅)-alkoxy, Aryl-(C₁-C₅)-alkoxy, (C₂-C₅)-Alkinyl, (C₂-C₅)-Alkenyl, Aryl-(C₁-C₅)-alkinyl, Tris-[(C₁-C₅)-alkyl]silyl-(C₂-C₅)-alkinyl, Bis-[(C₁-C₅)-alkyl](aryl)silyl-(C₂-C₅)-alkinyl, Bis-aryl[(C₁-C₅)-alkyl]silyl-(C₂-C₅)-alkinyl, (C₃-C₆)-Cycloalkyl-(C₂-C₆)-alkinyl, Aryl-(C₂-C₅)-alkenyl, Heteroaryl-(C₂-C₅)-alkenyl, (C₃-C₆)-Cycloalkyl-(C₂-C₅)-alkenyl, (C₂-C₅)-Haloalkinyl, (C₂-C₅)-Haloalkenyl, (C₄-C₅)-Cycloalkenyl, (C₁-C₅)-Alkoxy-(C₁-C₅)-alkoxy-(C₁-C₅)-alkyl, (C₁-C₅)-Alkylsulfonyl, Arylsulfonyl, Heteroarylsulfonyl, (C₁-C₅)-Alkylsulfonylamino, Arylsulfonylamino, Aryl-(C₁-C₅)-alkylsulfonylamino, Heteroarylsulfonylamino, Heteroaryl-(C₁-C₅)-alkylsulfonylamino, Bis-[(C₁-C₅)-Alkyl]aminosulfonyl stehen,
- R⁵: für Amino, (C₁-C₅)-Alkyl, (C₃-C₆)-Cycloalkyl, (C₃-C₆)-Cycloalkyl-(C₁-C₅)-alkyl, (C₁-C₅)-Haloalkyl, (C₃-C₆)-Halocycloalkyl, (C₄-C₆)-Cycloalkenyl, Aryl, Heteroaryl, Heterocyclyl, Aryl-(C₁-C₅)-alkyl, Heteroaryl-(C₁-C₅)-alkyl, Heterocyclyl-(C₁-C₅)-alkyl, (C₁-C₅)-Alkoxycarbonyl-(C₁-C₅)-alkyl, Aryl-(C₁-C₅)-alkoxycarbonyl-(C₁-C₅)-alkyl, (C₃-C₆)-Cycloalkoxycarbonyl-(C₁-C₅)-alkyl, (C₃-C₆)-Cycloalkyl-(C₁-C₅)-alkoxycarbonyl-(C₁-C₅)-alkyl, Heteroaryl-(C₁-C₅)-alkoxycarbonyl-(C₁-C₅)-alkyl, Aminocarbonyl-(C₁-C₅)-alkyl, (C₁-C₅)-Alkylaminocarbonyl-(C₁-C₅)-alkyl, (C₃-C₆)-Cycloalkylaminocarbonyl-(C₁-C₅)-alkyl, Aryl-(C₁-C₅)-alkylaminocarbonyl-(C₁-C₅)-alkyl, (C₁-C₅)-Alkylamino, Arylamino, (C₃-C₆)-Cycloalkylamino, Aryl-(C₁-C₅)-alkylamino, Heteroaryl-(C₁-C₅)-alkylamino, Heteroarylamino, Heterocyclylamino, Aryloxy-(C₁-C₅)-alkyl, (C₁-C₅)-Alkoxy-(C₁-C₅)-alkyl, Heteroaryloxy-(C₁-C₅)-alkyl, (C₂-C₅)-Alkenyl, (C₂-C₅)-Alkinyl, (C₂-C₅)-Alkenylamino, (C₂-C₅)-Alkinylamino, Bis-[(C₁-C₅)-alkyl]amino, Aryloxy, (C₃-C₆)-Cycloalkyl-(C₂-C₅)-alkyl, Bis-[(C₁-C₅)-Alkyl]amino, Aryl-(C₂-C₅)-alkenyl, Heteroaryl-(C₂-C₅)-alkenyl, Heterocyclyl-(C₂-C₅)-alkenyl steht,
- R⁶: für Wasserstoff, (C₁-C₅)-Alkyl, (C₃-C₆)-Cycloalkyl, Cyano-(C₁-C₅)-alkyl, (C₃-C₆)-Cycloalkyl-(C₁-C₅)-alkyl, (C₁-C₅)-Alkylsulfonyl, Arylsulfonyl, Heteroarylsulfonyl, (C₃-C₆)-Cycloalkylsulfonyl, Heterocyclylsulfonyl, Aryl-(C₁-C₅)-alkylsulfonyl, (C₁-C₅)-Alkylcarbonyl, Arylcarbonyl, Heteroarylcarbonyl, (C₃-C₆)-Cycloalkylcarbonyl, Heterocyclylcarbonyl, (C₁-C₅)-Alkoxycarbonyl, Aryl-(C₁-C₅)-alkoxycarbonyl, (C₁-C₅)-Haloalkylcarbonyl, (C₂-C₅)-Alkenyl, (C₂-C₅)-Alkinyl, (C₁-C₅)-Haloalkyl, Halo-(C₂-C₅)-alkinyl, Halo-(C₂-C₅)-alkenyl, (C₁-C₅)-Alkoxy-(C₁-C₅)-alkyl steht
- W: für Sauerstoff oder Schwefel, bevorzugt Sauerstoff, steht.

Im Speziellen bevorzugt ist die erfindungsgemäße Verwendung von Verbindungen der allgemeinen Formel (I), die durch die Formeln (Ia) bis (Iz) sowie (Iab) beschrieben werden, 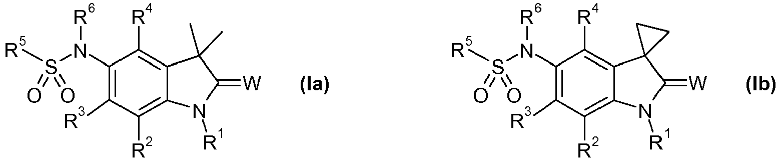 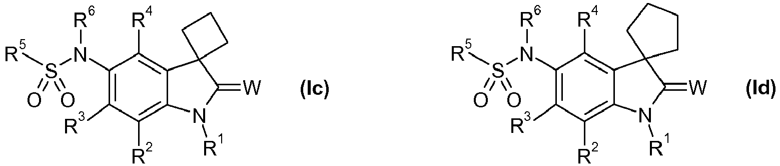 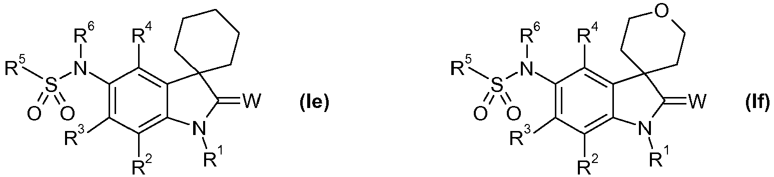 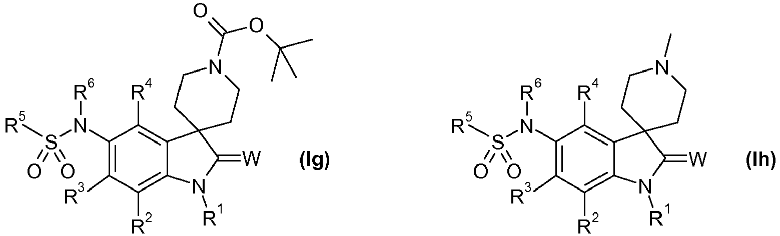 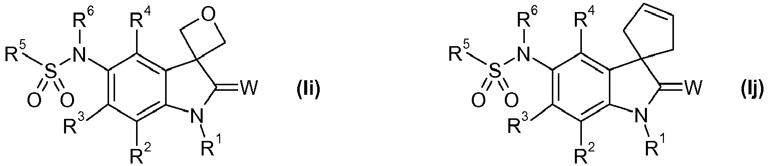 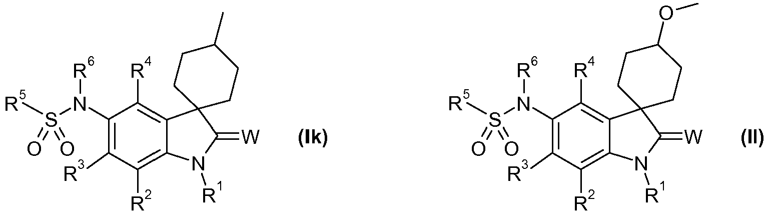 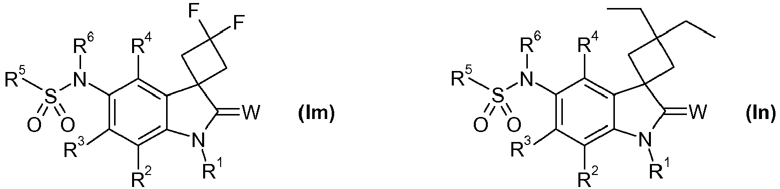 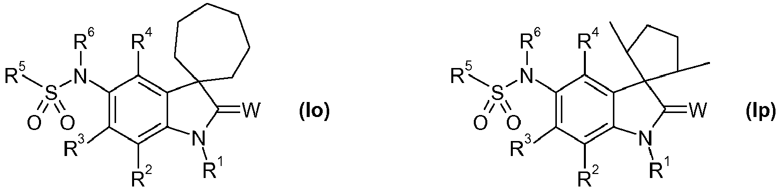 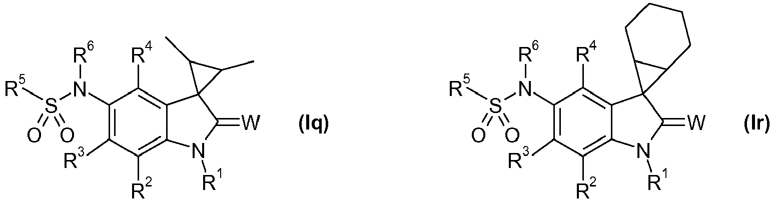 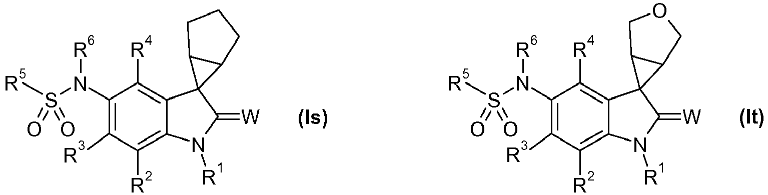 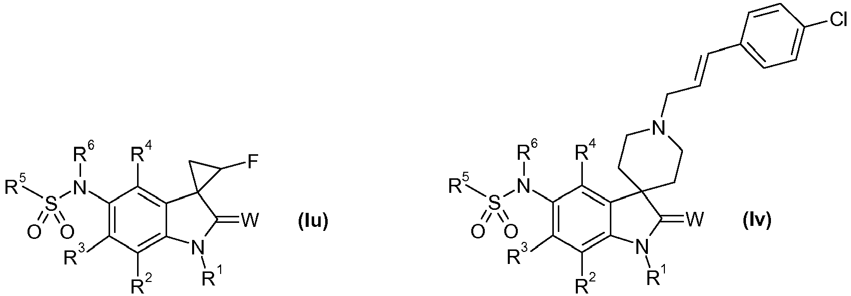 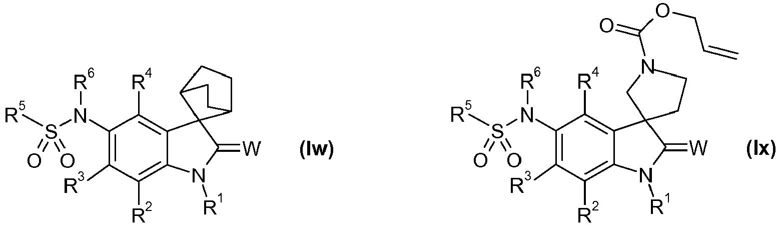 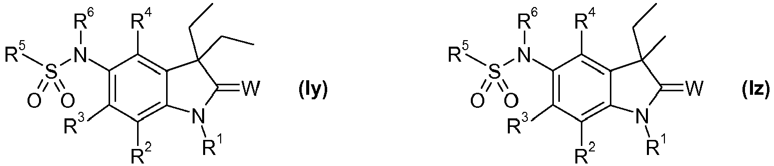 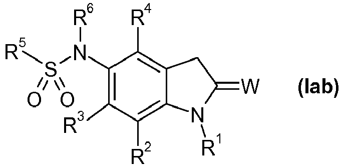 worin
- R¹: Wasserstoff, Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, Cyclopropyl, Cyclobutyl, Cyclopentyl, Cyclohexyl, Spiro[2.2]pent-1-yl, Spiro[2.3]hex-1-yl, Spiro[2.3]hex-4-yl, 3-Spiro[2.3]hex-5-yl, Spiro[3.3]hept-1-yl, Spiro[3.3]hept-2-yl, Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, Bicyclo[2.2.1]hept-2-yl, Bicyclo[2.2.2]octan-2-yl, Bicyclo[3.2.1]octan-2-yl, Bicyclo[3.2.2]nonan-2-yl, Adamantan-1-yl, Adamantan-2-yl, 1-Methylcyclopropyl, 2-Methylcyclopropyl, 2,2-Dimethylcyclopropyl, 2,3-Dimethylcyclopropyl, 1,1'-Bi(cyclopropyl)-1-yl, 1,1'-Bi(cyclopropyl)-2-yl, 2'-Methyl-1,1'-bi(cyclopropyl)-2-yl, 1-Cyanopropyl, 2-Cyanopropyl, 1-Methylcyclobutyl, 2-Methylcyclobutyl, 3-Methylcyclobutyl, 1-Cyanocyclobutyl, 2-Cyanocyclobutyl, 3-Cyanocyclobutyl, 1-Allylcyclopropyl, 1-Vinylcyclobutyl, 1-Vinylcyclopropyl, 1-Ethylcyclopropyl, 2-Ethylcyclopropyl, 1-Ethylcyclobutyl, 2-Ethylcyclobutyl, 3-Ethylcyclobutyl, 4-Methylcyclohexyl, 4-Methoxycyclohexyl, 4-Ethoxycyclohexyl, 4-n-Propyloxycyclohexyl, 4-Hydroxycyclohexyl, 4-Methoxycyclobutyl, 1-Cyclopropylcyclobutyl, 1-Prop-2-enylcyclobutyl, 2-Ethyl-3-Methylcyclobutyl,1-Propylcyclopropyl, 1-Methyl-2-propylcyclopropyl, 2-Propylcyclopropyl, 1-Propylcyclobutyl, 2-Propylcyclobutyl, 3-Propylcyclobutyl, 1-iso-Propylcyclobutyl, 1-iso-Propylcyclopropyl, 2-iso-Propylcyclopropyl, 3-iso-Propylcyclobutyl, 2-Dimethylaminocyclobutyl, 3-Dimethylaminocyclobutyl, 1-Butylcyclobutyl, 2-Butylcyclobutyl, 1-Butylcyclopropyl, 3-Butylcyclobutyl, 2-Butylcyclopropyl, 1-iso-Butylcyclobutyl, 3-tert-Butylcyclobutyl, 3,3-Diethylcyclobutyl, 2,2-Diethylcyclopropyl, 2-Methylidencyclopropyl, 1-Methoxymethylcyclopropyl, 1-iso-Butylcyclopropyl,2,2-Difluorethyl, 2,2,2-Trifluorethyl, 3,3,3-Trifluorpropyl, Ethenyl, 1-Propenyl, 2-Propenyl, 1-Methyl-ethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl, Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl, 1-Ethyl-1-methyl-2-propinyl, Cyanomethyl, Cyanoethyl, Cyano-n-Propyl, Cyano-n-Butyl, (C₂-C₆)-Haloalkenyl, (C₁-C₅)-Alkoxy-(C₁-C₅)-haloalkyl, gegebenenfalls substituiertes Phenyl, Aryl-(C₁-C₅)-alkyl, Heteroaryl, Heteroaryl-(C₁-C₅)-alkyl, (C₃-C₆)-Cycloalkyl-(C₁-C₅)-alkyl, (C₂-C₅)-Haloalkinyl, Heterocyclyl, Heterocyclyl-(C₁-C₅)-alkyl, (C₁-C₅)-Alkoxy-(C₁-C₅)-alkyl, (C₁-C₅)-Alkylcarbonyl-(C₁-C₅)-alkyl, Hydroxycarbonyl-(C₁-C₅)-alkyl, (C₁-C₅)-Alkoxycarbonyl-(C₁-C₅)-alkyl, (C₂-C₅)-Alkenyloxycarbonyl-(C₁-C₅)-alkyl, (C₂-C₅)-Alkinyloxycarbonyl-(C₁-C₅)-alkyl, Aryl-(C₁-C₅)-alkoxycarbonyl-(C₁-C₅)-alkyl, (C₃-C₆)-Cycloalkoxycarbonyl-(C₁-C₅)-alkyl, (C₃-C₆)-Cycloalkyl-(C₁-C₅)-alkoxycarbonyl-(C₁-C₅)-alkyl, Aminocarbonyl-(C₁-C₅)-alkyl, (C₁-C₅)-Alkylaminocarbonyl-(C₁-C₅)-alkyl, (C₃-C₆)-Cycloalkylaminocarbonyl-(C₁-C₅)-alkyl, Aryl-(C₁-C₅)-alkylaminocarbonyl-(C₁-C₅)-alkyl, Heteroaryl-(C₁-C₅)-alkylaminocarbonyl-(C₁-C₅)-alkyl, (C₁-C₅)-Alkoxy-(C₁-C₅)-alkoxy-(C₁-C₅)-alkyl, (C₁-C₅)-Alkylcarbonyl, (C₁-C₅)-Haloalkylcarbonyl, (C₃-C₆)-Cycloalkylcarbonyl, (C₁-C₅)-Alkoxycarbonyl, Aryl-(C₁-C₅)-alkoxycarbonyl, Arylcarbonyl, Heteroarylcarbonyl, Heterocyclylcarbonyl, Aryl-(C₁-C₆)-alkylcarbonyl, (C₁-C₆)-Alkylaminocarbonyl, (C₃-C₆)-Cycloalkylaminocarbonyl, Arylaminocarbonyl, Aryl-(C₁-C₆)-alkylaminocarbonyl, Heteroarylaminocarbonyl, Heterocyclylaminocarbonyl, Heteroaryl-(C₁-C₆)-alkylaminocarbonyl, Heterocyclyl-(C₁-C₆)-alkylaminocarbonyl, (C₁-C₆)-Alkylsulfonyl, (C₃-C₆)-Cycloalkylsulfonyl, Arylsulfonyl, Aryl-(C₁-C₆)-alkylsulfonyl, Heteroarylsulfonyl, Heterocyclylsulfonyl, Dimethylamino, Diethylamino, Methyl(ethyl)amino, Methyl(n-propyl)amino, Methyl(iso-Propyl)amino steht,
- R², R³, R⁴: unabhängig voneinander für Wasserstoff, Fluor, Chlor, Brom, Iod, Methoxy, Ethoxy, n-Propyloxy, iso-Propyloxy, Methyl, Ethyl, iso-Propyl, Trifluormethyl, Difluormethyl, Pentafluorethyl, Trifluormethoxy, Difluormethoxy, 2,2-Difluorethoxy, 3,3,3-Trifluorethoxy, Methylthio, Ethylthio, Trifluormethylthio, gegebenenfalls substituiertes Phenyl, Benzyl, Phenylethyl, p-Chlorphenylethyl, Heteroaryl, Heterocyclyl, Cyclopropyl, Cyclobutyl, Nitro, Hydroxy, Dimethylamino, Diethylamino, Formyl, Hydroxyiminomethyl, Methoxyiminomethyl, Ethoxyiminomethyl, Cyclopropylmethoxymethyl, Phenyloxy, p-Chlorphenyloxy, p-Trifluormethylphenyloxy, m-Chlorphenyloxy, m-Trifluormethylphenyloxy, 2,4-Dichlorphenyloxy, Heteroaryloxy, Benzyloxy, Ethinyl, Prop-1-inyl, (C₂-C₅)-Alkenyl, Phenylethinyl, p-Chlorphenylethinyl, p-Trifluormethylphenylethinyl, p-Methoxyphenylethinyl, p-Fluorphenylethinyl, m-Chlorphenylethinyl, m-Trifluormethylphenylethinyl, m-Methoxyphenylethinyl, m-Fluorphenylethinyl, Trimethylsilylethinyl, Triethylsilylethinyl, Triisopropylsilylethinyl, 2-Pyridylethinyl, 3-Pyridylethinyl, 4-Chlor-3-pyridylethinyl stehen,
- R⁵: für Amino, Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl. Cyclopropyl, Cyclobutyl, Cyclopentyl, Cyclohexyl, Cyclopropylmethyl, Cyclobutylmethyl, Cyclopentylmethyl, Cyclohexylmethyl, Trifluormethyl, Difluormethyl, 2,2-Difluorethyl, 2,2,2-Trifluorethyl, 3,3,3-Trifluorpropyl, Pentafluorethyl, Heptafluor-n-Propyl, Heptafluor-iso-propyl, Nonafluor-n-butyl, (C₃-C₆)-Halocycloalkyl, (C₄-C₆)-Cycloalkenyl, gegebenenfalls substituiertes Phenyl, Heteroaryl, Heterocyclyl, Aryl-(C₁-C₅)-alkyl, Heteroaryl-(C₁-C₅)-alkyl, Heterocyclyl-(C₁-C₅)-alkyl, (C₁-C₅)-Alkoxycarbonyl-(C₁-C₅)-alkyl, Aryl-(C₁-C₅)-alkoxycarbonyl-(C₁-C₅)-alkyl, (C₃-C₆)-Cycloalkoxycarbonyl-(C₁-C₅)-alkyl, (C₃-C₆)-Cycloalkyl-(C₁-C₅)-alkoxycarbonyl-(C₁-C₅)-alkyl, Heteroaryl-(C₁-C₅)-alkoxycarbonyl-(C₁-C₅)-alkyl, Aminocarbonyl-(C₁-C₅)-alkyl, (C₁-C₅)-Alkylaminocarbonyl-(C₁-C₅)-alkyl, (C₃-C₆)-Cycloalkylaminocarbonyl-(C₁-C₅)-alkyl, Aryl-(C₁-C₅)-alkylaminocarbonyl-(C₁-C₅)-alkyl, (C₁-C₅)-Alkylamino, Arylamino, (C₃-C₆)-Cycloalkylamino, Aryl-(C₁-C₅)-alkylamino, Heteroaryl-(C₁-C₅)-alkylamino, Heteroarylamino, Heterocyclylamino, Aryloxy-(C₁-C₅)-alkyl, (C₁-C₅)-Alkoxy-(C₁-C₅)-alkyl, Heteroaryloxy-(C₁-C₅)-alkyl, (C₂-C₅)-Alkenyl, (C₂-C₅)-Alkinyl, (C₂-C₅)-Alkenylamino, (C₂-C₅)-Alkinylamino, Bis-[(C₁-C₅)-alkyl]amino, Aryloxy, Bis-[(C₁-C₅)-Alkylamino, Aryl-(C₂-C₅)-alkenyl, Heteroaryl-(C₂-C₅)-alkenyl, Heterocyclyl-(C₂-C₅)-alkenyl steht,
- R⁶: für Wasserstoff, Methyl, Ethyl, iso-Propyl, n-Propyl, n-Butyl, iso-Butyl, tert.-Butyl, n-Pentyl, iso-Pentyl, neo-Pentyl, n- Hexyl, Cyclopropyl, Cyclobutyl, Cyclopentyl, Cyclohexyl, Cyclopropylmethyl, Cyclobutylmethyl, Cyclopentylmethyl, Cyclohexylmethyl, Cyanomethyl, Cyanoethyl, Cyano-n-propyl, (C₁-C₅)-Alkylsulfonyl, Arylsulfonyl, Heteroarylsulfonyl, (C₃-C₆)-Cycloalkylsulfonyl, Heterocyclylsulfonyl, Aryl-(C₁-C₅)-alkylsulfonyl, (C₁-C₅)-Alkylcarbonyl, Arylcarbonyl, Heteroarylcarbonyl, (C₃-C₆)-Cycloalkylcarbonyl, Heterocyclylcarbonyl, (C₁-C₅)-Alkoxycarbonyl, Aryl-(C₁-C₅)-alkoxycarbonyl, (C₁-C₅)-Haloalkylcarbonyl, (C₂-C₅)-Alkenyl, (C₂-C₅)-Alkinyl, 2,2-Difluorethyl, 2,2,2-Trifluorethyl, 3,3,3-Trifluorpropyl Halo-(C₂-C₅)-alkinyl, Halo-(C₂-C₅)-alkenyl, (C₁-C₅)-Alkoxy-(C₁-C₅)-alkyl steht und
- W: für Sauerstoff oder Schwefel, bevorzugt Sauerstoff, steht.

Die oben aufgeführten allgemeinen oder in Vorzugsbereichen aufgeführten Restedefinitionen gelten sowohl für die Endprodukte der allgemeinen Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Gemäß Stand der Technik sind die zuvor genannten substituierten Dihydrooxindolylsulfonamide der allgemeinen Formel (I) im Wesentlichen ebenfalls noch nicht bekannt. Somit gelten als weiterer Teil der Erfindung substituierte Dihydrooxindolylsulfonamide, der allgemeinen Formel (I) oder ihre Salze, die durch die Formeln (Ib) bis (If), (Ii) bis (Iu) und (Iw) beschrieben werden, 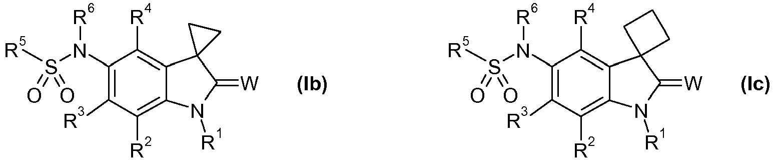 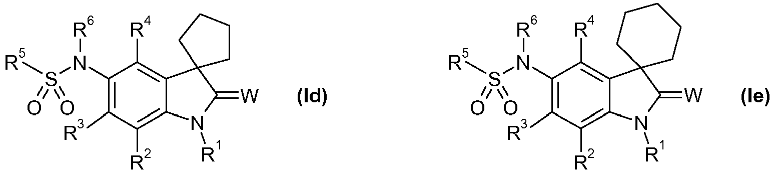 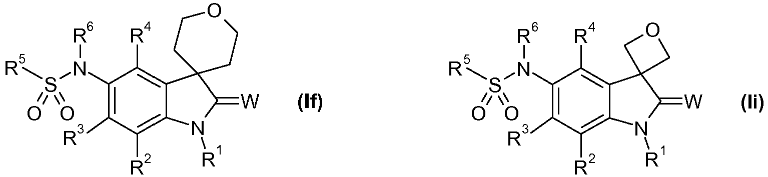 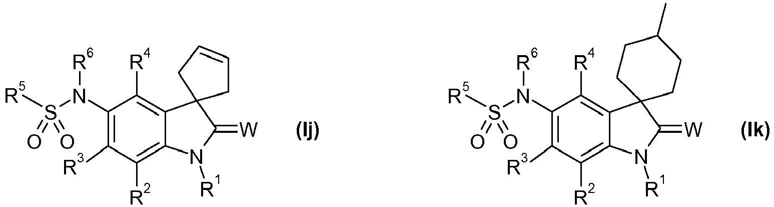 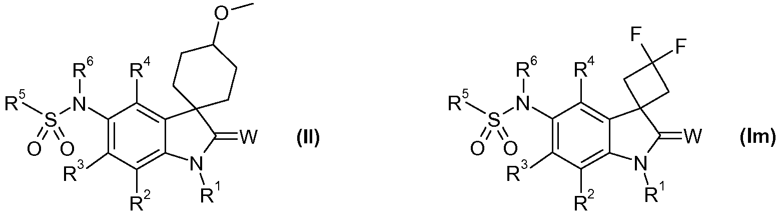 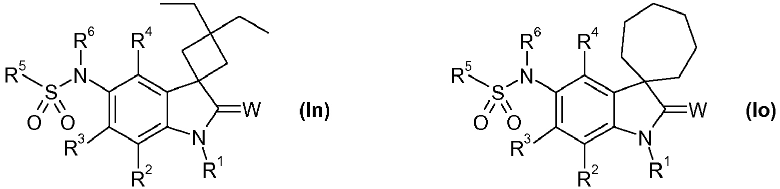 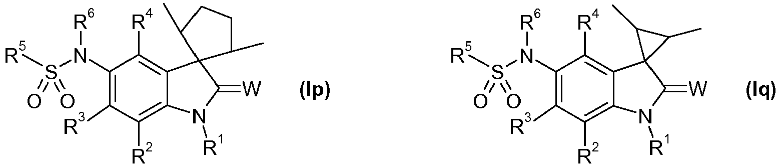 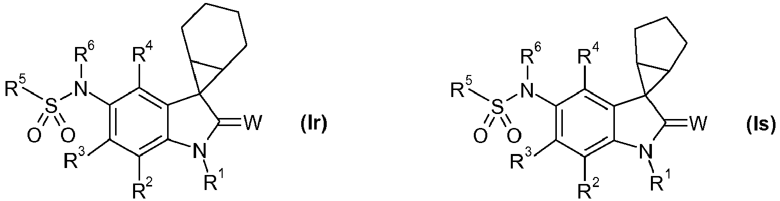 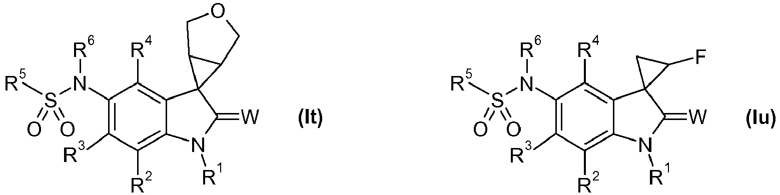 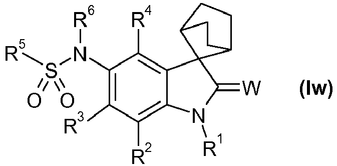 worin
- R¹: für Wasserstoff, (C₁-C₆)-Alkyl, (C₃-C₆)-Cycloalkyl, (C₁-C₆)-Haloalkyl, (C₃-C₆)-Halocycloalkyl, (C₂-C₆)-Alkenyl, (C₂-C₆)-Haloalkenyl, (C₁-C₅)-Alkoxy-(C₁-C₅)-haloalkyl, (C₂-C₆)-Alkinyl, Aryl-(C₁-C₅)-alkyl, Heteroaryl-(C₁-C₅)-alkyl, (C₃-C₆)-Cycloalkyl-(C₁-C₅)-alkyl, (C₂-C₅)-Haloalkinyl, Heterocyclyl, Heterocyclyl-(C₁-C₅)-alkyl, (C₁-C₅)-Alkoxy-(C₁-C₅)-alkyl, (C₁-C₅)-Alkylcarbonyl-(C₁-C₅)-alkyl, Hydroxycarbonyl-(C₁-C₅)-alkyl, (C₁-C₅)-Alkoxycarbonyl-(C₁-C₅)-alkyl, (C₂-C₅)-Alkenyloxycarbonyl-(C₁-C₅)-alkyl, (C₂-C₅)-Alkinyloxycarbonyl-(C₁-C₅)-alkyl, Aryl-(C₁-C₅)-alkoxycarbonyl-(C₁-C₅)-alkyl, (C₃-C₆)-Cycloalkoxycarbonyl-(C₁-C₅)-alkyl, (C₃-C₆)-Cycloalkyl-(C₁-C₅)-alkoxycarbonyl-(C₁-C₅)-alkyl, Aminocarbonyl-(C₁-C₅)-alkyl, (C₁-C₅)-Alkylaminocarbonyl-(C₁-C₅)-alkyl, (C₃-C₆)-Cycloalkylaminocarbonyl-(C₁-C₅)-alkyl, Aryl-(C₁-C₅)-alkylaminocarbonyl-(C₁-C₅)-alkyl, Heteroaryl-(C₁-C₅)-alkylaminocarbonyl-(C₁-C₅)-alkyl, (C₁-C₅)-Alkylthio-(C₁-C₅)-alkyl, (C₃-C₆)-Cycloalkylthio-(C₁-C₅)-alkyl, Arylthio-(C₁-C₅)-alkyl, Heterocyclylthio-(C₁-C₅)-alkyl, Heteroarylthio-(C₁-C₅)-alkyl, Aryl-(C₁-C₅)-alkylthio-(C₁-C₅)-alkyl, (C₁-C₅)-Alkylsulfinyl-(C₁-C₅)-alkyl, (C₁-C₅)-Alkylsulfonyl-(C₁-C₅)-alkyl, Arylsulfinyl-(C₁-C₅)-alkyl, Arylsulfonyl-(C₁-C₅)-alkyl, (C₃-C₆)-Cycloalkylsulfinyl-(C₁-C₅)-alkyl, (C₃-C₆)-Cycloalkylsulfonyl-(C₁-C₅)-alkyl, (C₁-C₅)-Alkoxy-(C₁-C₅)-alkoxy-(C₁-C₅)-alkyl, (C₁-C₅)-Alkylcarbonyl, (C₃-C₆)-Cycloalkylcarbonyl, (C₁-C₅)-Alkoxycarbonyl, Aryl-(C₁-C₅)-alkoxycarbonyl, Arylcarbonyl, Heteroarylcarbonyl, Heterocyclylcarbonyl, Aryl-(C₁-C₅)-alkylcarbonyl, (C₁-C₅)-Alkylaminocarbonyl, (C₃-C₆)-Cycloalkylaminocarbonyl, Arylaminocarbonyl, Aryl-(C₁-C₅)-alkylaminocarbonyl, (C₁-C₅)-Alkylsulfonyl, (C₃-C₆)-Cycloalkylsulfonyl, Arylsulfonyl, Heteroarylsulfonyl, Heterocyclylsulfonyl, Cyano-(C₁-C₅)-alkyl, Bis-[(C₁-C₅)-alkyl]amino, (C₃-C₆)-Cycloalkyl[(C₁-C₅)-alkyl]amino steht,
- R², R³, R⁴: unabhängig voneinander für Wasserstoff, Fluor, Chlor, Brom, Iod, (C₁-C₅)-Alkoxy, (C₁-C₅)-Alkyl, (C₁-C₅)-Haloalkyl, (C₁-C₅)-Haloalkoxy, (C₁-C₅)-Alkylthio, (C₁-C₅)-Haloalkylthio, Aryl, Heteroaryl, Heterocyclyl, (C₃-C₆)-Cycloalkyl stehen,
- R⁵: für Amino, (C₁-C₅)-Alkyl, (C₃-C₆)-Cycloalkyl, (C₃-C₆)-Cycloalkyl-(C₁-C₅)-alkyl, (C₁-C₅)-Haloalkyl, (C₃-C₆)-Halocycloalkyl, (C₄-C₆)-Cycloalkenyl, gegebenenfalls substituiertes Phenyl, Heteroaryl, Heterocyclyl, Aryl-(C₁-C₅)-alkyl, Heteroaryl-(C₁-C₅)-alkyl, Heterocyclyl-(C₁-C₅)-alkyl, (C₁-C₅)-Alkoxycarbonyl-(C₁-C₅)-alkyl, Aryl-(C₁-C₅)-alkoxycarbonyl-(C₁-C₅)-alkyl, (C₃-C₆)-Cycloalkoxycarbonyl-(C₁-C₆)-alkyl, (C₃-C₆)-Cycloalkyl-(C₁-C₅)-alkoxycarbonyl-(C₁-C₅)-alkyl, Heteroaryl-(C₁-C₅)-alkoxycarbonyl-(C₁-C₅)-alkyl, Aminocarbonyl-(C₁-C₅)-alkyl, (C₁-C₅)-Alkylaminocarbonyl-(C₁-C₅)-alkyl, (C₃-C₆)-Cycloalkylaminocarbonyl-(C₁-C₅)-alkyl, Aryl-(C₁-C₅)-alkylaminocarbonyl-(C₁-C₅)-alkyl, (C₁-C₅)-Alkylamino, Bis-[(C₁-C₅)-alkyl]amino, Arylamino, (C₃-C₆)-Cycloalkylamino, Aryl-(C₁-C₅)-alkylamino, Heteroaryl-(C₁-C₅)-alkylamino, Heteroarylamino, Heterocyclylamino, (C₂-C₅)-Alkenylamino, (C₂-C₅)-Alkinylamino, Aryloxy-(C₁-C₅)-alkyl, Heteroaryloxy-(C₁-C₈)-alkyl, (C₁-C₅)-Alkoxy-(C₁-C₅)-alkyl, (C₂-C₆)-Alkenyl, (C₂-C₆)-Alkinyl, Cyano-(C₁-C₅)-alkyl, Aryloxy, Aryl-(C₂-C₅)-alkenyl, Heteroaryl-(C₂-C₈)-alkenyl, Heterocyclyl-(C₂-C₅)-alkenyl steht,
- R⁶: für Wasserstoff, (C₁-C₅)-Alkyl, (C₃-C₆)-Cycloalkyl, Cyano-(C₁-C₅)-alkyl, (C₃-C₆)-Cycloalkyl-(C₁-C₅)-alkyl, (C₁-C₅)-Alkylsulfonyl, Arylsulfonyl, Aryl-(C₁-C₅)-alkylsulfonyl, Heteroarylsulfonyl, (C₃-C₆)-Cycloalkylsulfonyl, Heterocyclylsulfonyl, (C₁-C₅)-Alkylcarbonyl, Arylcarbonyl, Heteroarylcarbonyl, (C₃-C₆)-Cycloalkylcarbonyl, Heterocyclylcarbonyl, (C₁-C₅)-Alkoxycarbonyl, Aryl-(C₁-C₅)-alkoxycarbonyl, (C₁-C₅)-Haloalkylcarbonyl, (C₂-C₅)-Alkenyl, (C₂-C₅)-Alkinyl, (C₁-C₅)-Haloalkyl, Halo-(C₂-C₅)-alkinyl, Halo-(C₂-C₅)-alkenyl, (C₁-C₅)-Alkoxy-(C₁-C₅)-alkyl steht,
- W: für Sauerstoff oder Schwefel, bevorzugt Sauerstoff, steht.

Besonders bevorzugt sind Verbindungen der allgemeinen Formel (I), die durch die Formeln (Ib) bis (Ie), (Ij) bis (Is), (Iu) und (Iw) beschrieben werden, 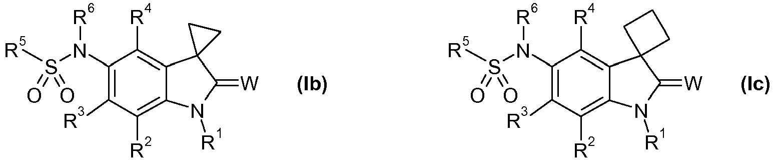 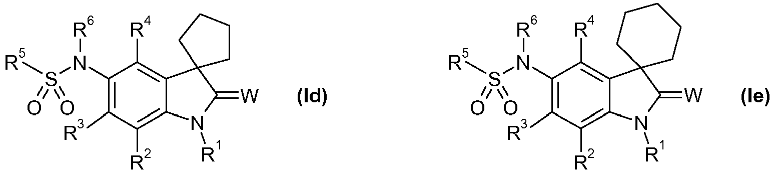 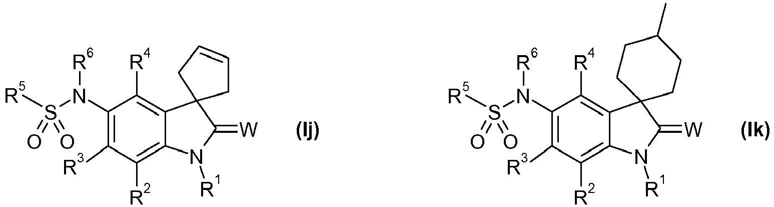 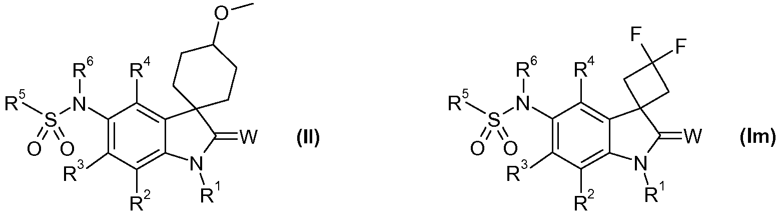 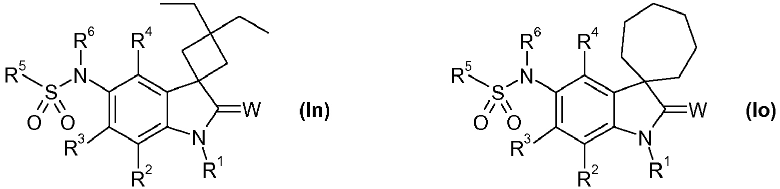 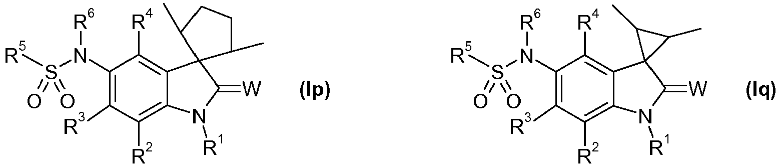 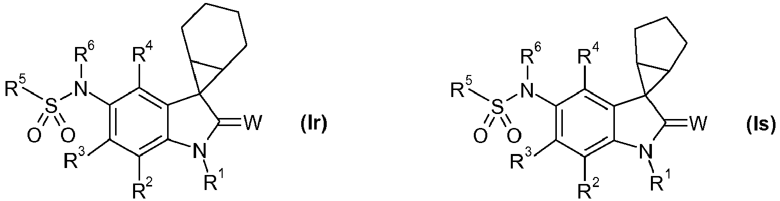 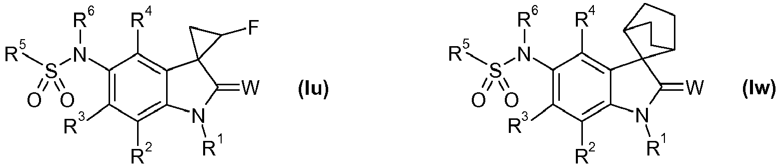 worin
- R¹: für Wasserstoff, Methyl, Ethyl, n-Propyl, 1-Methylethyl, n-Butyl, 1-Me-thylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropyl, Cyclobutyl, Cyclopentyl, Cyclohexyl, Spiro[2.2]pent-1-yl, Spiro[2.3]hex-1-yl, Spiro[2.3]hex-4-yl, 3-Spiro[2.3]hex-5-yl, Spiro[3.3]hept-1-yl, Spiro[3.3]hept-2-yl, Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, Bicyclo[2.2.1]hept-2-yl, Bicyclo[2.2.2]octan-2-yl, Bicyclo[3.2.1]octan-2-yl, Bicyclo[3.2.2]nonan-2-yl, Adamantan-1-yl, Adamantan-2-yl, 1-Methylcyclopropyl, 2-Methylcyclopropyl, 2,2-Dimethylcyclopropyl, 2,3-Dimethylcyclopropyl, 1,1'-Bi(cyclopropyl)-1-yl, 1,1'-Bi(cyclopropyl)-2-yl, 2'-Methyl-1,1'-bi(cyclopropyl)-2-yl, 1-Cyanopropyl, 2-Cyanopropyl, 1-Methylcyclobutyl, 2-Methylcyclobutyl, 3-Methylcyclobutyl, 1-Cyanocyclobutyl, 2-Cyanocyclobutyl, 3-Cyanocyclobutyl, 1-Allylcyclopropyl, 1-Vinylcyclobutyl, 1-Vinylcyclopropyl, 1-Ethylcyclopropyl, 2-Ethylcyclopropyl, 1-Ethylcyclobutyl, 2-Ethylcyclobutyl, 3-Ethylcyclobutyl, 4-Methylcyclohexyl, 4-Methoxycyclohexyl, 4-Ethoxycyclohexyl, 4-n-Propyloxycyclohexyl, 4-Hydroxycyclohexyl, 4-Methoxycyclobutyl, 1-Cyclopropylcyclobutyl, 1-Prop-2-enylcyclobutyl, 2-Ethyl-3-Methylcyclobutyl,1-Propylcyclopropyl, 1-Methyl-2-propylcyclopropyl, 2-Propylcyclopropyl, 1-Propylcyclobutyl, 2-Propylcyclobutyl, 3-Propylcyclobutyl, 1-iso-Propylcyclobutyl, 1-iso-Propylcyclopropyl, 2-iso-Propylcyclopropyl, 3-iso-Propylcyclobutyl, 2-Dimethylaminocyclobutyl, 3-Dimethylaminocyclobutyl, 1-Butylcyclobutyl, 2-Butylcyclobutyl, 1-Butylcyclopropyl, 3-Butylcyclobutyl, 2-Butylcyclopropyl, 1-iso-Butylcyclobutyl, 3-tert-Butylcyclobutyl, 3,3-Diethylcyclobutyl, 2,2-Diethylcyclopropyl, 2-Methylidencyclopropyl, 1-Methoxymethylcyclopropyl, 1-iso-Butylcyclopropyl,2,2-Difluorethyl, 2,2,2-Trifluorethyl, 3,3,3-Trifluorpropyl, Ethenyl, 1-Propenyl, 2-Propenyl, 1-Methyl-ethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, Ethinyl, 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl, 1-Ethyl-1-methyl-2-propinyl, (C₃-C₆)-Halocycloalkyl, (C₂-C₅)-Haloalkenyl, (C₁-C₅)-Alkoxy-(C₁-C₅)-haloalkyl, Benzyl, p-Chlorbenzyl, p-Methoxybenzyl, p-Trifluormethylbenzyl, p-Methylbenzyl, p-Fluorbenzyl, p-Brombenzyl, p-lodbenzyl, p-Methylthiobenzyl, p-Trifluormethoxybenzyl, p-Nitrobenzyl, p-Trifluormethylthiobenzyl, m-Chlorbenzyl, m-Methoxybenzyl, m-Trifluormethylbenzyl, m-Methylbenzyl, m-Fluorbenzyl, m-Brombenzyl, m-lodbenzyl, m-Methylthiobenzyl, m-Trifluormethoxybenzyl, m-Nitrobenzyl, m-Trifluormethylthiobenzyl, o-Chlorbenzyl, o-Methoxybenzyl, o-Trifluormethylbenzyl, o-Methylbenzyl, o-Fluorbenzyl, o-Brombenzyl, o-lodbenzyl, o-Methylthiobenzyl, o-Trifluormethoxybenzyl, o-Nitrobenzyl, o-Trifluormethylthiobenzyl, p-Methoxycarbonylbenzyl, p-Ethoxycarbonylbenzyl, m-Methoxycarbonylbenzyl, m-Ethoxycarbonylbenzyl, 2,4-Dichlorbenzyl, 3,5-Dichlorbenzyl, 2,4-Difluorbenzyl, 3,5-Difluorbenzyl, 3,4-Dichlorbenzyl, 3,4-Difluorbenzyl, 2,5-Dichlorbenzyl, Phenylethyl, p-Chlorphenylethyl, p-Methoxyphenylethyl, p-Trifluormethylphenylethyl, p-Fluorphenylethyl, p-Trifluormethoxyphenylethyl, p-Trifluormethylthiophenylethyl, p-Methylphenylethyl, p-Nitrophenylethyl, p-Methoxycarbonylphenylethyl, p-Ethoxycarbonylphenylethyl, m-Chlorphenylethyl, m-Methoxyphenylethyl, m-Trifluormethylphenylethyl, m-Fluorphenylethyl, m-Trifluormethoxyphenylethyl, m-Trifluormethylthiophenylethyl, m-Methylphenylethyl, m-Nitrophenylethyl, m-Methoxycarbonylphenylethyl, m-Ethoxycarbonylphenylethyl, o-Chlorphenylethyl, o-Methoxyphenylethyl, o-Trifluormethylphenylethyl, o-Fluorphenylethyl, o-Trifluormethoxyphenylethyl, o-Trifluormethylthiophenylethyl, o-Methylphenylethyl, o-Nitrophenylethyl, o-Methoxycarbonylphenylethyl, o-Ethoxycarbonylphenylethyl, Heteroaryl-(C₁-C₅)-alkyl, (C₁-C₅)-Haloalkenyl, Heterocyclyl, Heterocyclyl-(C₁-C₅)-alkyl, (C₁-C₅)-Alkoxy-(C₁-C₅)-alkyl, (C₁-C₅)-Alkylcarbonyl-(C₁-C₅)-alkyl, Hydroxycarbonyl-(C₁-C₅)-alkyl, (C₁-C₅)-Alkoxycarbonyl-(C₁-C₅)-alkyl, (C₂-C₅)-Alkenyloxycarbonyl-(C₁-C₅)-alkyl, (C₂-C₅)-Alkinyloxycarbonyl-(C₁-C₅)-alkyl, Aryl-(C₁-C₅)-alkoxycarbonyl-(C₁-C₅)-alkyl, (C₃-C₆)-Cycloalkoxycarbonyl-(C₁-C₅)-alkyl, (C₃-C₆)-Cycloalkyl-(C₁-C₅)-alkoxycarbonyl-(C₁-C₅)-alkyl, Aminocarbonyl-(C₁-C₅)-alkyl, (C₁-C₅)-Alkylaminocarbonyl-(C₁-C₅)-alkyl, (C₃-C₆)-Cycloalkylaminocarbonyl-(C₁-C₅)-alkyl, Aryl-(C₁-C₅)-alkylaminocarbonyl-(C₁-C₅)-alkyl, Heteroaryl-(C₁-C₅)-alkylaminocarbonyl-(C₁-C₅)-alkyl, (C₁-C₅)-Alkylthio-(C₁-C₅)-alkyl, (C₃-C₆)-Cycloalkylthio-(C₁-C₅)-alkyl, Arylthio-(C₁-C₅)-alkyl, Heterocyclylthio-(C₁-C₅)-alkyl, Heteroarylthio-(C₁-C₅)-alkyl, Aryl-(C₁-C₅)-alkylthio-(C₁-C₅)-alkyl, (C₁-C₅)-Alkylsulfinyl-(C₁-C₅)-alkyl, (C₁-C₅)-Alkylsulfonyl-(C₁-C₅)-alkyl, Arylsulfinyl-(C₁-C₅)-alkyl, Arylsulfonyl-(C₁-C₅)-alkyl, (C₃-C₆)-Cycloalkylsulfinyl-(C₁-C₅)-alkyl, (C₃-C₆)-Cycloalkylsulfonyl-(C₁-C₅)-alkyl, (C₁-C₅)-Alkoxy-(C₁-C₅)-alkoxy-(C₁-C₅)-alkyl, (C₁-C₅)-Alkylcarbonyl, (C₃-C₆)-Cycloalkylcarbonyl, (C₁-C₅)-Alkoxycarbonyl, Aryl-(C₁-C₅)-alkoxycarbonyl, Arylcarbonyl, Heteroarylcarbonyl, Heterocyclylcarbonyl, Aryl-(C₁-C₅)-alkylcarbonyl, (C₁-C₅)-Alkylaminocarbonyl, (C₃-C₆)-Cycloalkylaminocarbonyl, Arylaminocarbonyl, Aryl-(C₁-C₅)-alkylaminocarbonyl, (C₁-C₅)-Alkylsulfonyl, (C₃-C₆)-Cycloalkylsulfonyl, Arylsulfonyl, Heteroarylsulfonyl, Heterocyclylsulfonyl, Bis-[(C₁-C₅)-alkyl]amino, (C₃-C₆)-Cycloalkyl[(C₁-C₅)-alkyl]amino steht,
- R², R³, R⁴: unabhängig voneinander für Wasserstoff, Fluor, Chlor, Brom, Iod, Methoxy, Ethoxy, n-Propyloxy, iso-Propyloxy, Methyl, Ethyl, iso-Propyl, Trifluormethyl, Difluormethyl, Pentafluorethyl, Trifluormethoxy, Difluormethoxy,2,2-Difluorethoxy, 3,3,3-Trifluorethoxy, Methylthio, Ethylthio, Trifluormethylthio, gegebenenfalls substituiertes Phenyl, Heteroaryl, Heterocyclyl, Cyclopropyl, Cyclobutyl, Cyclopentyl, Cyclohexyl stehen,
- R⁵: für Amino, Methyl, Ethyl, n-Propyl, 1-Methylethyl, n-Butyl, 1-Me-thylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropyl, Cyclobutyl, Cyclopentyl, Cyclohexyl, Spiro[2.2]pent-1-yl, Spiro[2.3]hex-1-yl, Spiro[2.3]hex-4-yl, 3-Spiro[2.3]hex-5-yl, Cyclopropylmethyl, Cyclobutylmethyl, Cyclopentylmethyl, Cyclohexylmethyl, Trifluormethyl, Difluormethyl, 2,2-Difluorethyl, 2,2,2-Trifluorethyl, 3,3,3-Trifluorpropyl, Pentafluorethyl, Heptafluor-n-Propyl, Heptafluor-iso-propyl, Nonafluor-n-butyl, (C₃-C₆)-Halocycloalkyl, (C₄-C₆)-Cycloalkenyl, gegebenenfalls substituiertes Phenyl, Heteroaryl, Heterocyclyl, Aryl-(C₁-C₅)-alkyl, Heteroaryl-(C₁-C₅)-alkyl, Heterocyclyl-(C₁-C₅)-alkyl, (C₁-C₅)-Alkoxycarbonyl-(C₁-C₅)-alkyl, Aryl-(C₁-C₅)-alkoxycarbonyl-(C₁-C₅)-alkyl, (C₁-C₆)-Cycloalkoxycarbonyl-(C₁-C₅)-alkyl, (C₃-C₆)-Cycloalkyl-(C₁-C₅)-alkoxycarbonyl-(C₁-C₅)-alkyl, Heteroaryl-(C₁-C₅)-alkoxycarbonyl-(C₁-C₅)-alkyl, Aminocarbonyl-(C₁-C₅)-alkyl, (C₁-C₅)-Alkylaminocarbonyl-(C₁-C₅)-alkyl, (C₃-C₆)-Cycloalkylaminocarbonyl-(C₁-C₅)-alkyl, Aryl-(C₁-C₅)-alkylaminocarbonyl-(C₁-C₅)-alkyl, (C₁-C₅)-Alkylamino, Arylamino, (C₃-C₆)-Cycloalkylamino, Aryl-(C₁-C₅)-alkylamino, Heteroaryl-(C₁-C₅)-alkylamino, Heteroarylamino, Heterocyclylamino, (C₂-C₅)-Alkenylamino, (C₂-C₅)-Alkinylamino, Aryloxy-(C₁-C₅)-alkyl, Heteroaryloxy-(C₁-C₅)-alkyl, (C₁-C₅)-Alkoxy-(C₁-C₅)-alkyl, Phenylethenyl, p-Chlorphenylethenyl, p-Methylphenylethenyl, p-Methoxyphenylethenyl, p-Trifluormethylphenylethenyl, p-Fluorphenylethenyl, p-Cyanophenylethenyl, p-Trifluormethoxyphenylethenyl, p-Nitrophenylethenyl, p-Bromphenylethenyl, p-lodphenylethenyl, m-Chlorphenylethenyl, m-Methylphenylethenyl, m-Methoxyphenylethenyl, m-Trifluormethylphenylethenyl, m-Fluorphenylethenyl, m-Cyanophenylethenyl, m-Trifluormethoxyphenylethenyl, m-Nitrophenylethenyl, m-Bromphenylethenyl, m-lodphenylethenyl, p-Methoxycarbonylphenylethenyl, m-Methoxycarbonylphenylethenyl, o-Methoxycarbonylphenylethenyl, p-Ethoxycarbonylphenylethenyl, m-Ethoxycarbonylphenylethenyl, o-Ethoxycarbonylphenylethenyl, Ethenyl, 1-Propenyl, 2-Propenyl, 1-Methyl-ethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl, 1-Ethyl-1-methyl-2-propinyl, Cyanoethyl, Cyanomethyl, Cyano-n-propyl, Cyano-n-Butyl, Aryloxy, Bis-[(C₁-C₅)-Alkyl]amino, Aryl-(C₂-C₅)-alkenyl, Heteroaryl-(C₂-C₅)-alkenyl, Heterocyclyl-(C₂-C₅)-alkenyl steht,
- R⁶: für Wasserstoff, Methyl, Ethyl, n-Propyl, 1-Methylethyl, n-Butyl, 1-Me-thylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropyl, Cyclobutyl, Cyclopentyl, Cyclohexyl, Cyclopropylmethyl, Cyclopropylethyl, Cyclobutylmethyl, Cyclopentylmethyl, Cyclohexylmethyl, 2,2-Difluorethyl, 2,2,2-Trifluorethyl, 3,3,3-Trifluorpropyl, Cyanomethyl, Cyanoethyl, Cyano-n-propyl, Cyclopropyl,carbonyl, Cyclobutylcarbonyl, Cyclopentylcarbonyl, Cyclohexylcarbonyl, Methoxycarbonyl, (C₁-C₅)-Alkylsulfonyl, Arylsulfonyl, Aryl-(C₁-C₅)-alkylsulfonyl, Heteroarylsulfonyl, (C₃-C₆)-Cycloalkylsulfonyl, Heterocyclylsulfonyl, (C₁-C₅)-Alkylcarbonyl, Arylcarbonyl, Heteroarylcarbonyl, Heterocyclylcarbonyl, (C₁-C₅)-Alkoxycarbonyl, Aryl-(C₁-C₅)-alkoxycarbonyl, (C₁-C₅)-Haloalkylcarbonyl, (C₂-C₅)-Alkenyl, (C₂-C₅)-Alkinyl, Halo-(C₂-C₅)-alkinyl, Halo-(C₂-C₅)-alkenyl, (C₁-C₅)-Alkoxy-(C₁-C₅)-alkyl steht,
- W: für Sauerstoff oder Schwefel, bevorzugt Sauerstoff, steht.

Ganz besonders bevorzugt sind Verbindungen der allgemeinen Formel (I), die durch die Formeln (Ib) bis (Ie), (Ij) bis (Is), (Iu) und (Iw) beschrieben werden, 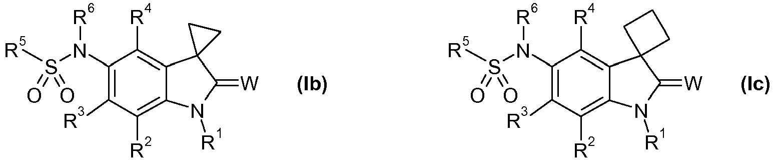 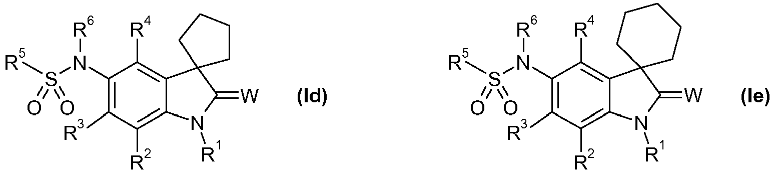 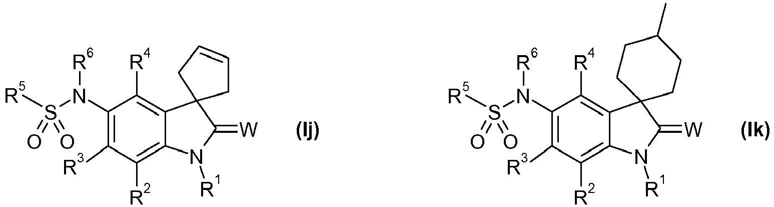 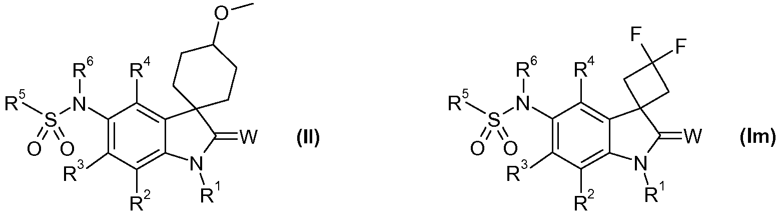 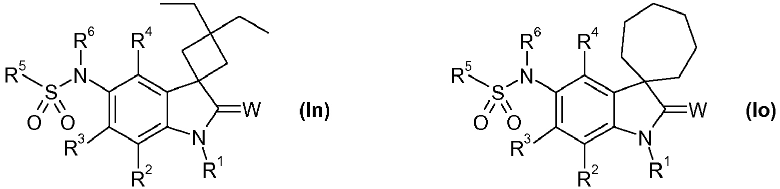 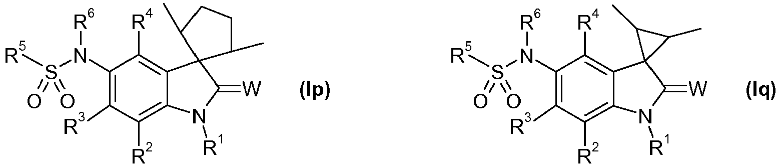 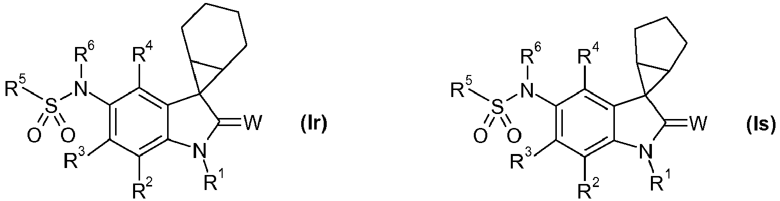 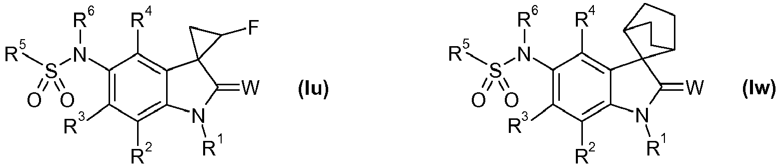 worin
- R¹: für Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropyl, Cyclobutyl, Cyclopentyl, Cyclohexyl, Spiro[2.2]pent-1-yl, Spiro[2.3]hex-1-yl, Spiro[2.3]hex-4-yl, 3-Spiro[2.3]hex-5-yl, Spiro[3.3]hept-1-yl, Spiro[3.3]hept-2-yl, Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, Bicyclo[2.2.1]hept-2-yl, Bicyclo[2.2.2]octan-2-yl, Bicyclo[3.2.1]octan-2-yl, Bicyclo[3.2.2]nonan-2-yl, Adamantan-1-yl, Adamantan-2-yl, 1-Methylcyclopropyl, 2-Methylcyclopropyl, 2,2-Dimethylcyclopropyl, 2,3-Dimethylcyclopropyl, 1,1'-Bi(cyclopropyl)-1-yl, 1,1'-Bi(cyclopropyl)-2-yl, 2'-Methyl-1,1'-bi(cyclopropyl)-2-yl, 1-Cyanopropyl, 2-Cyanopropyl, 1-Methylcyclobutyl, 2-Methylcyclobutyl, 3-Methylcyclobutyl, 1-Cyanocyclobutyl, 2-Cyanocyclobutyl, 3-Cyanocyclobutyl, 1-Allylcyclopropyl, 1-Vinylcyclobutyl, 1-Vinylcyclopropyl, 1-Ethylcyclopropyl, 2-Ethylcyclopropyl, 1-Ethylcyclobutyl, 2-Ethylcyclobutyl, 3-Ethylcyclobutyl, 4-Methylcyclohexyl, 4-Methoxycyclohexyl, 4-Ethoxycyclohexyl, 4-n-Propyloxycyclohexyl, 4-Hydroxycyclohexyl, 4-Methoxycyclobutyl, 1-Cyclopropylcyclobutyl, 1-Prop-2-enylcyclobutyl, 2-Ethyl-3-Methylcyclobutyl,1-Propylcyclopropyl, 1-Methyl-2-propylcyclopropyl, 2-Propylcyclopropyl, 1-Propylcyclobutyl, 2-Propylcyclobutyl, 3-Propylcyclobutyl, 1-iso-Propylcyclobutyl, 1-iso-Propylcyclopropyl, 2-iso-Propylcyclopropyl, 3-iso-Propylcyclobutyl, 2-Dimethylaminocyclobutyl, 3-Dimethylaminocyclobutyl, 1-Butylcyclobutyl, 2-Butylcyclobutyl, 1-Butylcyclopropyl, 3-Butylcyclobutyl, 2-Butylcyclopropyl, 1-iso-Butylcyclobutyl, 3-tert-Butylcyclobutyl, 3,3-Diethylcyclobutyl, 2,2-Diethylcyclopropyl, 2-Methylidencyclopropyl, 1-Methoxymethylcyclopropyl, 1-iso-Butylcyclopropyl,2,2-Difluorethyl, 2,2,2-Trifluorethyl, 3,3,3-Trifluorpropyl, Ethenyl, 1-Propenyl, 2-Propenyl, 1-Methyl-ethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, Ethinyl, 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl, 1-Ethyl-1-methyl-2-propinyl, (C₃-C₆)-Halocycloalkyl, (C₂-C₅)-Haloalkenyl, (C₁-C₅)-Alkoxy-(C₁-C₅)-haloalkyl, Benzyl, p-Chlorbenzyl, p-Methoxybenzyl, p-Trifluormethylbenzyl, p-Methylbenzyl, p-Fluorbenzyl, p-Brombenzyl, p-lodbenzyl, p-Methylthiobenzyl, p-Trifluormethoxybenzyl, p-Nitrobenzyl, p-Trifluormethylthiobenzyl, m-Chlorbenzyl, m-Methoxybenzyl, m-Trifluormethylbenzyl, m-Methylbenzyl, m-Fluorbenzyl, m-Brombenzyl, m-lodbenzyl, m-Methylthiobenzyl, m-Trifluormethoxybenzyl, m-Nitrobenzyl, m-Trifluormethylthiobenzyl, o-Chlorbenzyl, o-Methoxybenzyl, o-Trifluormethylbenzyl, o-Methylbenzyl, o-Fluorbenzyl, o-Brombenzyl, o-lodbenzyl, o-Methylthiobenzyl, o-Trifluormethoxybenzyl, o-Nitrobenzyl, o-Trifluormethylthiobenzyl, p-Methoxycarbonylbenzyl, p-Ethoxycarbonylbenzyl, m-Methoxycarbonylbenzyl, m-Ethoxycarbonylbenzyl, 2,4-Dichlorbenzyl, 3,5-Dichlorbenzyl, 2,4-Difluorbenzyl, 3,5-Difluorbenzyl, 3,4-Dichlorbenzyl, 3,4-Difluorbenzyl, 2,5-Dichlorbenzyl, Phenylethyl, p-Chlorphenylethyl, p-Methoxyphenylethyl, p-Trifluormethylphenylethyl, p-Fluorphenylethyl, p-Trifluormethoxyphenylethyl, p-Trifluormethylthiophenylethyl, p-Methylphenylethyl, p-Nitrophenylethyl, p-Methoxycarbonylphenylethyl, p-Ethoxycarbonylphenylethyl, m-Chlorphenylethyl, m-Methoxyphenylethyl, m-Trifluormethylphenylethyl, m-Fluorphenylethyl, m-Trifluormethoxyphenylethyl, m-Trifluormethylthiophenylethyl, m-Methylphenylethyl, m-Nitrophenylethyl, m-Methoxycarbonylphenylethyl, m-Ethoxycarbonylphenylethyl, o-Chlorphenylethyl, o-Methoxyphenylethyl, o-Trifluormethylphenylethyl, o-Fluorphenylethyl, o-Trifluormethoxyphenylethyl, o-Trifluormethylthiophenylethyl, o-Methylphenylethyl, o-Nitrophenylethyl, o-Methoxycarbonylphenylethyl, o-Ethoxycarbonylphenylethyl, Heteroaryl-(C₁-C₅)-alkyl, (C₁-C₅)-Haloalkenyl, Heterocyclyl, Heterocyclyl-(C₁-C₅)-alkyl, (C₁-C₅)-Alkoxy-(C₁-C₅)-alkyl, (C₁-C₅)-Alkylcarbonyl-(C₁-C₅)-alkyl, Hydroxycarbonyl-(C₁-C₅)-alkyl, (C₁-C₅)-Alkoxycarbonyl-(C₁-C₅)-alkyl, (C₂-C₅)-Alkenyloxycarbonyl-(C₁-C₅)-alkyl, (C₂-C₅)-Alkinyloxycarbonyl-(C₁-C₅)-alkyl, Aryl-(C₁-C₅)-alkoxycarbonyl-(C₁-C₅)-alkyl, (C₃-C₆)-Cycloalkoxycarbonyl-(C₁-C₅)-alkyl, (C₃-C₆)-Cycloalkyl-(C₁-C₅)-alkoxycarbonyl-(C₁-C₅)-alkyl, Aminocarbonyl-(C₁-C₅)-alkyl, (C₁-C₅)-Alkylaminocarbonyl-(C₁-C₅)-alkyl, (C₃-C₆)-Cycloalkylaminocarbonyl-(C₁-C₅)-alkyl, Aryl-(C₁-C₅)-alkylaminocarbonyl-(C₁-C₅)-alkyl, Heteroaryl-(C₁-C₅)-alkylaminocarbonyl-(C₁-C₅)-alkyl, (C₁-C₅)-Alkylthio-(C₁-C₅)-alkyl, (C₃-C₆)-Cycloalkylthio-(C₁-C₅)-alkyl, Arylthio-(C₁-C₅)-alkyl, Heterocyclylthio-(C₁-C₅)-alkyl, Heteroarylthio-(C₁-C₅)-alkyl, Aryl-(C₁-C₅)-alkylthio-(C₁-C₅)-alkyl, (C₁-C₅)-Alkylsulfinyl-(C₁-C₅)-alkyl, (C₁-C₅)-Alkylsulfonyl-(C₁-C₅)-alkyl, Arylsulfinyl-(C₁-C₅)-alkyl, Arylsulfonyl-(C₁-C₅)-alkyl, (C₃-C₆)-Cycloalkylsulfinyl-(C₁-C₅)-alkyl, (C₃-C₆)-Cycloalkylsulfonyl-(C₁-C₅)-alkyl, (C₁-C₅)-Alkoxy-(C₁-C₅)-alkoxy-(C₁-C₅)-alkyl, (C₁-C₅)-Alkylcarbonyl, (C₃-C₆)-Cycloalkylcarbonyl, (C₁-C₅)-Alkoxycarbonyl, Aryl-(C₁-C₅)-alkoxycarbonyl, Arylcarbonyl, Heteroarylcarbonyl, Heterocyclylcarbonyl, Aryl-(C₁-C₅)-alkylcarbonyl, (C₁-C₅)-Alkylaminocarbonyl, (C₃-C₆)-Cycloalkylaminocarbonyl, Arylaminocarbonyl, Aryl-(C₁-C₅)-alkylaminocarbonyl, (C₁-C₅)-Alkylsulfonyl, (C₃-C₆)-Cycloalkylsulfonyl, Arylsulfonyl, Heteroarylsulfonyl, Heterocyclylsulfonyl, Bis-[(C₁-C₅)-alkyl]amino, (C₃-C₆)-Cycloalkyl[(C₁-C₅)-alkyl]amino steht,
- R², R³, R⁴: unabhängig voneinander für Wasserstoff, Fluor, Chlor, Brom, Iod, Methoxy, Ethoxy, n-Propyloxy, iso-Propyloxy, Methyl, Ethyl, iso-Propyl, Trifluormethyl, Difluormethyl, Pentafluorethyl, Trifluormethoxy, Difluormethoxy,2,2-Difluorethoxy, 3,3,3-Trifluorethoxy, Methylthio, Ethylthio, Trifluormethylthio, gegebenenfalls substituiertes Phenyl, Heteroaryl, Heterocyclyl, Cyclopropyl, Cyclobutyl, Cyclopentyl, Cyclohexyl stehen,
- R⁵: für gegebenenfalls substituiertes Phenyl, Heteroaryl, Heterocyclyl, Aryl-(C₁-C₅)-alkyl, Heteroaryl-(C₁-C₅)-alkyl, Heterocyclyl-(C₁-C₅)-alkyl, (C₁-C₅)-Alkoxycarbonyl-(C₁-C₅)-alkyl, Aryl-(C₁-C₅)-alkoxycarbonyl-(C₁-C₅)-alkyl, (C₁-C₆)-Cycloalkoxycarbonyl-(C₁-C₅)-alkyl, (C₃-C₆)-Cycloalkyl-(C₁-C₅)-alkoxycarbonyl-(C₁-C₅)-alkyl, Heteroaryl-(C₁-C₅)-alkoxycarbonyl-(C₁-C₅)-alkyl, Aminocarbonyl-(C₁-C₅)-alkyl, (C₁-C₅)-Alkylaminocarbonyl-(C₁-C₅)-alkyl, (C₃-C₆)-Cycloalkylaminocarbonyl-(C₁-C₅)-alkyl, Aryl-(C₁-C₅)-alkylaminocarbonyl-(C₁-C₅)-alkyl, Aryloxy-(C₁-C₅)-alkyl, Heteroaryloxy-(C₁-C₅)-alkyl, Phenylethenyl, p-Chlorphenylethenyl, p-Methylphenylethenyl, p-Methoxyphenylethenyl, p-Trifluormethylphenylethenyl, p-Fluorphenylethenyl, p-Cyanophenylethenyl, p-Trifluormethoxyphenylethenyl, p-Nitrophenylethenyl, p-Bromphenylethenyl, p-lodphenylethenyl, m-Chlorphenylethenyl, m-Methylphenylethenyl, m-Methoxyphenylethenyl, m-Trifluormethylphenylethenyl, m-Fluorphenylethenyl, m-Cyanophenylethenyl, m-Trifluormethoxyphenylethenyl, m-Nitrophenylethenyl, m-Bromphenylethenyl, m-lodphenylethenyl, p-Methoxycarbonylphenylethenyl, m-Methoxycarbonylphenylethenyl, o-Methoxycarbonylphenylethenyl, p-Ethoxycarbonylphenylethenyl, m-Ethoxycarbonylphenylethenyl, o-Ethoxycarbonylphenylethenyl, Heteroaryl-(C₂-C₅)-alkenyl, Heterocyclyl-(C₂-C₅)-alkenyl steht,
- R⁶: für Wasserstoff, Methyl, Ethyl, n-Propyl, 1-Methylethyl, n-Butyl, 1-Me-thylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropyl, Cyclobutyl, Cyclopentyl, Cyclohexyl, Cyclopropylmethyl, Cyclopropylethyl, Cyclobutylmethyl, Cyclopentylmethyl, Cyclohexylmethyl, 2,2-Difluorethyl, 2,2,2-Trifluorethyl, 3,3,3-Trifluorpropyl, Cyanomethyl, Cyanoethyl, Cyano-n-propyl, Cyclopropyl,carbonyl, Cyclobutylcarbonyl, Cyclopentylcarbonyl, Cyclohexylcarbonyl, Methoxycarbonyl, (C₁-C₅)-Alkylsulfonyl, Arylsulfonyl, Aryl-(C₁-C₅)-alkylsulfonyl, Heteroarylsulfonyl, (C₃-C₆)-Cycloalkylsulfonyl, Heterocyclylsulfonyl, (C₁-C₅)-Alkylcarbonyl, Arylcarbonyl, Heteroarylcarbonyl, Heterocyclylcarbonyl, (C₁-C₅)-Alkoxycarbonyl, Aryl-(C₁-C₅)-alkoxycarbonyl, (C₁-C₅)-Haloalkylcarbonyl, (C₂-C₅)-Alkenyl, (C₂-C₅)-Alkinyl, Halo-(C₂-C₅)-alkinyl, Halo-(C₂-C₅)-alkenyl, (C₁-C₅)-Alkoxy-(C₁-C₅)-alkyl steht,
- W: für Sauerstoff oder Schwefel, bevorzugt Sauerstoff, steht.

Im Speziellen bevorzugt sind Verbindungen der allgemeinen Formel (I), die durch die Formeln (Ib) bis (Ie), (Ij) bis (Il) sowie (Io) bis (Iq) beschrieben werden, 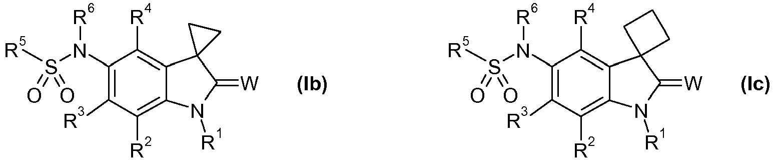 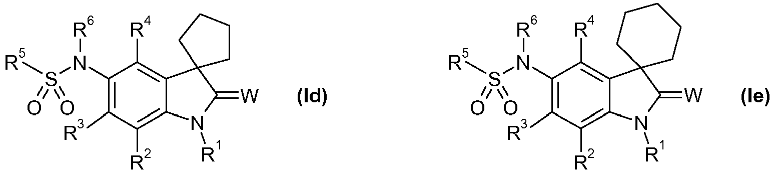 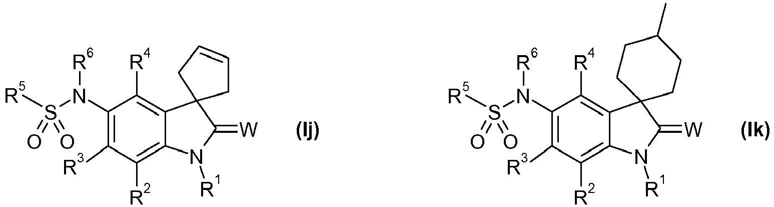 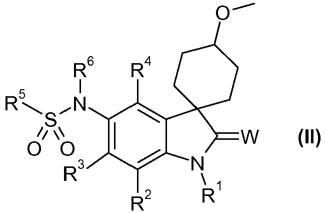 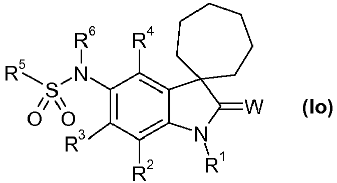 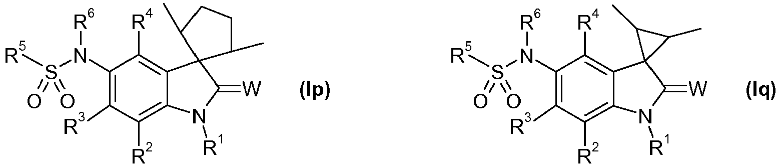 worin
- R¹: für Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropyl, Cyclobutyl, Cyclopentyl, Cyclohexyl, Spiro[2.2]pent-1-yl, Spiro[2.3]hex-1-yl, Spiro[2.3]hex-4-yl, 3-Spiro[2.3]hex-5-yl, Spiro[3.3]hept-1-yl, Spiro[3.3]hept-2-yl, Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, Bicyclo[2.2.1]hept-2-yl, Bicyclo[2.2.2]octan-2-yl, Bicyclo[3.2.1]octan-2-yl, Bicyclo[3.2.2]nonan-2-yl, Adamantan-1-yl, Adamantan-2-yl, 1-Methylcyclopropyl, 2-Methylcyclopropyl, 2,2-Dimethylcyclopropyl, 2,3-Dimethylcyclopropyl, 1,1'-Bi(cyclopropyl)-1-yl, 1,1'-Bi(cyclopropyl)-2-yl, 2'-Methyl-1,1'-bi(cyclopropyl)-2-yl, 1-Cyanopropyl, 2-Cyanopropyl, 1-Methylcyclobutyl, 2-Methylcyclobutyl, 3-Methylcyclobutyl, 1-Cyanocyclobutyl, 2-Cyanocyclobutyl, 3-Cyanocyclobutyl, 1-Allylcyclopropyl, 1-Vinylcyclobutyl, 1-Vinylcyclopropyl, 1-Ethylcyclopropyl, 2-Ethylcyclopropyl, 1-Ethylcyclobutyl, 2-Ethylcyclobutyl, 3-Ethylcyclobutyl, 4-Methylcyclohexyl, 4-Methoxycyclohexyl, 4-Ethoxycyclohexyl, 4-n-Propyloxycyclohexyl, 4-Hydroxycyclohexyl, 4-Methoxycyclobutyl, 1-Cyclopropylcyclobutyl, 1-Prop-2-enylcyclobutyl, 2-Ethyl-3-Methylcyclobutyl,1-Propylcyclopropyl, 1-Methyl-2-propylcyclopropyl, 2-Propylcyclopropyl, 1-Propylcyclobutyl, 2-Propylcyclobutyl, 3-Propylcyclobutyl, 1-iso-Propylcyclobutyl, 1-iso-Propylcyclopropyl, 2-iso-Propylcyclopropyl, 3-iso-Propylcyclobutyl, 2-Dimethylaminocyclobutyl, 3-Dimethylaminocyclobutyl, 1-Butylcyclobutyl, 2-Butylcyclobutyl, 1-Butylcyclopropyl, 3-Butylcyclobutyl, 2-Butylcyclopropyl, 1-iso-Butylcyclobutyl, 3-tert-Butylcyclobutyl, 3,3-Diethylcyclobutyl, 2,2-Diethylcyclopropyl, 2-Methylidencyclopropyl, 1-Methoxymethylcyclopropyl, 1-iso-Butylcyclopropyl,2,2-Difluorethyl, 2,2,2-Trifluorethyl, 3,3,3-Trifluorpropyl, Ethenyl, 1-Propenyl, 2-Propenyl, 1-Methyl-ethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, Ethinyl, 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl, 1-Ethyl-1-methyl-2-propinyl, (C₃-C₆)-Halocycloalkyl, (C₂-C₅)-Haloalkenyl, (C₁-C₅)-Alkoxy-(C₁-C₅)-haloalkyl, Benzyl, p-Chlorbenzyl, p-Methoxybenzyl, p-Trifluormethylbenzyl, p-Methylbenzyl, p-Fluorbenzyl, p-Brombenzyl, p-lodbenzyl, p-Methylthiobenzyl, p-Trifluormethoxybenzyl, p-Nitrobenzyl, p-Trifluormethylthiobenzyl, m-Chlorbenzyl, m-Methoxybenzyl, m-Trifluormethylbenzyl, m-Methylbenzyl, m-Fluorbenzyl, m-Brombenzyl, m-lodbenzyl, m-Methylthiobenzyl, m-Trifluormethoxybenzyl, m-Nitrobenzyl, m-Trifluormethylthiobenzyl, o-Chlorbenzyl, o-Methoxybenzyl, o-Trifluormethylbenzyl, o-Methylbenzyl, o-Fluorbenzyl, o-Brombenzyl, o-lodbenzyl, o-Methylthiobenzyl, o-Trifluormethoxybenzyl, o-Nitrobenzyl, o-Trifluormethylthiobenzyl, p-Methoxycarbonylbenzyl, p-Ethoxycarbonylbenzyl, m-Methoxycarbonylbenzyl, m-Ethoxycarbonylbenzyl, 2,4-Dichlorbenzyl, 3,5-Dichlorbenzyl, 2,4-Difluorbenzyl, 3,5-Difluorbenzyl, 3,4-Dichlorbenzyl, 3,4-Difluorbenzyl, 2,5-Dichlorbenzyl, Phenylethyl, p-Chlorphenylethyl, p-Methoxyphenylethyl, p-Trifluormethylphenylethyl, p-Fluorphenylethyl, p-Trifluormethoxyphenylethyl, p-Trifluormethylthiophenylethyl, p-Methylphenylethyl, p-Nitrophenylethyl, p-Methoxycarbonylphenylethyl, p-Ethoxycarbonylphenylethyl, m-Chlorphenylethyl, m-Methoxyphenylethyl, m-Trifluormethylphenylethyl, m-Fluorphenylethyl, m-Trifluormethoxyphenylethyl, m-Trifluormethylthiophenylethyl, m-Methylphenylethyl, m-Nitrophenylethyl, m-Methoxycarbonylphenylethyl, m-Ethoxycarbonylphenylethyl, o-Chlorphenylethyl, o-Methoxyphenylethyl, o-Trifluormethylphenylethyl, o-Fluorphenylethyl, o-Trifluormethoxyphenylethyl, o-Trifluormethylthiophenylethyl, o-Methylphenylethyl, o-Nitrophenylethyl, o-Methoxycarbonylphenylethyl, o-Ethoxycarbonylphenylethyl, Heteroaryl-(C₁-C₅)-alkyl, (C₁-C₅)-Haloalkenyl, Heterocyclyl, Heterocyclyl-(C₁-C₅)-alkyl, (C₁-C₅)-Alkoxy-(C₁-C₅)-alkyl, (C₁-C₅)-Alkylcarbonyl-(C₁-C₅)-alkyl, Hydroxycarbonyl-(C₁-C₅)-alkyl, (C₁-C₅)-Alkoxycarbonyl-(C₁-C₅)-alkyl, (C₂-C₅)-Alkenyloxycarbonyl-(C₁-C₅)-alkyl, (C₂-C₅)-Alkinyloxycarbonyl-(C₁-C₅)-alkyl, Aryl-(C₁-C₅)-alkoxycarbonyl-(C₁-C₅)-alkyl, (C₃-C₆)-Cycloalkoxycarbonyl-(C₁-C₅)-alkyl, (C₃-C₆)-Cycloalkyl-(C₁-C₅)-alkoxycarbonyl-(C₁-C₅)-alkyl, Aminocarbonyl-(C₁-C₅)-alkyl, (C₁-C₅)-Alkylaminocarbonyl-(C₁-C₅)-alkyl, (C₃-C₆)-Cycloalkylaminocarbonyl-(C₁-C₅)-alkyl, Aryl-(C₁-C₅)-alkylaminocarbonyl-(C₁-C₅)-alkyl, Heteroaryl-(C₁-C₅)-alkylaminocarbonyl-(C₁-C₅)-alkyl, (C₁-C₅)-Alkylthio-(C₁-C₅)-alkyl, (C₃-C₆)-Cycloalkylthio-(C₁-C₅)-alkyl, Arylthio-(C₁-C₅)-alkyl, Heterocyclylthio-(C₁-C₅)-alkyl, Heteroarylthio-(C₁-C₅)-alkyl, Aryl-(C₁-C₅)-alkylthio-(C₁-C₅)-alkyl, (C₁-C₅)-Alkylsulfinyl-(C₁-C₅)-alkyl, (C₁-C₅)-Alkylsulfonyl-(C₁-C₅)-alkyl, Arylsulfinyl-(C₁-C₅)-alkyl, Arylsulfonyl-(C₁-C₅)-alkyl, (C₃-C₆)-Cycloalkylsulfinyl-(C₁-C₅)-alkyl, (C₃-C₆)-Cycloalkylsulfonyl-(C₁-C₅)-alkyl, (C₁-C₅)-Alkoxy-(C₁-C₅)-alkoxy-(C₁-C₅)-alkyl, (C₁-C₅)-Alkylcarbonyl, (C₃-C₆)-Cycloalkylcarbonyl, (C₁-C₅)-Alkoxycarbonyl, Aryl-(C₁-C₅)-alkoxycarbonyl, Arylcarbonyl, Heteroarylcarbonyl, Heterocyclylcarbonyl, Aryl-(C₁-C₅)-alkylcarbonyl, (C₁-C₅)-Alkylaminocarbonyl, (C₃-C₆)-Cycloalkylaminocarbonyl, Arylaminocarbonyl, Aryl-(C₁-C₅)-alkylaminocarbonyl, (C₁-C₅)-Alkylsulfonyl, (C₃-C₆)-Cycloalkylsulfonyl, Arylsulfonyl, Heteroarylsulfonyl, Heterocyclylsulfonyl, Bis-[(C₁-C₅)-alkyl]amino, (C₃-C₆)-Cycloalkyl[(C₁-C₅)-alkyl]amino steht,
- R², R³, R⁴: unabhängig voneinander für Wasserstoff, Fluor, Chlor, Brom, Iod, Methoxy, Ethoxy, n-Propyloxy, iso-Propyloxy, Methyl, Ethyl, iso-Propyl, Trifluormethyl, Difluormethyl, Pentafluorethyl, Trifluormethoxy, Difluormethoxy,2,2-Difluorethoxy, 3,3,3-Trifluorethoxy, Methylthio, Ethylthio, Trifluormethylthio, gegebenenfalls substituiertes Phenyl, Heteroaryl, Heterocyclyl, Cyclopropyl, Cyclobutyl, Cyclopentyl, Cyclohexyl stehen,
- R⁵: für Aryl-(C₁-C₅)-alkyl, Heteroaryl-(C₁-C₅)-alkyl, Heterocyclyl-(C₁-C₅)-alkyl, Aryloxy-(C₁-C₅)-alkyl, Heteroaryloxy-(C₁-C₅)-alkyl, Phenylethenyl, p-Chlorphenylethenyl, p-Methylphenylethenyl, p-Methoxyphenylethenyl, p-Trifluormethylphenylethenyl, p-Fluorphenylethenyl, p-Cyanophenylethenyl, p-Trifluormethoxyphenylethenyl, p-Nitrophenylethenyl, p-Bromphenylethenyl, p-lodphenylethenyl, m-Chlorphenylethenyl, m-Methylphenylethenyl, m-Methoxyphenylethenyl, m-Trifluormethylphenylethenyl, m-Fluorphenylethenyl, m-Cyanophenylethenyl, m-Trifluormethoxyphenylethenyl, m-Nitrophenylethenyl, m-Bromphenylethenyl, m-lodphenylethenyl, p-Methoxycarbonylphenylethenyl, m-Methoxycarbonylphenylethenyl, o-Methoxycarbonylphenylethenyl, p-Ethoxycarbonylphenylethenyl, m-Ethoxycarbonylphenylethenyl, o-Ethoxycarbonylphenylethenyl, Heteroaryl-(C₂-C₅)-alkenyl, Heterocyclyl-(C₂-C₅)-alkenyl steht,
- R⁶: für Wasserstoff, Methyl, Ethyl, n-Propyl, 1-Methylethyl, n-Butyl, 1-Me-thylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropyl, Cyclobutyl, Cyclopentyl, Cyclohexyl, Cyclopropylmethyl, Cyclopropylethyl, Cyclobutylmethyl, Cyclopentylmethyl, Cyclohexylmethyl, 2,2-Difluorethyl, 2,2,2-Trifluorethyl, 3,3,3-Trifluorpropyl, Cyanomethyl, Cyanoethyl, Cyano-n-propyl, Cyclopropyl,carbonyl, Cyclobutylcarbonyl, Cyclopentylcarbonyl, Cyclohexylcarbonyl, Methoxycarbonyl, (C₁-C₅)-Alkylsulfonyl, Arylsulfonyl, Aryl-(C₁-C₅)-alkylsulfonyl, Heteroarylsulfonyl, (C₃-C₆)-Cycloalkylsulfonyl, Heterocyclylsulfonyl, (C₁-C₅)-Alkylcarbonyl, Arylcarbonyl, Heteroarylcarbonyl, Heterocyclylcarbonyl, (C₁-C₅)-Alkoxycarbonyl, Aryl-(C₁-C₅)-alkoxycarbonyl, (C₁-C₅)-Haloalkylcarbonyl, (C₂-C₅)-Alkenyl, (C₂-C₅)-Alkinyl, Halo-(C₂-C₅)-alkinyl, Halo-(C₂-C₅)-alkenyl, (C₁-C₅)-Alkoxy-(C₁-C₅)-alkyl steht,
- W: für Sauerstoff oder Schwefel, bevorzugt Sauerstoff, steht.

Im Hinblick auf die erfindungsgemäßen Verbindungen werden die vorstehend und weiter unten verwendeten Bezeichnungen erläutert. Diese sind dem Fachmann geläufig und haben insbesondere die im Folgenden erläuterten Bedeutungen:

Erfindungsgemäß steht "Arylsulfonyl" für gegebenenfalls substituiertes Phenylsulfonyl oder gegebenenfalls substituiertes polycyclisches Arylsulfonyl, hier insbesondere gegebenenfalls substituiertes Naphthyl-sulfonyl, beispielsweise substituiert durch Fluor, Chlor, Brom, Iod, Cyano, Nitro, Alkyl-, Haloalkyl-, Haloalkoxy-, Amino-, Alkylamino-, Alkylcarbonylamino-, Dialkylamino- oder Alkoxy-gruppen.

Erfindungsgemäß steht "Cycloalkylsulfonyl" - in Alleinstellung oder als Bestandteil einer chemischen Gruppe - für gegebenenfalls substituiertes Cycloalkylsulfonyl, vorzugsweise mit 3 bis 6 Kohlenstoffatomen wie beispielsweise Cyclopropylsulfonyl, Cyclobutylsulfonyl, Cyclopentylsulfonyl oder Cyclohexylsulfonyl.

Erfindungsgemäß steht "Alkylsulfonyl" - in Alleinstellung oder als Bestandteil einer chemischen Gruppe - für geradkettiges oder verzweigtes Alkylsulfonyl, vorzugsweise mit 1 bis 8, oder mit 1 bis 6 Kohlenstoffatomen, z.B. (aber nicht beschränkt auf) (C₁-C₆)-Alkylsulfonyl wie Methylsulfonyl, Ethylsulfonyl, Propylsulfonyl, 1-Methylethylsulfonyl, Butylsulfonyl, 1-Methylpropylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl.

Erfindungsgemäß steht "Heteroarylsulfonyl" für gegebenenfalls substituiertes Pyridylsulfonyl, Pyrimidinylsulfonyl, Pyrazinylsulfonyl oder gegebenenfalls substituiertes polycyclisches Heteroarylsulfonyl, hier insbesondere gegebenenfalls substituiertes Chinolinylsulfonyl, beispielsweise substituiert durch Fluor, Chlor, Brom, Iod, Cyano, Nitro, Alkyl-, Haloalkyl-, Haloalkoxy-, Amino-, Alkylamino-, Alkylcarbonylamino-, Dialkylamino- oder Alkoxygruppen.

Erfindungsgemäß steht "Alkylthio" - in Alleinstellung oder als Bestandteil einer chemischen Gruppe - für geradkettiges oder verzweigtes S-Alkyl, vorzugsweise mit 1 bis 8, oder mit 1 bis 6 Kohlenstoffatomen, wie (C₁-C₁₀)-, (C₁-C₆)- oder (C₁-C₄)-Alkylthio, z.B. (aber nicht beschränkt auf) (C₁-C₆)-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 1,1-Dimethylpropylthio, 1,2-Dimethylpropylthio, 2,2-Dimethylpropylthio, 1-Ethylpropylthio, Hexylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio.

Alkenylthio bedeutet erfindungsgemäßt ein über ein Schwefelatom gebundenen Alkenylrest, Alkinylthio bedeutet ein über ein Schwefelatom gebundenen Alkinylrest, Cycloalkylthio bedeutet ein über ein Schwefelatom gebundenen Cycloalkylrest und Cycloalkenylthio bedeutet ein über ein Schwefelatom gebundenen Cycloalkenylrest.

Alkylsulfinyl (Alkyl-S(=O)-), soweit nicht an anderer Stelle anders definiert steht erfindungsgemäß für Alkylreste, die über -S(=O)- an das Gerüst gebunden sind, wie (C₁-C₁₀)-, (C₁-C₆)- oder (C₁-C₄)-Alkylsulfinyl, z. B. (aber nicht beschränkt auf) (C₁-C₆)-Alkylsulfinyl wie Methylsulfinyl, Ethylsulfinyl, Propylsulfinyl, 1-Methylethylsulfinyl, Butylsulfinyl, 1-Methylpropylsulfinyl, 2-Methylpropylulfinyl, 1,1-Dimethylethylsulfinyl, Pentylsulfinyl, 1-Methylbutylsulfinyl, 2-Methylbutylsulfinyl, 3-Methylbutylsulfinyl, 1,1-Dimethylpropylsulfinyl, 1,2-Dimethylpropylsulfinyl, 2,2-Dimethylpropylsulfinyl, 1-Ethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl.

Analog sind Alkenylsulfinyl und Alkinylsulfinyl, erfindungsgemäß definiert als Alkenyl- bzw. Alkinylreste, die über -S(=O)- an das Gerüst gebunden sind, wie (C₂-C₁₀)-, (C₂-C₆)- oder (C₂-C₄)-Alkenylsulfinyl bzw. (C₃-C₁₀)-, (C₃-C₆)- oder (C₃-C₄)-Alkinylsulfinyl.

Analog sind Alkenylsulfonyl und Alkinylsulfonyl erfindungsgemäß definiert als Alkenyl- bzw. Alkinylreste, die über -S(=O)₂- an das Gerüst gebunden sind, wie (C₂-C₁₀)-, (C₂-C₆)- oder (C₂-C₄)-Alkenylsulfonyl bzw. (C₃-C₁₀)-, (C₃-C₆)- oder (C₃-C₄)-Alkinylsulfonyl.

"Alkoxy" bedeutet ein über ein Sauerstoffatom gebundenen Alkylrest, z. B. (aber nicht beschränkt auf) (C₁-C₆)-Alkoxy wie Methoxy, Ethoxy, Propoxy, 1-Methylethoxy, Butoxy, 1-Methylpropoxy, 2-Methylpropoxy, 1,1-Dimethylethoxy, Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy. Alkenyloxy bedeutet ein über ein Sauerstoffatom gebundenen Alkenylrest, Alkinyloxy bedeutet ein über ein Sauerstoffatom gebundenen Alkinylrest wie (C₂-C₁₀)-, (C₂-C₆)- oder (C₂-C₄)-Alkenoxy bzw. (C₃-C₁₀)-, (C₃-C₆)- oder (C₃-C₄)-Alkinoxy.

"Cycloalkyloxy" bedeutet ein über ein Sauerstoffatom gebundenen Cycloalkylrest und Cycloalkenyloxy bedeutet ein über ein Sauerstoffatom gebundenen Cycloalkenylrest.

"Alkylcarbonyl" (Alkyl-C(=O)-), soweit nicht an anderer Stelle anders definiert, steht erfindungsgemäß für Alkylreste, die über -C(=O)- an das Gerüst gebunden sind, wie (C₁-C₁₀)-, (C₁-C₆)- oder (C₁-C₄)-Alkylcarbonyl. Die Anzahl der C-Atome bezieht sich dabei auf den Alkylrest in der Alkylcarbonylgruppe.

Analog stehen "Alkenylcarbonyl" und "Alkinylcarbonyl", soweit nicht an anderer Stelle anders definiert, erfindungsgemäß für Alkenyl- bzw. Alkinylreste, die über -C(=O)- an das Gerüst gebunden sind, wie (C₂-C₁₀)-, (C₂-C₆)- oder (C₂-C₄)-Alkenylcarbonyl bzw. (C₂-C₁₀)-, (C₂-C₆)- oder (C₂-C₄)-Alkinylcarbonyl. Die Anzahl der C-Atome bezieht sich dabei auf den Alkenyl- bzw. Alkinylrest in der Alkenyl- bzw. Alkinylcarbonylgruppe. Alkoxycarbonyl (Alkyl-O-C(=O)-), soweit nicht an anderer Stelle anders definiert: Alkylreste, die über -O-C(=O)- an das Gerüst gebunden sind, wie (C₁-C₁₀)-, (C₁-C₆)- oder (C₁-C₄)-Alkoxycarbonyl. Die Anzahl der C-Atome bezieht sich dabei auf den Alkylrest in der Alkoxycarbonylgruppe.
Analog stehen "Alkenyloxycarbonyl" und "Alkinyloxycarbonyl", soweit nicht an anderer Stelle anders definiert, erfindungsgemäß für Alkenyl- bzw. Alkinylreste, die über -O-C(=O)- an das Gerüst gebunden sind, wie (C₂-C₁₀)-, (C₂-C₆)- oder (C₂-C₄)-Alkenyloxycarbonyl bzw. (C₃-C₁₀)-, (C₃-C₆)- oder (C₃-C₄)-Alkinyloxycarbonyl. Die Anzahl der C-Atome bezieht sich dabei auf den Alkenyl- bzw. Alkinylrest in der Alken- bzw. Alkinyloxycarbonylgruppe.

Der Begriff "Alkylcarbonyloxy" (Alkyl-C(=O)-O-) steht erfindungsgemäß, soweit nicht an anderer Stelle anders definiert, für Alkylreste, die über eine Carbonyloxygruppe (-C(=O)-O-) mit dem Sauerstoff an das Gerüst gebunden sind, wie (C₁-C₁₀)-, (C₁-C₆)- oder (C₁-C₄)-Alkylcarbonyloxy. Die Anzahl der C-Atome bezieht sich dabei auf den Alkylrest in der Alkylcarbonyloxygruppe.

Analog sind "Alkenylcarbonyloxy" und "Alkinylcarbonyloxy" erfindungsgemäß definiert als Alkenyl- bzw. Alkinylreste, die über (-C(=O)-O-) mit dem Sauerstoff an das Gerüst gebunden sind, wie (C₂-C₁₀)-, (C₂-C₆)- oder (C₂-C₄)-Alkenylcarbonyloxy bzw. (C₂-C₁₀)-, (C₂-C₆)- oder (C₂-C₄)-Alkinylcarbonyloxy. Die Anzahl der C-Atome bezieht sich dabei auf den Alkenyl- bzw. Alkinylrest in der Alkenyl- bzw. Alkinylcarbonyloxygruppe.

Der Begriff "Aryl" bedeutet ein gegebenenfalls substituiertes mono-, bi- oder polycyclisches aromatisches System mit vorzugsweise 6 bis 14, insbesondere 6 bis 10 Ring-C-Atomen, beispielsweise Phenyl, Naphthyl, Anthryl, Phenanthrenyl, und ähnliches, vorzugsweise Phenyl.

Vom Begriff "gegebenenfalls substituiertes Aryl" sind auch mehrcyclische Systeme, wie Tetrahydronaphtyl, Indenyl, Indanyl, Fluorenyl, Biphenylyl, umfasst, wobei die Bindungsstelle am aromatischen System ist. Von der Systematik her ist "Aryl" in der Regel auch von dem Begriff "gegebenenfalls substituiertes Phenyl" umfasst. Bevorzugte Aryl-Substituenten sind hier zum Beispiel Wasserstoff, Halogen, Alkyl, Cycloalkyl, Cycloalkylalkyl, Cycloalkenyl, Halocycloalkyl, Alkenyl, Alkinyl, Aryl, Arylalkyl, Arylalkenyl, Heteroaryl, Heteroarylalkyl, Heterocyclyl, Heterocyclylalkyl, Alkoxyalkyl, Alkylthio, Haloalkylthio, Haloalkyl, Alkoxy, Haloalkoxy, Cycloalkoxy, Cycloalkylalkoxy, Aryloxy, Heteroraryloxy, Alkoxyalkoxy, Alkinylalkoxy, Alkenyloxy, Bisalkylaminoalkoxy, Tris-[alkyl]silyl, Bis-[alkyl]arylsilyl, Bis-[alkyl]alkylsilyl, Tris-[alkyl]silylalkinyl, Arylalkinyl, Heteroarylalkinyl, Alkylalkinyl, Cycloalkylalkinyl, Haloalkylalkinyl, Heterocyclyl-N-alkoxy, Nitro, Cyano, Amino, Alkylamino, Bisalkylamino, Alkylcarbonylamino, Cycloalkylcarbonylamino, Arylcarbonylamino, Alkoxycarbonylamino, Alkoxycarbonylalkylamino, Arylalkoxycarbonylalkylamino, Hydroxycarbonyl, Alkoxycarbonyl, Aminocarbonyl, Alkylaminocarbonyl, Cycloalkylaminocarbonyl, Bis-Alkylaminocarbonyl, Heteroarylalkoxy, Arylalkoxy

Ein heterocyclischer Rest (Heterocyclyl) enthält mindestens einen heterocyclischen Ring (=carbocyclischer Ring, in dem mindestens ein C-Atom durch ein Heteroatom ersetzt ist, vorzugsweise durch ein Heteroatom aus der Gruppe N, O, S, P) der gesättigt, ungesättigt, teilgesättigt oder heteroaromatisch ist und dabei unsubstituiert oder substituiert sein kann, wobei die Bindungsstelle an einem Ringatom lokalisiert ist. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden auch mehrcyclische Systeme umfaßt, wie beispielsweise 8-Aza-bicyclo[3.2.1]octanyl, 8-Aza-bicyclo[2.2.2]octanyl oder 1-Aza-bicyclo[2.2.1]heptyl. Im Falle von gegebenenfalls substituiertem Heterocyclyl werden auch spirocyclische Systeme umfaßt, wie beispielsweise 1-Oxa-5-aza-spiro[2.3]hexyl. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen, wie beispielsweise mit einem Heteroatom aus der Gruppe N, O und S 1- oder 2- oder 3-Pyrrolidinyl, 3,4-Dihydro-2H-pyrrol-2- oder 3-yl, 2,3-Dihydro-1H-pyrrol-1- oder 2- oder 3- oder 4- oder 5-yl; 2,5-Dihydro-1H-pyrrol-1- oder 2- oder 3-yl, 1- oder 2- oder 3- oder 4-Piperidinyl; 2,3,4,5-Tetrahydropyridin-2- oder 3- oder 4- oder 5-yl oder 6-yl; 1,2,3,6-Tetrahydropyridin-1- oder 2- oder 3- oder 4- oder 5- oder 6-yl; 1,2,3,4-Tetrahydropyridin-1- oder 2- oder 3- oder 4- oder 5- oder 6-yl; 1,4-Dihydropyridin-1- oder 2- oder 3- oder 4-yl; 2,3-Dihydropyridin-2- oder 3- oder 4- oder 5- oder 6-yl; 2,5-Dihydropyridin-2- oder 3- oder 4- oder 5- oder 6-yl, 1- oder 2- oder 3- oder 4-Azepanyl; 2,3,4,5-Tetrahydro-1H-azepin-1- oder 2- oder 3- oder 4- oder 5- oder 6- oder 7-yl; 2,3,4,7-Tetrahydro-1H-azepin-1- oder 2- oder 3- oder 4- oder 5- oder 6- oder 7-yl; 2,3,6,7-Tetrahydro-1H-azepin-1- oder 2- oder 3- oder 4-yl; 3,4,5,6-Tetrahydro-2H-azepin-2- oder 3- oder 4- oder 5- oder 6- oder 7-yl; 4,5-Dihydro-1H-azepin-1- oder 2- oder 3- oder 4-yl; 2,5-Dihydro-1H-azepin-1- oder -2- oder 3- oder 4- oder 5- oder 6- oder 7-yl; 2,7-Dihydro-1H-azepin-1- oder -2- oder 3- oder 4-yl; 2,3-Dihydro-1H-azepin-1- oder -2- oder 3- oder 4- oder 5- oder 6- oder 7-yl; 3,4-Dihydro-2H-azepin-2- oder 3- oder 4- oder 5- oder 6- oder 7-yl; 3,6-Dihydro-2H-azepin-2- oder 3- oder 4- oder 5- oder 6- oder 7-yl; 5,6-Dihydro-2H-azepin-2- oder 3- oder 4- oder 5- oder 6- oder 7-yl; 4,5-Dihydro-3H-azepin-2- oder 3- oder 4- oder 5- oder 6- oder 7-yl; 1H-Azepin-1- oder -2- oder 3- oder 4- oder 5- oder 6- oder 7-yl; 2H-Azepin-2- oder 3- oder 4- oder 5- oder 6- oder 7-yl; 3H-Azepin-2- oder 3- oder 4- oder 5- oder 6- oder 7-yl; 4H-Azepin-2- oder 3- oder 4- oder 5- oder 6- oder 7-yl, 2- oder 3-Oxolanyl (= 2- oder 3-Tetrahydrofuranyl); 2,3-Dihydrofuran-2- oder 3- oder 4- oder 5-yl; 2,5-Dihydrofuran-2- oder 3-yl, 2- oder 3- oder 4-Oxanyl (= 2- oder 3- oder 4-Tetrahydropyranyl); 3,4-Dihydro-2H-pyran-2- oder 3- oder 4- oder 5- oder 6-yl; 3,6-Dihydro-2H-pyran-2- oder 3-oder 4- oder 5- oder 6-yl; 2H-Pyran-2- oder 3- oder 4- oder 5- oder 6-yl; 4H-Pyran-2- oder 3- oder 4-yl, 2- oder 3- oder 4-Oxepanyl; 2,3,4,5-Tetrahydrooxepin-2- oder 3- oder 4- oder 5- oder 6- oder 7-yl; 2,3,4,7-Tetrahydrooxepin-2- oder 3- oder 4- oder 5- oder 6- oder 7-yl; 2,3,6,7-Tetrahydrooxepin-2- oder 3- oder 4-yl; 2,3-Dihydrooxepin-2- oder 3- oder 4- oder 5- oder 6- oder 7-yl; 4,5-Dihydrooxepin-2- oder 3- oder 4-yl; 2,5-Dihydrooxepin-2- oder 3- oder 4- oder 5- oder 6- oder 7-yl; Oxepin-2- oder 3- oder 4- oder 5- oder 6- oder 7-yl; 2- oder 3-Tetrahydrothiophenyl; 2,3-Dihydrothiophen-2- oder 3- oder 4- oder 5-yl; 2,5-Dihydrothiophen-2- oder 3-yl; Tetrahydro-2H-thiopyran-2- oder 3- oder 4-yl; 3,4-Dihydro-2H-thiopyran-2- oder 3- oder 4- oder 5- oder 6-yl; 3,6-Dihydro-2H-thiopyran-2- oder 3- oder 4- oder 5- oder 6-yl; 2H-Thiopyran-2- oder 3- oder 4- oder 5- oder 6-yl; 4H-Thiopyran-2- oder 3- oder 4-yl. Bevorzugte 3-Ring und 4-Ring-Heterocyclen sind beispielsweise 1- oder 2-Aziridinyl, Oxiranyl, Thiiranyl, 1- oder 2- oder 3-Azetidinyl, 2- oder 3-Oxetanyl, 2- oder 3-Thietanyl, 1,3-Dioxetan-2-yl. Weitere Beispiele für "Heterocyclyl" sind ein partiell oder vollständig hydrierter heterocyclischer Rest mit zwei Heteroatomen aus der Gruppe N, O und S, wie beispielsweise 1- oder 2- oder 3- oder 4-Pyrazolidinyl; 4,5-Dihydro-3H-pyrazol- 3- oder 4- oder 5-yl; 4,5-Dihydro-1H-pyrazol-1- oder 3- oder 4- oder 5-yl; 2,3-Dihydro-1H-pyrazol-1- oder 2- oder 3- oder 4- oder 5-yl; 1- oder 2- oder 3- oder 4- Imidazolidinyl; 2,3-Dihydro-1H-imidazol-1- oder 2- oder 3- oder 4-yl; 2,5-Dihydro-1H-imidazol-1- oder 2- oder 4- oder 5-yl; 4,5-Dihydro-1H-imidazol-1- oder 2- oder 4- oder 5-yl; Hexahydropyridazin-1- oder 2- oder 3- oder 4-yl; 1,2,3,4-Tetrahydropyridazin-1- oder 2- oder 3- oder 4- oder 5- oder 6-yl; 1,2,3,6-Tetrahydropyridazin-1- oder 2- oder 3- oder 4- oder 5- oder 6-yl; 1,4,5,6-Tetrahydropyridazin-1- oder 3- oder 4- oder 5- oder 6-yl; 3,4,5,6-Tetrahydropyridazin-3- oder 4- oder 5-yl; 4,5-Dihydropyridazin-3- oder 4-yl; 3,4-Dihydropyridazin-3- oder 4- oder 5- oder 6-yl; 3,6-Dihydropyridazin-3- oder 4-yl; 1,6-Dihydropyriazin-1- oder 3- oder 4- oder 5- oder 6-yl; Hexahydropyrimidin-1- oder 2- oder 3- oder 4-yl; 1,4,5,6-Tetrahydropyrimidin-1- oder 2- oder 4- oder 5- oder 6-yl; 1,2,5,6-Tetrahydropyrimidin-1- oder 2- oder 4- oder 5- oder 6-yl; 1,2,3,4-Tetrahydropyrimidin-1- oder 2- oder 3- oder 4- oder 5- oder 6-yl; 1,6-Dihydropyrimidin-1- oder 2- oder 4- oder 5- oder 6-yl; 1,2-Dihydropyrimidin-1- oder 2- oder 4- oder 5- oder 6-yl; 2,5-Dihydropyrimidin-2- oder 4- oder 5-yl; 4,5-Dihydropyrimidin- 4- oder 5- oder 6-yl; 1,4-Dihydropyrimidin-1- oder 2- oder 4- oder 5- oder 6-yl; 1- oder 2- oder 3-Piperazinyl; 1,2,3,6-Tetrahydropyrazin-1- oder 2- oder 3- oder 5- oder 6-yl; 1,2,3,4-Tetrahydropyrazin-1- oder 2- oder 3- oder 4- oder 5- oder 6-yl; 1,2-Dihydropyrazin-1- oder 2- oder 3- oder 5- oder 6-yl; 1,4-Dihydropyrazin-1- oder 2- oder 3-yl; 2,3-Dihydropyrazin-2- oder 3- oder 5- oder 6-yl; 2,5-Dihydropyrazin-2- oder 3-yl; 1,3-Dioxolan-2- oder 4- oder 5-yl; 1,3-Dioxol-2- oder 4-yl; 1,3-Dioxan-2- oder 4- oder 5-yl; 4H-1,3-Dioxin-2- oder 4- oder 5- oder 6-yl; 1,4-Dioxan-2- oder 3- oder 5- oder 6-yl; 2,3-Dihydro-1,4-dioxin-2- oder 3- oder 5- oder 6-yl; 1,4-Dioxin-2- oder 3-yl; 1,2-Dithiolan-3- oder 4-yl; 3H-1,2-Dithiol-3- oder 4- oder 5-yl; 1,3-Dithiolan-2- oder 4-yl; 1,3-Dithiol-2- oder 4-yl; 1,2-Dithian-3- oder 4-yl; 3,4-Dihydro1,2-dithiin-3- oder 4- oder 5- oder 6-yl; 3,6-Dihydro-1,2-dithiin-3- oder 4-yl; 1,2-Dithiin-3- oder 4-yl; 1,3-Dithian-2- oder 4- oder 5-yl; 4H-1,3-Dithiin-2- oder 4- oder 5- oder 6-yl; Isoxazolidin-2- oder 3- oder 4- oder 5-yl; 2,3-Dihydroisoxazol-2- oder 3- oder 4- oder 5-yl; 2,5-Dihydroisoxazol-2- oder 3- oder 4- oder 5-yl; 4,5-Dihydroisoxazol-3- oder 4- oder 5-yl; 1,3-Oxazolidin-2- oder 3- oder 4- oder 5-yl; 2,3-Dihydro-1,3-oxazol-2- oder 3- oder 4- oder 5-yl; 2,5-Dihydro-1,3-oxazol-2- oder 4- oder 5-yl; 4,5-Dihydro-1,3-oxazol-2- oder 4- oder 5-yl; 1,2-Oxazinan-2- oder 3- oder 4- oder 5- oder 6-yl; 3,4-Dihydro-2H-1,2-oxazin-2- oder 3- oder 4- oder 5- oder 6-yl; 3,6-Dihydro-2H-1,2-oxazin-2- oder 3- oder 4- oder 5- oder 6-yl; 5,6-Dihydro-2H-1,2-oxazin-2- oder 3- oder 4- oder 5- oder 6-yl; 5,6-Dihydro-4H-1,2-oxazin-3- oder 4- oder 5- oder 6-yl; 2H-1,2-Oxazin-2- oder 3- oder 4- oder 5- oder 6-yl; 6H-1,2-Oxazin-3- oder 4- oder 5- oder 6-yl; 4H-1,2-Oxazin-3- oder 4- oder 5- oder 6-yl; 1,3-Oxazinan-2- oder 3- oder 4- oder 5- oder 6-yl; 3,4-Dihydro-2H-1,3-oxazin-2- oder 3- oder 4- oder 5- oder 6-yl; 3,6-Dihydro-2H-1,3-oxazin-2- oder 3- oder 4- oder 5- oder 6-yl; 5,6-Dihydro-2H-1,3-oxazin-2- oder 4- oder 5- oder 6-yl; 5,6-Dihydro-4H-1,3-oxazin-2- oder 4- oder 5- oder 6-yl; 2H-1,3-Oxazin-2- oder 4- oder 5- oder 6-yl; 6H-1,3-Oxazin-2- oder 4- oder 5- oder 6-yl; 4H-1,3-Oxazin-2- oder 4- oder 5- oder 6-yl; Morpholin-2- oder 3- oder 4-yl; 3,4-Dihydro-2H-1,4-oxazin-2- oder 3- oder 4- oder 5- oder 6-yl; 3,6-Dihydro-2H-1,4-oxazin-2- oder 3- oder 5- oder 6-yl; 2H-1,4-oxazin-2- oder 3- oder 5- oder 6-yl; 4H-1,4-oxazin-2- oder 3-yl; 1,2-Oxazepan-2- oder 3- oder 4- oder 5- oder 6- oder 7-yl; 2,3,4,5-Tetrahydro-1,2-oxazepin-2- oder 3- oder 4- oder 5- oder 6- oder 7-yl; 2,3,4,7-Tetrahydro-1,2-oxazepin-2- oder 3- oder 4- oder 5- oder 6- oder 7-yl; 2,3,6,7-Tetrahydro-1,2-oxazepin-2- oder 3- oder 4- oder 5- oder 6- oder 7-yl; 2,5,6,7-Tetrahydro-1,2-oxazepin-2- oder 3- oder 4- oder 5- oder 6- oder 7-yl; 4,5,6,7-Tetrahydro-1,2-oxazepin-3- oder 4- oder 5- oder 6- oder 7-yl; 2,3-Dihydro-1,2-oxazepin-2- oder 3- oder 4- oder 5- oder 6- oder 7-yl; 2,5-Dihydro-1,2-oxazepin-2- oder 3- oder 4- oder 5- oder 6- oder 7-yl; 2,7-Dihydro-1,2-oxazepin-2- oder 3- oder 4- oder 5- oder 6- oder 7-yl; 4,5-Dihydro-1,2-oxazepin-3- oder 4- oder 5- oder 6- oder 7-yl; 4,7-Dihydro-1,2-oxazepin-3- oder 4- oder 5- oder 6- oder 7-yl; 6,7-Dihydro-1,2-oxazepin-3- oder 4- oder 5- oder 6- oder 7-yl; 1,2-Oxazepin-3- oder 4- oder 5- oder 6- oder 7-yl; 1,3-Oxazepan-2- oder 3- oder 4- oder 5- oder 6- oder 7-yl; 2,3,4,5-Tetrahydro-1,3-oxazepin-2- oder 3- oder 4- oder 5- oder 6- oder 7-yl; 2,3,4,7-Tetrahydro-1,3-oxazepin-2- oder 3- oder 4- oder 5- oder 6- oder 7-yl; 2,3,6,7-Tetrahydro-1,3-oxazepin-2- oder 3- oder 4- oder 5- oder 6- oder 7-yl; 2,5,6,7-Tetrahydro-1,3-oxazepin-2- oder 4- oder 5- oder 6- oder 7-yl; 4,5,6,7-Tetrahydro-1,3-oxazepin-2- oder 4- oder 5- oder 6- oder 7-yl; 2,3-Dihydro-1,3-oxazepin-2- oder 3- oder 4- oder 5- oder 6- oder 7-yl; 2,5-Dihydro-1,3-oxazepin-2- oder 4- oder 5- oder 6- oder 7-yl; 2,7-Dihydro-1,3-oxazepin-2- oder 4- oder 5- oder 6- oder 7-yl; 4,5-Dihydro-1,3-oxazepin-2- oder 4- oder 5- oder 6- oder 7-yl; 4,7-Dihydro-1,3-oxazepin-2- oder 4- oder 5- oder 6- oder 7-yl; 6,7-Dihydro-1,3-oxazepin-2- oder 4- oder 5- oder 6- oder 7-yl; 1,3-Oxazepin-2- oder 4- oder 5- oder 6- oder 7-yl; 1,4-Oxazepan-2- oder 3- oder 5- oder 6- oder 7-yl; 2,3,4,5-Tetrahydro-1,4-oxazepin-2- oder 3- oder 4- oder 5- oder 6- oder 7-yl; 2,3,4,7-Tetrahydro-1,4-oxazepin-2- oder 3- oder 4- oder 5- oder 6- oder 7-yl; 2,3,6,7-Tetrahydro-1,4-oxazepin-2- oder 3- oder 5- oder 6- oder 7-yl; 2,5,6,7-Tetrahydro-1,4-oxazepin-2- oder 3- oder 5- oder 6- oder 7-yl; 4,5,6,7-Tetrahydro-1,4-oxazepin-2- oder 3- oder 4- oder 5- oder 6- oder 7-yl; 2,3-Dihydro-1,4-oxazepin-2- oder 3- oder 5- oder 6- oder 7-yl; 2,5-Dihydro-1,4-oxazepin-2- oder 3- oder 5- oder 6- oder 7-yl; 2,7-Dihydro-1,4-oxazepin-2- oder 3- oder 5- oder 6- oder 7-yl; 4,5-Dihydro-1,4-oxazepin-2- oder 3- oder 4- oder 5- oder 6- oder 7-yl; 4,7-Dihydro-1,4-oxazepin-2- oder 3- oder 4- oder 5- oder 6- oder 7-yl; 6,7-Dihydro-1,4-oxazepin-2- oder 3- oder 5- oder 6- oder 7-yl; 1,4-Oxazepin-2- oder 3- oder 5- oder 6- oder 7-yl; Isothiazolidin-2- oder 3- oder 4- oder 5-yl; 2,3-Dihydroisothiazol-2- oder 3- oder 4- oder 5-yl; 2,5-Dihydroisothiazol-2- oder 3- oder 4- oder 5-yl; 4,5-Dihydroisothiazol-3- oder 4- oder 5-yl; 1,3-Thiazolidin-2- oder 3- oder 4- oder 5-yl; 2,3-Dihydro-1,3-thiazol-2- oder 3- oder 4- oder 5-yl; 2,5-Dihydro-1,3-thiazol-2- oder 4- oder 5-yl; 4,5-Dihydro-1,3-thiazol-2- oder 4- oder 5-yl; 1,3-Thiazinan-2- oder 3- oder 4- oder 5- oder 6-yl; 3,4-Dihydro-2H-1,3-thiazin-2- oder 3- oder 4- oder 5- oder 6-yl; 3,6-Dihydro-2H-1,3-thiazin-2- oder 3- oder 4- oder 5- oder 6-yl; 5,6-Dihydro-2H-1,3-thiazin-2- oder 4- oder 5- oder 6-yl; 5,6-Dihydro-4H-1,3-thiazin-2- oder 4- oder 5- oder 6-yl; 2H-1,3-Thiazin-2- oder 4- oder 5- oder 6-yl; 6H-1,3-Thiazin-2- oder 4- oder 5- oder 6-yl; 4H-1,3-Thiazin-2- oder 4- oder 5- oder 6-yl. Weitere Beispiele für "Heterocyclyl" sind ein partiell oder vollständig hydrierter heterocyclischer Rest mit 3 Heteroatomen aus der Gruppe N, O und S, wie beispielsweise 1,4,2-Dioxazolidin-2- oder 3- oder 5-yl; 1,4,2-Dioxazol-3- oder 5-yl; 1,4,2-Dioxazinan-2- oder -3- oder 5- oder 6-yl; 5,6-Dihydro-1,4,2-dioxazin-3- oder 5- oder 6-yl; 1,4,2-Dioxazin-3- oder 5- oder 6-yl; 1,4,2-Dioxazepan-2- oder 3- oder 5- oder 6- oder 7-yl; 6,7-Dihydro-5H-1,4,2-Dioxazepin-3- oder 5- oder 6- oder 7-yl; 2,3-Dihydro-7H-1,4,2-Dioxazepin-2- oder 3- oder 5- oder 6- oder 7-yl; 2,3-Dihydro-5H-1,4,2-Dioxazepin-2- oder 3- oder 5- oder 6- oder 7-yl; 5H-1,4,2-Dioxazepin-3- oder 5- oder 6- oder 7-yl; 7H-1,4,2-Dioxazepin-3- oder 5- oder 6- oder 7-yl. Strukturbeispiele für gegebenenfalls weiter substituierte Heterocyclen sind auch im Folgenden aufgeführt:

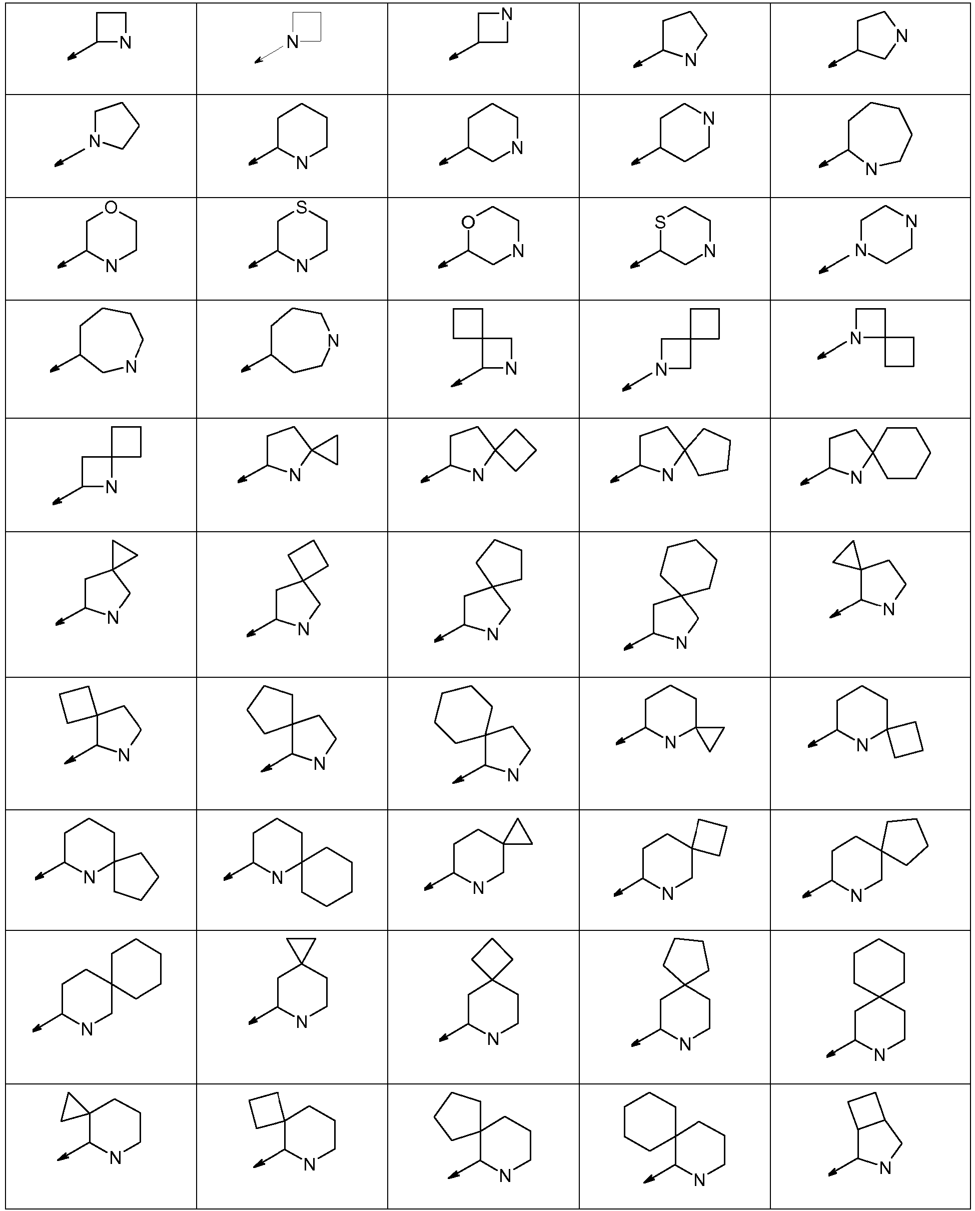

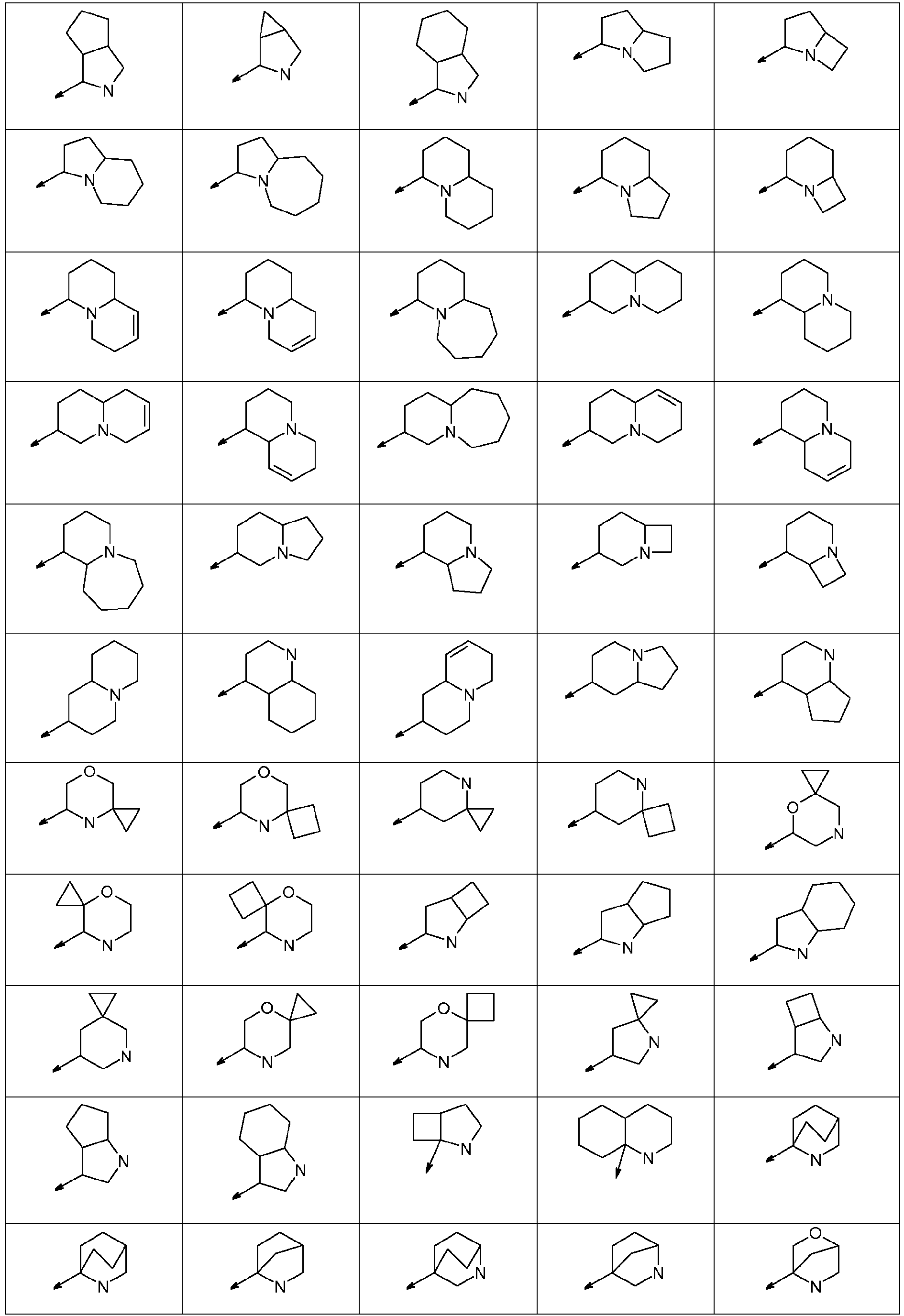

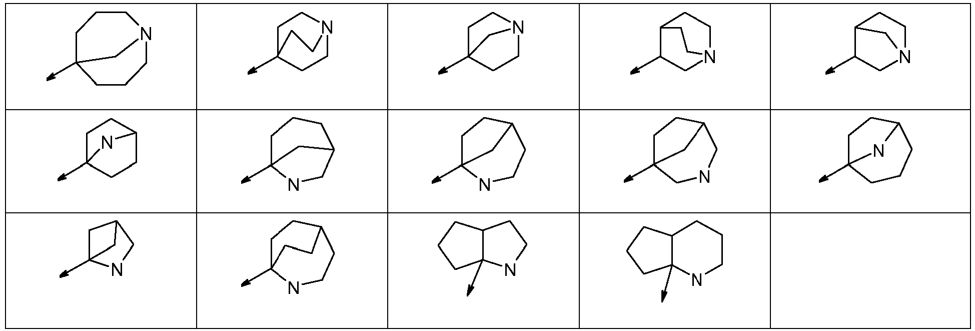

Die oben aufgeführten Heterocyclen sind bevorzugt beispielsweise durch Wasserstoff, Halogen, Alkyl, Haloalkyl, Hydroxy, Alkoxy, Cycloalkoxy, Aryloxy, Alkoxyalkyl, Alkoxyalkoxy, Cycloalkyl, Halocycloalkyl, Aryl, Arylalkyl, Heteroaryl, Heterocyclyl, Alkenyl, Alkylcarbonyl, Cycloalkylcarbonyl, Arylcarbonyl, Heteroarylcarbonyl, Alkoxycarbonyl, Hydroxycarbonyl, Cycloalkoxycarbonyl, Cycloalkylalkoxycarbonyl, Alkoxycarbonylalkyl, Arylalkoxycarbonyl, Arylalkoxycarbonylalkyl, Alkinyl, Alkinylalkyl, Alkylalkinyl, Tris-alkylsilylalkinyl, Nitro, Amino, Cyano, Haloalkoxy, Haloalkylthio, Alkylthio, Hydrothio, Hydroxyalkyl, Oxo, Heteroarylalkoxy, Arylalkoxy, Heterocyclylalkoxy, Heterocyclylalkylthio, Heterocyclyloxy, Heterocyclylthio, Heteroaryloxy, Bis-alkylamino, Alkylamino, Cycloalkylamino, Hydroxycarbonylalkylamino, Alkoxycarbonylalkylamino, Arylalkoxycarbonylalkylamino, Alkoxycarbonylalkyl(alkyl)amino, Aminocarbonyl, Alkylaminocarbonyl, Bisalkylaminocarbonyl, Cycloalkylaminocarbonyl, Hydroxycarbonylalkylaminocarbonyl, Alkoxycarbonylalkylaminocarbonyl, Arylalkoxycarbonylalkylaminocarbonyl substituiert.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Handelt es sich es sich um einen teilweise oder vollständig gesättigten StickstoffHeterocyclus, so kann dieser sowohl über Kohlenstoff als auch über den Stickstoff mit dem Rest des Moleküls verknüpft sein.

Als Substituenten für einen substituierten heterocyclischen Rest kommen die weiter unten genannten Substituenten in Frage, zusätzlich auch Oxo und Thioxo. Die Oxogruppe als Substituent an einem Ring-C-Atom bedeutet dann beispielsweise eine Carbonylgruppe im heterocyclischen Ring. Dadurch sind vorzugsweise auch Lactone und Lactame umfasst. Die Oxogruppe kann auch an den Heteroringatomen, die in verschiedenen Oxidationsstufen existieren können, z.B. bei N und S, auftreten und bilden dann beispielsweise die divalenten Gruppen N(O), S(O) (auch kurz SO) und S(O)2 (auch kurz SO2) im heterocyclischen Ring. Im Fall von -N(O)- und -S(O)-Gruppen sind jeweils beide Enantiomere umfasst.

Erfindungsgemäß steht der Ausdruck "Heteroaryl" für heteroaromatische Verbindungen, d. h. vollständig ungesättigte aromatische heterocyclische Verbindungen, vorzugsweise für 5- bis 7-gliedrige Ringe mit 1 bis 4, vorzugsweise 1 oder 2 gleichen oder verschiedenen Heteroatomen, vorzugsweise O, S oder N. Erfindungsgemäße Heteroaryle sind beispielsweise 1H-Pyrrol-1-yl; 1H-Pyrrol-2-yl; 1H-Pyrrol-3-yl; Furan-2-yl; Furan-3-yl; Thien-2-yl; Thien-3-yl, 1H-Imidazol-1-yl; 1H-Imidazol-2-yl; 1H-Imidazol-4-yl; 1H-Imidazol-5-yl; 1H-Pyrazol-1-yl; 1H-Pyrazol-3-yl; 1H-Pyrazol-4-yl; 1H-Pyrazol-5-yl, 1H-1,2,3-Triazol-1-yl, 1H-1,2,3-Triazol-4-yl, 1H-1,2,3-Triazol-5-yl, 2H-1,2,3-Triazol-2-yl, 2H-1,2,3-Triazol-4-yl, 1H-1,2,4-Triazol-1-yl, 1H-1,2,4-Triazol-3-yl, 4H-1,2,4-Triazol-4-yl, 1,2,4-Oxadiazol-3-yl, 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl, Azepinyl, Pyridin-2-yl, Pyridin-3-yl, Pyridin-4-yl, Pyrazin-2-yl, Pyrazin-3-yl, Pyrimidin-2-yl, Pyrimidin-4-yl, Pyrimidin-5-yl, Pyridazin-3-yl, Pyridazin-4-yl, 1,3,5-Triazin-2-yl, 1,2,4-Triazin-3-yl, 1,2,4-Triazin-5-yl, 1,2,4-Triazin-6-yl, 1,2,3-Triazin-4-yl, 1,2,3-Triazin-5-yl, 1,2,4-, 1,3,2-, 1,3,6- und 1,2,6-Oxazinyl, Isoxazol-3-yl, Isoxazol-4-yl, Isoxazol-5-yl, 1,3-Oxazol-2-yl, 1,3-Oxazol-4-yl, 1,3-Oxazol-5-yl, Isothiazol-3-yl, Isothiazol-4-yl, Isothiazol-5-yl, 1,3-Thiazol-2-yl, 1,3-Thiazol-4-yl, 1,3-Thiazol-5-yl, Oxepinyl, Thiepinyl, 1,2,4-Triazolonyl und 1,2,4-Diazepinyl, 2H-1,2,3,4-Tetrazol-5-yl, 1H-1,2,3,4-Tetrazol-5-yl, 1,2,3,4-Oxatriazol-5-yl, 1,2,3,4-Thiatriazol-5-yl, 1,2,3,5-Oxatriazol-4-yl, 1,2,3,5-Thiatriazol-4-yl. Die erfindungsgemäßen Heteroarylgruppen können ferner mit einem oder mehreren, gleichen oder verschiedenen Resten substituiert sein. Sind zwei benachbarte Kohlenstoffatome Bestandteil eines weiteren aromatischen Rings, so handelt es sich um annellierte heteroaromatische Systeme, wie benzokondensierte oder mehrfach annellierte Heteroaromaten. Bevorzugt sind beispielsweise Chinoline (z. B. Chinolin-2-yl, Chinolin-3-yl, Chinolin-4-yl, Chinolin-5-yl, Chinolin-6-yl, Chinolin-7-yl, Chinolin-8-yl); Isochinoline (z. B. Isochinolin-1-yl, Isochinolin-3-yl, Isochinolin-4-yl, Isochinolin-5-yl, Isochinolin-6-yl, Isochinolin-7-yl, Isochinolin-8-yl); Chinoxalin; Chinazolin; Cinnolin; 1,5-Naphthyridin; 1,6-Naphthyridin; 1,7-Naphthyridin; 1,8-Naphthyridin; 2,6-Naphthyridin; 2,7-Naphthyridin; Phthalazin; Pyridopyrazine; Pyridopyrimidine; Pyridopyridazine; Pteridine; Pyrimidopyrimidine. Beispiele für Heteroaryl sind auch 5- oder 6-gliedrige benzokondensierte Ringe aus der Gruppe 1H-Indol-1-yl, 1H-Indol-2-yl, 1H-Indol-3-yl, 1H-Indol-4-yl, 1H-Indol-5-yl, 1H-Indol-6-yl, 1H-Indol-7-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1H-Indazol-1-yl, 1H-Indazol-3-yl, 1H-Indazol-4-yl, 1H-Indazol-5-yl, 1H-Indazol-6-yl, 1H-Indazol-7-yl, 2H-Indazol-2-yl, 2H-Indazol-3-yl, 2H-Indazol-4-yl, 2H-Indazol-5-yl, 2H-Indazol-6-yl, 2H-Indazol-7-yl, 2H-Isoindol-2-yl, 2H-Isoindol-1-yl, 2H-Isoindol-3-yl, 2H-Isoindol-4-yl, 2H-Isoindol-5-yl, 2H-Isoindol-6-yl; 2H-Isoindol-7-yl, 1H-Benzimidazol-1-yl, 1H-Benzimidazol-2-yl, 1H-Benzimidazol-4-yl, 1H-Benzimidazol-5-yl, 1H-Benzimidazol-6-yl, 1H-Benzimidazol-7-yl, 1,3-Benzoxazol-2-yl, 1,3-Benzoxazol-4-yl, 1,3-Benzoxazol-5-yl, 1,3-Benzoxazol-6-yl, 1,3-Benzoxazol-7-yl, 1,3-Benzthiazol-2-yl, 1,3-Benzthiazol-4-yl, 1,3-Benzthiazol-5-yl, 1,3-Benzthiazol-6-yl, 1,3-Benzthiazol-7-yl, 1,2-Benzisoxazol-3-yl, 1,2-Benzisoxazol-4-yl, 1,2-Benzisoxazol-5-yl, 1,2-Benzisoxazol-6-yl, 1,2-Benzisoxazol-7-yl, 1,2-Benzisothiazol-3-yl, 1,2-Benzisothiazol-4-yl, 1,2-Benzisothiazol-5-yl, 1,2-Benzisothiazol-6-yl, 1,2-Benzisothiazol-7-yl.

Die Bezeichnung "Halogen" bedeutet beispielsweise Fluor, Chlor, Brom oder Iod. Wird die Bezeichnung für einen Rest verwendet, dann bedeutet "Halogen" beispielsweise ein Fluor-, Chlor-, Brom- oder lodatom.

Erfindungsgemäß bedeutet "Alkyl" einen geradkettigen oder verzweigten offenkettigen, gesättigten Kohlenwasserstoffrest, der gegebenenfalls ein- oder mehrfach substituiert ist. Bevorzugte Substituenten sind Halogenatome, Alkoxy-, Haloalkoxy-, Cyano-, Alkylthio, Haloalkylthio-, Amino- oder Nitrogruppen, besonders bevorzugt sind Methoxy, Methyl, Fluoralkyl, Cyano, Nitro, Fluor, Chlor, Brom oder Iod. Die Vorsilbe "Bis" schließt auch die Kombination unterschiedlicher Alkylreste ein, z. B. Ethyl(Methyl) oder Methyl(Ethyl).

"Haloalkyl", "-alkenyl" und "-alkinyl" bedeuten durch gleiche oder verschiedene Halogenatome, teilweise oder vollständig substituiertes Alkyl, Alkenyl bzw. Alkinyl, z.B. Monohaloalkyl (= Monohalogenalkyl) wie z. B. CH₂CH₂Cl, CH₂CH₂Br, CHClCH₃, CH₂Cl, CH₂F; Perhaloalkyl wie z. B. CCl₃, CClF₂, CFCl₂,CF₂CClF₂, CF₂CClFCF₃; Polyhaloalkyl wie z. B. CH₂CHFCl, CF₂CClFH, CF₂CBrFH, CH₂CF₃; Der Begriff Perhaloalkyl umfasst dabei auch den Begriff Perfluoralkyl.

Teilfluoriertes Alkyl bedeutet einen geradkettigen oder verzweigten, gesättigten Kohlenwasserstoff, der einfach oder mehrfach durch Fluor substituiert ist, wobei sich die entsprechenden Fluoratome als Substituenten an einem oder mehreren verschiedenen Kohlenstoffatomen der geradkettigen oder verzweigten Kohlenwasserstoffkette befinden können, wie z. B. CHFCH₃, CH₂CH₂F, CH₂CH₂CF₃, CHF₂, CH₂F, CHFCF₂CF₃

Teilfluoriertes Haloalkyl bedeutet einen geradkettigen oder verzweigten, gesättigten Kohlenwasserstoff, der durch verschiedenene Halogenatomen mit mindestens einem Fluoratom substituiert ist, wobei alle anderen gegebenenfalls vorhandenen Halogenatome ausgewählt sind aus der Gruppe Fluor, Chlor oder Brom, Iod. Die entsprechenden Halogenatome können sich dabei als Substituenten an einem oder mehreren verschiedenen Kohlenstoffatomen der geradkettigen oder verzweigten Kohlenwasserstoffkette befinden. Teilfluoriertes Haloalkyl schließt auch die vollständige Substitution der geradkettigen oder verzweigten Kette durch Halogen unter Beteiligung von mindestens einem Fluoratom ein.

Haloalkoxy ist z.B. OCF₃, OCHF₂, OCH₂F, OCF₂CF₃, OCH₂CF₃ und OCH₂CH₂Cl; Entsprechendes gilt für Haloalkenyl und andere durch Halogen substituierten Reste.

Der hier beispielhaft genannte Ausdruck "(C₁-C₄)-Alkyl" bedeutet eine Kurzschreibweise für geradkettiges oder verzweigtes Alkyl mit einem bis 4 Kohlenstoffatomen entsprechend der Bereichsangabe für C-Atome, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "(C₁-C₆)-Alkyl", umfassen entsprechend auch geradkettige oder verzweigte Alkylreste mit einer größeren Zahl von C-Atomen, d. h. gemäß Beispiel auch die Alkylreste mit 5 und 6 C-Atomen.

Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Resten wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste, wobei mindestens eine Doppelbindung bzw. Dreifachbindung enthalten ist. Bevorzugt sind Reste mit einer Doppelbindung bzw. Dreifachbindung.

Der Begriff "Alkenyl" schließt insbesondere auch geradkettige oder verzweigte offenkettige Kohlenwasserstoffreste mit mehr als einer Doppelbindung ein, wie 1,3-Butadienyl und 1,4-Pentadienyl, aber auch Allenyl- oder Kumulenyl-reste mit einer bzw. mehreren kumulierten Doppelbindungen, wie beispielsweise Allenyl (1,2-Propadienyl), 1,2-Butadienyl und 1,2,3-Pentatrienyl. Alkenyl bedeutet z.B. Vinyl, welches ggf. durch weitere Alkylreste substituiert sein kann, z B. (aber nicht beschränkt auf) (C₂-C₆)-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

Der Begriff "Alkinyl" schließt insbesondere auch geradkettige oder verzweigte offenkettige Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl bzw. 3-Penten-1-in-1-yl. (C₂-C₆)-Alkinyl bedeutet z.B. Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Me¬thyl-1-pentinyl, 4-Methyl-2-pentinyl, 1,1-Di-methyl-2-butinyl, 1,1-Dimethyl-3-butinyl, 1,2-Di¬methyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl.

Der Begriff "Cycloalkyl" bedeutet ein carbocyclisches, gesättigtes Ringsystem mit vorzugsweise 3-8 Ring-C-Atomen, z.B. Cyclopropyl, Cyclobutyl, Cyclopentyl oder Cyclohexyl. Im Falle von gegebenenfalls substituiertem Cycloalkyl werden cyclische Systeme mit Substituenten umfasst, wobei auch Substituenten mit einer Doppelbindung am Cycloalkylrest, z. B. eine Alkylidengruppe wie Methyliden, umfasst sind. Im Falle von gegebenenfalls substituiertem Cycloalkyl werden auch mehrcyclische aliphatische Systeme umfaßt, wie beispielsweise Bicyclo[1.1.0]butan-1-yl, Bicyclo[1.1.0]butan-2-yl, Bicyclo[2.1.0]pentan-1-yl, Bicyclo[2.1.0]pentan-2-yl, Bicyclo[2.1.0]pentan-5-yl, Bicyclo[2.2.1]hept-2-yl (Norbornyl), Bicyclo[2.2.2]octan-2-yl, Adamantan-1-yl und Adamantan-2-yl. Der Ausdruck "(C₃-C₇)-Cycloalkyl" bedeutet eine Kurzschreibweise für Cycloalkyl mit drei bis 7 Kohlenstoffatomen entsprechend der Bereichsangabe für C-Atome.

Im Falle von substituiertem Cycloalkyl werden auch spirocyclische aliphatische Systeme umfaßt, wie beispielsweise Spiro[2.2]pent-1-yl, Spiro[2.3]hex-1-yl, Spiro[2.3]hex-4-yl, 3-Spiro[2.3]hex-5-yl.

"Cycloalkenyl" bedeutet ein carbocyclisches, nicht aromatisches, partiell ungesättigtes Ringsystem mit vorzugsweise 4-8 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl, wobei auch Substituenten mit einer Doppelbindung am Cycloalkenylrest, z. B. eine Alkylidengruppe wie Methyliden, umfasst sind. Im Falle von gegebenenfalls substituiertem Cycloalkenyl gelten die Erläuterungen für substituiertes Cycloalkyl entsprechend.

Der Begriff "Alkyliden", z. B. auch in der Form (C₁-C₁₀)-Alkyliden, bedeutet den Rest eines geradkettigen oder verzweigten offenkettigen Kohlenwasserstoffrests, der über eine Zweifachbindung gebunden ist. Als Bindungsstelle für Alkyliden kommen naturgemäß nur Positionen am Grundkörper in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H₅. Cycloalkyliden bedeutet ein carbocyclischer Rest, der über eine Zweifachbindung gebunden ist.

Die Verbindungen der allgemeinen Formel (I) können je nach Art und Verknüpfung der Substituenten als Stereoisomere vorliegen. Die durch ihre spezifische Raumform definierten möglichen Stereoisomere, wie Enantiomere, Diastereomere, Z- und E-Isomere sind alle von der Formel (I) umfasst. Sind beispielsweise eine oder mehrere Alkenylgruppen vorhanden, so können Diastereomere (Z- und E-Isomere) auftreten. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden erhalten. Die chromatographische Trennung kann sowohl im analytischen Maßstab zur Feststellung des Enantiomerenüberschusses bzw. des Diastereomerenüberschusses, wie auch im präparativen Maßstab zur Herstellung von Prüfmustern für die biologische Ausprüfung erfolgen. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft somit auch alle Stereoisomeren, die von der allgemeinen Formel (I) umfasst, jedoch nicht mit ihrer spezifischen Stereoform angegeben sind, sowie deren Gemische.

### Synthese von Dihydrooxindolylsulfonamiden:

Die erfindungsgemäßen gegebenenfalls weiter substituierten Dihydrooxindolylsulfonamide der allgemeinen Formel (I) können nach bekannten Verfahren hergestellt werden. Die eingesetzten und untersuchten Syntheserouten gehen dabei von kommerziell erhältlichen oder leicht herstellbaren Dihydrooxindolylaminen und den entsprechenden Sulfonylchloriden aus. Die Synthese von Dihydrooxindolylaminen wird im Folgenden beispielhaft aber nicht einschränkend anhand der Herstellung von spiro-Cyclopropyl- und spiro-Cyclobutyldihydrooxindolylaminen erläutert. Die weiteren Dihydrooxindolylamine, die zur Herstellung der erfindungsgemäßen Sulfonamide der allgemeinen Formel (I) benötigt werden, sind auf analogen Synthesewegen herstellbar. Ein entsprechend am Stickstoff mit R¹ mono-substituiertes und gegebenenfalls an den anderen Positionen weiter substituiertes Anilin wird mit Chloressigsäurechlorid oder Bromessigsäurebromid unter Verwendung einer geeigneten Base in einem polar-aprotischen Lösungsmittel umgesetzt. Das entsprechende Reaktionsprodukt wird unter Friedel-Crafts-Bedingungen mit einer geeigneten Lewissäure zum gewünschten gegebenenfalls weiter substituierten Dihydrooxindol (**A**) cyclisiert (Schema 1). Alternativ können die Dihydrooxindole (**A**) ausgehend von einem gegegebenenfalls weiter substituierten Isatin hergestellt werden (vgl. WO2006106426), indem zuerst am Stickstoff der entsprechende Substituent R¹ eingeführt wird (im Fall von R¹ = n-Propyl mit n-Propyliodid und einer geeigneten Carbonatbase, z. B. Kaliumcarbonat oder Caesiumcarbonat in einem geeigneten polar-aprotischen Lösemittel, z. B. N,N-Dimethylformamid, vgl. Tetrahedron:Asymmetry 2009, 20(14), 1697) und anschließend durch Umsetzung mit Hydrazin-Hydrat bei erhöhter Temperatur entsprechend einer Wolff-Kishner Reaktion die zweite Carbonylgruppe in eine CH₂-Gruppe überführt wird. In Schema 1 werden die Reaktionssequenzen zur Herstellung von spiro-Cyclopropyldihydrooxindolylaminen beispielhaft, aber nicht einschränkend mit R¹ = n-Propyl, sowie R², R³, R⁴ = Wasserstoff dargestellt.

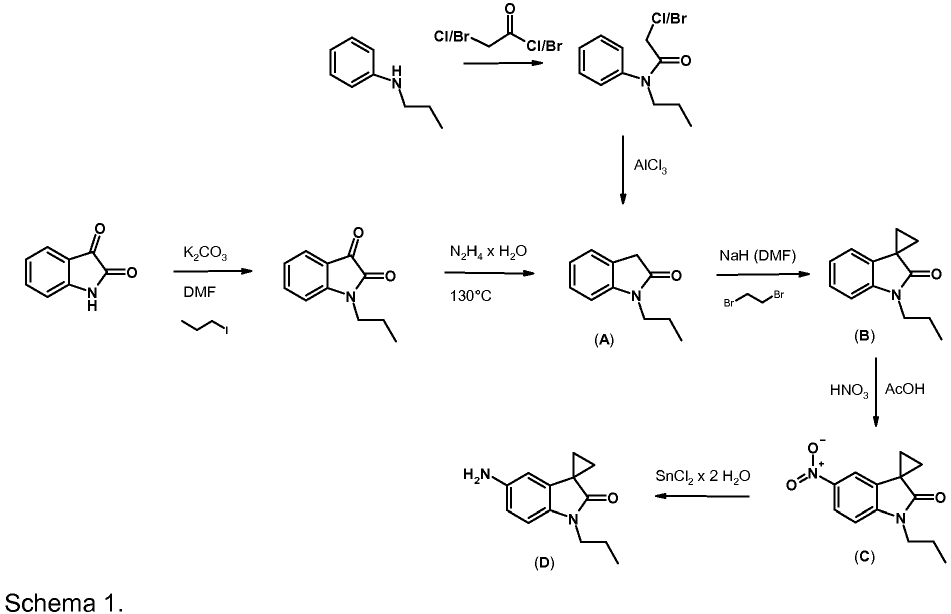

Entsprechend substituierte Dihydrooxindole (A) werden danach mit einer geeigneten Base (z. B. Natriumhydrid) in einem geeigneten polar-aprotischen Lösemittel (z.B. N,N-Dimethylformamid oder Tetrahydrofuran) beispielhaft aber nicht einschränkend mit 1,2-Dibromethan in das entsprechende Spiro-Cyclopropyldihydrooxindol (B) überführt. Im nächsten Schritt kann das Produkt (B) mit Hilfe von Salpetersäure in Essigsäure nitriert werden (vgl. US20070037791, J. Am. Chem. Soc. 1953, 75, 2572). Das entsprechende Nitroderivat (C) kann danach mit Hilfe eines geeigneten Reduktionsmittels (z. B. Zinn(II)chlorid-Dihydrat) zum gewünschten beispielhaften spiro-Cyclopropyldihydrooxindolylamin (D) umgesetzt werden (vgl. EP1598353 und Farmaco Ed. Sci. 1977, 32, 703) (Schema 1).
Einen weiteren alternativen Herstellweg für substituierte Dihydrooxindolylamine bietet die Umsetzung von einem gegebenenfalls weiter substituierten p-Acetylaminoanilin mit einem gegebenenfalls substituierten Bromessigsäurebromid und nachfolgender Lewissäure-vermittelter Cyclisierung (z. B. mit Aluminiumtrichlorid) sowie abschließender Entfernung der Acetylschutzgruppe mit einer geeigneten Säure (z. B. Salzsäure, vgl. EP1598353). In Schema 2 wird diese Reaktionssequenz zur Herstellung von substituierten Dihydrooxindolylaminen beispielhaft aber nicht einschränkend mit R², R³, R⁴ = Wasserstoff dargestellt, und X, Y sowie R¹ haben die zuvor definierten Bedeutungen.

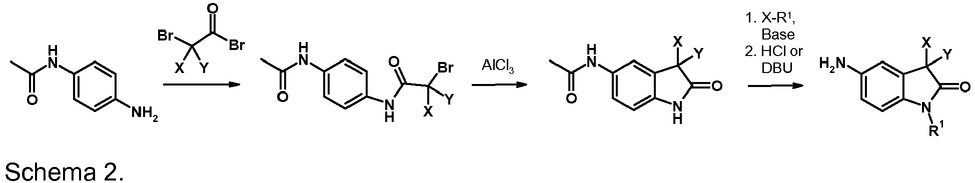

Gegebenenfalls weiter substituierte spiro-Cyclobutyldihydrooxindolylamine (E) können analog über die in Schema 1 und 2 beschriebenen Syntheserouten hergestellt werden, wobei in diesem Fall gegebenenfalls weiter substituierte 1,3-Dibrompropane eingesetzt werden. In Schema 3 wird diese Reaktionssequenz zur Herstellung von gegebenenfalls substituierten spiro-Cyclobutyldihydrooxindolyl-aminen beispielhaft, aber nicht einschränkend mit R¹ = Methylund R², R³, R⁴ = Wasserstoff dargestellt.

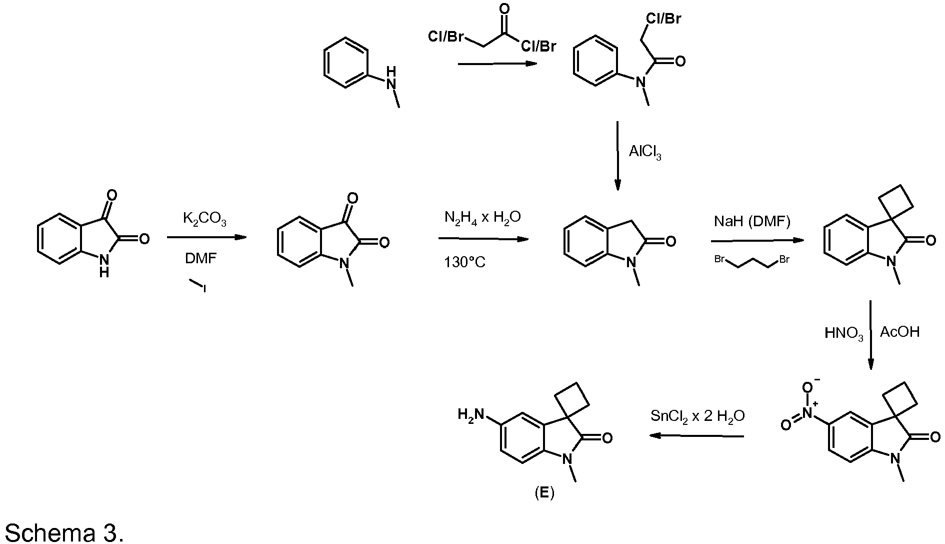

Aryl- und Heteroarylsulfonylchloridvorstufen können beispielsweise über direkte Chlorsulfonierung der entsprechenden substituierten Aromaten und Heteroaromaten (vgl. Eur J. Med. Chem. 2010, 45, 1760) oder über Diazotierung eines Aminosubstituierten Aromaten oder Heteroaromaten und anschließende Chlorsulfonierung hergestellt werden (vgl. WO2005035486). Die Kupplung der entsprechenden substituierten Sulfonylchloridvorstufen mit den entsprechenden gegebenenfalls weiter substituierten Dihydrooxindolylaminen mit Hilfe einer geeigneten Base (z. B. Triethylamin, Pyridin oder Natriumhydroxid) in einem geeigneten Lösemittel (z. B. Tetrahydrofuran, Acetonitril, DMSO oder Dichlormethan) liefert die erfindungsgemäßen gegebenenfalls weiter substituierten Dihydrooxindolylsulfonamide (beispielsweise Subklassen (Ic), (Id)). R¹, R², R³, R⁴, R⁵ und R⁵ haben im folgenden Schema 4 die zuvor definierten Bedeutungen. X und Y werden beispielhaft, aber nicht einschränkend durch CH₂, eine spiro-Cyclopropylgruppe und eine spiro-Cyclobutylgruppe dargestellt.

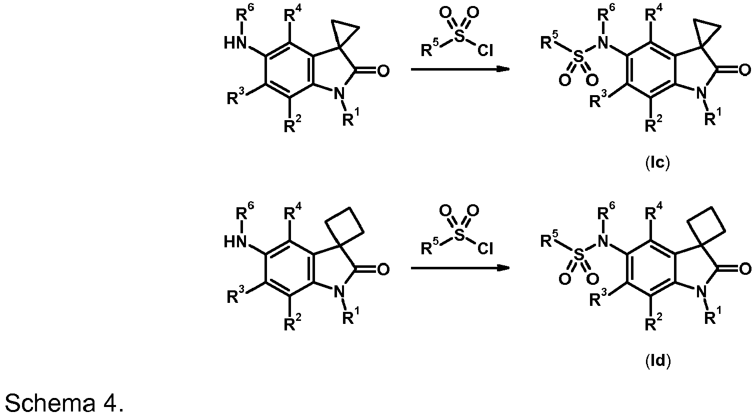

Ausgewählte detaillierte Synthesebeispiele für die erfindungsgemäßen Verbindungen der allgemeinen Formel (I) sind im Folgenden aufgeführt. Die angegebenen Beispielnummern entsprechen den in den nachstehenden Tabellen A1 bis J3 genannten Numerierungen. Die ¹H-NMR-, ¹³C-NMR- und ¹⁹F-NMR-spektroskopischen Daten, die für die in den nachfolgenden Abschnitten beschriebenen chemischen Beispiele angegeben sind, (400 MHz bei ¹H-NMR und 150 MHz bei ¹³C-NMR und 375 MHz bei ¹⁹F-NMR, Lösungsmittel CDCl₃, CD₃OD oder d₆-DMSO, interner Standard: Tetramethylsilan δ = 0.00 ppm), wurden mit einem Gerät der Firma Bruker erhalten, und die bezeichneten Signale haben die nachfolgend aufgeführten Bedeutungen: br = breit(es); s = Singulett, d = Dublett, t = Triplett, dd = Doppeldublett, ddd = Dublett eines Doppeldubletts, m = Multiplett, q = Quartett, quint = Quintett, sext = Sextett, sept = Septett, dq = Doppelquartett, dt = Doppeltriplett. Bei Diastereomerengemischen werden entweder die jeweils signifikanten Signale beider Diastereomere oder das charakteristische Signal des Hauptdiastereomers angegeben. Die verwendeten Abkürzungen für chemische Gruppen haben die nachfolgenden Bedeutungen: Me = CH₃, Et = CH₂CH₃, t-Hex = C(CH₃)₂CH(CH₃)₂, t-Bu = C(CH₃)₃, n-Bu = unverzweigtes Butyl, n-Pr = unverzweigtes Propyl, c-Hex = Cyclohexyl.

### No. A1-173: 1-(3-Bromphenyl)-N-(1'-methyl-2'-oxo-1',2'-dihydrospiro[cyclopropan-1,3'-indol]-5'-yl)methansulfonamid

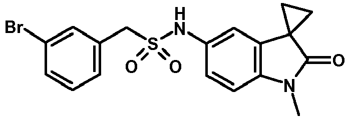

1-Methyl-1,3-dihydro-2H-indol-2-on (2.00 g, 14 mmol) wurde zusammen mit 1,2-Dibromethan (3.83 g, 20 mmol) in abs. N,N-Dimethylformamid (15 ml) gelöst und danach bei einer Temperatur von 10-15°C vorsichtig portionsweise mit Natriumhydrid (1.68 g, 42 mmol, 60%ige Dispersion) versetzt sowie anderthalb Stunden lang nachgerührt. Danach erfolgte die Zugabe von Methanol und wäßriger Ammoniumchloridlösung zum Reaktionsgemisch, und die wäßrige Phase wurde intensiv mit Ethylacetat extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1'-Methylspiro[cyclopropan-1,3'-indol]-2'(1'H)-on (2500 mg, 92 % der Theorie) isoliert. ¹H-NMR (400 MHz, CDCl₃ δ, ppm) 7.25 (m, 1H), 7.03 (m, 1H), 6.91 (d, 1H), 6.83 (d, 1H), 3.30 (s, 3H), 1.73 (m, 2H), 1.52 (m, 2H). 1'-Methylspiro[cyclopropan-1,3'-indol]-2'(1'H)-on (2.50 g, 13 mmol) wurde in Eisessig (23 ml) gegeben und danach langsam und vorsichtig mit rauchender Salpetersäure (4 ml) versetzt. Das resultierende Reaktionsgemisch wurde 30 Minuten lang bei Raumtemperatur gerührt und danach langsam mit Eiswasser verdünnt. Die wäßrige Phase wurde daraufhin mehrfach mit Ethylacetat extrahiert, und die vereinigten organischen Phasen wurden mit gesättigter Natriumcarbonatlösung gewaschen und anschließend über Magnesiumsulfat getrocknet, filtriert und unter vermindertem Druck eingeengt. Durch säulenchromatographische Reinigung des erhaltenen Rohproduktes (Gradient Essigester/Heptan) wurde 1'-Methyl-5'-nitrospiro[cyclopropan-1,3'-indol]-2'(1'H)-on (1800 mg, 57 % der Theorie) als farbloser Feststoff isoliert. ¹H-NMR (400 MHz, CDCl₃ δ, ppm) 8.25 (dd, 1H), 7.74 (d, 1H), 6.97 (d, 1H), 3.36 (s, 3H), 1.88 (m, 2H), 1.69 (m, 2H). Im folgenden Schritt wurde 1'-Methyl-5'-nitrospiro[cyclopropan-1,3'-indol]-2'(1'H)-on (1.80 g, 8 mmol) zusammen mit Zinn(II)chloriddihydrat (7.45 g, 33 mmol) in abs. Ethanol gegeben und 5 h lang unter Argon bei einer Temperatur von 80 °C gerührt. Nach dem Abkühlen auf Raumtemperatur wurde das Reaktionsgemisch auf Eiswasser gegossen und anschließend mit wäßriger NaOH auf pH12 eingestellt. Die wäßrige Phase wurde daraufhin mehrfach mit Ethylacetat extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 5'-Amino-1'-methylspiro[cyclopropan-1,3'-indol]-2'(1'H)-on (1226 mg, 79 % der Theorie) als farbloser Feststoff isoliert. ¹H-NMR (400 MHz, CDCl₃ δ, ppm) 6.71 (d, 1H), 6.60 (dd, 1H), 6.25 (d, 1H), 3.52 (br. s, 2H, NH), 3.24 (t, 2H), 1.71 (m, 2H), 1.43 (m, 2H). 5'-Amino-1'-methylspiro[cyclopropan-1,3'-indol]-2'(1'H)-on (130 mg, 1.0 equiv.) wurde zusammen mit (3-Bromphenyl)methansulfonylchlorid (261 mg, 1.4 equiv.) in einem Rundkolben unter Argon in abs. Acetonitril gelöst, danach mit Pyridin (0.11 ml, 2.0 equiv.) sowie Dimethylsulfoxid (0.03 ml, 0.60 mmol) versetzt, und 6 h lang bei Raumtemperatur gerührt. Das Reaktionsgemisch wurde daraufhin unter vermindertem Druck eingeengt, der verbleibende Rückstand mit Wasser und Dichlormethan versetzt und die wässrige Phase mehrfach mit Dichlormethan extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1-(3-Bromphenyl)-N-(1'-methyl-2'-oxo-1',2'-dihydrospiro[cyclopropan-1,3'-indol]-5'-yl)methansulfonamid (235 mg, 81 % der Theorie) als farbloser Feststoff isoliert. ¹H-NMR (400 MHz, CDCl₃ δ, ppm) 7.52 (m, 1H), 7.39 (m, 1H), 7.29 (m, 1H), 7.24 (m, 1H), 7.04 (m, 1H), 6.87 (d, 1H), 6.65 (d, 1H), 6.26 (s, 1H, NH), 4.13 (s, 2H), 3.30 (s, 3H), 1.78 (m, 2H), 1.54 (m, 2H). ¹³C-NMR (150 MHz, CDCl₃ δ, ppm) 176.8, 141.7, 133.7, 132.6, 132.1, 130.8, 130.4, 129.5, 122.6, 120.8, 113.3, 108.3, 56.8, 27.4, 26.7, 19.7.

### No. A1-181: 1-(4-Cyanophenyl)-N-(1'-methyl-2'-oxo-1',2'-dihydrospiro[cyclopropan-1,3'-indol]-5'-yl)methansulfonamid

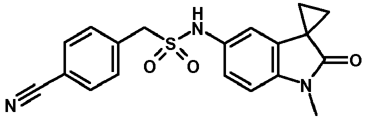

5-Amino-1-methyl-spiro[cyclopropan-1,3-indol]-2(1H)-on (150 mg, 0.79 mmol) wurde zusammen mit (4-Cyanophenyl)methansulfonylchlorid (258 mg, 1.19 mmol) in einem Rundkolben unter Argon in abs. Acetonitril gelöst, danach mit Pyridin (0.13 ml, 1.59 mmol) sowie Dimethylsulfoxid (0.03 ml, 0.48 mmol) versetzt, und 6 h lang bei Raumtemperatur gerührt. Das Reaktionsgemisch wurde daraufhin unter vermindertem Druck eingeengt, der verbleibende Rückstand mit Wasser und Dichlormethan versetzt und die wässrige Phase mehrfach mit Dichlormethan extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1-(4-Cyanophenyl)-N-(1'-methyl-2'-oxo-1',2'-dihydrospiro[cyclopropan-1,3'-indol]-5'-yl)methansulfonamid (245 mg, 84 % der Theorie) als farbloser Feststoff isoliert. ¹H-NMR (400 MHz, d₆-DMSO δ, ppm) 9.64 (s, 1H, NH), 7.84 (d, 2H), 7.48 (d, 2H), 7.05 (m, 2H), 6.78 (d, 1H), 4.54 (s, 2H), 3.20 (s, 3H), 1.57 (m, 2H), 1.53 (m, 2H).

### No. A2-176: N-(1'-Ethyl-2'-oxo-1',2'-dihydrospiro[cyclopropan-1,3'-indol]-5'-yl)-1-(3-nitrophenyl)methansulfonamid

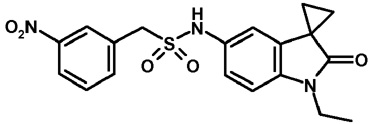

Isatin (5.00 g, 34 mmol) wurde in einem Rundkolben unter Argon in N,N-Dimethylformamid (50 ml) gelöst und mit 1-lodethan (68 mmol) und Kaliumcarbonat (9.39 g, 68 mmol) versetzt. Das resultierende Reaktionsgemisch wurde 6 h lang bei Raumtemperatur gerührt und danach mit Wasser und Essigester versetzt. Die wässrige Phase wurde daraufhin mehrfach mit Essigester extrahiert, und die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1-Ethyl-1H-indol-2,3-dion isoliert, das danach zusammen mit Hydrazin-Hydrat (30.96 g, 612 mmol) 4 h lang auf 130 °C erhitzt und nach dem Abkühlen auf Raumtemperatur auf Eiswasser gegeben wurde. Die wässrige Phase wurde im Anschluß mehrfach mit Essigester extrahiert, und die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1-Ethyl-1,3-dihydro-2H-indol-2-on isoliert, das im nächsten Schritt bei einer Temperatur von 15°C zusammen mit 1,2-Dibromethan (6.27 g, 33 mmol) in einem Gemisch aus abs. Tetrahydrofuran (25 ml) und abs. N,N-Dimethylformamid (1 ml) gelöst und danach vorsichtig portionsweise mit Natriumhydrid (2.76 g, 69 mmol, 60%ige Dispersion) versetzt sowie 1 h lang unter Rückflußbedingungen gerührt wurde. Nach dem Abkühlen auf Raumtemperatur erfolgte die Zugabe von Methanol und Wasser zum Reaktionsgemisch. Die wäßrige Phase wurde intensiv mit Ethylacetat extrahiert, und die vereinigten organischen Phasen wurden zusätzlich jeweils einmal mit gesättigter Natriumcarbonatlösung und Wasser gewaschen. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1'-Ethylspiro[cyclopropan-1,3'-indol]-2'(1'H)-on isoliert. 1'-Ethylspiro[cyclopropan-1,3'-indol]-2'(1'H)-on (5.00 g, 21 mmol) wurde in Eisessig (35 ml) gegeben und danach langsam und vorsichtig mit rauchender Salpetersäure (7 ml) versetzt. Das resultierende Reaktionsgemisch wurde 2h lang bei Raumtemperatur gerührt und danach langsam mit Eiswasser verdünnt. Die wäßrige Phase wurde daraufhin mehrfach mit Ethylacetat extrahiert, und die vereinigten organischen Phasen wurden mit gesättigter Natriumcarbonatlösung gewaschen sowie anschließend über Magnesiumsulfat getrocknet, filtriert und unter vermindertem Druck eingeengt. Durch säulenchromatographische Reinigung des erhaltenen Rohproduktes (Gradient Essigester/Heptan) wurde 1'-Ethyl 5'-nitro-spiro[cyclopropan-1,3'-indol]-2'(1'H)-on (3900 mg, 79 % der Theorie) als farbloser Feststoff isoliert. Im folgenden Schritt wurde 1'-Ethyl 5'-nitro-spiro[cyclopropan-1,3'-indol]-2'(1'H)-on (3.90 g, 17 mmol) zusammen mit Zinn(II)chloriddihydrat (15.16 g, 67 mmol) in abs. Ethanol gegeben und 5 h lang unter Argon bei einer Temperatur von 80°C gerührt. Nach dem Abkühlen auf Raumtemperatur wurde das Reaktionsgemisch auf Eiswasser gegossen und anschließend mit wäßriger NaOH auf pH12 eingestellt. Die wäßrige Phase wurde daraufhin mehrfach mit Ethylacetat extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 5'-Amino-1'-ethylspiro[cyclopropan-1,3'-indol]-2'(1'H)-on (2700 mg, 79 % der Theorie) als farbloser Feststoff isoliert. ¹H-NMR (400 MHz, CDCl₃ δ, ppm) 6.73 (d, 1H), 6.59 (dd, 1H), 6.26 (d, 1H), 3.80 (q, 2H), 1.71 (m, 2H), 1.42 (m, 2H), 1.28 (t, 3H). 5'-Amino-1'-ethylspiro[cyclopropan-1,3'-indol]-2'(1'H)-on (110 mg, 1.0 equiv.) wurde zusammen mit (3-Nitrophenyl)methansulfonylchlorid (161 mg, 1.4 equiv.) in einem Rundkolben unter Argon in abs. Acetonitril gelöst, danach mit Pyridin (0.08 ml, 2.1 equiv.) sowie Dimethylsulfoxid (0.02 ml, 0.60 mmol) versetzt, und 6 h lang bei Raumtemperatur gerührt. Das Reaktionsgemisch wurde daraufhin unter vermindertem Druck eingeengt, der verbleibende Rückstand mit Wasser und Dichlormethan versetzt und die wässrige Phase mehrfach mit Dichlormethan extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde N-(1'-Ethyl-2'-oxo-1',2'-dihydrospiro[cyclopropan-1,3'-indol]-5'-yl)-1-(3-nitrophenyl)methansulfonamid (123 mg, 55 % der Theorie) als farbloser Feststoff isoliert. ¹H-NMR (400 MHz, CDCl₃ δ, ppm) 8.23 (m, 1H), 8.12 (m, 1H), 7.75 (d, 1H), 7.58 (m, 1H), 7.08 (dd, 1H), 6.91 (d, 1H), 6.77 (d, 1H), 6.32 (s, 1H, NH), 4.38 (s, 2H), 3.86 (q, 2H), 1.80 (m, 2H), 1.57 (m, 2H), 1.32 (t, 3H).

### No. A3-167: 1-(3,4-Dichlorphenyl)-N-(2'-oxo-1'-propyl-1',2'-dihydrospiro[cyclopropan-1,3'-indol]-5'-yl)methansulfonamid

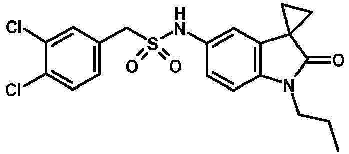

Isatin (5.00 g, 34 mmol) wurde in einem Rundkolben unter Argon in N,N-Dimethylformamid (50 ml) gelöst und mit 1-lodpropan (11.56 g, 68 mmol) und Kaliumcarbonat (9.39 g, 68 mmol) versetzt. Das resultierende Reaktionsgemisch wurde 6 h lang bei Raumtemperatur gerührt und danach mit Wasser und Essigester versetzt. Die wässrige Phase wurde daraufhin mehrfach mit Essigester extrahiert, und die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1-Propyl-1H-indol-2,3-dion (6.10 g, 93 % der Theorie) isoliert. ¹H-NMR (400 MHz, CDCl₃ δ, ppm) 7.59 (m, 2H), 7.11 (m, 1H), 6.89 (d, 1H), 3.71 (t, 2H), 1.76 (sext, 2H), 1.00 (t, 3H). 1-Propyl-1H-indol-2,3-dion (6.10 g, 32 mmol) wurde danach zusammen mit Hydrazin-Hydrat (30.96 g, 612 mmol) 4 h lang auf 130°C erhitzt und nach dem Abkühlen auf Raumtemperatur auf Eiswasser gegeben. Die wässrige Phase wurde im Anschluß mehrfach mit Essigester extrahiert, und die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1-Propyl-1,3-dihydro-2H-indol-2-on (4.50 g, 81 % der Theorie) isoliert. ¹H-NMR (400 MHz, CDCl₃ δ, ppm) 7.28 (m, 2H), 7.02 (m, 1H), 6.84 (d, 1H), 3.68 (t, 2H), 3.52 (s, 2H), 1.71 (sext, 2H), 0.97 (t, 3H). 1-Propyl-1,3-dihydro-2H-indol-2-on (3.90 g, 22 mmol) wurde im nächsten Schritt bei einer Temperatur von 15 °C zusammen mit 1,2-Dibromethan (6.27 g, 33 mmol) in einem Gemisch aus abs. Tetrahydrofuran (25 ml) und abs. N,N-Dimethylformamid (1 ml) gelöst und danach vorsichtig portionsweise mit Natriumhydrid (2.76 g, 69 mmol, 60%ige Dispersion) versetzt sowie 1 h lang unter Rückflußbedingungen gerührt. Nach dem Abkühlen auf Raumtemperatur erfolgte die Zugabe von Methanol und Wasser zum Reaktionsgemisch. Die wäßrige Phase wurde intensiv mit Ethylacetat extrahiert, und die vereinigten organischen Phasen wurden zusätzlich jeweils einmal mit gesättigter Natriumcarbonatlösung und Wasser gewaschen. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1'-Propylspiro[cyclopropan-1,3'-indol]-2'(1'H)-on (4000 mg, 89 % der Theorie) isoliert. ¹H-NMR (400 MHz, CDCl₃ δ, ppm) 7.22 (m, 1H), 7.00 (m, 1H), 6.92 (d, 1H), 6.83 (d, 1H), 3.75 (t, 2H), 1.74 (m, 2H), 1.50 (m, 2H), 1.26 (m, 2H), 0.98 (t, 3H). 1'-Propylspiro[cyclopropan-1,3'-indol]-2'(1'H)-on (5.20 g, 21 mmol) wurde in Eisessig (35 ml) gegeben und danach langsam und vorsichtig mit rauchender Salpetersäure (7 ml) versetzt. Das resultierende Reaktionsgemisch wurde 2h lang bei Raumtemperatur gerührt und danach langsam mit Eiswasser verdünnt. Die wäßrige Phase wurde daraufhin mehrfach mit Ethylacetat extrahiert, und die vereinigten organischen Phasen wurden mit gesättigter Natriumcarbonatlösung gewaschen sowie anschließend über Magnesiumsulfat getrocknet, filtriert und unter vermindertem Druck eingeengt. Durch säulenchromatographische Reinigung des erhaltenen Rohproduktes (Gradient Essigester/Heptan) wurde 5'-Nitro-1'-propylspiro[cyclopropan-1,3'-indol]-2'(1'H)-on (3600 mg, 71 % der Theorie) als farbloser Feststoff isoliert. ¹H-NMR (400 MHz, CDCl₃ δ, ppm) 8.26 (dd, 1H), 7.73 (d, 1H), 6.98 (d, 1H), 3.30 (t, 2H), 1.87 (m, 2H), 1.75 (sext, 1H), 1.68 (m, 2H), 1.00 (t, 3H). Im folgenden Schritt wurde 5'-Nitro-1'-propylspiro[cyclopropan-1,3'-indol]-2'(1'H)-on (3.60 g, 15 mmol) zusammen mit Zinn(II)chloriddihydrat (13.19 g, 58 mmol) in abs. Ethanol gegeben und 5 h lang unter Argon bei einer Temperatur von 80°C gerührt. Nach dem Abkühlen auf Raumtemperatur wurde das Reaktionsgemisch auf Eiswasser gegeossen und anschließend mit wäßriger NaOH auf pH12 eingestellt. Die wäßrige Phase wurde daraufhin mehrfach mit Ethylacetat extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 5'-Amino-1'-propylspiro[cyclopropan-1,3'-indol]-2'(1'H)-on (3100 mg, 98 % der Theorie) als farbloser Feststoff isoliert. ¹H-NMR (400 MHz, CDCl₃ δ, ppm) 6.71 (d, 1H), 6.58 (dd, 1H), 6.25 (d, 1H), 3.71 (t, 2H), 1.73 (m, 2H), 1.42 (m, 2H), 1.25 (m, 2H), 0.98 (t, 3H). 5'-Amino-1'-propylspiro[cyclopropan-1,3'-indol]-2'(1'H)-on (100 mg, 1.0 equiv.) wurde zusammen mit (3,4-Dichlorphenyl)methansulfonylchlorid (168 mg, 1.4 equiv.) in einem Rundkolben unter Argon in abs. Acetonitril gelöst, danach mit Pyridin (0.08 ml, 2.1 equiv.) sowie Dimethylsulfoxid (0.04 ml, 0.60 mmol) versetzt, und 6 h lang bei Raumtemperatur gerührt. Das Reaktionsgemisch wurde daraufhin unter vermindertem Druck eingeengt, der verbleibende Rückstand mit Wasser und Dichlormethan versetzt und die wässrige Phase mehrfach mit Dichlormethan extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1-(3,4-Dichlorphenyl)-N-(2'-oxo-1'-propyl-1',2'-dihydrospiro[cyclopropan-1,3'-indol]-5'-yl)methansulfonamid (147 mg, 68 % der Theorie) als farbloser Feststoff isoliert. ¹H-NMR (400 MHz, CDCl₃ δ, ppm) 7.42 (d, 1H), 7.36 (d, 1H), 7.19 (m, 1H), 7.01 (m, 1H), 6.85 (d, 1H), 6.67 (d, 1H), 6.33 (s, 1H, NH), 4.24 (s, 2H), 3.74 (t, 2H), 1.78 (m, 2H), 1.73 (sext, 2H), 1.52 (m, 2H), 0.99 (t, 3H).

### No. B1-152: N-(1'-Methyl-2'-oxo-1',2'-dihydrospiro[cyclobutan-1,3'-indol]-5'-yl)-1-(4-methylphenyl)methansulfonamid

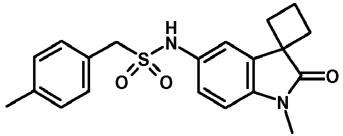

1-Methyl-1,3-dihydro-2H-indol-2-on (1.00 g, 7 mmol) und 1,3-Dibrompropan (2.06 g, 10 mmol) wurden in einem ausgeheizten Rundkolben unter Argon in abs. N,N-Dimethylformamid gelöst und 5 Min lang bei Raumtemperatur gerührt. Danach wurde die Reaktionslösung auf 0°C eingekühlt, und im Anschluß erfolgte die portionsweise Zugabe von Natriumhydrid (0.82 g, 20 mmol, 60%ige Dispersion). Das resultierende Reaktionsgemisch wurde ca. 2 h lang nachgerührt, danach mit Methanol (4ml) versetzt und nach weiteren 5 Min mit ges. Ammoniumchloridlösung (15 ml) sowie Wasser (200 ml) versetzt. Die wäßrige Phase wurde intensiv mit Ethylacetat extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1'-Methylspiro[cyclobutan-1,3'-indol]-2'(1'H)-on (360 mg, 29 % der Theorie) isoliert. ¹H-NMR (400 MHz, CDCl₃ δ, ppm) 7.52 (d, 1H), 7.27 (m, 1H), 7.09 (m, 1H), 6.77 (d, 1H), 3.20 (s, 3H), 2.67 (m, 2H), 2.33 (m, 4H). 1'-Methylspiro[cyclobutan-1,3'-indol]-2'(1'H)-on (360 mg, 1.92 mmol) wurde in konz. Essigsäure (5 ml) gegeben und danach vorsichtig mit rauchender Salpetersäure (0.21 ml, 5.06 mmol) versetzt. Das resultierende Reaktionsgemisch wurde 2h lang bei Raumtemperatur gerührt und danach mit Eiswasser verdünnt. Die wäßrige Phase wurde daraufhin mehrfach mit Ethylacetat extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1'-Methyl-5'-nitrospiro[cyclobutan-1,3'-indol]-2'(1'H)-on (380 mg, 85 % der Theorie) als farbloser Feststoff isoliert. ¹H-NMR (400 MHz, CDCl₃ δ, ppm) 8.38 (d, 1H), 8.26 (dd, 1H), 6.86 (d, 1H), 3.25 (s, 3H), 2.70 (m, 2H), 2.42 (m, 4H). Im nächsten Schritt wurde 1'-Methyl-5'-nitrospiro[cyclobutan-1,3'-indol]-2'(1'H)-on (450 mg, 1.55 mmol) zusammen mit Zinn(II)chloriddihydrat (1.40 g, 6.20 mmol) in abs. Ethanol gegeben und 5 h lang unter Argon bei einer Temperatur von 80°C gerührt. Nach dem Abkühlen auf Raumtemperatur wurde das Reaktionsgemisch auf Eiswasser gegeossen und anschließend mit wäßriger NaOH auf pH12 eingestellt. Die wäßrige Phase wurde daraufhin mehrfach mit Ethylacetat extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 5'-Amino-1'-methylspiro[cyclobutan-1,3'-indol]-2'(1'H)-on (230 mg, 66 % der Theorie) als farbloser Feststoff isoliert. ¹H-NMR (400 MHz, CDCl₃ δ, ppm) 6.98 (d, 1H), 6.62 (m, 2H), 3.14 (s, 3H), 2.64 (m, 2H), 2.38-2.20 (m, 4H). 5'-Amino-1'-methylspiro[cyclobutan-1,3'-indol]-2'(1'H)-on (150 mg, 0.79 mmol) wurde zusammen mit (4-Methylphenyl)methansulfonylchlorid (156 mg, 0.76 mmol) in einem ausgeheizten Rundkolben unter Argon in abs. Acetonitril (5 ml) gelöst, danach mit Pyridin (0.11 ml, 1.38 mmol) sowie Dimethylsulfoxid (0.03 ml, 0.42 mmol) versetzt, und 6 h lang bei Raumtemperatur gerührt. Das Reaktionsgemisch wurde daraufhin unter vermindertem Druck eingeengt, der verbleibende Rückstand mit Wasser, verd. Salzsäure und Dichlormethan versetzt und die wässrige Phase mehrfach mit Dichlormethan extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde N-(1'-Methyl-2'-oxo-1',2'-dihydrospiro[cyclobutan-1,3'-indol]-5'-yl)-1-(4-methylphenyl)methansulfonamid (118 mg, 46 % der Theorie) als farbloser Feststoff isoliert. ¹H-NMR (400 MHz, CDCl₃ δ, ppm) 7.29 (m, 3H), 7.16 (d, 1H), 6.96 (br. s, 1H, NH), 6.61 (d, 1H), 4.30 (s, 2H), 3.13 (s, 3H), 2.62 (m, 2H), 2.33 (s, 3H), 2.32-2.17 (m, 4H).

### No. E1-152: N-(1'-Methyl-2'-oxo-1',2'-dihydrospiro[cyclopent-3-en-1,3'-indol]-5'-yl)-1-(4-methylphenyl)methansulfonamid

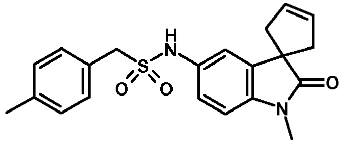

3,3-Diallyl-5-nitro-1,3-dihydro-2H-indol-2-on (340 mg, 1.0 equiv) wurde in einem Rundkolben unter Argon in N,N-Dimethylformamid (5 ml) gelöst und mit Methyliodid (0.16 ml, 2.0 equiv) und Kaliumcarbonat (364 mg, 2.0 equiv) versetzt. Das resultierende Reaktionsgemisch wurde 6 h lang bei Raumtemperatur gerührt und danach mit Wasser und Essigester versetzt. Die wässrige Phase wurde daraufhin mehrfach mit Essigester extrahiert, und die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 3,3-Diallyl-1-methyl-5-nitro-1,3-dihydro-2H-indol-2-on isoliert, das nach der Reinigung direkt weiter umgesetzt wurde. ¹H-NMR (400 MHz, CDCl₃ δ, ppm) 8.28 (m, 1H), 8.09 (d, 1H), 6.90 (d, 1H), 5.38 (m, 2H), 5.04 (m, 2H), 4.96 (m, 2H), 3.25 (s, 3H), 2.62 (m, 4H), 1.00. 3,3-Diallyl-1-methyl-5-nitro-1,3-dihydro-2H-indol-2-on (550 mg, 2.00 mmol) wurde in abs. Toluol (10 ml) gegeben und danach unter Argon mit (1,3-Bis-(2,4,6-trimethylphenyl)-2-imidazolidinyliden)dichlor(o-isopropoxyphenylmethylen)ruthenium (2.5 mg 2 mol%) versetzt. Das resultierende Reaktionsgemisch wurde einen Tag lang bei einer Temperatur von 90-100°C gerührt und nach dem Abkühlen auf Raumtemperatur mit Wasser und Ammoniumchloridlösung verdünnt. Die wäßrige Phase wurde daraufhin mehrfach mit Ethylacetat extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1'-Methyl-5'-nitrospiro[cyclopent-3-en-1,3'-indol]-2'(1'H)-on isoliert. ¹H-NMR (400 MHz, CDCl₃ δ, ppm) 8.27 (dd, 1H), 8.14 (d, 1H), 6.91 (d, 1H), 5.88 (m, 2H), 3.30 (s, 3H), 3.07 (m, 2H), 2.64 (m, 2H). Im nächsten Schritt wurde 1'-Methyl-5'-nitrospiro[cyclopent-3-en-1,3'-indol]-2'(1'H)-on (500 mg, 2.03 mmol) zusammen mit Zinn(II)chloriddihydrat (1.66 g, 4.0 equiv.) in abs. Ethanol (15 ml) gegeben und 3 h lang unter Argon bei einer Temperatur von 80°C gerührt. Nach dem Abkühlen auf Raumtemperatur wurde das Reaktionsgemisch auf Eiswasser gegossen und anschließend mit wäßriger NaOH auf pH12 eingestellt. Die wäßrige Phase wurde daraufhin mehrfach mit Ethylacetat extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 5'-Amino-1'-methylspiro[cyclopent-3-en-1,3'-indol]-2'(1'H)-on isoliert. ¹H-NMR (400 MHz, CDCl₃ δ, ppm) 6.71 (d, 1H), 6.62 (m, 2H), 5.82 (m, 2H), 3.18 (s, 3H), 3.01 (m, 2H), 2.58 (m, 2H). 5'-Amino-1'-methylspiro[cyclopent-3-en-1,3'-indol]-2'(1'H)-on (200 mg, 0.94 mmol) wurde zusammen mit (4-Methylphenyl)methansulfonylchlorid (248 mg, 1.3 equiv) in einem ausgeheizten Rundkolben unter Argon in abs. Acetonitril (5 ml) gelöst, danach mit Pyridin (0.23 ml, 3.1 equiv) versetzt und 1 h lang bei 70°C gerührt. Das Reaktionsgemisch wurde daraufhin unter vermindertem Druck eingeengt, der verbleibende Rückstand mit Wasser, verd. Salzsäure und Dichlormethan versetzt und die wässrige Phase mehrfach mit Dichlormethan extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde N-(1'-Methyl-2'-oxo-1',2'-dihydrospiro[cyclopent-3-en-1,3'-indol]-5'-yl)-1-(4-methylphenyl)methansulfonamid (169 mg, 47 % der Theorie) als farbloser Feststoff isoliert. ¹H-NMR (400 MHz, CDCl₃ δ, ppm) 7.20 (d, 2H), 7.18 (d, 2H), 7.11 (dd, 1H), 6.99 (d, 1H), 6.79 (d, 1H), 5.99 (br. s, 1H, NH), 5.85 (m, 2H), 4.25 (s, 2H), 3.23 (s, 3H), 3.04 (m, 2H), 2.59 (m, 2H).

### No. H1-181: 1-(4-Cyanophenyl)-N-(1-methyl-2-oxo-1,2,2',3',5',6'-hexahydrospiro[indol-3,4'-pyran]-5-yl)methansulfonamid

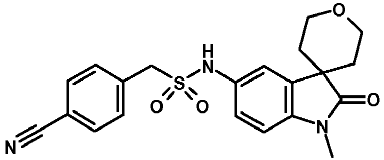

1-Methyl-1,3-dihydro-2H-indol-2-on (2.50 g, 17 mmol) wurde in einem ausgeheizten Rundkolben unter Argon in abs. N,N-Dimethylformamid gelöst und 5 Min lang bei Raumtemperatur gerührt. Danach wurde die Reaktionslösung auf 0 °C eingekühlt, und im Anschluß erfolgte die portionsweise Zugabe von Natriumhydrid (2.11 g, 53 mmol, 60%ige Dispersion). Das resultierende Reaktionsgemisch wurde ca. 1 h lang bei Raumtemperatur nachgerührt, dann mit 2-[2-(4-Methylphenyl)sulfonyloxyethoxy]-ethyl-4-methylphenylsulfonat (5.63 g, 14 mmol) versetzt und weitere 4 h bei einer Temperatur von 50°C gerührt. Nach dem Abkühlen auf Raumtemperatur wurde das reaktionsgemisch mit Methanol (4ml) versetzt und nach weiteren 5 Min mit ges. Ammoniumchloridlösung (15 ml) sowie Wasser (200 ml) versetzt. Die wäßrige Phase wurde intensiv mit Ethylacetat extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1-Methyl-2',3',5',6'-tetrahydrospiro[indol-3,4'-pyran]-2(1H)-on (1.60 g, 43 % der Theorie) isoliert. ¹H-NMR (400 MHz, CDCl₃ δ, ppm) 7.37 (d, 1H), 7.30 (m, 1H), 7.09 (m, 1H), 6.86 (d, 1H), 4.28 (m, 2H), 3.93 (m, 2H), 3.21 (s, 3H), 1.86 (m, 4H). 1-Methyl-2',3',5',6'-tetrahydrospiro[indol-3,4'-pyran]-2(1H)-on (1.60 g, 7.37 mmol) wurde in konz. Essigsäure (12 ml) gegeben und danach vorsichtig mit rauchender Salpetersäure (3.0 ml) versetzt. Das resultierende Reaktionsgemisch wurde 2h lang bei Raumtemperatur gerührt und danach mit Eiswasser verdünnt. Die wäßrige Phase wurde daraufhin mehrfach mit Ethylacetat extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1-Methyl-5-nitro-2',3',5',6'-tetrahydrospiro[indol-3,4'-pyran]-2(1H)-on (1.90 g, 98 % der Theorie) als farbloser Feststoff isoliert. ¹H-NMR (400 MHz, CDCl₃ δ, ppm) 8.30 (d, 1H), 8.27 (dd, 1H), 6.94 (d, 1H), 4.29 (m, 2H), 3.96 (m, 2H), 3.28 (s, 3H), 1.95-1.86 (m, 4H). Im nächsten Schritt wurde 1-Methyl-5-nitro-2',3',5',6'-tetrahydrospiro[indol-3,4'-pyran]-2(1H)-on (1.90 g, 7.25 mmol) zusammen mit Zinn(II)chloriddihydrat (6.19 g, 27 mmol) in abs. Ethanol (30 ml) gegeben und 3 h lang unter Argon bei einer Temperatur von 80 °C gerührt. Nach dem Abkühlen auf Raumtemperatur wurde das Reaktionsgemisch auf Eiswasser gegeossen und anschließend mit wäßriger NaOH auf pH12 eingestellt. Die wäßrige Phase wurde daraufhin mehrfach mit Ethylacetat extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 5-Amino-1-methyl-2',3',5',6'-tetrahydrospiro[indol-3,4'-pyran]-2(1H)-on (1.06 g, 63 % der Theorie) isoliert. ¹H-NMR (400 MHz, CDCl₃ δ, ppm) 6.79 (d, 1H), 6.64 (m, 2H), 4.28 (m, 2H), 3.91 (m, 2H), 3.16 (s, 3H), 1.87-1.80 (m, 4H). 5-Amino-1-methyl-2',3',5',6'-tetrahydrospiro[indol-3,4'-pyran]-2(1H)-on (150 mg, 0.64 mmol) wurde zusammen mit (4-Cyanophenyl)methansulfonylchlorid (181 mg, 1.3 equiv) in einem ausgeheizten Rundkolben unter Argon in abs. Acetonitril (5 ml) gelöst, danach mit Pyridin (0.16 ml, 3.1 equiv) versetzt und 1 h lang bei 70°C gerührt. Das Reaktionsgemisch wurde daraufhin unter vermindertem Druck eingeengt, der verbleibende Rückstand mit Wasser, verd. Salzsäure und Dichlormethan versetzt und die wässrige Phase mehrfach mit Dichlormethan extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1-(4-Cyanophenyl)-N-(1-methyl-2-oxo-1,2,2',3',5',6'-hexahydrospiro[indol-3,4'-pyran]-5-yl)methansulfonamid (181 mg, 68 % der Theorie) als farbloser Feststoff isoliert. ¹H-NMR (400 MHz, CDCl₃ δ, ppm) 7.69 (d, 2H), 7.48 (d, 2H), 7.22 (d, H), 7.07 (dd, 1H), 6.82 (d, 1H), 6.24 (br. s, 1H, NH), 4.36 (s, 2H), 4.28 (m, 2H), 3.92 (m, 2H), 3.22 (s, 3H), 1.86 (m, 4H).

In Analogie zu den oben angeführten und an entsprechender Stelle rezitierten Herstellungsbeispielen und unter Berücksichtigung der allgemeinen Angaben zur Herstellung von substituierten Dihydrooxindolylsulfonamiden der allgemeinen Formel (I) erhält man die nachfolgend genannten Verbindungen.

A1. Verbindungen A1-1 bis A1-600 der allgemeinen Formel (Ib), worin R¹ für Methyl, R², R³ und R⁴ für Wasserstoff stehen und W, R⁵, R⁶ den Definitionen (Nos 1 bis 600; entsprechend Verbindungen A1-1 bis A1-600) in der folgenden Tabelle 1 entsprechen. Ein Pfeil in einer der in Tabelle 1 aufgeführten Definitionen für R⁵, R⁶ steht für eine Bindung des betreffenden Restes zur Kernstruktur (Ib).

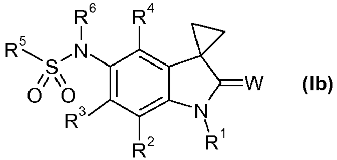

**Tabelle 1**

| No. | R⁵ | W | R⁶ |
|---|---|---|---|
| 1 | CH₃ | O | H |
| 2 | Ethyl | O | H |
| 3 | n-Propyl | O | H |
| 4 | iso-Propyl | O | H |
| 5 | n-Butyl | O | H |
| 6 | c-Propyl | O | H |
| 7 | c-Butyl | O | H |
| 8 | c-Pentyl | O | H |
| 9 | c-Hexyl | O | H |
| 10 | CH₃ | S | H |
| 11 | CH₃ | O | 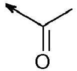 |
| 12 | CH₃ | O | 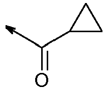 |
| 13 | CH₃ | O | 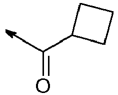 |
| 14 | CH₃ | O | CH₃ |
| 15 | CH₃ | O | 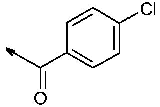 |
| 16 | CH₃ | O | Ethyl |
| 17 | Ethyl | O | CH₃ |
| 18 | iso-Propyl | O | CH₃ |
| 19 | c-Propyl | O | CH₃ |
| 20 | 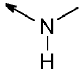 | O | H |
| 21 | 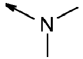 | O | H |
| 22 | 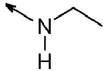 | O | H |
| 23 | 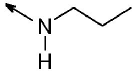 | O | H |
| 24 | 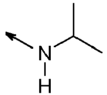 | O | H |
| 25 | 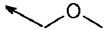 | O | H |
| 26 | 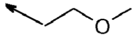 | O | H |
| 27 | 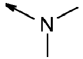 | S | H |
| 28 | 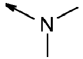 | O | CH₃ |
| 29 | 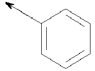 | O | H |
| 30 | 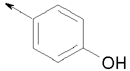 | O | H |
| 31 | 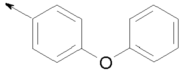 | O | H |
| 32 | 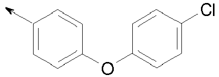 | O | H |
| 33 | 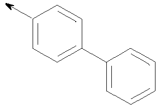 | O | H |
| 34 | 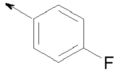 | O | H |
| 35 | 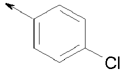 | O | H |
| 36 | 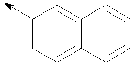 | O | H |
| 37 | 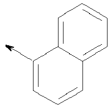 | O | H |
| 38 | 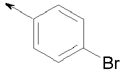 | O | H |
| 39 | 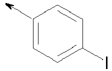 | O | H |
| 40 | 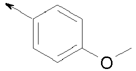 | O | H |
| 41 | 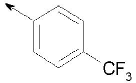 | O | H |
| 42 | 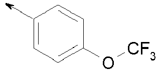 | O | H |
| 43 | 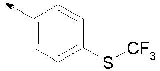 | O | H |
| 44 | 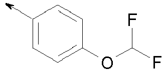 | O | H |
| 45 | 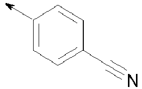 | O | H |
| 46 | 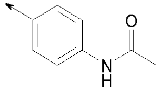 | O | H |
| 47 | 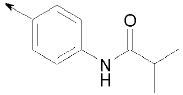 | O | H |
| 48 | 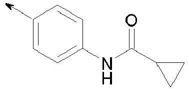 | O | H |
| 49 | NH₂ | O | H |
| 50 | 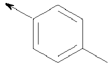 | O | H |
| 51 | 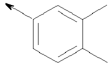 | O | H |
| 52 | 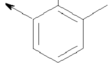 | O | H |
| 53 | 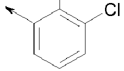 | O | H |
| 54 | 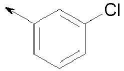 | O | H |
| 55 | 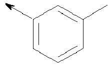 | O | H |
| 56 | 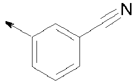 | O | H |
| 57 | 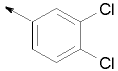 | O | H |
| 58 | 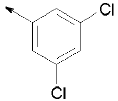 | O | H |
| 59 | 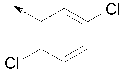 | O | H |
| 60 | 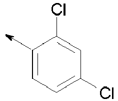 | O | H |
| 61 | 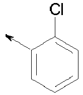 | O | H |
| 62 | 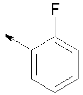 | O | H |
| 63 | 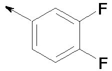 | O | H |
| 64 | 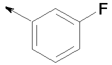 | O | H |
| 65 | 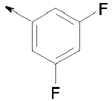 | O | H |
| 66 | 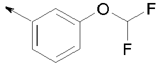 | O | H |
| 67 | 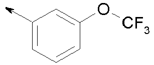 | O | H |
| 68 | 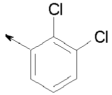 | O | H |
| 69 | 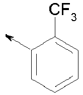 | O | H |
| 70 | 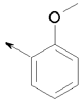 | O | H |
| 71 | 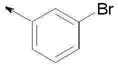 | O | H |
| 72 | 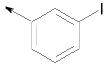 | O | H |
| 73 | 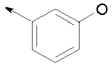 | O | H |
| 74 | 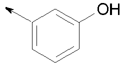 | O | H |
| 75 | 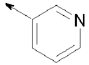 | O | H |
| 76 | 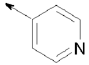 | O | H |
| 77 | 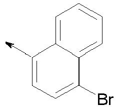 | O | H |
| 78 | 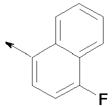 | O | H |
| 79 | 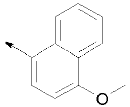 | O | H |
| 80 | 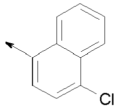 | O | H |
| 81 | 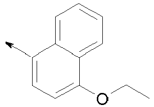 | O | H |
| 82 | 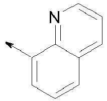 | O | H |
| 83 | 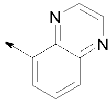 | O | H |
| 84 | 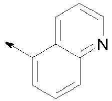 | O | H |
| 85 | 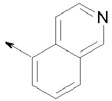 | O | H |
| 86 | 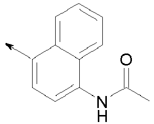 | O | H |
| 87 | 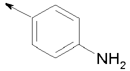 | O | H |
| 88 | 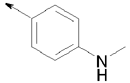 | O | H |
| 89 | 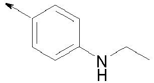 | O | H |
| 90 | 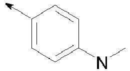 | O | H |
| 91 | 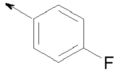 | S | H |
| 92 | 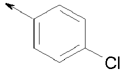 | S | H |
| 93 | 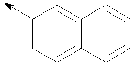 | S | H |
| 94 | 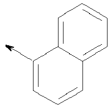 | S | H |
| 95 | 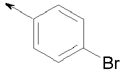 | S | H |
| 96 | 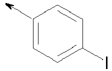 | S | H |
| 97 | 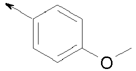 | S | H |
| 98 | 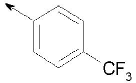 | S | H |
| 99 | 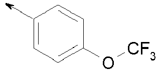 | S | H |
| 100 | 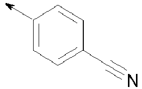 | S | H |
| 101 | 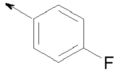 | O | CH₃ |
| 102 | 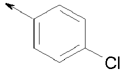 | O | CH₃ |
| 103 | 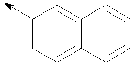 | O | CH₃ |
| 104 | 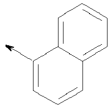 | O | CH₃ |
| 105 | 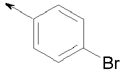 | O | CH₃ |
| 106 | 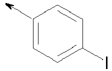 | O | CH₃ |
| 107 | 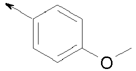 | O | CH₃ |
| 108 | 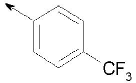 | O | CH₃ |
| 109 | 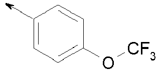 | O | CH₃ |
| 110 | 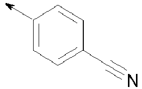 | O | 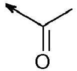 |
| 111 | 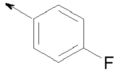 | O | 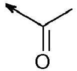 |
| 112 | 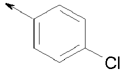 | O | 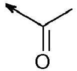 |
| 113 | 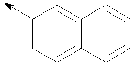 | O | 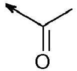 |
| 114 | 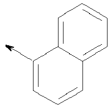 | O | 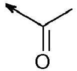 |
| 115 | 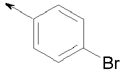 | O | 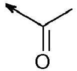 |
| 116 | 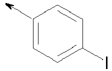 | O | 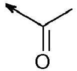 |
| 117 | 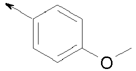 | O | 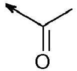 |
| 118 | 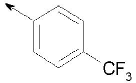 | O | 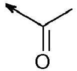 |
| 119 | 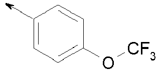 | O | 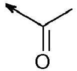 |
| 120 | 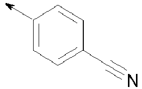 | O | 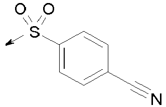 |
| 121 | 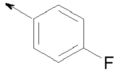 | O | 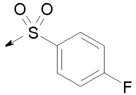 |
| 122 | 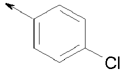 | O | 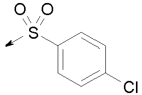 |
| 123 | 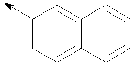 | O | 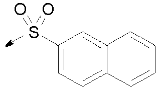 |
| 124 | 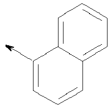 | O | 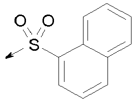 |
| 125 | 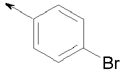 | O | 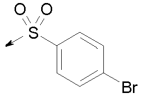 |
| 126 | 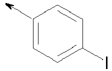 | O | 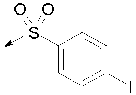 |
| 127 | 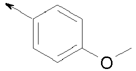 | O | 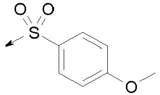 |
| 128 | 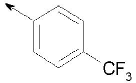 | O | 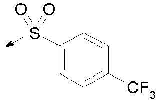 |
| 129 | 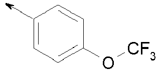 | O | 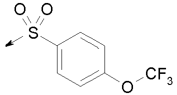 |
| 130 | 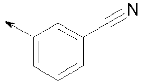 | O | 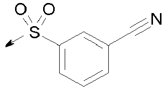 |
| 131 | 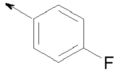 | O | 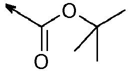 |
| 132 | 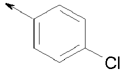 | O | 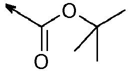 |
| 133 | 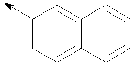 | O | 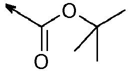 |
| 134 | 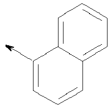 | O | 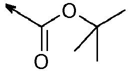 |
| 135 | 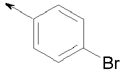 | O | 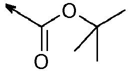 |
| 136 | 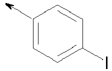 | O | 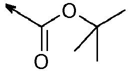 |
| 137 | 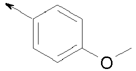 | O | 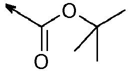 |
| 138 | 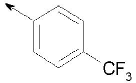 | O | 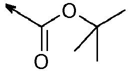 |
| 139 | 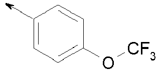 | O | 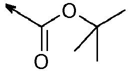 |
| 140 | 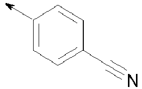 | O | 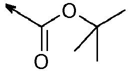 |
| 141 | 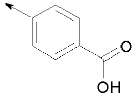 | O | H |
| 142 | 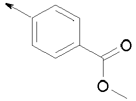 | O | H |
| 143 | 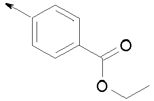 | O | H |
| 144 | 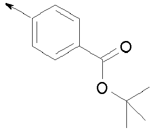 | O | H |
| 145 | 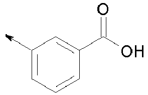 | O | H |
| 146 | 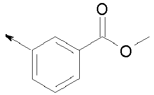 | O | H |
| 147 | 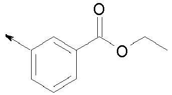 | O | H |
| 148 | 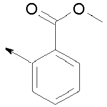 | O | H |
| 149 | 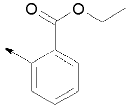 | O | H |
| 150 | 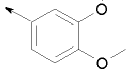 | O | H |
| 151 | 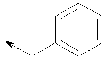 | O | H |
| 152 | 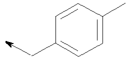 | O | H |
| 153 | 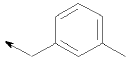 | O | H |
| 154 | 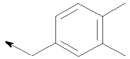 | O | H |
| 155 | 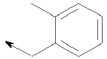 | O | H |
| 156 | 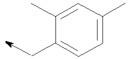 | O | H |
| 157 | 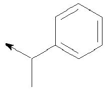 | O | H |
| 158 | 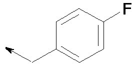 | O | H |
| 159 | 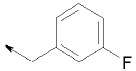 | O | H |
| 160 | 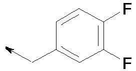 | O | H |
| 161 | 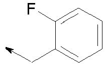 | O | H |
| 162 | 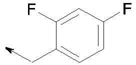 | O | H |
| 163 | 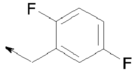 | O | H |
| 164 | 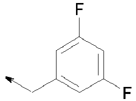 | O | H |
| 165 | 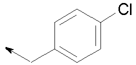 | O | H |
| 166 | 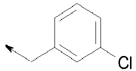 | O | H |
| 167 | 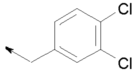 | O | H |
| 168 | 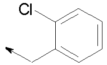 | O | H |
| 169 | 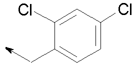 | O | H |
| 170 | 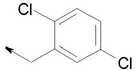 | O | H |
| 171 | 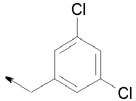 | O | H |
| 172 | 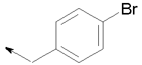 | O | H |
| 173 | 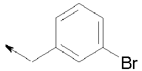 | O | H |
| 174 | 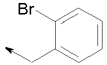 | O | H |
| 175 | 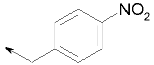 | O | H |
| 176 | 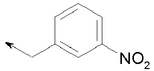 | O | H |
| 177 | 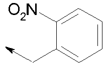 | O | H |
| 178 | 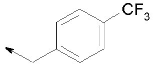 | O | H |
| 179 | 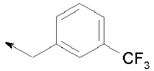 | O | H |
| 180 | 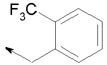 | O | H |
| 181 | 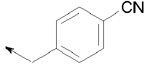 | O | H |
| 182 | 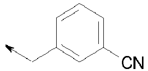 | O | H |
| 183 | 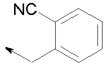 | O | H |
| 184 | 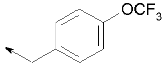 | O | H |
| 185 | 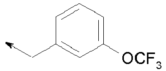 | O | H |
| 186 | 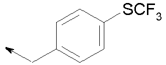 | O | H |
| 187 | 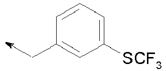 | O | H |
| 188 | 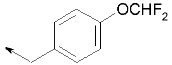 | O | H |
| 189 | 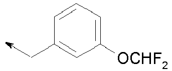 | O | H |
| 190 | 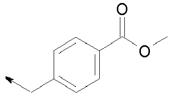 | O | H |
| 191 | 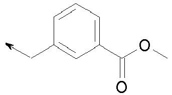 | O | H |
| 192 | 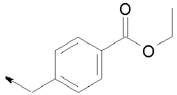 | O | H |
| 193 | 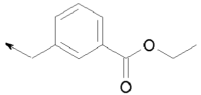 | O | H |
| 194 | 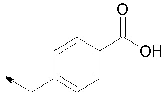 | O | H |
| 195 | 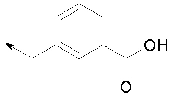 | O | H |
| 196 | 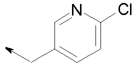 | O | H |
| 197 | 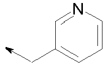 | O | H |
| 198 | 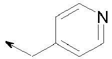 | O | H |
| 199 | 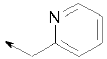 | O | H |
| 200 | 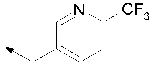 | O | H |
| 201 | 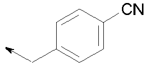 | S | H |
| 202 | 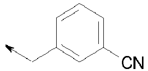 | S | H |
| 203 | 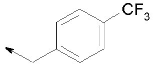 | S | H |
| 204 | 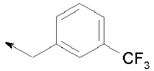 | S | H |
| 205 | 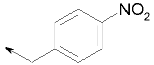 | S | H |
| 206 | 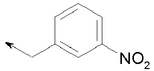 | S | H |
| 207 | 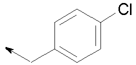 | S | H |
| 208 | 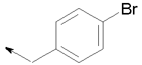 | S | H |
| 209 | 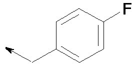 | S | H |
| 210 | 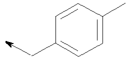 | S | H |
| 211 | 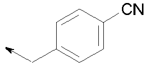 | O | CH₃ |
| 212 | 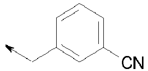 | O | CH₃ |
| 213 | 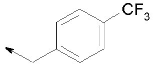 | O | CH₃ |
| 214 | 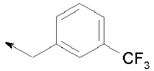 | O | CH₃ |
| 215 | 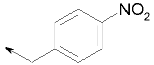 | O | CH₃ |
| 216 | 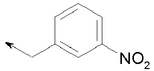 | O | CH₃ |
| 217 | 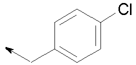 | O | CH₃ |
| 218 | 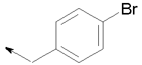 | O | CH₃ |
| 219 | 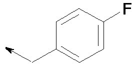 | O | CH₃ |
| 220 | 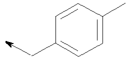 | O | CH₃ |
| 221 | 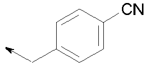 | O | Ethyl |
| 222 | 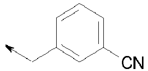 | O | Ethyl |
| 223 | 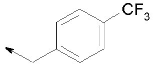 | O | Ethyl |
| 224 | 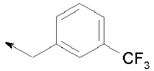 | O | Ethyl |
| 225 | 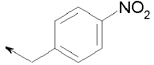 | O | Ethyl |
| 226 | 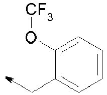 | O | H |
| 227 | 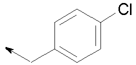 | O | Ethyl |
| 228 | 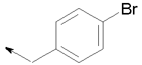 | O | Ethyl |
| 229 | 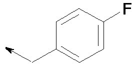 | O | Ethyl |
| 230 | 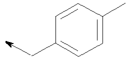 | O | Ethyl |
| 231 | 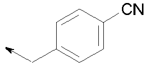 | O | 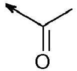 |
| 232 | 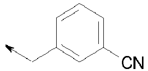 | O | 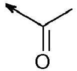 |
| 233 | 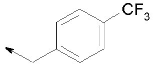 | O | 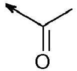 |
| 234 | 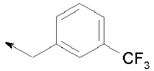 | O | 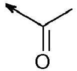 |
| 235 | 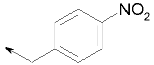 | O | 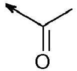 |
| 236 | 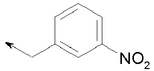 | O | 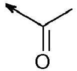 |
| 237 | 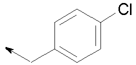 | O | 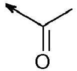 |
| 238 | 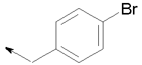 | O | 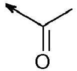 |
| 239 | 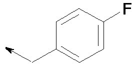 | O | 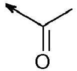 |
| 240 | 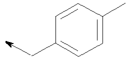 | O | 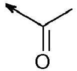 |
| 241 | 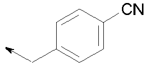 | O | 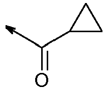 |
| 242 | 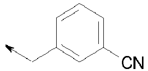 | O | 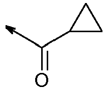 |
| 243 | 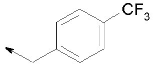 | O | 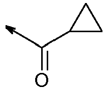 |
| 244 | 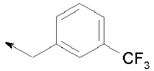 | O | 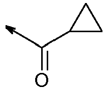 |
| 245 | 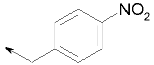 | O | 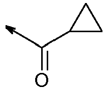 |
| 246 | 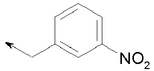 | O | 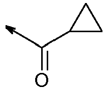 |
| 247 | 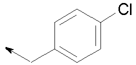 | O | 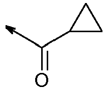 |
| 248 | 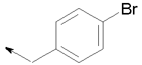 | O | 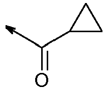 |
| 249 | 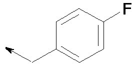 | O | 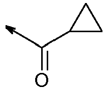 |
| 250 | 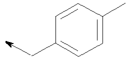 | O | 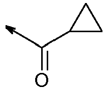 |
| 251 | 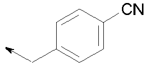 | O | 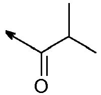 |
| 252 | 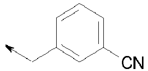 | O | 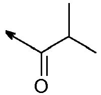 |
| 253 | 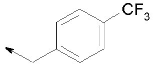 | O | 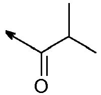 |
| 254 | 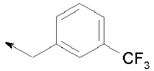 | O | 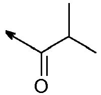 |
| 255 | 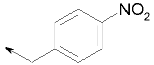 | O | 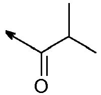 |
| 256 | 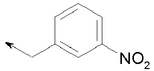 | O | 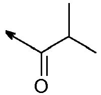 |
| 257 | 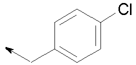 | O | 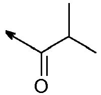 |
| 258 | 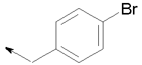 | O | 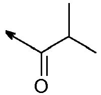 |
| 259 | 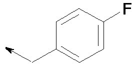 | O | 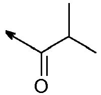 |
| 260 | 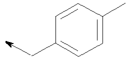 | O | 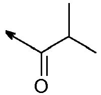 |
| 261 | 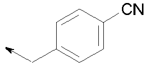 | O | 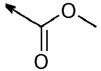 |
| 262 | 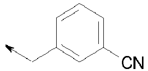 | O | 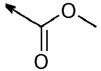 |
| 263 | 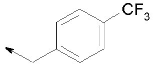 | O | 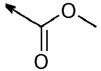 |
| 264 | 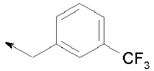 | O | 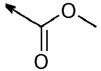 |
| 265 | 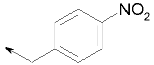 | O | 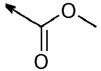 |
| 266 | 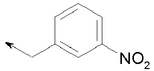 | O | 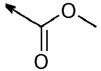 |
| 267 | 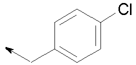 | O | 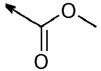 |
| 268 | 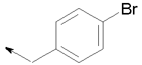 | O | 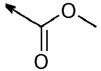 |
| 269 | 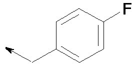 | O | 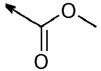 |
| 270 | 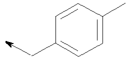 | O | 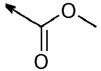 |
| 271 | 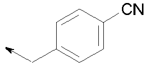 | O | 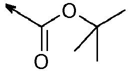 |
| 272 | 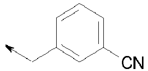 | O | 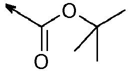 |
| 273 | 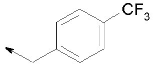 | O | 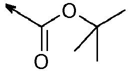 |
| 274 | 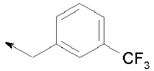 | O | 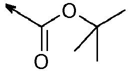 |
| 275 | 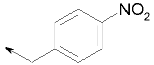 | O | 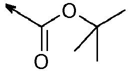 |
| 276 | 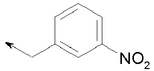 | O | 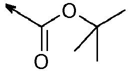 |
| 277 | 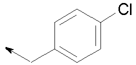 | O | 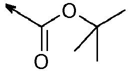 |
| 278 | 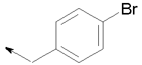 | O | 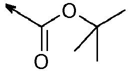 |
| 279 | 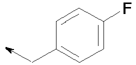 | O | 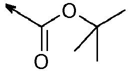 |
| 280 | 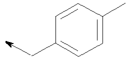 | O | 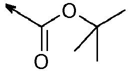 |
| 281 | 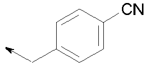 | O | 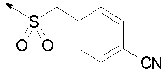 |
| 282 | 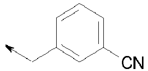 | O | 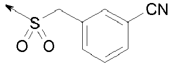 |
| 283 | 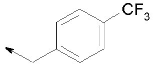 | O | 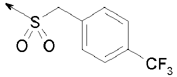 |
| 284 | 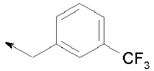 | O | 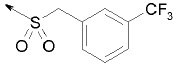 |
| 285 | 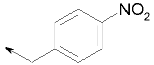 | O | 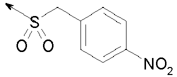 |
| 286 | 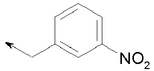 | O | 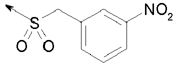 |
| 287 | 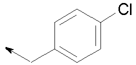 | O | 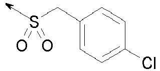 |
| 288 | 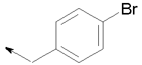 | O | 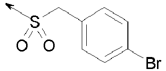 |
| 289 | 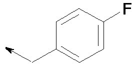 | O | 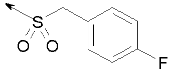 |
| 290 | 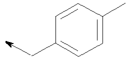 | O | 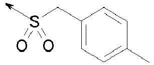 |
| 291 | 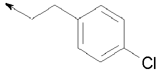 | O | H |
| 292 | 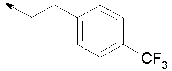 | O | H |
| 293 | 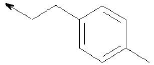 | O | H |
| 294 | 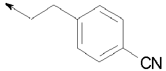 | O | H |
| 295 | 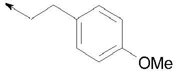 | O | H |
| 296 | 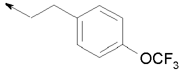 | O | H |
| 297 | 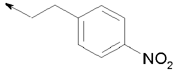 | O | H |
| 298 | 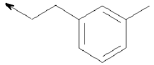 | O | H |
| 299 | 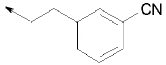 | O | H |
| 300 | 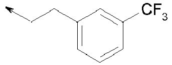 | O | H |
| 301 | 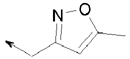 | O | H |
| 302 | Pyrimidin-4-ylmethyl | O | H |
| 303 | Pyrazin-2-ylmethyl | O | H |
| 304 | Pyridazin-3-ylmethyl | O | H |
| 305 | Pyridazin-4-ylmethyl | O | H |
| 306 | Pyrimidin-2-ylmethyl | O | H |
| 307 | Pyrimidin-5-ylmethyl | O | H |
| 308 | (6-Methylpyridin-2-yl)methyl | O | H |
| 309 | 1-(Pyridin-3-yl)ethyl | O | H |
| 310 | 1-(Pyridin-2-yl)ethyl | O | H |
| 311 | (2-Methylpyridin-4-yl)methyl | O | H |
| 312 | (4-Hydroxyphenyl)methyl | O | H |
| 313 | (3-Hydroxyphenyl)methyl | O | H |
| 314 | 1-(Pyrazin-2-yl)ethyl | O | H |
| 315 | (5-Methylpyrazin-2-yl)methyl | O | H |
| 316 | (2-Methylpyrimidin-2-yl)methyl | O | H |
| 317 | (2-Cyanopyridin-4-yl)methyl | O | H |
| 318 | (4-Ethenylphenyl)methyl | O | H |
| 319 | 2,3-Dihydro-1H-indan-1-yl | O | H |
| 320 | (2-Formylphenyl)methyl | O | H |
| 321 | (3-Formylphenyl)methyl | O | H |
| 322 | (4-Formylphenyl)methyl | O | H |
| 323 | (2-Ethylphenyl)methyl | O | H |
| 324 | (3-Ethylphenyl)methyl | O | H |
| 325 | (4-Ethylphenyl)methyl | O | H |
| 326 | 1-Phenylpropan-1-yl | O | H |
| 327 | (2-iso-Propylphenyl)methyl | O | H |
| 328 | (3-iso-Propylphenyl)methyl | O | H |
| 329 | (4-iso-Propylphenyl)methyl | O | H |
| 330 | (2-tert-Butylphenyl)methyl | O | H |
| 331 | (3-tert-Butylphenyl)methyl | O | H |
| 332 | (4-tert-Butylphenyl)methyl | O | H |
| 333 | (2-n-Propylphenyl)methyl | O | H |
| 334 | (3-n-Propylphenyl)methyl | O | H |
| 335 | (4-n-Propylphenyl)methyl | O | H |
| 336 | (2-c-Propylphenyl)methyl | O | H |
| 337 | (3-c-Propylphenyl)methyl | O | H |
| 338 | (4-c-Propylphenyl)methyl | O | H |
| 339 | 1-(4-Methylphenyl)ethyl | O | H |
| 340 | 1-(3-Methylphenyl)ethyl | O | H |
| 341 | 1-(2-Methylphenyl)ethyl | O | H |
| 342 | (2,5-Dimethylphenyl)methyl | O | H |
| 343 | (3,5-Dimethylphenyl)methyl | O | H |
| 344 | (2,3-Dimethylphenyl)methyl | O | H |
| 345 | (2,6-Dimethylphenyl)methyl | O | H |
| 346 | (2-Methoxyphenyl)methyl | O | H |
| 347 | (3-Methoxyphenyl)methyl | O | H |
| 348 | (4-Methoxyphenyl)methyl | O | H |
| 349 | (2,5-Dimethoxyphenyl)methyl | O | H |
| 350 | (3,5-Dimethoxyphenyl)methyl | O | H |
| 351 | (2,4-Dimethoxyphenyl)methyl | O | H |
| 352 | (6-Methoxypyridin-2-yl)methyl | O | H |
| 353 | (5-Methoxypyridin-2-yl)methyl | O | H |
| 354 | (6-Methoxypyridin-3-yl)methyl | O | H |
| 355 | (5-Methoxypyrazin-2-yl)methyl | O | H |
| 356 | (2-Methoxypyrimidin-5-yl)methyl | O | H |
| 357 | (3-Fluor-4-Methylphenyl)methyl | O | H |
| 358 | (2-Fluor-4-Methylphenyl)methyl | O | H |
| 359 | (4-Fluor-2-Methylphenyl)methyl | O | H |
| 360 | (4-Fluor-3-Methylphenyl)methyl | O | H |
| 361 | 1-(3-Fluorphenyl)ethyl | O | H |
| 362 | 1-(4-Fluorphenyl)ethyl | O | H |
| 363 | 1-(2-Fluorphenyl)ethyl | O | H |
| 364 | 1-(2-Chlorphenyl)ethyl | O | H |
| 365 | 1-(3-Chlorphenyl)ethyl | O | H |
| 366 | 1-(4-Chlorphenyl)ethyl | O | H |
| 367 | 1-(2-Bromphenyl)ethyl | O | H |
| 368 | 1-(3-Bromphenyl)ethyl | O | H |
| 369 | 1-(4-Bromphenyl)ethyl | O | H |
| 370 | 1-(2-Cyanophenyl)ethyl | O | H |
| 371 | 1-(3-Cyanophenyl)ethyl | O | H |
| 372 | 1-(4-Cyanophenyl)ethyl | O | H |
| 373 | 1-(2-Trifluormethylphenyl)ethyl | O | H |
| 374 | 1-(3-Trifluormethylphenyl)ethyl | O | H |
| 375 | 1-(4-Trifluormethylphenyl)ethyl | O | H |
| 376 | 1-(2-Methoxyphenyl)ethyl | O | H |
| 377 | 1-(3-Methoxyphenyl)ethyl | O | H |
| 378 | 1-(4-Methoxyphenyl)ethyl | O | H |
| 379 | (4-Chloropyridin-2-yl)methyl | O | H |
| 380 | (3-Chloropyridin-4-yl)methyl | O | H |
| 381 | (2-Chloropyridin-3-yl)methyl | O | H |
| 382 | (2-Chloropyridin-4-yl)methyl | O | H |
| 383 | (2,6-Difluorphenyl)methyl | O | H |
| 384 | (2,3-Difluorphenyl)methyl | O | H |
| 385 | (5-Chlorpyrazin-2-yl)methyl | O | H |
| 386 | (2-Chlorpyrimidin-5-yl)methyl | O | H |
| 387 | 1-Benzofuran-5-ylmethyl | O | H |
| 388 | Cyclopropyl(phenyl)methyl | O | H |
| 389 | Cyclopropyl(4-chlorphenyl)methyl | O | H |
| 390 | Cyclopropyl(4-methylphenyl)methyl | O | H |
| 391 | Cyclopropyl(4-cyanophenyl)methyl | O | H |
| 392 | Cyclopropyl(4-fluorphenyl)methyl | O | H |
| 393 | Indan-5-ylmethyl | O | H |
| 394 | (2,4,6-Trimethylphenyl)methyl | O | H |
| 395 | (2,6-Dichlor-4-methylphenyl)methyl | O | H |
| 396 | 1-(3-Fluorphenyl)propyl | O | H |
| 397 | 1-(4-Fluorphenyl)propyl | O | H |
| 398 | 1-(2-Fluorphenyl)propyl | O | H |
| 399 | 1-(2-Chlorphenyl)propyl | O | H |
| 400 | 1-(3-Chlorphenyl)propyl | O | H |
| 401 | 1-(4-Chlorphenyl)propyl | O | H |
| 402 | 1-(2-Bromphenyl)propyl | O | H |
| 403 | 1-(3-Bromphenyl)propyl | O | H |
| 404 | 1-(4-Bromphenyl)propyl | O | H |
| 405 | 1-(2-Cyanophenyl)propyl | O | H |
| 406 | 1-(3-Cyanophenyl)propyl | O | H |
| 407 | 1-(4-Cyanophenyl)propyl | O | H |
| 408 | 1-(2-Trifluormethylphenyl)propyl | O | H |
| 409 | 1-(3-Trifluormethylphenyl)propyl | O | H |
| 410 | 1-(4-Trifluormethylphenyl)propyl | O | H |
| 411 | 1-(2-Methoxyphenyl)propyl | O | H |
| 412 | 1-(3-Methoxyphenyl)propyl | O | H |
| 413 | 1-(4-Methoxyphenyl)propyl | O | H |
| 414 | 1-(2-Methylphenyl)propyl | O | H |
| 415 | 1-(3-Methylphenyl)propyl | O | H |
| 416 | 1-(4-Methylphenyl)propyl | O | H |
| 417 | 1-(2,4-dimethylphenyl)ethyl | O | H |
| 418 | 1-(4-ethylphenyl)ethyl | O | H |
| 419 | 1-(3,4-Dimethylphenyl)ethyl | O | H |
| 420 | 1-(2,5-Dimethylphenyl)ethyl | O | H |
| 421 | 1-(Phenyl)butyl | O | H |
| 422 | 2-Methyl-1-(Phenyl)propyl | O | H |
| 423 | (2,4,5-Trimethylphenyl)methyl | O | H |
| 424 | (5-Cyano-2-fluorphenyl)methyl | O | H |
| 425 | (4-Cyano-2-fluorphenyl)methyl | O | H |
| 426 | (2-Cyano-4-fluorphenyl)methyl | O | H |
| 427 | (2-Cyano-5-fluorphenyl)methyl | O | H |
| 428 | 4-(Dimethylamino)phenylmethyl | O | H |
| 429 | 3-(Dimethylamino)phenylmethyl | O | H |
| 430 | Benzo[1,3]dioxol-5-yl-methyl | O | H |
| 431 | 4-(Methoxymethyl)phenylmethyl | O | H |
| 432 | 3-(Methoxymethyl)phenylmethyl | O | H |
| 433 | 2-(Methoxymethyl)phenylmethyl | O | H |
| 434 | (2-Methoxy-5-methyl-phenyl)methyl | O | H |
| 435 | (3-Fluor-4-methoxyphenyl)methyl | O | H |
| 436 | (2-Fluor-4-methoxyphenyl)methyl | O | H |
| 437 | (2-Fluor-5-methoxyphenyl)methyl | O | H |
| 438 | 1-(2,6-Difluorphenyl)ethyl | O | H |
| 439 | 1-(2,5-Difluorphenyl)ethyl | O | H |
| 440 | 1-(2,4-Difluorphenyl)ethyl | O | H |
| 441 | 1-(2,6-Dichlorphenyl)ethyl | O | H |
| 442 | 1-(2,5-Dichlorphenyl)ethyl | O | H |
| 443 | 1-(2,4-Dichlorphenyl)ethyl | O | H |
| 444 | 1-(2,3-Dichlorphenyl)ethyl | O | H |
| 445 | 1-(3,5-Dichlorphenyl)ethyl | O | H |
| 446 | 2-Naphthylmethyl | O | H |
| 447 | 1-Naphthylmethyl | O | H |
| 448 | Chinolin-4-ylmethyl | O | H |
| 449 | Chinolin-6-ylmethyl | O | H |
| 450 | Chinolin-8-ylmethyl | O | H |
| 451 | Chinolin-2-ylmethyl | O | H |
| 452 | Ch inoxal in-2-yl methyl | O | H |
| 453 | (5-Chlor-2-fluorphenyl)methyl | O | H |
| 454 | (4-Chlor-2-fluorphenyl)methyl | O | H |
| 455 | (2-Chlor-4-fluorphenyl)methyl | O | H |
| 456 | (2-Chlor-5-fluorphenyl)methyl | O | H |
| 457 | (3-Chlor-2-fluorphenyl)methyl | O | H |
| 458 | (3-Chlor-4-fluorphenyl)methyl | O | H |
| 459 | (3-Chlor-5-fluorphenyl)methyl | O | H |
| 460 | (4-Chlor-3-fluorphenyl)methyl | O | H |
| 461 | (2-Chlor-6-fluorphenyl)methyl | O | H |
| 462 | (2,4,5-Trifluorphenyl)methyl | O | H |
| 463 | (2,4,6-Trifluorphenyl)methyl | O | H |
| 464 | (3,4,5-Trifluorphenyl)methyl | O | H |
| 465 | (3-Cyano-4-methoxyphenyl)methyl | O | H |
| 466 | (4-Cyano-3-methoxyphenyl)methyl | O | H |
| 467 | (4-Cyano-2-methoxyphenyl)methyl | O | H |
| 468 | (4-Cyclopropoxyphenyl)methyl | O | H |
| 469 | 1-Benzothiophen-6-ylmethyl | O | H |
| 470 | 1-Benzothiophen-5-ylmethyl | O | H |
| 471 | 1-(2,4,5-Trimethylphenyl)ethyl | O | H |
| 472 | 1-(4-Ethylphenyl)propyl | O | H |
| 473 | 1-(4-Propan-2-ylphenyl)ethyl | O | H |
| 474 | 3-Methyl-1-phenylbutan-1-yl | O | H |
| 475 | (3-Acetamidophenyl)methyl | O | H |
| 476 | (4-Acetamidophenyl)methyl | O | H |
| 477 | [4-(Methylcarbamoyl)phenyl)methyl | O | H |
| 478 | [3-(Methylcarbamoyl)phenyl)methyl | O | H |
| 479 | [4-(Ethylcarbamoyl)phenyl)methyl | O | H |
| 480 | [3-(Ethylcarbamoyl)phenyl)methyl | O | H |
| 481 | 1-(2,4,6-trimethylpyridin-3-yl)ethyl | O | H |
| 482 | [4-(propan-2-yloxy)phenyl]methyl | O | H |
| 483 | [3-(propan-2-yloxy)phenyl]methyl | O | H |
| 484 | (2-Methyl-6-nitrophenyl)methyl | O | H |
| 485 | (4-Methyl-3-nitrophenyl)methyl | O | H |
| 486 | (2-Methyl-3-nitrophenyl)methyl | O | H |
| 487 | (2-Methyl-4-nitrophenyl)methyl | O | H |
| 488 | 1-(2-Nitrophenyl)ethyl | O | H |
| 489 | 1-(3-Nitrophenyl)ethyl | O | H |
| 490 | 1-(4-Nitrophenyl)ethyl | O | H |
| 491 | (3,4-Dimethoxyphenyl)methyl | O | H |
| 492 | (4-Methoxy-3,5-dimethylpyridin-2-yl)methyl | O | H |
| 493 | (4,5-Dimethoxypyridin-2-yl)methyl | O | H |
| 494 | 1-(2-Naphthyl)methyl | O | H |
| 495 | 1-(1-Naphthyl)methyl | O | H |
| 496 | (3-Chlor-4-methoxyphenyl)methyl | O | H |
| 497 | (4-Chlor-3-methoxyphenyl)methyl | O | H |
| 498 | (4-Chlor-2-methoxyphenyl)methyl | O | H |
| 499 | (5-Chlor-2-methoxyphenyl)methyl | O | H |
| 500 | (3-Chlor-5-methoxyphenyl)methyl | O | H |
| 501 | (2-Methylchinolin-4-yl)methyl | O | H |
| 502 | 1-(5-Chlor-2-fluorphenyl)ethyl | O | H |
| 503 | 1-(4-Chlor-2-fluorphenyl)ethyl | O | H |
| 504 | 1-(2-Chlor-4-fluorphenyl)ethyl | O | H |
| 505 | 1-(2-Chlor-5-fluorphenyl)ethyl | O | H |
| 506 | 1-(3-Chlor-2-fluorphenyl)ethyl | O | H |
| 507 | 1-(3-Chlor-4-fluorphenyl)ethyl | O | H |
| 508 | 1-(3-Chlor-5-fluorphenyl)ethyl | O | H |
| 509 | 1-(4-Chlor-3-fluorphenyl)ethyl | O | H |
| 510 | 1-(2-Chlor-6-fluorphenyl)ethyl | O | H |
| 511 | (2-Hydroxychinolin-3-yl)methyl | O | H |
| 512 | 1-(5,6,7,8-tetrahydronaphthalin-2-yl)ethyl | O | H |
| 513 | [5-(Trifluormethyl)pyridin-2-yl]methyl | O | H |
| 514 | [2-(Trifluormethyl)pyridin-4-yl]methyl | O | H |
| 515 | (3,6-Dichlorpyridin-2-yl)methyl | O | H |
| 516 | [5-(Trifluormethyl)pyrazin-2-yl]methyl | O | H |
| 517 | [2-(Trifluormethyl)pyrimidin-2-yl]methyl | O | H |
| 518 | 1-Phenylhexan-1-yl | O | H |
| 519 | 1-(3-tert-Butylphenyl)ethyl | O | H |
| 520 | 1-(4-tert-Butylphenyl)ethyl | O | H |
| 521 | 1-(2-Nitrophenyl)propyl | O | H |
| 522 | 1-(3-Nitrophenyl)propyl | O | H |
| 523 | 1-(4-Nitrophenyl)propyl | O | H |
| 524 | (2-Methoxy-5-nitrophenyl)methyl | O | H |
| 525 | (4-Methoxy-3-nitrophenyl)methyl | O | H |
| 526 | (2-Methoxy-4-nitrophenyl)methyl | O | H |
| 527 | (3-Methoxy-4-nitrophenyl)methyl | O | H |
| 528 | Diphenylmethyl | O | H |
| 529 | (4-Phenylphenyl)methyl | O | H |
| 530 | Phenyl(pyridin-2-yl)methyl | O | H |
| 531 | Phenyl(pyridin-3-yl)methyl | O | H |
| 532 | Phenyl(pyridin-4-yl)methyl | O | H |
| 533 | (5-Chlor-2-Ethoxyphenyl)methyl | O | H |
| 534 | (5-Chlor-2-nitrophenyl)methyl | O | H |
| 535 | (4-Chlor-2-nitrophenyl)methyl | O | H |
| 536 | (2-Chlor-4-nitrophenyl)methyl | O | H |
| 537 | (2-Chlor-5-nitrophenyl)methyl | O | H |
| 538 | (3-Chlor-2-nitrophenyl)methyl | O | H |
| 539 | (3-Chlor-4-nitrophenyl)methyl | O | H |
| 540 | (3-Chlor-5-nitrophenyl)methyl | O | H |
| 541 | (4-Chlor-3-nitrophenyl)methyl | O | H |
| 542 | (2-Chlor-6-nitrophenyl)methyl | O | H |
| 543 | (5-Brompyridin-2-yl)methyl | O | H |
| 544 | (2-Brompyridin-4-yl)methyl | O | H |
| 545 | (6-Brompyridin-2-yl)methyl | O | H |
| 546 | (2,4-Difluor-5-nitrophenyl)methyl | O | H |
| 547 | (3-Methyl-2-trifluormethylphenyl)methyl | O | H |
| 548 | 3,3,3-Trifluor-1-phenylpropyl | O | H |
| 549 | Cyclohexyl(phenyl)methyl | O | H |
| 550 | Cyclopentyl(phenyl)methyl | O | H |
| 551 | 1-(3,4-Dichlorphenyl)ethyl | O | H |
| 552 | [4-(Cyclopentyloxy)phenyl]methyl | O | H |
| 553 | [2-Fluor-4-(trifluormethyl)phenyl]methyl | O | H |
| 554 | [3-Fluor-4-(trifluormethyl)phenyl]methyl | O | H |
| 555 | [2-Fluor-5-(trifluormethyl)phenyl]methyl | O | H |
| 556 | [3-Fluor-5-(trifluormethyl)phenyl]methyl | O | H |
| 557 | 1-(2-Nitrophenyl)butyl | O | H |
| 558 | 1-(3-Nitrophenyl)butyl | O | H |
| 559 | 1-(4-Nitrophenyl)butyl | O | H |
| 560 | 1-(2-Cyanophenyl)butyl | O | H |
| 561 | 1-(3-Cyanohenyl)butyl | O | H |
| 562 | 1-(4-Cyanophenyl)butyl | O | H |
| 563 | 1-(2-Fluorphenyl)butyl | O | H |
| 564 | 1-(3-Fluorphenyl)butyl | O | H |
| 565 | 1-(4-Fluorphenyl)butyl | O | H |
| 566 | 1-(2-Chlorphenyl)butyl | O | H |
| 567 | 1-(3-Chlorphenyl)butyl | O | H |
| 568 | 1-(4-Chlorphenyl)butyl | O | H |
| 569 | (2,4-Dinitrophenyl)methyl | O | H |
| 570 | (2-Methylphenyl)(phenyl)methyl | O | H |
| 571 | 1,2-Diphenylethyl | O | H |
| 572 | 1-(4-Phenylphenyl)ethyl | O | H |
| 573 | (4-Brom-3-methylphenyl)methyl | O | H |
| 574 | (4-Brom-3-fluorphenyl)methyl | O | H |
| 575 | (4-Brom-3-chlorphenyl)methyl | O | H |
| 576 | (3-Brom-4-chlorphenyl)methyl | O | H |
| 577 | (3-Brom-5-chlorphenyl)methyl | O | H |
| 578 | 4-Brom-3-methyl phenyl | O | H |
| 579 | 4-Brom-3-fluorphenyl | O | H |
| 580 | 4-Brom-3-chlorphenyl | O | H |
| 581 | 3-Brom-4-chlorphenyl | O | H |
| 582 | 3-Brom-5-chlorphenyl | O | H |
| 583 | 4-Brom-2-fluorphenyl | O | H |
| 584 | (5-Brom-2-fluorphenyl)methyl | O | H |
| 585 | (2-Brom-4-fluorphenyl)methyl | O | H |
| 586 | (4-Brom-2-fluorphenyl)methyl | O | H |
| 587 | (3-Brom-5-fluorphenyl)methyl | O | H |
| 588 | 5-Brom-2-fluorphenyl | O | H |
| 589 | 2-Brom-4-fluorphenyl | O | H |
| 590 | 3-Brom-5-fluorphenyl | O | H |
| 591 | 1-(2,4-Dichlorphenyl)propyl | O | H |
| 592 | 1-(3,4-Dichlorphenyl)propyl | O | H |
| 593 | 1-(2,6-Dichlor-3-fluorphenyl)ethyl | O | H |
| 594 | 1-(2,4-Dichlor-5-fluorphenyl)ethyl | O | H |
| 595 | (2-Chlor-6-Trifluormethylphenyl)methyl | O | H |
| 596 | (2-Chlor-4-Trifluormethylphenyl)methyl | O | H |
| 597 | (4-Chlor-3-Trifluormethylphenyl)methyl | O | H |
| 598 | (2-Chlor-4-Trifluormethylphenyl)methyl | O | H |
| 599 | (3-Brom-4-Methoxyphenyl)methyl | O | H |
| 600 | 4-Brom-3-Methoxyphenyl | O | H |

A2. Verbindungen A2-1 bis A2-600 der allgemeinen Formel (Ib), worin R¹ für Ethyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen A2-1 bis A2-600) entsprechen.

A3. Verbindungen A3-1 bis A3-600 der allgemeinen Formel (Ib), worin R¹ für n-Propyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen A3-1 bis A3-600) entsprechen.

A4. Verbindungen A4-1 bis A4-600 der allgemeinen Formel (Ib), worin R¹ für iso-Propyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen A4-1 bis A4-600) entsprechen.

A5. Verbindungen A5-1 bis A5-600 der allgemeinen Formel (Ib), worin R¹ für n-Butyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen A5-1 bis A5-600) entsprechen.

A6. Verbindungen A6-1 bis A6-600 der allgemeinen Formel (Ib), worin R¹ für 3-Methylbutyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen A6-1 bis A6-600) entsprechen.

A7. Verbindungen A7-1 bis A7-600 der allgemeinen Formel (Ib), worin R¹ für 2-Methylbutyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen A7-1 bis A7-600) entsprechen.

A8. Verbindungen A8-1 bis A8-600 der allgemeinen Formel (Ib), worin R¹ für Methyl, R² für Fluor, R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen A8-1 bis A8-600) entsprechen.

A9. Verbindungen A9-1 bis A9-600 der allgemeinen Formel (Ib), worin R¹ für Ethyl, R² für Fluor, R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen A9-1 bis A9-600) entsprechen.

A10. Verbindungen A10-1 bis A10-600 der allgemeinen Formel (Ib), worin R¹ für n-Propyl, R² für Fluor, R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen A10-1 bis A10-600) entsprechen.

A11. Verbindungen A11-1 bis A11-600 der allgemeinen Formel (Ib), worin R¹ für iso-Propyl, R² für Fluor, R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen A11-1 bis A11-600) entsprechen.

A12. Verbindungen A12-1 bis A12-600 der allgemeinen Formel (Ib), worin R¹ für Methyl, R³ für Fluor, R² und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen A12-1 bis A12-600) entsprechen.

A13. Verbindungen A13-1 bis A13-600 der allgemeinen Formel (Ib), worin R¹ für Ethyl, R³ für Fluor, R² und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen A13-1 bis A13-600) entsprechen.

A14. Verbindungen A14-1 bis A14-600 der allgemeinen Formel (Ib), worin R¹ für n-Propyl, R³ für Fluor, R² und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen A14-1 bis A14-600) entsprechen.

A15. Verbindungen A15-1 bis A15-600 der allgemeinen Formel (Ib), worin R¹ für iso-Propyl, R³ für Fluor, R² und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen A15-1 bis A15-600) entsprechen.

A16. Verbindungen A16-1 bis A16-600 der allgemeinen Formel (Ib), worin R¹ für Methyl, R² für Methyl, R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen A16-1 bis A16-600) entsprechen.

A17. Verbindungen A17-1 bis A17-600 der allgemeinen Formel (Ib), worin R¹ für Methyl, R³ für Methyl, R² und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen A17-1 bis A17-600) entsprechen.

A18. Verbindungen A18-1 bis A18-600 der allgemeinen Formel (Ib), worin R¹ für Benzyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen A18-1 bis A18-600) entsprechen.

A19. Verbindungen A19-1 bis A19-600 der allgemeinen Formel (Ib), worin R¹ für Cyclopropylmethyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen A19-1 bis A19-600) entsprechen.

A20. Verbindungen A20-1 bis A20-600 der allgemeinen Formel (Ib), worin R¹ für Methyl, R³ für Chlor, R² und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen A20-1 bis A20-600) entsprechen.

A21. Verbindungen A21-1 bis A21-600 der allgemeinen Formel (Ib), worin R¹ für n-Pentyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen A21-1 bis A21-600) entsprechen.

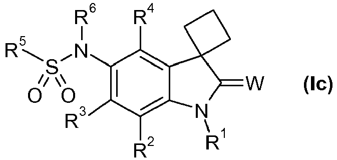

B1. Verbindungen B1-1 bis B1-600 der oben abgebildeten allgemeinen Formel (Ic), worin R¹ für Methyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen B1-1 bis B1-600) entsprechen.

B2. Verbindungen B2-1 bis B2-600 der allgemeinen Formel (Ic), worin R¹ für Ethyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen B2-1 bis B2-600) entsprechen.

B3. Verbindungen B3-1 bis B3-600 der allgemeinen Formel (Ic), worin R¹ für n-Propyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen B3-1 bis B3-600) entsprechen.

B4. Verbindungen B4-1 bis B4-600 der allgemeinen Formel (Ic), worin R¹ für iso-Propyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen B4-1 bis B4-600) entsprechen.

B5. Verbindungen B5-1 bis B5-600 der allgemeinen Formel (Ic), worin R¹ für n-Butyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen B5-1 bis B5-600) entsprechen.

B6. Verbindungen B6-1 bis B6-600 der allgemeinen Formel (Ic), worin R¹ für 3-Methylbutyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen B6-1 bis B6-600) entsprechen.

B7. Verbindungen B7-1 bis B7-600 der allgemeinen Formel (Ic), worin R¹ für 2-Methylbutyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen B7-1 bis B7-600) entsprechen.

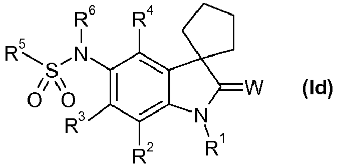

C1. Verbindungen C1-1 bis C1-600 der oben abgebildeten allgemeinen Formel (Id), worin R¹ für Methyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen C1-1 bis C1-600) entsprechen.

C2. Verbindungen C2-1 bis C2-600 der allgemeinen Formel (Id), worin R¹ für Ethyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen C2-1 bis C2-600) entsprechen.

C3. Verbindungen C3-1 bis C3-600 der allgemeinen Formel (Id), worin R¹ für n-Propyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen C3-1 bis C3-600) entsprechen.

C4. Verbindungen C4-1 bis C4-600 der allgemeinen Formel (Id), worin R¹ für iso-Propyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen C4-1 bis C4-600) entsprechen.

C5. Verbindungen C5-1 bis C5-600 der allgemeinen Formel (Id), worin R¹ für n-Butyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen C5-1 bis C5-600) entsprechen.

C6. Verbindungen C6-1 bis C6-600 der allgemeinen Formel (Id), worin R¹ für 3-Methylbutyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen C6-1 bis C6-600) entsprechen.

C7. Verbindungen C7-1 bis C7-600 der allgemeinen Formel (Id), worin R¹ für 2-Methylbutyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen C7-1 bis C7-600) entsprechen.

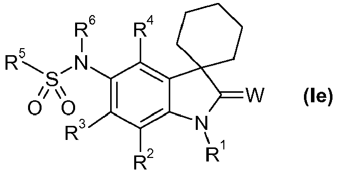

D1. Verbindungen D1-1 bis D1-600 der oben abgebildeten allgemeinen Formel (Ie), worin R¹ für Methyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen D1-1 bis D1-600) entsprechen.

D2. Verbindungen D2-1 bis D2-600 der allgemeinen Formel (Ie), worin R¹ für Ethyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen D2-1 bis D2-600) entsprechen.

D3. Verbindungen D3-1 bis D3-600 der allgemeinen Formel (Ie), worin R¹ für n-Propyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen D3-1 bis D3-600) entsprechen.

D4. Verbindungen D4-1 bis D4-600 der allgemeinen Formel (Ie), worin R¹ für iso-Propyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen D4-1 bis D4-600) entsprechen.

D5. Verbindungen D5-1 bis D5-600 der allgemeinen Formel (Ie), worin R¹ für n-Butyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen D5-1 bis D5-600) entsprechen.

D6. Verbindungen D6-1 bis D6-600 der allgemeinen Formel (Ie), worin R¹ für 3-Methylbutyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen D6-1 bis D6-600) entsprechen.

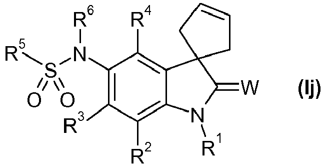

E1. Verbindungen E1-1 bis E1-600 der oben abgebildeten allgemeinen Formel (Ij), worin R¹ für Methyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen E1-1 bis E1-600) entsprechen.

E2. Verbindungen E2-1 bis E2-600 der allgemeinen Formel (Ij), worin R¹ für Ethyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen E2-1 bis E2-600) entsprechen.

E3. Verbindungen E3-1 bis E3-600 der allgemeinen Formel (Ij), worin R¹ für n-Propyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen E3-1 bis E3-600) entsprechen.

E4. Verbindungen E4-1 bis E4-600 der allgemeinen Formel (Ij), worin R¹ für iso-Propyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen E4-1 bis E4-600) entsprechen.

E5. Verbindungen E5-1 bis E5-600 der allgemeinen Formel (Ij), worin R¹ für n-Butyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen E5-1 bis E5-600) entsprechen.

E6. Verbindungen E6-1 bis E6-600 der allgemeinen Formel (Ij), worin R¹ für 3-Methylbutyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen E6-1 bis E6-600) entsprechen.

E7. Verbindungen E7-1 bis E7-600 der allgemeinen Formel (Ij), worin R¹ für Allyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen E7-1 bis E7-600) entsprechen.

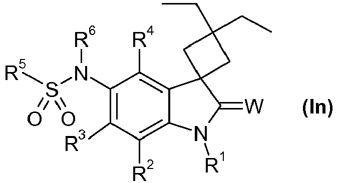

F1. Verbindungen F1-1 bis F1-600 der oben abgebildeten allgemeinen Formel (In), worin R¹ für Methyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen F1-1 bis F1-600) entsprechen.

F2. Verbindungen F2-1 bis F2-600 der allgemeinen Formel (In), worin R¹ für Ethyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen F2-1 bis F2-600) entsprechen.

F3. Verbindungen F3-1 bis F3-600 der allgemeinen Formel (In), worin R¹ für n-Propyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen F3-1 bis F3-600) entsprechen.

F4. Verbindungen F4-1 bis F4-600 der allgemeinen Formel (In), worin R¹ für iso-Propyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen F4-1 bis F4-600) entsprechen.

F5. Verbindungen F5-1 bis F5-600 der allgemeinen Formel (In), worin R¹ für n-Butyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen F5-1 bis F5-600) entsprechen.

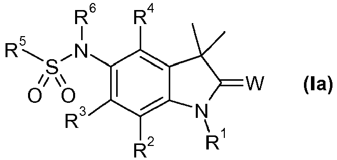

G1. Verbindungen G1-1 bis G1-600 der allgemeinen Formel (la), worin R¹ für Methyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen G1-1 bis G1-600) entsprechen.

G2. Verbindungen G2-1 bis G2-600 der allgemeinen Formel (la), worin R¹ für Ethyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen G2-1 bis G2-600) entsprechen.

G3. Verbindungen G3-1 bis G3-600 der allgemeinen Formel (la), worin R¹ für n-Propyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen G3-1 bis G3-600) entsprechen.

G4. Verbindungen G4-1 bis G4-600 der allgemeinen Formel (la), worin R¹ für iso-Propyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen G4-1 bis G4-600) entsprechen.

G5. Verbindungen G5-1 bis G5-600 der allgemeinen Formel (la), worin R¹ für n-Butyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen G5-1 bis G5-600) entsprechen.

G6. Verbindungen G6-1 bis G6-600 der allgemeinen Formel (la), worin R¹ für 3-Methylbutyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen G6-1 bis G6-600) entsprechen.

G7. Verbindungen G7-1 bis G7-600 der allgemeinen Formel (la), worin R¹ für 2-Methylbutyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen G7-1 bis G7-600) entsprechen.

G8. Verbindungen G8-1 bis G8-600 der allgemeinen Formel (la), worin R¹ für Methyl, R² für Fluor, R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen G8-1 bis G8-600) entsprechen.

G9. Verbindungen G9-1 bis G9-600 der allgemeinen Formel (la), worin R¹ für Ethyl, R² für Fluor, R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen G9-1 bis G9-600) entsprechen.

G10. Verbindungen G10-1 bis G10-600 der allgemeinen Formel (la), worin R¹ für n-Propyl, R² für Fluor, R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen G10-1 bis G10-600) entsprechen.

G11. Verbindungen G11-1 bis G11-600 der allgemeinen Formel (la), worin R¹ für iso-Propyl, R² für Fluor, R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen G11-1 bis G11-600) entsprechen.

G12. Verbindungen G12-1 bis G12-600 der allgemeinen Formel (la), worin R¹ für Methyl, R³ für Fluor, R² und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen G12-1 bis G12-600) entsprechen.

G13. Verbindungen G13-1 bis G13-600 der allgemeinen Formel (la), worin R¹ für Ethyl, R³ für Fluor, R² und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen G13-1 bis G13-600) entsprechen.

G14. Verbindungen G14-1 bis G14-600 der allgemeinen Formel (la), worin R¹ für n-Propyl, R³ für Fluor, R² und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen G14-1 bis G14-600) entsprechen.

G15. Verbindungen G15-1 bis G15-600 der allgemeinen Formel (la), worin R¹ für iso-Propyl, R³ für Fluor, R² und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen G15-1 bis G15-600) entsprechen.

G16. Verbindungen G16-1 bis G16-600 der allgemeinen Formel (la), worin R¹ für Methyl, R² für Methyl, R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen G16-1 bis G16-600) entsprechen.

G17. Verbindungen G17-1 bis G17-600 der allgemeinen Formel (la), worin R¹ für Methyl, R³ für Methyl, R² und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen G17-1 bis G17-600) entsprechen.

G18. Verbindungen G18-1 bis G18-600 der allgemeinen Formel (la), worin R¹ für Benzyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen G18-1 bis G18-600) entsprechen.

G19. Verbindungen G19-1 bis G19-600 der allgemeinen Formel (la), worin R¹ für Cyclopropylmethyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen G19-1 bis G19-600) entsprechen.

G20. Verbindungen G20-1 bis G20-600 der allgemeinen Formel (la), worin R¹ für Methyl, R³ für Chlor, R² und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen G20-1 bis G20-600) entsprechen.

G21. Verbindungen G21-1 bis G21-600 der allgemeinen Formel (la), worin R¹ für n-Pentyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen G21-1 bis G21-600) entsprechen.

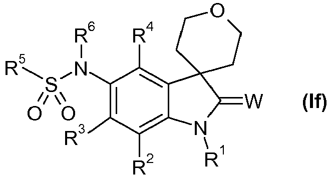

H1. Verbindungen H1-1 bis H1-600 der oben abgebildeten allgemeinen Formel (If), worin R¹ für Methyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen H1-1 bis H1-600) entsprechen.

H2. Verbindungen H2-1 bis H2-600 der allgemeinen Formel (If), worin R¹ für Ethyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen H2-1 bis H2-600) entsprechen.

H3. Verbindungen H3-1 bis H3-600 der allgemeinen Formel (If), worin R¹ für n-Propyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen H3-1 bis H3-600) entsprechen.

H4. Verbindungen H4-1 bis H4-600 der allgemeinen Formel (If), worin R¹ für iso-Propyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen H4-1 bis H4-600) entsprechen.

H5. Verbindungen H5-1 bis H5-600 der allgemeinen Formel (If), worin R¹ für n-Butyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen H5-1 bis H5-600) entsprechen.

H6. Verbindungen H6-1 bis H6-600 der allgemeinen Formel (If), worin R¹ für 3-Methylbutyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen H6-1 bis H6-600) entsprechen.

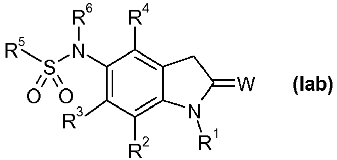

I1. Verbindungen I1-1 bis I1-600 der allgemeinen Formel (Iab), worin R¹ für Methyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen I1-1 bis I1-600) entsprechen.

I2. Verbindungen I2-1 bis 12-600 der allgemeinen Formel (Iab), worin R¹ für Ethyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen I2-1 bis I2-600) entsprechen.

I3. Verbindungen I3-1 bis I3-600 der allgemeinen Formel (Iab), worin R¹ für n-Propyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen I3-1 bis I3-600) entsprechen.

I4. Verbindungen I4-1 bis I4-600 der allgemeinen Formel (Iab), worin R¹ für iso-Propyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen I4-1 bis I4-600) entsprechen.

I5. Verbindungen I5-1 bis I5-600 der allgemeinen Formel (Iab), worin R¹ für n-Butyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen I5-1 bis I5-600) entsprechen.

I6. Verbindungen 16-1 bis 16-600 der allgemeinen Formel (Iab), worin R¹ für 3-Methylbutyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen I6-1 bis I6-600) entsprechen.

I7. Verbindungen I7-1 bis I7-600 der allgemeinen Formel (Iab), worin R¹ für 2-Methylbutyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen I7-1 bis I7-600) entsprechen.

I8. Verbindungen I8-1 bis I8-600 der allgemeinen Formel (Iab), worin R¹ für Methyl, R² für Fluor, R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen 18-1 bis 18-600) entsprechen.

I9. Verbindungen I9-1 bis I9-600 der allgemeinen Formel (Iab), worin R¹ für Ethyl, R² für Fluor, R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen I9-1 bis I9-600) entsprechen.

I10. Verbindungen I10-1 bis I10-600 der allgemeinen Formel (Iab), worin R¹ für n-Propyl, R² für Fluor, R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen I10-1 bis I10-600) entsprechen.

I11. Verbindungen I11-1 bis I11-600 der allgemeinen Formel (Iab), worin R¹ für iso-Propyl, R² für Fluor, R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen I11-1 bis I11-600) entsprechen.

I12. Verbindungen I12-1 bis 112-600 der allgemeinen Formel (Iab), worin R¹ für Methyl, R³ für Fluor, R² und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen I12-1 bis I12-600) entsprechen.

113. Verbindungen 113-1 bis 113-600 der allgemeinen Formel (Iab), worin R¹ für Ethyl, R³ für Fluor, R² und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen 113-1 bis 113-600) entsprechen.

114. Verbindungen 114-1 bis 114-600 der allgemeinen Formel (Iab), worin R¹ für n-Propyl, R³ für Fluor, R² und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen 114-1 bis 114-600) entsprechen.

115. Verbindungen 115-1 bis 115-600 der allgemeinen Formel (Iab), worin R¹ für isoPropyl, R³ für Fluor, R² und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen 115-1 bis 115-600) entsprechen.

116. Verbindungen 116-1 bis 116-600 der allgemeinen Formel (Iab), worin R¹ für Methyl, R² für Methyl, R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen 116-1 bis 116-600) entsprechen.

117. Verbindungen 117-1 bis 117-600 der allgemeinen Formel (Iab), worin R¹ für Methyl, R³ für Methyl, R² und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen 117-1 bis 117-600) entsprechen.

118. Verbindungen 118-1 bis 118-600 der allgemeinen Formel (Iab), worin R¹ für Benzyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen 118-1 bis 118-600) entsprechen.

119. Verbindungen 119-1 bis 119-600 der allgemeinen Formel (Iab), worin R¹ für Cyclopropylmethyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen 119-1 bis 119-600) entsprechen.

120. Verbindungen I20-1 bis I20-600 der allgemeinen Formel (Iab), worin R¹ für Methyl, R³ für Chlor, R² und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen I20-1 bis I20-600) entsprechen.

121. Verbindungen 121-1 bis 121-600 der allgemeinen Formel (Iab), worin R¹ für n-Pentyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen 121-1 bis 121-600) entsprechen.

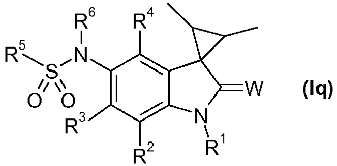

J1. Verbindungen J1-1 bis J1-600 der oben dargestellten allgemeinen Formel (Iq), worin R¹ für Methyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen J1-1 bis J1-600) entsprechen.

J2. Verbindungen J2-1 bis J2-600 der allgemeinen Formel (Iq), worin R¹ für Ethyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen J2-1 bis J2-600) entsprechen.

J3. Verbindungen J3-1 bis J3-600 der allgemeinen Formel (Iq), worin R¹ für n-Propyl, R², R³ und R⁴ für Wasserstoff stehen und W, R⁵, R⁶ den Definitionen für die jeweilige Einzelverbindung den in Tabelle 1 genannten Restedefinitionen (Nos 1 bis 600; entsprechend Verbindungen J3-1 bis J3-600) entsprechen.

### Spektroskopische Daten ausgewählter Tabellenbeispiele:

### Beispiel No. A1-1:

¹H-NMR (400 MHz, CDCl₃) δ 7.11 (dd, 1H), 6.87 (d, 1H), 6.83 (d, 1H), 6.22 (br. s, 1H, NH), 3.29 (s, 3H), 2.96 (s, 3H), 1.78 (m, 2H), 1.55 (m, 2H),

### Beispiel No. A1-3:

¹H-NMR (400 MHz, CDCl₃) δ 7.01 (dd, 1H), 6.96 (d, 1H), 6.81 (d, 1H), 6.11 (br. s, 1H, NH), 3.18 (s, 3H), 2.78 (t, 2H), 1.55 (m, 2H), 1.52 (m, 2H), 1.38 (sext, 2H), 0.77 (t, 3H).

### Beispiel No. A1-20:

¹H-NMR (400 MHz, d₆-DMSO) δ 9.36 (br. s, 1H, NH), 7.12 (br. q, 1H, NH), 7.04 (dd, 1H), 6.99 (d, 1H), 6.82 (d, 1H), 3.18 (s, 3H), 2.42 (d, 3H) 1.54 (m, 2H), 1.51 (m, 2H).

### Beispiel No. A1-21:

¹H-NMR (400 MHz, CDCl₃) δ 7.08 (dd, 1H), 6.82 (d, 1H), 6.80 (d, 1H), 6.13 (br. s, 1H, NH), 3.28 (s, 3H), 2.82 (s, 6H), 1.77 (m, 2H), 1.53 (m, 2H).

### Beispiel No. A1-23:

¹H-NMR (400 MHz, d₆-DMSO) δ 9.33 (br. s, 1H, NH), 7.26 (br. t, 1H, NH), 7.01 (dd, 1H), 6.96 (d, 1H), 6.81 (d, 1H), 6.11 (br. s, 1H, NH), 3.18 (s, 3H), 2.78 (t, 2H), 1.55 (m, 2H), 1.52 (m, 2H), 1.38 (sext, 2H), 0.77 (t, 3H).

### Beispiel No. A1-26:

¹H-NMR (400 MHz, CDCl₃) δ 7.11 (dd, 1H), 6.84 (d, 1H), 6.83 (d, 1H), 6.29 (br. s, 1H, NH), 3.85 (m 2H), 3.46 (s, 3H), 3.29 (s, 3H), 3.19 (m, 2H), 1.77 (m, 2H), 1.54 (m, 2H).

### Beispiel No. A1-37:

¹H-NMR (400 MHz, CHCl₃) d 8.69 (d, 1H), 8.07 (m, 1H), 8.04 (d, 1H), 7.96 (m, 1H), 7.69-7.60 (m, 2H), 7.41 (m, 1H), 6.68 (m, 1H), 6.64 (br. s, 1H, NH), 6.59 (m, 1H), 6.37 (d, 1H), 3.18 (s, 3H), 1.65 (m, 2H), 1.26 (m, 2H).

### Beispiel No. A1-53:

¹H-NMR (400 MHz, CHCl₃) d 7.81 (m, 1H), 7.57 (m, 1H), 7.18 (m, 1H), 6.83 (dd, 1H), 6.73 (dd, 1H), 6.58 (d, 1H), 6.51 (br. s, 1H, NH), 3.23 (s, 3H), 2.70 (s, 3H), 1.74 (m, 2H), 1.43 (m, 2H).

### Beispiel No. A1-66:

¹H-NMR (400 MHz, CHCl₃) d 7.53 (m, 1H), 7.45 (m, 2H), 7.31 (m, 1H), 6.86 (dd, 1H), 6.73 (d, 1H), 6.62 (d, 1H), 6.52 (br. s, 1H, NH), 6.67-6.31 (t, 1H, OCHF₂), 3.25 (s, 3H), 1.74 (m, 2H), 1.45 (m, 2H).

### Beispiel No. A1-152:

¹H-NMR (400 MHz, d₆-DMSO) δ 9.55 (br. s, 1H, NH), 7.30 (d, 2H), 7.17 (d, 2H), 6.97 (m, 2H), 6.83 (d, 1H), 4.34 (s, 2H), 3.17 (s, 3H), 2.28 (s, 3H), 1.53 (m, 4H).

### Beispiel No. A1-153:

¹H-NMR (400 MHz, d₆-DMSO) δ 9.53 (br. s, 1H, NH), 7.23 (m, 1H), 7.16 (m, 1H), 7.97 (m, 2H), 7.05 (m, 2H), 6.75 (d, 1H), 4.33 (s, 2H), 3.20 (s, 3H), 2.27 (s, 3H), 1.53 (m, 4H).

### Beispiel No. A1-158:

¹H-NMR (400 MHz, d₆-DMSO) δ 9.54 (br. s, 1H, NH), 7.32 (m, 2H), 7.19 (m, 2H), 7.05 (m, 2H), 6.75 (d, 1H), 4.40 (s, 2H), 3.20 (s, 3H), 1.55 (m, 4H).

### Beispiel No A1-159:

¹H-NMR (400 MHz, CDCl₃) d 7.50 (m, 1H), 7.39 (m, 1H), 7.30 (m, 1H), 7.20 (m, 1H), 7.09 (m, 2H), 6.83 (d, 1H), 6.40 (br. s, 1H, NH), 4.37 (s, 2H), 3.25 (s, 3H), 1.78 (m, 2H), 1.58 (m, 2H).

### Beispiel No A1-161:

¹H-NMR (400 MHz, CDCl₃) d 7.47 (m, 1H), 7.38 (m, 1H), 7.18 (m, 1H), 7.09 (m, 1H), 7.02 (dd, 1H), 6.84 (d, 1H), 6.67 (d, 1H), 6.13 (br. s, 1H, NH), 4.39 (s, 2H), 3.29 (s, 3H), 1.77 (m, 2H), 1.51 (m, 2H).

### Beispiel No. A1-165:

¹H-NMR (400 MHz, d₆-DMSO) δ 9.56 (br. s, 1H, NH), 7.41 (d, 2H), 7.30 (d, 2H), 7.06 (dd, 1H), 7.03 (d, 1H), 6.75 (d, 1H), 4.42 (s, 2H), 3.20 (s, 3H), 1.54 (m, 4H).

### Beispiel No A1-166:

¹H-NMR (400 MHz, CDCl₃) d 7.39-7.29 (m, 3H), 7.23 (m, 1H), 7.03 (dd, 1H), 6.87 (d, 1H), 6.65 (d, 1H), 6.12 (br. s, 1H, NH), 4.26 (s, 2H), 3.30 (s, 3H), 1.78 (m, 2H), 1.53 (m, 2H).

### Beispiel No. A1-167:

¹H-NMR (400 MHz, CDCl₃) d 7.45 (d, 1H), 7.34 (d, 1H), 7.20 (dd, 1H), 7.02 (dd, 1H), 6.86 (d, 1H), 6.67 (d, 1H), 6.13 (br. s, 1H, NH), 4.24 (s, 2H), 3.30 (s, 3H), 1.78 (m, 2H), 1.52 (m, 2H).

### Beispiel No. A1-169:

¹H-NMR (400 MHz, CDCl₃) d 7.46 (d, 1H), 7.39 (m, 1H), 7.24 (m, 1H), 6.99 (dd, 1H), 6.81 (d, 1H), 6.66 (d, 1H), 6.20 (br. s, 1H, NH), 4.52 (s, 2H), 3.28 (s, 3H), 1.77 (m, 2H), 1.49 (m, 2H).

### Beispiel No. A1-170:

¹H-NMR (400 MHz, CDCl₃) d 7.45 (m, 1H), 7.31 (m, 1H), 7.26 (m, 1H), 7.05 (dd, 1H), 6.84 (d, 1H), 6.68 (d, 1H), 6.27 (br. s, 1H, NH), 4.51 (s, 2H), 3.29 (s, 3H), 1.77 (m, 2H), 1.51 (m, 2H).

### Beispiel No. A1-171:

¹H-NMR (400 MHz, CDCl₃) d 7.36 (m, 1H), 7.19 (m, 1H), 7.06 (m, 1H), 6.97 (dd, 1H), 6.85 (d, 1H), 6.67 (d, 1H), 6.21 (br. s, 1H, NH), 4.26 (s, 2H), 3.30 (s, 3H), 1.78 (m, 2H), 1.51 (m, 2H).

### Beispiel No. A1-176:

¹H-NMR (400 MHz, CDCl₃) δ 8.25 (m, 1H), 8.11 (m, 1H), 7.75 (m, 1H), 7.59 (m, 1H), 7.08 (dd, 1H), 6.89 (d, 1H), 6.76 (d, 1H), 6.16 (br. s, 1H, NH), 4.38 (s, 2H), 3.31 (s, 3H), 1.80 (m, 2H), 1.56 (m, 2H).

### Beispiel No. A1-177:

¹H-NMR (400 MHz, d₆-DMSO) δ 9.82 (br. s, 1H, NH), 8.02 (m, 1H), 7.71 (m, 1H), 7.63 (m, 1H), 7.50 (m, 1H), 7.07 (m, 1H), 7.04 (m, 1H), 6.79 (d, 1H), 4.86 (s, 2H), 3.21 (s, 3H), 1.57 (m, 2H), 1.54 (m, 2H).

### Beispiel No. A1-178:

¹H-NMR (400 MHz, d₆-DMSO) δ 9.63 (br. s, 1H, NH), 7.72 (d, 2H), 7.51 (d, 2H), 7.08 (dd, 1H), 7.02 (d, 1H), 6.79 (d, 1H), 4.54 (s, 2H), 3.20 (s, 3H), 1.56 (m, 2H), 1.53 (m, 2H).

### Beispiel No. A1-179:

¹H-NMR (400 MHz, CDCl₃) d 7.66 (m, 1H), 7.59 (m, 1H), 7.54 (m, 1H), 7.49 (m, 1H), 7.01 (dd, 1H), 6.86 (d, 1H), 6.70 (d, 1H), 6.14 (br. s, 1H, NH), 4.34 (s, 2H), 3.30 (s, 3H), 1.78 (m, 2H), 1.52 (m, 2H).

### Beispiel No. A1-180:

¹H-NMR (400 MHz, CDCl₃) d 7.71 (m, 2H), 7.56 (m, 1H), 7.48 (m, 1H), 6.96 (dd, 1H), 6.80 (d, 1H), 6.63 (d, 1H), 6.24 (br. s, 1H, NH), 4.57 (s, 2H), 3.28 (s, 3H), 1.77 (m, 2H), 1.50 (m, 2H).

### Beispiel No A1-182:

¹H-NMR (400 MHz, CDCl₃) d 7.67 (m, 1H), 7.61 (m, 1H), 7.55 (m, 1H), 7.50 (m, 1H), 7.02 (dd, 1H), 6.88 (d, 1H), 6.73 (d, 1H), 6.22 (br. s, 1H, NH), 4.31 (s, 2H), 3.30 (s, 3H), 1.80 (m, 2H), 1.52 (m, 2H).

### Beispiel No. A1-190:

¹H-NMR (400 MHz, CDCl₃) d 8.04 (d, 2H), 7.40 (d, 2H), 6.97 (dd, 1 H), 6.83 (d, 1H), 6.66 (d, 1H), 6.09 (br. s, 1H, NH), 4.35 (s, 2H), 3.94 (s, 3H), 3.30 (s, 3H), 1.78 (m, 2H), 1.50 (m, 2H).

### Beispiel No. A1-191:

¹H-NMR (400 MHz, CDCl₃) d 8.06 (m, 1H), 7.94 (m, 1H), 7.59 (m, 1H), 7.49 (m, 1H), 7.07 (dd, 1H), 6.87 (d, 1H), 6.69 (d, 1H), 6.12 (br. s, 1H, NH), 4.33 (s, 2H), 3.93 (s, 3H), 3.30 (s, 3H), 1.78 (m, 2H), 1.54 (m, 2H).

### Beispiel No. A1-192:

¹H-NMR (400 MHz, CDCl₃) d 8.27 (d, 2H), 7.40 (d, 2H), 6.98 (dd, 1H), 6.85 (d, 1H), 6.66 (d, 1H), 6.24 (br. s, 1H, NH), 4.42 (q, 2H), 4.35 (s, 2H), 3.30 (s, 3H), 1.78 (m, 2H), 1.52 (m, 2H), 1.42 (t, 3H).

### Beispiel No. A1-226:

¹H-NMR (400 MHz, d₆-DMSO) δ 9.76 (br. s, 1H, NH), 7.51-7.46 (m, 2H), 7.40-7.35 (m, 2H), 7.10 (dd, 1H), 7.03 (d, 1H), 6.80 (d, 1H), 4.45 (s, 2H), 3.20 (s, 3H), 1.53 (m, 4H).

### Beispiel No. A1-291:

¹H-NMR (400 MHz, CDCl₃) d 7.29 (d, 2H), 7.11 (d, 2H), 6.98 (dd, 1H), 6.83 (d, 1H), 6.65 (d, 1H), 6.30 (br. s, 1H, NH), 3.28 (s, 3H), 3.26 (m, 2H), 3.12 (m, 2H), 1.77 (m, 2H), 1.53 (m, 2H).

### Beispiel No. A1-292:

¹H-NMR (400 MHz, CDCl₃) d 7.58 (d, 2H), 7.30 (d, 2H), 6.94 (dd, 1H), 6.83 (d, 1H), 6.71 (d, 1H), 6.10 (br. s, 1H, NH), 3.31 (m, 2H), 3.28 (s, 3H), 3.21 (m, 2H), 1.78 (m, 2H), 1.52 (m, 2H).

### Beispiel No. A1-301:

¹H-NMR (400 MHz, CDCl₃) δ 7.27 (dd, 1H), 6.90 (d, 1H), 6.88 (d, 1H), 6.31 (br. s, 1H, NH), 6.18 (s, 1H), 4.27 (s, 2H), 3.29 (s, 3H), 2.45 (s, 3H), 1.78 (m, 2H), 1.57 (m, 2H),

### Beispiel No A1-332:

¹H-NMR (400 MHz, CDCl₃) d 7.39 (d, 2H), 7.23 (d, 2H), 6.99 (dd, 1H), 6.85 (d, 1H), 6.74 (d, 1H), 6.08 (br. s, 1H, NH), 4.26 (s, 2H), 3.30 (s, 3H), 1.78 (m, 2H), 1.52 (m, 2H), 1.21 (s, 9H).

### Beispiel No. A1-461:

¹H-NMR (400 MHz, d₆-DMSO) δ 9.91 (br. s, 1H, NH), 7.43 (m, 1H), 7.38 (m, 1H), 7.27 (m, 1H), 7.13 (dd, 1H), 7.04 (d, 1H), 6.86 (d, 1H), 4.54 (s, 2H), 3.20 (s, 3H), 1.54 (m, 2H), 1.52 (m, 2H).

### Beispiel No. A2-45:

¹H-NMR (400 MHz, CDCl₃) δ 7.82 (d, 2H), 7.74 (d, 2H), 6.79 (m, 2H), 6.68 (d, 1H), 6.50 (br. s, 1H, NH), 3.81 (q, 2H), 1.77 (m, 2H), 1.47 (m, 2H), 1.28 (t, 3H).

### Beispiel No. A2-56:

¹H-NMR (400 MHz, CDCl₃) δ 8.01 (d, 1H), 7.91 (m, 1H), 7.85 (m, 1H), 7.61 (m, 1H), 6.82 (dd, 1H), 6.78 (d, 1H), 6.66 (d, 1H), 6.65 (br. s, 1H, NH), 3.82 (q, 2H), 1.77 (m, 2H), 1.47 (m, 2H), 1.28 (t, 3H).

### Beispiel No. A2-152:

¹H-NMR (400 MHz, CDCl₃) δ 7.21 (d, 2H), 7.18 (d, 2H), 7.02 (dd, 1 H), 6.85 (d, 1H), 6.65 (d, 1H), 6.18 (br. s, 1H, NH), 4.25 (s, 2H), 3.85 (q, 2H), 2.36 (s, 3H), 1.76 (m, 2H), 1.50 (m, 2H), 1.30 (t, 3H).

### Beispiel No. A2-153:

¹H-NMR (400 MHz, CDCl₃) δ 7.26 (m, 1H), 7.19 (m, 1H), 7.11 (m, 2H), 6.99 (dd, 1H), 6.86 (d, 1H), 6.62 (d, 1H), 6.18 (br. s, 1H, NH), 4.26 (s, 2H), 3.85 (q, 2H), 2.34 (s, 2H), 1.78 (m, 2H), 1.49 (m, 2H), 1.30 (t, 3H).

### Beispiel No. A2-158:

¹H-NMR (400 MHz, CDCl₃) δ 7.31 (m, 2H), 7.08 (m, 2H), 6.98 (dd, 1H), 6.87 (d, 1H), 6.68 (d, 1H), 6.23 (br. s, 1H, NH), 4.27 (s, 2H), 3.85 (q, 2H), 1.78 (m, 2H), 1.51 (m, 2H), 1.29 (t, 3H).

### Beispiel No. A2-165:

¹H-NMR (400 MHz, CDCl₃) δ 7.35 (d, 2H), 7.27 (d, 2H), 6.98 (dd, 1H), 6.87 (d, 1H), 6.67 (d, 1H), 6.28 (br. s, 1H, NH), 4.26 (s, 2H), 3.85 (q, 2H), 1.78 (m, 2H), 1.51 (m, 2H), 1.30 (t, 3H).

### Beispiel No. A2-166:

¹H-NMR (400 MHz, CDCl₃) δ 7.36 (m, 1H), 7.34 (m, 1H), 7.32 (m, 1H), 7.25 (m, 1H), 7.04 (dd, 1H), 6.87 (d, 1H), 6.66 (d, 1H), 6.32 (br. s, 1H, NH), 4.26 (s, 2H), 3.85 (q, 2H), 1.78 (m, 2H), 1.51 (m, 2H), 1.31 (t, 3H).

### Beispiel No. A2-167:

¹H-NMR (400 MHz, CDCl₃) δ 7.45 (d, 1H), 7.36 (d, 1H), 7.21 (dd, 1H), 7.02 (dd, 1H), 6.88 (d, 1H), 6.67 (d, 1H), 6.18 (br. s, 1H, NH), 4.24 (s, 2H), 3.86 (q, 2H), 1.79 (m, 2H), 1.52 (m, 2H), 1.30 (t, 3H).

### Beispiel No. A2-178:

¹H-NMR (400 MHz, CDCl₃) δ 7.63 (d, 2H), 7.47 (d, 2H), 6.98 (dd, 1H), 6.87 (d, 1H), 6.73 (d, 1H), 6.33 (br. s, 1H, NH), 4.35 (s, 2H), 3.85 (q, 2H), 1.78 (m, 2H), 1.51 (m, 2H), 1.29 (t, 3H).

### Beispiel No. A2-181:

¹H-NMR (400 MHz, CDCl₃) δ 7.68 (d, 2H), 7.47 (d, 2H), 6.98 (dd, 1H), 6.89 (d, 1H), 6.75 (d, 1H), 6.18 (br. s, 1H, NH), 4.33 (s, 2H), 3.86 (q, 2H), 1.81 (m, 2H), 1.53 (m, 2H), 1.31 (t, 3H).

### Beispiel No. A2-182:

¹H-NMR (400 MHz, CDCl₃) δ 7.68 (m, 1H), 7.62 (m, 1H), 7.58 (m, 1H), 7.50 (m, 1H), 7.01 (dd, 1H), 6.90 (d, 1H), 6.73 (d, 1H), 6.21 (br. s, 1H, NH), 4.32 (s, 2H), 3.86 (q, 2H), 1.80 (m, 2H), 1.52 (m, 2H), 1.31 (t, 3H).

### Beispiel No. A2-291:

¹H-NMR (400 MHz, CDCl₃) δ 7.29 (d, 2H), 7.12 (d, 2H), 6.95 (dd, 1H), 6.84 (d, 1H), 6.63 (d, 1H), 6.03 (br. s, 1H, NH), 3.84 (q, 2H), 3.28 (m, 2H), 3.12 (m, 2H), 1.77 (m, 2H), 1.52 (m, 2H), 1.28 (t, 3H).

### Beispiel No. A2-292:

¹H-NMR (400 MHz, CDCl₃) δ 7.58 (d, 2H), 7.30 (d, 2H), 6.95 (dd, 1H), 6.85 (d, 1H), 6.71 (d, 1H), 6.17 (br. s, 1H, NH), 3.84 (q, 2H), 3.32 (m, 2H), 3.21 (m, 2H), 1.78 (m, 2H), 1.51 (m, 2H), 1.28 (t, 3H).

### Beispiel No. A3-45:

¹H-NMR (400 MHz, CDCl₃) δ 7.81 (d, 2H), 7.74 (d, 2H), 6.75 (m, 2H), 6.68 (d, 1H), 6.49 (br. s, 1H, NH), 3.70 (t, 2H), 1.77 (m, 2H), 1.70 (sext, 2H), 1.48 (m, 2H), 0.96 (t, 3H).

### Beispiel No. A3-56:

¹H-NMR (400 MHz, CDCl₃) δ 8.01 (m, 1H), 7.91 (m, 1H), 7.83 (m, 1H), 7.58 (m, 1H), 6.80 (dd, 1H), 6.75 (d, 1H), 6.65 (d, 1H), 6.57 (br. s, 1H, NH), 3.71 (t, 2H), 1.76 (m, 2H), 1.70 (sext, 2H), 1.48 (m, 2H), 0.96 (t, 3H).

### Beispiel No. A3-152:

¹H-NMR (400 MHz, CDCl₃) δ 7.20 (d, 2H), 7.17 (d, 2H), 7.01 (dd, 1H), 6.96 (br. s, 1H, NH), 6.84 (d, 1H), 6.65 (d, 1H), 4.25 (s, 2H), 3.75 (t, 2H), 2.36 (s, 3H), 1.77 (m, 2H), 1.73 (sext, 2H), 1.49 (m, 2H), 0.98 (t, 3H).

### Beispiel No. A3-153:

¹H-NMR (400 MHz, CDCl₃) δ 7.25 (m, 1H), 7.18 (m, 1H), 7.11 (m, 2H), 6.98 (dd, 1H), 6.85 (d, 1H), 6.62 (d, 1H), 6.23 (br. s, 1H, NH), 4.26 (s, 2H), 3.74 (t, 2H), 1.78 (m, 2H), 1.73 (sext, 2H), 1.49 (m, 2H), 0.99 (t, 3H).

### Beispiel No. A3-158:

¹H-NMR (400 MHz, CDCl₃) δ 7.32 (d, 2H), 7.08 (d, 2H), 6.96 (dd, 1H), 6.85 (d, 1H), 6.67 (d, 1H), 6.14 (br. s, 1H, NH), 4.27 (s, 2H), 3.74 (t, 2H), 1.78 (m, 2H), 1.74 (sext, 2H), 1.51 (m, 2H), 0.98 (t, 3H).

### Beispiel No. A3-165:

¹H-NMR (400 MHz, CDCl₃) δ 7.35 (d, 2H), 7.25 (d, 2H), 6.96 (dd, 1H), 6.85 (d, 1H), 6.66 (d, 1H), 6.20 (br. s, 1H, NH), 4.26 (s, 2H), 3.74 (t, 2H), 1.79 (m, 2H), 1.73 (sext, 2H), 1.51 (m, 2H), 0.99 (t, 3H).

### Beispiel No. A3-166:

¹H-NMR (400 MHz, CDCl₃) δ 7.34 (m, 1H), 7.31 (m, 1H), 7.29 (m, 1H), 7.24 (m, 1H), 7.01 (dd, 1H), 6.87 (d, 1H), 6.65 (d, 1H), 6.18 (br. s, 1H, NH), 4.26 (s, 2H), 3.75 (t, 2H), 1.78 (m, 2H), 1.75 (sext, 2H), 1.53 (m, 2H), 1.00 (t, 3H).

### Beispiel No. A3-176:

¹H-NMR (400 MHz, CDCl₃) δ 8.24 (m, 1H), 8.13 (m, 1H), 7.73 (d, 1H), 7.58 (m, 1H), 7.07 (dd, 1H), 6.87 (d, 1H), 6.76 (d, 1H), 6.33 (br. s, 1H, NH), 4.38 (s, 2H), 3.75 (t, 2H), 1.78 (m, 2H), 1.75 (sext, 2H), 1.55 (m, 2H), 1.01 (t, 3H).

### Beispiel No. A3-178:

¹H-NMR (400 MHz, CDCl₃) δ 7.64 (d, 2H), 7.47 (d, 2H), 6.96 (dd, 1H), 6.86 (d, 1H), 6.72 (d, 1H), 6.16 (br. s, 1H, NH), 4.35 (s, 2H), 3.74 (t, 2H), 1.78 (m, 2H), 1.75 (sext, 2H), 1.52 (m, 2H), 0.99 (t, 3H).

### Beispiel No. A3-181:

¹H-NMR (400 MHz, CDCl₃) δ 7.67 (d, 2H), 7.47 (d, 2H), 6.97 (dd, 1H), 6.87 (d, 1H), 6.75 (d, 1H), 6.29 (br. s, 1H, NH), 4.33 (s, 2H), 3.75 (t, 2H), 1.81 (m, 2H), 1.73 (sext, 2H), 1.53 (m, 2H), 0.99 (t, 3H).

### Beispiel No. A3-182:

¹H-NMR (400 MHz, CDCl₃) δ 7.68 (m, 1H), 7.61 (m, 2H), 7.50 (m, 1H), 7.01 (dd, 1H), 6.89 (d, 1H), 6.73 (d, 1H), 6.31 (br. s, 1H, NH), 4.31 (s, 2H), 3.75 (t, 2H), 1.79 (m, 2H), 1.74 (sext, 2H), 1.54 (m, 2H), 0.99 (t, 3H).

### Beispiel No. A3-291:

¹H-NMR (400 MHz, CDCl₃) δ 7.30 (d, 2H), 7.12 (d, 2H), 6.93 (dd, 1H), 6.83 (d, 1H), 6.63 (d, 1H), 6.10 (br. s, 1H, NH), 3.73 (t, 2H), 3.27 (m, 2H), 3.12 (m, 2H), 1.78 (m, 2H), 1.72 (sext, 2H), 1.51 (m, 2H), 0.98 (t, 3H).

### Beispiel No. A3-292:

¹H-NMR (400 MHz, CDCl₃) δ 7.58 (d, 2H), 7.31 (d, 2H), 6.94 (dd, 1H), 6.83 (d, 1H), 6.71 (d, 1H), 6.21 (br. s, 1H, NH), 3.73 (t, 2H), 3.30 (m, 2H), 3.21 (m, 2H), 1.78 (m, 2H), 1.71 (sext, 2H), 1.50 (m, 2H), 0.97 (t, 3H).

### Beispiel No. A4-45:

¹H-NMR (400 MHz, CDCl₃) δ 7.82 (d, 2H), 7.47 (d, 2H), 6.91 (d, 1H), 6.76 (dd, 1H), 6.66 (d, 1H), 6.56 (br. s, 1H, NH), 4.70 (sept, 1H), 1.76 (m, 2H), 1.47 (d, 6H), 1.45 (m, 2H).

### Beispiel No. A4-56:

¹H-NMR (400 MHz, CDCl₃) δ 8.02 (m, 1H), 7.92 (m, 1H), 7.83 (m, 1H), 7.60 (m, 1H), 6.91 (d, 1H), 6.78 (dd, 1H), 6.63 (d, 1H), 6.55 (br. s, 1H, NH), 4.70 (sept, 1H), 1.75 (m, 2H), 1.47 (d, 6H), 1.45 (m, 2H).

### Beispiel No. A4-152:

¹H-NMR (400 MHz, CDCl₃) δ 7.21 (d, 2H), 7.18 (d, 2H), 7.00 (d, 1H), 6.96 (dd, 1H), 6.59 (d, 1H), 6.15 (br. s, 1H, NH), 4.74 (sept, 1H), 4.26 (s, 2H), 2.37 (s, 3H), 1.74 (m, 2H), 1.50 (d, 6H), 1.47 (m, 2H).

### Beispiel No. A4-153:

¹H-NMR (400 MHz, CDCl₃) δ 7.26 (m, 1H), 7.19 (m, 1H), 7.12 (m, 2H), 7.01 (d, 1H), 6.95 (dd, 1H), 6.60 (d, 1H), 6.13 (br. s, 1H, NH), 4.75 (sept, 1H), 4.27 (s, 2H), 2.35 (s, 3H), 1.74 (m, 2H), 1.51 (d, 6H), 1.47 (m, 2H).

### Beispiel No. A4-158:

¹H-NMR (400 MHz, CDCl₃) δ 7.31 (d, 2H), 7.07 (d, 2H), 7.00 (d, 1H), 6.94 (dd, 1 H), 6.66 (d, 1H), 6.23 (br. s, 1H, NH), 4.74 (sept, 1H), 4.27 (s, 2H), 1.76 (m, 2H), 1.51 (m, 8H).

### Beispiel No. A4-165:

¹H-NMR (400 MHz, CDCl₃) δ 7.35 (d, 2H), 7.28 (d, 2H), 7.02 (d, 1H), 6.94 (dd, 1H), 6.64 (d, 1H), 6.20 (br. s, 1H, NH), 4.73 (sept, 1H), 4.27 (s, 2H), 1.77 (m, 2H), 1.51 (d, 6H), 1.48 (m, 2H).

### Beispiel No. A4-166:

¹H-NMR (400 MHz, CDCl₃) δ 7.35 (m, 1H), 7.30 (m, 1H), 7.25 (m, 2H), 7.01 (m, 2H), 6.63 (d, 1H), 6.27 (br. s, 1H, NH), 4.74 (sept, 1H), 4.26 (s, 2H), 1.76 (m, 2H), 1.50 (m, 8H).

### Beispiel No. A4-167:

¹H-NMR (400 MHz, CDCl₃) δ 7.42 (d, 1H), 7.36 (d, 1H), 7.19 (dd, 1H), 7.00 (m, 2H), 6.66 (d, 1H), 6.31 (br. s, 1H, NH), 4.74 (sept, 1H), 4.24 (s, 2H), 1.77 (m, 2H), 1.51 (d, 6H), 1.49 (m, 2H).

### Beispiel No. A4-176:

¹H-NMR (400 MHz, CDCl₃) δ 8.25 (m, 1H), 8.13 (d, 1H), 7.75 (d, 1H), 7.58 (m, 1H), 7.04 (m, 2H), 6.74 (d, 1H), 6.33 (br. s, 1H, NH), 4.75 (sept, 1H), 4.38 (s, 2H), 1.78 (m, 2H), 1.54 (m, 2H), 1.52 (d, 6H).

### Beispiel No. A4-178:

¹H-NMR (400 MHz, CDCl₃) δ 7.63 (d, 2H), 7.46 (d, 2H), 7.02 (d, 1H), 6.95 (dd, 1H), 6.71 (d, 1H), 6.28 (br. s, 1H, NH), 4.74 (sept, 1H), 4.35 (s, 2H), 1.76 (m, 2H), 1.52 (m, 8H).

### Beispiel No. A4-181:

¹H-NMR (400 MHz, CDCl₃) δ 7.69 (d, 2H), 7.47 (d, 2H), 7.01 (m, 1H), 6.93 (dd, 1H), 6.74 (d, 1H), 6.16 (br. s, 1H, NH), 4.74 (sept, 1H), 4.34 (s, 2H), 1.78 (m, 2H), 1.51 (m, 8H).

### Beispiel No. A4-182:

¹H-NMR (400 MHz, CDCl₃) δ 7.68 (m, 1H), 7.60 (m, 2H), 7.49 (m, 1H), 7.03 (d, 1H), 6.97 (dd, 1H), 6.72 (d, 1H), 6.32 (br. s, 1H, NH), 4.73 (sept, 1H), 4.31 (s, 2H), 1.78 (m, 2H), 1.54 (m, 2H), 1.51 (d, 6H).

### Beispiel No. A4-291:

¹H-NMR (400 MHz, CDCl₃) δ 7.24 (d, 2H), 7.13 (d, 2H), 6.98 (d, 1H), 6.91 (dd, 1H), 6.61 (d, 1H), 6.07 (br. s, 1H, NH), 4.73 (sept, 1H), 3.27 (m, 2H), 3.12 (m, 2H), 1.77 (m, 2H), 1.50 (m, 8H).

### Beispiel No. A4-292:

¹H-NMR (400 MHz, CDCl₃) δ 7.58 (d, 2H), 7.31 (d, 2H), 7.00 (d, 1H), 6.92 (dd, 1H), 6.70 (d, 1H), 6.26 (br. s, 1H, NH), 4.73 (sept, 1H), 3.33 (m, 2H), 3.21 (m, 2H), 1.77 (m, 2H), 1.49 (m, 8H).

### Beispiel No. B1-165:

¹H-NMR (400 MHz, CDCl₃ δ, ppm) 7.38 (d, 2H), 7.20 (m, 3H), 7.02 (m, 1H), 6.74 (d, 1H), 6.11 (br. s, 1H, NH), 4.30 (s, 2H), 3.19 (s, 3H), 2.67 (m, 2H), 2.43-2.20 (m, 4H).

### Beispiel No. B1-178:

¹H-NMR (400 MHz, CDCl₃ δ, ppm) 7.65 (d, 2H), 7.50 (m, 2H), 7.34 (m, 1H), 7.01 (m, 1H), 6.74 (d, 1H), 6.14 (br. s, 1H, NH), 4.38 (s, 2H), 3.19 (s, 3H), 2.67 (m, 2H), 2.44-2.18 (m, 4H).

### Beispiel No. B1-181:

¹H-NMR (400 MHz, CDCl₃ δ, ppm) 7.68 (d, 2H), 7.47 (d, 2H), 7.29 (m, 1H), 7.01 (m, 1 H), 6.75 (d, 1H), 6.14 (br. s, 1H, NH), 4.37 (s, 2H), 3.19 (s, 3H), 2.68 (m, 2H), 2.42-2.20 (m, 4H).

### Beispiel No. C1-45:

¹H-NMR (400 MHz, CDCl₃) δ 7.81 (d, 2H), 7.75 (d, 2H), 6.91 (d, 1H), 6.86 (dd, 1H), 6.69 (d, 1H), 6.39 (br. s, 1H, NH), 3.17 (s, 3H), 2.18-2.05 (m, 4H), 1.87 (m, 2H), 1.71 (m, 2H).

### Beispiel No. C1-152:

¹H-NMR (400 MHz, CDCl₃) δ 7.21 (d, 2H), 7.19 (d, 2H), 7.06 (dd, 1H), 6.95 (d, 1H), 6.77 (d, 1H), 6.04 (br. s, 1H, NH), 4.27 (s, 2H), 3.21 (s, 3H), 2.37 (s, 3H), 2.20-2.06 (m, 4H), 1.95 (m, 2H), 1.82 (m, 2H).

### Beispiel No. C1-165:

¹H-NMR (400 MHz, d₆-DMSO) δ 9.55 (br. s, 1H, NH), 7.41 (d, 2H), 7.29 (d, 2H), 7.09 (dd, 1H), 6.99 (d, 1H), 6.93 (d, 1H), 4.42 (s, 2H), 3.31 (s, 3H), 2.00-1.92 (m, 4H), 1.89 (m, 2H), 1.72 (m, 2H).

### Beispiel No. C1-166:

¹H-NMR (400 MHz, CDCl₃) δ 7.36 (m, 1H), 7.30 (m, 1 H), 7.28 (m, 2H), 7.09 (dd, 1H), 6.98 (d, 1H), 6.79 (d, 1H), 6.23 (br. s, 1H, NH), 4.27 (s, 2H), 3.21 (s, 3H), 2.20-2.07 (m, 4H), 1.96 (m, 2H), 1.82 (m, 2H).

### Beispiel No. C1-181:

¹H-NMR (400 MHz, CDCl₃) δ 7.67 (d, 2H), 7.47 (d, 2H), 7.04 (m, 2H), 6.79 (d, 1H), 6.33 (br. s, 1H, NH), 4.35 (s, 2H), 3.21 (s, 3H), 2.21-2.08 (m, 4H), 1.97 (m, 2H), 1.81 (m,2H).

### Beispiel No. C1-182:

¹H-NMR (400 MHz, CDCl₃) δ 7.68 (m, 1H), 7.60 (m, 2H), 7.52 (m, 1H), 7.06 (m, 1H), 7.02 (d, 1H), 6.80 (d, 1H), 6.12 (br. s, 1H, NH), 4.32 (s, 2H), 3.22 (s, 3H), 2.20-2.08 (m, 4H), 1.97 (m, 2H), 1.82 (m, 2H).

### Beispiel No. C1-291:

¹H-NMR (400 MHz, CDCl₃) δ 7.29 (d, 2H), 7.12 (d, 2H), 7.02 (dd, 1H), 6.99 (d, 1H), 6.75 (d, 1H), 6.16 (br. s, 1H, NH), 3.30 (m, 2H), 3.17 (s, 3H), 3.13 (m, 2H), 2.18-2.07 (m, 4H), 1.95 (m, 2H), 1.80 (m, 2H).

### Beispiel No. C2-45:

¹H-NMR (400 MHz, d₆-DMSO) δ 10.15 (br. s, 1H, NH), 8.05 (d, 2H), 7.82 (d, 2H), 6.92 (m, 2H), 6.83 (d, 1H), 3.63 (q, 2H), 1.98-1.87 (m, 4H), 1.77 (m, 2H), 1.57 (m, 2H), 1.09 (t, 3H).

### Beispiel No. C2-152:

¹H-NMR (400 MHz, CDCl₃) δ 7.22 (d, 2H), 7.19 (d, 2H), 7.07 (dd, 1H), 6.96 (d, 1H), 6.79 (d, 1H), 6.09 (br. s, 1H, NH), 4.27 (s, 2H), 3.78 (q, 2H), 2.37 (s, 3H), 2.20-2.08 (m, 4H), 1.95 (m, 2H), 1.81 (m, 2H), 1.27 (t, 3H).

### Beispiel No. C2-165:

¹H-NMR (400 MHz, CDCl₃) δ 7.35 (d, 2H), 7.28 (d, 2H), 7.04 (dd, 1H), 6.98 (d, 1H), 6.79 (d, 1H), 6.22 (br. s, 1H, NH), 4.28 (s, 2H), 3.76 (q, 2H), 2.20-2.07 (m, 4H), 1.94 (m, 2H), 1.80 (m, 2H), 1.28 (t, 3H).

### Beispiel No. C2-166:

¹H-NMR (400 MHz, CDCl₃) δ 7.36 (m, 1H), 7.32 (m, 1H), 7.28 (m, 2H), 7.08 (dd, 1H), 6.98 (d, 1H), 6.80 (d, 1H), 6.09 (br. s, 1H, NH), 4.28 (s, 2H), 3.77 (q, 2H), 2.20-2.08 (m, 4H), 1.96 (m, 2H), 1.81 (m, 2H), 1.28 (t, 3H).

### Beispiel No. C2-181:

¹H-NMR (400 MHz, CDCl₃) δ 7.68 (d, 2H), 7.48 (d, 2H), 7.04 (m, 2H), 6.80 (d, 1H), 6.29 (br. s, 1H, NH), 4.35 (s, 2H), 3.76 (q, 2H), 2.20-2.09 (m, 4H), 1.95 (m, 2H), 1.80 (m, 2H), 1.27 (t, 3H).

### Beispiel No. C2-182:

¹H-NMR (400 MHz, CDCl₃) δ 7.68 (m, 1H), 7.61 (m, 2H), 7.52 (m, 1H), 7.04 (m, 2H), 6.81 (d, 1H), 6.14 (br. s, 1H, NH), 4.33 (s, 2H), 3.77 (q, 2H), 2.21-2.08 (m, 4H), 1.97 (m, 2H), 1.81 (m, 2H), 1.27 (t, 3H).

### Beispiel No. C2-291:

¹H-NMR (400 MHz, CDCl₃) δ 7.29 (d, 2H), 7.13 (d, 2H), 7.00 (m, 2H), 6.75 (d, 1H), 6.17 (br. s, 1H, NH), 3.74 (q, 2H), 3.30 (m, 2H), 3.13 (m, 2H), 2.19-2.07 (m, 4H), 1.96 (m, 2H), 1.81 (m, 2H), 1.26 (t, 3H).

### Beispiel No. C3-45:

¹H-NMR (400 MHz, CDCl₃) δ 7.84 (d, 2H), 7.75 (d, 2H), 6.89 (d, 1H), 6.85 (dd, 1H), , 6.70 (d, 1H), 6.53 (br. s, 1H, NH), 3.64 (t, 2H), 2.17-2.03 (m, 4H), 1.87 (m, 2H), 1.72 (m, 2H), 1.68 (sext, 2H), 0.93 (t, 3H).

### Beispiel No. C3-152:

¹H-NMR (400 MHz, CDCl₃) δ 7.22 (d, 2H), 7.18 (d, 2H), 7.05 (dd, 1H), 6.95 (d, 1H), 6.77 (d, 1H), 6.10 (br. s, 1H, NH), 4.27 (s, 2H), 3.68 (t, 2H), 2.38 (s, 3H), 2.19-2.06 (m, 4H), 1.94 (m, 2H), 1.82 (m, 2H), 1.71 (sext, 2H), 0.97 (t, 3H).

### Beispiel No. C3-165:

¹H-NMR (400 MHz, CDCl₃) δ 7.36 (d, 2H), 7.27 (d, 2H), 7.02 (dd, 1H), 6.97 (d, 1H), 6.77 (d, 1H), 6.14 (br. s, 1H, NH), 4.28 (s, 2H), 3.66 (t, 2H), 2.20-2.06 (m, 4H), 1.94 (m, 2H), 1.81 (m, 2H), 1.70 (sext, 2H), 0.97 (t, 3H).

### Beispiel No. C3-166:

¹H-NMR (400 MHz, CDCl₃) δ 7.37 (m, 1H), 7.33 (m, 1H), 7.29 (m, 2H), 7.07 (dd, 1H), 6.98 (d, 1H), 6.79 (d, 1H), 6.24 (br. s, 1H, NH), 4.28 (s, 2H), 3.68 (t, 2H), 2.20-2.07 (m, 4H), 1.96 (m, 2H), 1.83 (m, 2H), 1.72 (sext, 2H), 0.96 (t, 3H).

### Beispiel No. C3-181:

¹H-NMR (400 MHz, CDCl₃) δ 7.68 (d, 2H), 7.48 (d, 2H), 7.02 (m, 2H), 6.79 (d, 1H), 6.26 (br. s, 1H, NH), 4.35 (s, 2H), 3.67 (t, 2H), 2.21-2.08 (m, 4H), 1.95 (m, 2H), 1.81 (m, 2H), 1.71 (sext, 2H), 0.96 (t, 3H).

### Beispiel No. C3-182:

¹H-NMR (400 MHz, CDCl₃) δ 7.68 (m, 1H), 7.61 (m, 2H), 7.50 (m, 1H), 7.04 (m, 2H), 6.80 (d, 1H), 6.30 (br. s, 1H, NH), 4.33 (s, 2H), 3.69 (t, 2H), 2.20-2.09 (m, 4H), 1.96 (m, 2H), 1.82 (m, 2H), 1.72 (sext, 2H), 0.96 (t, 3H).

### Beispiel No. C3-291:

¹H-NMR (400 MHz, CDCl₃) δ 7.29 (d, 2H), 7.13 (d, 2H), 6.99 (m, 2H), 6.74 (d, 1H), 6.09 (br. s, 1H, NH), 3.65 (t, 2H), 3.29 (m, 2H), 3.14 (m, 2H), 2.19-2.05 (m, 4H), 1.95 (m, 2H), 1.80 (m, 2H), 1.70 (sext, 2H), 0.96 (t, 3H).

### Beispiel No. C4-45:

¹H-NMR (400 MHz, CDCl₃) δ 7.84 (d, 2H), 7.76 (d, 2H), 6.86 (m, 1H), 6.83 (m, 2H), 6.30 (br. s, 1H, NH), 4.60 (sept, 1H), 2.14-2.02 (m, 4H), 1.86 (m, 2H), 1.70 (m, 2H), 1.45 (d, 6H).

### Beispiel No. C4-152:

¹H-NMR (400 MHz, CDCl₃) δ 7.22 (d, 2H), 7.19 (d, 2H), 7.01 (m, 1H), 6.93 (m, 2H), 5.99 (br. s, 1H, NH), 4.64 (sept, 1H), 4.28 (s, 2H), 2.38 (s, 3H), 2.19-2.07 (m, 4H), 1.94 (m, 2H), 1.80 (m, 2H), 1.49 (d, 6H).

### Beispiel No. C4-165:

¹H-NMR (400 MHz, CDCl₃) δ 7.36 (d, 2H), 7.29 (d, 2H), 7.00 (m, 1H), 6.96 (d, 1H), 6.93 (d, 1H), 6.12 (br. s, 1H, NH), 4.65 (sept, 1H), 4.28 (s, 2H), 2.18-2.08 (m, 4H), 1.93 (m, 2H), 1.79 (m, 2H), 1.28 (d, 6H).

### Beispiel No. C4-166:

¹H-NMR (400 MHz, CDCl₃) δ 7.36 (m, 1H), 7.32 (m, 1H), 7.29 (m, 2H), 7.05 (dd, 1H), 6.97 (d, 1H), 6.95 (d, 1H), 6.09 (br. s, 1H, NH), 4.65 (sept, 1H), 4.28 (s, 2H), 2.19-2.05 (m, 4H), 1.94 (m, 2H), 1.81 (m, 2H), 1.48 (d, 6H).

### Beispiel No. C4-181:

¹H-NMR (400 MHz, CDCl₃) δ 7.68 (d, 2H), 7.49 (d, 2H), 7.01 (m, 2H), 6.93 (d, 1H), 6.14 (br. s, 1H, NH), 4.65 (sept, 1H), 4.35 (s, 2H), 2.19-2.08 (m, 4H), 1.96 (m, 2H), 1.80 (m, 2H), 1.48 (d, 6H).

### Beispiel No. C4-182:

¹H-NMR (400 MHz, CDCl₃) δ 7.68 (m, 1H), 7.62 (m, 2H), 7.52 (m, 1H), 7.01 (m, 2H), 6.94 (d, 1H), 6.11 (br. s, 1H, NH), 4.65 (sept, 1H), 4.33 (s, 2H), 2.19-2.08 (m, 4H), 1.96 (m, 2H), 1.81 (m, 2H).

### Beispiel No. C4-291:

¹H-NMR (400 MHz, CDCl₃) δ 7.29 (d, 2H), 7.14 (d, 2H), 6.94 (m, 2H), 6.90 (d, 1H), 5.97 (br. s, 1H, NH), 4.63 (sept, 1H), 3.30 (m, 2H), 3.14 (m, 2H), 2.18-2.05 (m, 4H), 1.94 (m, 2H), 1.78 (m, 2H).

### Beispiel No. D1-45:

¹H-NMR (400 MHz, CDCl₃) δ 7.79 (d, 2H), 7.74 (d, 2H), 7.12 (d, 1H), 6.92 (dd, 1H), 6.71 (d, 1H), 6.33 (br. s, 1H, NH), 3.17 (s, 3H), 2.06 (m, 2H), 1.81 (m, 2H), 1.72-1.64 (m, 4H), 1.47 (m, 2H).

### Beispiel No. D1-152:

¹H-NMR (400 MHz, CDCl₃) δ 7.23 (d, 2H), 7.20 (d, 2H), 7.10 (dd, 1H), 6.99 (d, 1H), 6.79 (d, 1H), 6.03 (br. s, 1H, NH), 4.28 (s, 2H), 3.20 (s, 3H), 2.37 (s, 3H), 1.97 (m, 2H), 1.85 (m, 2H), 1.75-1.66 (m, 4H), 1.58 (m, 2H).

### Beispiel No. D1-165:

¹H-NMR (400 MHz, CDCl₃) δ 7.36 (d, 2H), 7.28 (d, 2H), 7.22 (d, 1H), 7.08 (dd, 1H), 6.80 (d, 1H), 6.08 (br. s, 1H, NH), 4.29 (s, 2H), 3.20 (s, 3H), 1.98 (m, 2H), 1.84 (m, 2H), 1.74-1.66 (m, 4H), 1.57 (m, 2H).

### Beispiel No. D1-166:

¹H-NMR (400 MHz, CDCl₃) δ 7.36 (m, 1H), 7.31 (m, 1H), 7.29 (m, 2H), 7.23 (d, 1H), 7.11 (dd, 1H), 6.81 (d, 1H), 6.12 (br. s, 1H, NH), 4.28 (s, 2H), 3.20 (s, 3H), 1.98 (m, 2H), 1.85 (m, 2H), 1.73-1.66 (m, 4H), 1.57 (m, 2H).

### Beispiel No. D1-181:

¹H-NMR (400 MHz, CDCl₃) δ 7.67 (d, 2H), 7.47 (d, 2H), 7.30 (d, 1H), 7.09 (dd, 1H), 6.81 (d, 1H), 6.33 (br. s, 1H, NH), 4.36 (s, 2H), 3.21 (s, 3H), 2.21-1.08 (m, 4H), 1.99 (m, 2H), 1.86 (m, 2H), 1.75-1.65 (m, 4H), 1.56 (m, 2H).

### Beispiel No. D1-182:

¹H-NMR (400 MHz, CDCl₃) δ 7.67 (m, 1H), 7.60 (m, 2H), 7.50 (m, 1H), 7.31 (d, 1H), 7.11 (dd, 1H), 6.83 (d, 1H), 6.39 (br. s, 1H, NH), 4.34 (s, 2H), 3.21 (s, 3H), 1.98 (m, 2H), 1.84 (m, 2H), 1.75-1.63 (m, 4H), 1.57 (m, 2H).

### Beispiel No. D1-291:

¹H-NMR (400 MHz, CDCl₃) δ 7.30 (d, 2H), 7.24 (d, 1H), 7.13 (d, 2H), 7.02 (dd, 1H), 6.78 (d, 1H), 6.01 (br. s, 1H, NH), 3.31 (m, 2H), 3.18 (s, 3H), 3.14 (m, 2H), 1.97 (m, 2H), 1.82 (m, 2H), 1.75-1.65 (m, 4H), 1.56 (m, 2H).

### Beispiel No. E7-152

¹H-NMR (400 MHz, CDCl₃) d 7.21 (d, 2H), 7.19 (d, 2H), 7.07 (dd, 1H), 7.02 (d, 1H), 6.78 (d, 1H), 5.99 (br. s, 1H, NH), 5.88-5.81 (m, 3H), 5.24-5.20 (m, 2H), 4.36 (m, 2H), 4.26 (s, 2H), 3.02 (m, 2H), 2.61 (m, 2H), 2.37 (s, 3H).

### Beispiel No. G1-1

¹H-NMR (400 MHz, CDCl₃) d 7.16 (d, 1H), 7.14 (dd, 1H), 6.82 (d, 1H), 6.39 (br. s, 1H, NH), 3.22 (s, 3H), 2.99 (s, 3H), 1.38 (s, 6H).

### Beispiel No. G1-34:

¹H-NMR (400 MHz, CHCl₃) d 7.71 (m, 2H), 7.11 (m, 2H), 6.91 (m, 2H), 6.71 (d, 1H), 6.39 (br. s, 1H, NH), 3.18 (s, 3H), 1.29 (s, 6H).

### Beispiel No. G1-37:

¹H-NMR (400 MHz, CHCl₃) d 8.68 (d, 1H), 8.09 (m, 1H), 8.04 (d, 1H), 7.94 (m, 1H), 7.68-7.59 (m, 2H), 7.42 (m, 1H), 6.81 (m, 1H), 6.65 (br. s, 1H, NH), 6.59 (m, 1H), 6.57 (d, 1H), 3.10 (s, 3H), 1.12 (s, 6H).

### Beispiel No. G1-38:

¹H-NMR (400 MHz, CHCl₃) d 7.58 (d, 2H), 7.54 (d, 2H), 6.90 (m, 2H), 6.72 (d, 1H), 6.40 (br. s, 1H, NH), 3.18 (s, 3H), 1.30 (s, 6H).

### Beispiel No. G1-42:

¹H-NMR (400 MHz, CHCl₃) d 7.76 (d, 2H), 7.27 (d, 2H), 6.99 (dd, 1H), 6.88 (d, 1H), 6.82 (br. s, 1H, NH), 6.74 (d, 1H), 3.18 (s, 3H), 1.28 (s, 6H).

### Beispiel No. G1-56:

¹H-NMR (400 MHz, CHCl₃) d 7.97 (d, 1H), 7.90 (m, 1H), 7.83 (m, 1H), 6.94 (dd, 1H), 6.89 (d, 1H), 6.74 (d, 1H), 6.52 (br. s, 1H, NH), 3.19 (s, 3H), 1.30 (s, 6H).

### Beispiel No. G1-66:

¹H-NMR (400 MHz, CHCl₃) d 7.56 (m, 1H), 7.44 (m, 2H), 7.30 (m, 1H), 6.96 (dd, 1H), 6.90 (d, 1H), 6.72 (d, 1H), 6.53 (br. s, 1H, NH), 6.66-6.30 (t, 1H, OCHF₂), 3.18 (s, 3H), 1.28 (s, 6H).

### Beispiel No. G1-67

¹H-NMR (400 MHz, CDCl₃) d 7.65 (m, 1H), 7.53 (m, 1H), 7.49 (d, 1H), 7.40 (m, 1H), 6.95 (dd, 1H), 6.91 (d, 1H), 6.72 (d, 1H), 6.58 (br. s, 1 H, NH), 3.18 (s, 3H), 1.28 (s, 6H).

### Beispiel No. G1-152

¹H-NMR (400 MHz, CDCl₃) d 7.22 (d, 2H), 7.19 (d, 2H), 7.08 (dd, 1H), 6.99 (d, 1H), 6.82 (d, 1H), 6.16 (br. s, 1H, NH), 4.28 (s, 2H), 3.21 (s, 3H), 2.33 (s, 3H), 1.37 (s, 6H).

### Beispiel No. G1-153

¹H-NMR (400 MHz, CDCl₃) d 7.25 (m, 1H), 7.20 (m, 1H), 7.15 (m, 1H), 7.10 (m, 1H), 7.08 (dd, 1H), 6.95 (d, 1H), 6.80 (d, 1H), 6.14 (br. s, 1H, NH), 4.28 (s, 2H), 3.22 (s, 3H), 2.35 (s, 3H), 1.36 (s, 6H).

### Beispiel No. G1-158

¹H-NMR (400 MHz, CDCl₃) d 7.30 (m, 2H), 7.08-7.02 (m, 3H), 6.98 (d, 1H), 6.80 (d, 1H), 6.33 (br. s, 1 H, NH), 4.28 (s, 2H), 3.21 (s, 3H), 1.37 (s, 6H).

### Beispiel No G1-159:

¹H-NMR (400 MHz, CDCl₃) d 7.35 (m, 1H), 7.11 (m, 3H), 7.04 (m, 1H), 6.98 (d, 1H), 6.81 (d, 1H), 6.33 (br. s, 1H, NH), 4.30 (s, 2H), 3.22 (s, 3H), 1.37 (s, 6H).

### Beispiel No. G1-161:

¹H-NMR (400 MHz, CHCl₃) d 7.48 (m, 1H), 7.37 (m, 1 H), 7.18 (m, 1H), 7.08 (m, 2H), 6.99 (d, 1H), 6.78 (d, 1H), 6.15 (br. s, 1H, NH), 4.41 (s, 2H), 3.21 (s, 3H), 1.36 (s, 6H).

### Beispiel No. G1-165

¹H-NMR (400 MHz, CDCl₃) d 7.34 (d, 2H), 7.26 (d, 2H), 7.06 (dd, 1H), 6.98 (d, 1H), 6.80 (d, 1H), 6.32 (br. s, 1H, NH), 4.28 (s, 2H), 3.22 (s, 3H), 1.37 (s, 6H).

### Beispiel No. G1-166

¹H-NMR (400 MHz, CDCl₃) d 7.38 (m, 1H), 7.33 (m, 1H), 7.30-7.23 (m, 2H), 7.10 (dd, 1H), 6.98 (d, 1H), 6.82 (d, 1H), 6.22 (br. s, H, NH), 4.28 (s, 2H), 3.22 (s, 3H), 1.37 (s, 6H).

### Beispiel No. G1-167

¹H-NMR (400 MHz, CDCl₃) d 7.44 (d, 1H), 7.37 (d, 1H), 7.19 (dd, 2H), 7.10 (dd, 1H), 7.01 (d, 1H), 6.81 (d, 1H), 6.42 (br. s, 1H, NH), 4.26 (s, 2H), 3.22 (s, 3H), 1.37 (s, 6H).

### Beispiel No. G1-169

¹H-NMR (400 MHz, CDCl₃) d 7.46 (d, 1H), 7.37 (d, 1H), 7.24 (dd, 2H), 7.07 (dd, 1H), 7.01 (d, 1H), 6.76 (d, 1H), 6.48 (br. s, 1H, NH), 4.54 (s, 2H), 3.20 (s, 3H), 1.35 (s, 6H).

### Beispiel No. G1-171

¹H-NMR (400 MHz, CDCl₃) d 7.38 (m, 1H), 7.21 (m, 2H), 7.12 (dd, 1H), 7.00 (d, 1H), 6.83 (d, 1H), 6.28 (br. s, 1H, NH), 4.24 (s, 2H), 3.22 (s, 3H), 1.38 (s, 6H).

### Beispiel No. G1-172

¹H-NMR (400 MHz, CDCl₃) d 7.51 (d, 2H), 7.20 (d, 2H), 7.04 (dd, 1H), 6.98 (d, 1H), 6.80 (d, 1H), 6.22 (br. s, 1 H, NH), 4.26 (s, 2H), 3.22 (s, 3H), 1.37 (s, 6H).

### Beispiel No. G1-175

¹H-NMR (400 MHz, CDCl₃) d 8.24 (d, 2H), 7.53 (d, 2H), 7.06 (m, 2H), 6.83 (d, 1H), 6.18 (br. s, 1H, NH), 4.40 (s, 2H), 3.22 (s, 3H), 1.38 (s, 6H).

### Beispiel No. G1-178

¹H-NMR (400 MHz, CDCl₃) d 7.63 (m, 1H), 7.47 (m, 1H), 7.07 (dd, 1H), 7.04 (d, 1H), 6.80 (d, 1H), 6.31 (br. s, 1H, NH), 4.36 (s, 2H), 3.22 (s, 3H), 1.37 (s, 6H).

### Beispiel No. G1-179

¹H-NMR (400 MHz, CDCl₃) d 7.66 (d, 1H), 7.58 (m, 1H), 7.52 (m, 2H), 7.08 (dd, 1H), 7.03 (d, 1H), 6.81 (d, 1H), 6.38 (br. s, 1H, NH), 4.36 (s, 2H), 3.22 (s, 3H), 1.36 (s, 6H).

### Beispiel No. G1-180

¹H-NMR (400 MHz, CDCl₃) d 7.75 (d, 1H), 7.68 (d, 1H), 7.55 (m, 1H), 7.47 (m, 1H), 7.02 (dd, 1H), 6.99 (d, 1H), 6.75 (d, 1H), 6.31 (br. s, 1H, NH), 4.59 (s, 2H), 3.20 (s, 3H), 1.36 (s, 6H).

### Beispiel No. G1-181

¹H-NMR (400 MHz, CDCl₃) d 7.68 (d, 2H), 7.47 (d, 2H), 7.05 (m, 2H), 6.82 (d, 1H), 6.18 (br. s, 1H, NH), 4.35 (s, 2H), 3.22 (s, 3H), 1.38 (s, 6H).

### Beispiel No. G1-182

¹H-NMR (400 MHz, CDCl₃) d 7.69 (m, 1H), 7.60 (m, 2H), 7.52 (m, 1H), 7.08 (dd, 1H), 7.04 (d, 1H), 6.84 (d, 1H), 6.17 (br. s, 1H, NH), 4.33 (s, 2H), 3.23 (s, 3H), 1.38 (s, 6H).

### Beispiel No. G1-184

¹H-NMR (400 MHz, CDCl₃) d 7.37 (d, 2H), 7.21 (d, 2H), 7.06 (dd, 1H), 7.03 (d, 1H), 6.80 (d, 1H), 6.38 (br. s, 1H, NH), 4.31 (s, 2H), 3.22 (s, 3H), 1.37 (s, 6H).

### Beispiel No. G1-190

¹H-NMR (400 MHz, CDCl₃) d 8.03 (d, 2H), 7.41 (d, 2H), 7.08 (dd, 1H), 7.00 (d, 1H), 6.81 (d, 1H), 6.41 (br. s, 1H, NH), 4.36 (s, 2H), 3.92 (s, 3H), 3.22 (s, 3H), 1.36 (s, 6H).

### Beispiel No. G1-191

¹H-NMR (400 MHz, CDCl₃) d 8.05 (d, 1H), 7.98 (m, 1H), 7.57 (m, 1H), 7.46 (m, 1H), 7.11 (dd, 1H), 7.04 (d, 1H), 6.82 (d, 1H), 6.32 (br. s, 1H, NH), 4.35 (s, 2H), 3.92 (s, 3H), 3.22 (s, 3H), 1.37 (s, 6H).

### Beispiel No. G1-192

¹H-NMR (400 MHz, CDCl₃) d 8.03 (d, 2H), 7.41 (d, 2H), 7.08 (dd, 1H), 6.98 (d, 1H), 6.80 (d, 1H), 6.38 (br. s, 1H, NH), 4.39 (q, 2H), 4.36 (s, 2H), 3.21 (s, 3H), 1.40 (t, 3H), 1.36 (s, 6H).

### Beispiel No. G1-290:

¹H-NMR (400 MHz, CDCl₃) δ 7.15 (d, 4H), 7.13 (d, 4H), 6.71 (d, 1H), 6.63 (m, 2H), 4.52 (s, 4H), 3.14 (s, 3H), 2.33 (s, 6H), 1.28 (s, 6H),

### Beispiel No. G1-291

¹H-NMR (400 MHz, CDCl₃) d 7.29 (d, 2H), 7.13 (d, 2H), 7.02 (dd, 1H), 6.94 (d, 1H), 6.78 (d, 1H), 6.06 (br. s, 1H, NH), 3.30 (m, 2H), 3.20 (s, 3H), 3.12 (m, 2H), 1.36 (s, 6H).

### Beispiel No. G1-292

¹H-NMR (400 MHz, CDCl₃) d 7.58 (d, 2H), 7.31 (d, 2H), 7.03 (m, 2H), 6.79 (d, 1H), 6.32 (br. s, 1H, NH), 3.33 (m, 2H), 3.23 (s, 3H), 3.13 (m, 2H), 1.36 (s, 6H).

### Beispiel No. G1-332

¹H-NMR (400 MHz, CDCl₃) d 7.40 (d, 2H), 7.26 (d, 2H), 7.08 (dd, 1H), 7.05 (d, 1H), 6.80 (d, 1H), 6.14 (br. s, 1H, NH), 4.27 (s, 2H), 3.22 (s, 3H), 1.38 (s, 6H), 1.32 (s, 9H).

### Beispiel No. G16-152:

¹H-NMR (400 MHz, d₆-DMSO) δ 9.46 (br. s, 1H, NH), 7.16 (m, 4H), 6.84 (m, 1H), 6.78 (m, 1H), 4.33 (s, 2H), 3.38 (s, 3H), 2.52 (s, 3H), 2.29 (s, 3H), 1.21 (s, 6H).

### Beispiel No. G16-165:

¹H-NMR (400 MHz, CDCl₃) δ 7.34 (d, 2H), 7.26 (d, 2H), 6.78 (m, 1H), 6.74 (m, 1H), 6.20 (br. s, 1H, NH), 4.28 (s, 2H), 3.49 (s, 3H), 2.61 (s, 3H), 1.34 (s, 6H).

### Beispiel No. G16-181:

¹H-NMR (400 MHz, CDCl₃) δ 7.68 (d, 2H), 7.46 (d, 2H), 6.84 (m, 1H), 6.77 (m, 1H), 6.21 (br. s, 1H, NH), 4.35 (s, 2H), 3.50 (s, 3H), 2.62 (s, 3H), 1.35 (s, 6H).

### Beispiel No. H1-54:

¹H-NMR (400 MHz, CDCl₃ δ, ppm) 7.81 (d, 2H), 7.76 (d, 2H), 7.08 (d, 1H), 6.94 (dd, 1H), 6.74 (d, 1H), 6.45 (br. s, 1H, NH), 4.25 (m, 2H), 3.85 (m, 2H), 3.18 (s, 3H), 1.78 (m,4H).

### Beispiel No. H1-152:

¹H-NMR (400 MHz, CDCl₃ δ, ppm) 7.21 (d, 2H), 7.19 (d, 2H), 7.11 (dd, 1H), 7.08 (d, 1H), 6.81 (d, 1H), 6.09 (br. s, 1H, NH), 4.28 (s, 2H), 4.25 (m, 2H), 3.91 (m, 2H), 3.21 (s, 3H), 1.85 (m, 4H).

### Beispiel No. H1-165:

¹H-NMR (400 MHz, CDCl₃ δ, ppm) 7.37 (d, 2H), 7.29 (d, 2H), 7.14 (d, 1H), 7.07 (dd, 1H), 6.81 (d, 1H), 6.16 (br. s, 1H, NH), 4.29 (s, 2H), 4.27 (m, 2H), 3.90 (m, 2H), 3.21 (s, 3H), 1.84 (m, 4H).

### Beispiel No. H1-166:

¹H-NMR (400 MHz, CDCl₃ δ, ppm) 7.39-7.25 (m, 4H), 7.11 (d, 1H), 7.10 (dd, 1H), 6.82 (d, 1H), 6.18 (br. s, 1H, NH), 4.29 (s, 2H), 4.28 (m, 2H), 3.91 (m, 2H), 3.21 (s, 3H), 1.84 (m, 4H).

### Beispiel No. H1-182:

¹H-NMR (400 MHz, CDCl₃ δ, ppm) 7.69 (m, 1H), 7.64 (m, 1H), 7.57 (m, 1H), 7.52 (m, 1H), 7.20 (d, 1H), 7.11 (dd, 1H), 6.84 (d, 1H), 6.35 (br. s, 1H, NH), 4.35 (s, 2H), 4.27 (m, 2H), 3.90 (m, 2H), 3.22 (s, 3H), 1.85 (m, 4H).

### Beispiel No. H1-291:

¹H-NMR (400 MHz, CDCl₃ δ, ppm) 7.31 (d, 2H), 7.14 (d, 1H), 7.12 (d, 2H), 7.03 (dd, 1H), 6.79 (d, 1H), 6.16 (br. s, 1H, NH), 4.27 (m, 2H), 3.90 (m, 2H), 3.31 (m, 2H), 3.19 (s, 3H), 3.13 (m, 2H), 1.84 (m, 4H).

### Beispiel No. 11-152:

¹H-NMR (400 MHz, d₆-DMSO δ, ppm) 9.54 (s, 1H, NH), 7.18 (d, 2H), 7.13 (d, 2H), 7.10 (m, 2H), 6.94 (d, 1H), 4.31 (s, 2H), 3.55 (s, 2H), 3.10 (s, 3H), 2.29 (s, 3H).

### Beispiel No. 11-165:

¹H-NMR (400 MHz, d₆-DMSO δ, ppm) 9.60 (s, 1H, NH), 7.43 (d, 2H), 7.29 (d, 2H), 7.10 (m, 2H), 6.93 (d, 1H), 4.40 (s, 2H), 3.55 (s, 2H), 3.10 (s, 3H).

### Beispiel No. 11-181:

¹H-NMR (400 MHz, d₆-DMSO δ, ppm) 9.68 (s, 1H, NH), 7.84 (d, 2H), 7.48 (d, 2H), 7.10 (m, 2H), 6.94 (d, 1H), 4.53 (s, 2H), 3.55 (s, 2H), 3.11 (s, 3H).

Weiterer Gegenstand der vorliegenden Erfindung ist die erfindungsgemäße Verwendung mindestens eines substituierten Dihydrooxindolylsulfonamids der allgemeinen Formel (I), sowie von beliebigen Mischungen dieser substituierten Dihydrooxindolylsulfonamide der allgemeinen Formel (I) mit weiteren agrochemischen Wirkstoffen, zur Steigerung der Widerstandsfähigkeit von Pflanzen gegenüber abiotischen Stressfaktoren, bevorzugt Trockenstress, sowie zur Stärkung des Pflanzenwachstums und/oder zur Erhöhung des Pflanzenertrags.

Weiterer Gegenstand der vorliegenden Erfindung ist eine Sprühlösung zur Behandlung von Pflanzen, enthaltend eine zur Steigerung der Widerstandsfähigkeit von Pflanzen gegenüber abiotischen Stressfaktoren wirksame Menge von mindestens einer Verbindung, ausgewählt aus der Gruppe, bestehend aus den substituierten Dihydrooxindolylsulfonamiden, der allgemeinen Formel (I). Zu den dabei relativierbaren abiotischen Streßbedingungen können zum Beispiel Hitze, Dürre, Kälte- und Trockenstress (Stress verursacht durch Trockenheit und/oder Wassermangel), osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen zählen.

In einer Ausführungsform kann beispielsweise vorgesehen sein, dass die erfindungsgemäßer Verwendung vorgesehenen Verbindungen, d. h. die entsprechenden substituierten Dihydrooxindolylsulfonamide der allgemeinen Formel (I), durch eine Sprühapplikation auf entsprechende zu behandelnde Pflanzen oder Pflanzenteile aufgebracht werden. Die erfindungsgemäß vorgesehene Verwendung der Verbindungen der allgemeinen Formel (I) oder deren Salze erfolgt vorzugsweise mit einer Dosierung zwischen 0,00005 und 3 kg/ha, besonders bevorzugt zwischen 0,0001 und 2 kg/ha, insbesondere bevorzugt zwischen 0,0005 und 1 kg/ha, im Speziellen bevorzugt zwischen 0,001 und 0,25 kg/ha.

Unter der Bezeichnung Resistenz bzw. Widerstandsfähigkeit gegenüber abiotischem Stress werden im Rahmen der vorliegenden Erfindung verschiedenartige Vorteile für Pflanzen verstanden. Solche vorteilhaften Eigenschaften äußern sich beispielsweise in den nachfolgend genannten verbesserten Pflanzencharakteristika: verbessertes Wurzelwachstum hinsichtlich Oberfläche und Tiefe, vermehrte Ausläuferbildung oder Bestockung, stärkere und produktivere Ausläufer und Bestockungstriebe, Verbesserung des Sproßwachstums, erhöhte Standfestigkeit, vergrößerte Sprossbasisdurchmesser, vergrößerte Blattfläche, höhere Erträge an Nähr- und Inhaltsstoffen, wie z.B. Kohlenhydrate, Fette, Öle, Proteine, Vitamine, Mineralstoffe, ätherische Öle, Farbstoffe, Fasern, bessere Faserqualität, früheres Blühen, gesteigerte Blütenanzahl, reduzierter Gehalt an toxischen Produkten wie Mycotoxine, reduzierter Gehalt an Rückständen oder unvorteilhaften Bestandteilen jeglicher Art oder bessere Verdaulichkeit, verbesserte Lagerstabilität des Erntegutes, verbesserter Toleranz gegenüber unvorteilhaften Temperaturen, verbesserter Toleranz gegenüber Dürre und Trockenheit, wie auch Sauerstoffmangel durch Wasserüberschuß, verbesserte Toleranz gegenüber erhöhten Salzgehalten in Böden und Wasser, gesteigerte Toleranz gegenüber Ozonstress, verbesserte Verträglichkeit gegenüber Herbiziden und anderen Pflanzenbehandlungsmitteln, verbesserte Wasseraufnahme und Photosyntheseleistung, vorteilhafte Pflanzeneigenschaften, wie beispielsweise Beschleunigung der Reifung, gleichmäßigere Abreife, größere Anziehungskraft für Nützlinge, verbesserte Bestäubung oder andere Vorteile, die einem Fachmann durchaus bekannt sind.

Insbesondere zeigt die erfindungsgemäße Verwendung einer oder mehrerer Verbindungen der allgemeinen Formel (I) in der Sprühapplikation auf Pflanzen und Pflanzenteilen die beschriebenen Vorteile. Die kombinierte Verwendung von substituierten Dihydrooxindolylsulfonamiden der allgemeinen Formel (I) mit gentechnisch veränderten Sorten in Bezug auf erhöhte abiotische Stresstoleranz ist darüber hinaus ebenfalls möglich.

Die weiter oben genannten verschiedenartigen Vorteile für Pflanzen lassen sich bekannterweise partiell zusammenfassen und mit allgemein gültigen Begriffen belegen. Soche Begriffe sind beispielsweise die nachfolgend aufgeführten Bezeichnungen: phytotonischer Effekt, Widerstandsfähigkeit gegenüber Stressfaktoren, weniger Pflanzenstress, Pflanzengesundheit, gesunde Pflanzen, Pflanzenfitness, ("Plant Fitness"), "Plant Wellness", "Plant Concept", "Vigor Effect", "Stress Shield", Schutzschild, "Crop Health", "Crop Health Properties", "Crop Health Products", "Crop Health Management", "Crop Health Therapy", "Plant Health", Plant Health Properties", Plant Health Products", "Plant Health Management", "Plant Health Therapy", Grünungseffekt ("Greening Effect" oder "Re-greening Effect"), "Freshness" oder andere Begriffe, die einem Fachmann durchaus bekannt sind.

Im Rahmen der vorliegenden Erfindung wird unter einem guten Effekt auf die Widerstandsfähigkeit gegenüber abiotischem Stress nicht beschränkend
- mindestens ein um im Allgemeinen 3 %, insbesondere größer als 5 % besonders bevorzugt größer als 10 % verbessertes Auflaufen,
- mindestens einen im Allgemeinen 3 %, insbesondere größer als 5 % besonders bevorzugt größer als 10 % gesteigerten Ertrag,
- mindestens eine um im Allgemeinen 3 %, insbesondere größer als 5 % besonders bevorzugt größer als 10 % verbesserte Wurzelentwicklung,
- mindestens eine um im Allgemeinen 3 %, insbesondere größer als 5 % besonders bevorzugt größer als 10 % ansteigende Sproßgröße,
- mindestens eine um im Allgemeinen 3 %, insbesondere größer als 5 % besonders bevorzugt größer als 10 % vergrößerte Blattfläche,
- mindestens eine um im Allgemeinen 3 %, insbesondere größer als 5 % besonders bevorzugt größer als 10 % verbesserte Photosyntheseleistung und/oder
- mindestens eine um im Allgemeinen 3 %, insbesondere größer als 5 % besonders bevorzugt größer als 10 % verbesserte Blütenausbildung
verstanden, wobei die Effekte einzeln oder aber in beliebiger Kombination von zwei oder mehreren Effekten auftreten können.

Weiterer Gegenstand der vorliegenden Erfindung ist eine Sprühlösung zur Behandlung von Pflanzen, enthaltend eine zur Steigerung der Widerstandsfähigkeit von Pflanzen gegenüber abiotischen Stressfaktoren wirksame Menge von mindestens einer Verbindung aus der Gruppe der substituierten Dihydrooxindolylsulfonamide der allgemeinen Formel (I). Die Sprühlösung kann andere übliche Bestandteile aufweisen, wie Lösungsmittel, Formulierhilfsstoffe, insbesondere Wasser, enthalten. Weitere Bestandteile können unter anderem agrochemische Wirkstoffe sein, welche unten noch weiter beschrieben werden.

Weiterer Gegenstand der vorliegenden Erfindung ist die erfindungsgemäße Verwendung von entsprechenden Sprühlösungen zur Steigerung der Widerstandsfähigkeit von Pflanzen gegenüber abiotischen Stressfaktoren. Die nachfolgenden Ausführungen gelten sowohl für die erfindungsgemäße Verwendung einer oder mehrerer Verbindungen der allgemeinen Formel (I) an sich als auch für die entsprechenden Sprühlösungen.

Bevorzugt ist die erfindungsgemäße Verwendung von Verbindungen der allgemeinen Formel (I) auf Pflanzen aus der Gruppe der Nutzpflanzen, Zierpflanzen, Rasenarten, allgemein genutzte Bäume, die in öffentlichen und privaten Bereichen als Zierpflanzen Verwendungen finden, und Forstbestand. Der Forstbestand umfasst Bäume für die Herstellung von Holz, Zellstoff, Papier und Produkten die aus Teilen der Bäume hergestellt werden. Der Begriff Nutzpflanzen, wie hier verwendet, bezeichnet Kulturpflanzen, die als Pflanzen für die Gewinnung von Nahrungsmitteln, Futtermitteln, Treibstoffe oder für technische Zwecke eingesetzt werden.

Zu den Nutzpflanzen zählen z. B. folgende Pflanzenarten: Triticale, Durum (Hartweizen), Turf, Reben, Getreide, beispielsweise Weizen, Gerste, Roggen, Hafer, Reis, Mais und Hirse; Rüben, beispielsweise Zuckerrüben und Futterrüben; Früchte, beispielsweise Kernobst, Steinobst und Beerenobst, beispielsweise Äpfel, Birnen, Pflaumen, Pfirsiche, Mandeln, Kirschen und Beeren, z. B. Erdbeeren, Himbeeren, Brombeeren; Hülsenfrüchte, beispielsweise Bohnen, Linsen, Erbsen und Sojabohnen; Ölkulturen, beispielsweise Raps, Senf, Mohn, Oliven, Sonnenblumen, Kokos, Castorölpflanzen, Kakaobohnen und Erdnüsse; Gurkengewächse, beispielsweise Kürbis, Gurken und Melonen; Fasergewächse, beispielsweise Baumwolle, Flachs, Hanf und Jute; Citrusfrüchte, beispielsweise Orangen, Zitronen, Pampelmusen und Mandarinen; Gemüsesorten, beispielsweise Spinat, (Kopf)-Salat, Spargel, Kohlarten, Möhren, Zwiebeln, Tomaten, Kartoffeln und Paprika; Lorbeergewächse, beispielsweise Avocado, Cinnamomum, Kampfer, oder ebenso Pflanzen wie Tabak, Nüsse, Kaffee, Aubergine, Zuckerrohr, Tee, Pfeffer, Weinreben, Hopfen, Bananen, Naturkautschukgewächse sowie Zierpflanzen, beispielsweise Blumen, Sträucher, Laubbäume und Nadelbäume wie Koniferen. Diese Aufzählung stellt keine Limitierung dar.

Als besonders geeignete Zielkulturen für die Anwendung des erfindungsgemäßen Verfahrens sind folgende Pflanzen anzusehen: Hafer, Roggen, Triticale, Durum, Baumwolle, Aubergine, Turf, Kernobst, Steinobst, Beerenobst, Mais, Weizen, Gerste, Gurke, Tabak, Reben, Reis, Getreide, Birne, Pfeffer, Bohnen, Sojabohnen, Raps, Tomate, Paprika, Melonen, Kohl, Kartoffel und Apfel.

Als Bäume, die entsprechend dem erfindungsgemäßen Verfahren verbessert werden können, seien beispielhaft genannt: Abies sp., Eucalyptus sp., Picea sp., Pinus sp., Aesculus sp., Platanus sp., Tilia sp., Acer sp., Tsuga sp., Fraxinus sp., Sorbus sp., Betula sp., Crataegus sp., Ulmus sp., Quercus sp., Fagus sp., Salix sp., Populus sp..

Als bevorzugte Bäume, die entsprechend dem erfindungsgemäßen Verfahren verbessert werden können, können genannt werden: Aus der Baumart Aesculus: A. hippocastanum, A. pariflora, A. carnea; aus der Baumart Platanus: P. aceriflora, P. occidentalis, P. racemosa; aus der Baumart Picea: P. abies; aus der Baumart Pinus: P. radiate, P. ponderosa, P. contorta, P. sylvestre, P. elliottii, P. montecola, P. albicaulis, P. resinosa, P. palustris, P. taeda, P. flexilis, P. jeffregi, P. baksiana, P. strobes; aus der Baumart Eucalyptus: E. grandis, E. globulus, E. camadentis, E. nitens, E. obliqua, E. regnans, E. pilularus.

Als besonders bevorzugte Bäume, die entsprechend dem erfindungsgemäßen Verfahren verbessert werden können, können genannt werden: Aus der Baumart Pinus: P. radiate, P. ponderosa, P. contorta, P. sylvestre, P. strobes; aus der Baumart Eucalyptus: E. grandis, E. globulus und E. camadentis.

Als besonders bevorzugte Bäume, die entsprechend dem erfindungsgemäßen Verfahren verbessert werden können, können genannt werden: Rosskastanie, Platanengewächs, Linde und Ahornbaum.

Die vorliegende Erfindung kann auch an beliebigen Rasenarten ("turfgrasses") durchgeführt werden, einschließlich "cool season turfgrasses" und "warm season turfgrasses". Beispiele für Rasenarten für die kalte Jahreszeit sind Blaugräser ("blue grasses"; Poa spp.), wie "Kentucky bluegrass" (Poa pratensis L.), "rough bluegrass" (Poa trivialis L.), "Canada bluegrass" (Poa compressa L.), "annual bluegrass" (Poa annua L.), "upland bluegrass" (Poa glaucantha Gaudin), "wood bluegrass" (Poa nemoralis L.) und "bulbous bluegrass" (Poa bulbosa L.); Straussgräser ("Bentgrass", Agrostis spp.), wie "creeping bentgrass" (Agrostis palustris Huds.), "colonial bentgrass" (Agrostis tenuis Sibth.), "velvet bentgrass" (Agrostis canina L.), "South German Mixed Bentgrass" (Agrostis spp. einschließlich Agrostis tenius Sibth., Agrostis canina L., und Agrostis palustris Huds.), und "redtop" (Agrostis alba L.);
Schwingel ("Fescues", Festucu spp.), wie "red fescue" (Festuca rubra L. spp. rubra), "creeping fescue" (Festuca rubra L.), "chewings fescue" (Festuca rubra commutata Gaud.), "sheep fescue" (Festuca ovina L.), "hard fescue" (Festuca longifolia Thuill.), "hair fescue" (Festucu capillata Lam.), "tall fescue" (Festuca arundinacea Schreb.) und "meadow fescue" (Festuca elanor L.);
Lolch ("ryegrasses", Lolium spp.), wie "annual ryegrass" (Lolium multiflorum Lam.), "perennial ryegrass" (Lolium perenne L.) und "italian ryegrass" (Lolium multiflorum Lam.);
und Weizengräser ("wheatgrasses", Agropyron spp..), wie "fairway wheatgrass" (Agropyron cristatum (L.) Gaertn.), "crested wheatgrass" (Agropyron desertorum (Fisch.) Schult.) und "western wheatgrass" (Agropyron smithii Rydb.).

Beispiele für weitere "cool season turfgrasses" sind "beachgrass" (Ammophila breviligulata Fern.), "smooth bromegrass" (Bromus inermis Leyss.), Schilf ("cattails") wie "Timothy" (Phleum pratense L.), "sand cattail" (Phleum subulatum L.), "orchardgrass" (Dactylis glomerata L.), "weeping alkaligrass" (Puccinellia distans (L.) Parl.) und "crested dog's-tail" (Cynosurus cristatus L.).

Beispiele für "warm season turfgrasses" sind "Bermudagrass" (Cynodon spp. L. C. Rich), "zoysiagrass" (Zoysia spp. Willd.), "St. Augustine grass" (Stenotaphrum secundatum Walt Kuntze), "centipedegrass" (Eremochloa ophiuroides Munro Hack.), "carpetgrass" (Axonopus affinis Chase), "Bahia grass" (Paspalum notatum Flugge), "Kikuyugrass" (Pennisetum clandestinum Hochst. ex Chiov.), "buffalo grass" (Buchloe dactyloids (Nutt.) Engelm.), "Blue gramma" (Bouteloua gracilis (H.B.K.) Lag. ex Griffiths), "seashore paspalum" (Paspalum vaginatum Swartz) und "sideoats grama" (Bouteloua curtipendula (Michx. Torr.). "Cool season turfgrasses" sind für die erfindungsgemäße Verwendung im Allgemeinen bevorzugt. Besonders bevorzugt sind Blaugras, Straussgras und "redtop", Schwingel und Lolch. Straussgras ist insbesondere bevorzugt.

Besonders bevorzugt werden mit den Verbindungen der allgemeinen Formel (I) Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder mit Hilfe rekombinanter DNA-Techniken, gezüchtet worden sind. Kulturpflanzen können demnach Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten.

Das erfindungsgemäße Behandlungsverfahren kann somit auch für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, dass es ein interessierendes Protein oder Polypeptid exprimiert oder dasses ein anderes Gen, das in der Pflanze vorliegt bzw. andere Gene, die in der Pflanze vorliegen, herunterreguliert oder abschaltet (zum Beispiel mittels Antisense-Technologie, Co-suppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

Zu Pflanzen und Pflanzensorten, die vorzugsweise mit den Verbindungen der allgemeinen Formel (I) behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls mit den Verbindungen der allgemeinen Formel (I) behandelt werden können, sind solche Pflanzen, die gegen einen oder mehrere abiotische Streßfaktoren resistent sind. Zu den abiotischen Streßbedingungen können zum Beispiel Hitze, Dürre, Kälte- und Trockenstress" osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls mit den Verbindungen der allgemeinen Formel (I) behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die ebenfalls mit den Verbindungen der allgemeinen Formel (I) behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, dass man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, dass die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, dass die Pollenfertilität in Hybridpflanzen, die die für die Pollensterilität verantwortlichen genetischen Determinanten enthalten, völlig restoriert wird. Dies kann erreicht werden, indem sichergestellt wird, dass die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben (WO 92/005251, WO 95/009910, WO 98/27806, WO 05/002324, WO 06/021972 und US 6,229,072). Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden (z. B. WO 91/002069).

Pflanzen oder Pflanzensorten (die mit Methoden der Pflanzenbiotechnologie, wie der Gentechnik, erhalten werden), die ebenfalls mit den Verbindungen der allgemeinen Formel (I) behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium Salmonella typhimurium (Comai et al., Science (1983), 221, 370-371), das CP4-Gen des Bakteriums Agrobacterium sp. (Barry et al., Curr. Topics Plant Physiol. (1992), 7, 139-145), die Gene, die für eine EPSPS aus der Petunie (Shah et al., Science (1986), 233, 478-481), für eine EPSPS aus der Tomate (Gasser et al., J. Biol. Chem. (1988), 263, 4280-4289) oder für eine EPSPS aus Eleusine (WO 01/66704) kodieren. Es kann sich auch um eine mutierte EPSPS handeln, wie sie zum Beispiel in EP-A 0837944, WO 00/066746, WO 00/066747 oder WO 02/026995 beschrieben ist. Glyphosatetolerante Pflanzen können auch dadurch erhalten werden, dass man ein Gen exprimiert, das für ein Glyphosate-Oxidoreduktase-Enzym, wie es in US 5,776,760 und US 5,463,175 beschrieben ist, kodiert. Glyphosatetolerante Pflanzen können auch dadurch erhalten werden, dass man ein Gen exprimiert, das für ein Glyphosate-acetyltransferase-Enzym, wie es in z. B. WO 02/036782, WO 03/092360, WO 05/012515 und WO 07/024782 beschrieben ist, kodiert. Glyphosatetolerante Pflanzen können auch dadurch erhalten werden, dass man Pflanzen, die natürlich vorkommende Mutationen der oben erwähnten Gene, wie sie zum Beispiel in WO 01/024615 oder WO 03/013226 beschrieben sind, enthalten, selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, dass man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind zum Beispiel in US 5,561,236; US 5,648,477; US 5,646,024; US 5,273,894; US 5,637,489; US 5,276,268; US 5,739,082; US 5,908,810 und US 7,112,665 beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym gemäß WO 96/038567, WO 99/024585 und WO 99/024586 kodiert, transformiert werden. Eine Toleranz gegenüber HPPD-Hemmern kann auch dadurch erzielt werden, dass man Pflanzen mit Genen transformiert, die für gewisse Enzyme kodieren, die die Bildung von Homogentisat trotz Hemmung des nativen HPPD-Enzyms durch den HPPD-Hemmer ermöglichen. Solche Pflanzen und Gene sind in WO 99/034008 und WO 2002/36787 beschrieben. Die Toleranz von Pflanzen gegenüber HPPD-Hemmern kann auch dadurch verbessert werden, dass man Pflanzen zusätzlich zu einem Gen, das für ein HPPD-tolerantes Enzym kodiert, mit einem Gen transformiert, das für ein Prephenatdehydrogenase-Enzym kodiert, wie dies in WO 2004/024928 beschrieben ist.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, dass verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen, wie dies zum Beispiel bei Tranel und Wright, Weed Science (2002), 50, 700-712, jedoch auch in US 5,605,011, US 5,378,824, US 5,141,870 und US 5,013,659, beschrieben ist. Die Herstellung von sulfonylharnstofftoleranten Pflanzen und imidazolinontoleranten Pflanzen ist in US 5,605,011; US 5,013,659; US 5,141,870; US 5,767,361; US 5,731,180; US 5,304,732; US 4,761,373; US 5,331,107; US 5,928,937; und US 5,378,824; sowie in der internationalen Veröffentlichung WO 96/033270 beschrieben. Weitere imidazolinontolerante Pflanzen sind auch in z. B. WO 2004/040012, WO 2004/106529, WO 2005/020673, WO 2005/093093, WO 2006/007373, WO 2006/015376, WO 2006/024351 und WO 2006/060634 beschrieben. Weitere sulfonylharnstoff- und imidazolinontolerante Pflanzen sind auch in z.B. WO 2007/024782 beschrieben.

Weitere Pflanzen, die gegenüber ALS-Inhibitoren, insbesondere gegenüber Imidazolinonen, Sulfonylharnstoffen und/oder Sulfamoylcarbonyltriazolinonen tolerant sind, können durch induzierte Mutagenese, Selektion in Zellkulturen in Gegenwart des Herbizids oder durch Mutationszüchtung erhalten werden, wie dies zum Beispiel für die Sojabohne in US 5,084,082, für Reis in WO 97/41218, für die Zuckerrübe in US 5,773,702 und WO 99/057965, für Salat in US 5,198,599 oder für die Sonnenblume in WO 2001/065922 beschrieben ist.

Pflanzen oder Pflanzensorten (die nach Methoden der pflanzlichen Biotechnologie, wie der Gentechnik, erhalten wurden), die ebenfalls mit den Verbindungen der allgemeinen Formel (I) behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus Bacillus thuringiensis oder einen insektiziden Teil davon, wie die insektiziden Kristallproteine, die von Crickmore et al., Microbiology and Molecular Biology Reviews (1998), 62, 807-813, zusammengestellt wurden, von Crickmore et al. (2005) in der Bacillus thuringiensis-Toxinnomenklatur aktualisiert (online bei: http://www.lifesci.sussex.ac.uk/Home/Neil_Crickmore/Bt/), oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus Bacillus thuringiensis oder einen Teil davon, der in Gegenwart eines zweiten, anderen Kristallproteins als Bacillus thuringiensis oder eines Teils davon insektizid wirkt, wie das binäre Toxin, das aus den Kristallproteinen Cy34 und Cy35 besteht (Moellenbeck et al., Nat. Biotechnol. (2001), 19, 668-72; Schnepf et al., Applied Environm. Microb. (2006), 71, 1765-1774); oder
3) ein insektizides Hybridprotein, das Teile von zwei unterschiedlichen insektiziden Kristallproteinen aus Bacillus thuringiensis umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604; oder
5) ein insektizides sezerniertes Protein aus Bacillus thuringiensis oder Bacillus cereus oder einen insektiziden Teil davon, wie die vegetativ wirkenden insektentoxischen Proteine (vegetative insecticidal proteins, VIP), die unter folgendem Link angeführt sind, z. B. Proteine der Proteinklasse VIP3Aa: http://www.lifesci.sussex.ac.uk/Home/Neil_Crickmore/Bt/vip.html oder
6) ein sezerniertes Protein aus Bacillus thuringiensis oder Bacillus cereus, das in Gegenwart eines zweiten sezernierten Proteins aus Bacillus thuringiensis oder B. cereus insektizid wirkt, wie das binäre Toxin, das aus den Proteinen VIP1A und VIP2A besteht (WO 94/21795); oder
7) ein insektizides Hybridprotein, das Teile von verschiedenen sezernierten Proteinen von Bacillus thuringiensis oder Bacillus cereus umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, dass man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls mit den Verbindungen der allgemeinen Formel (I) behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag, wie dies in WO 2000/004173 oder EP 04077984.5 oder EP 06009836.5 beschrieben ist.
b. Pflanzen, die ein streßtoleranzförderndes Transgen enthalten, das die Expression und/oder Aktivität der für PARG kodierenden Gene der Pflanzen oder Pflanzenzellen zu reduzieren vermag, wie dies z.B. in WO 2004/090140 beschrieben ist;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotid-adenyltransferase, Nicotinamidadenindinukleotidsynthetase oder Nicotinamidphosphoribosyl-transferase, wie dies z. B. in EP 04077624.7 oder WO 2006/133827 oder PCT/EP07/002433 beschrieben ist.

Pflanzen oder Pflanzensorten (die nach Methoden der pflanzlichen Biotechnologie, wie der Gentechnik, erhalten wurden), die ebenfalls mit den Verbindungen der allgemeinen Formel (I) behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so dass sich diese modifizierte Stärke besser für bestimmte Anwendungen eignet. Diese transgenen Pflanzen, die eine modifizierte Stärke synthetisieren, sind zum Beispiel in EP 0571427, WO 95/004826, EP 0719338, WO 96/15248, WO 96/19581, WO 96/27674, WO 97/11188, WO 97/26362, WO 97/32985, WO 97/42328, WO 97/44472, WO 97/45545, WO 98/27212, WO 98/40503, WO 99/58688, WO 99/58690, WO 99/58654, WO 2000/008184, WO 2000/008185, WO 2000/28052, WO 2000/77229, WO 2001/12782, WO 2001/12826, WO 2002/101059, WO 2003/071860, WO 2004/056999, WO 2005/030942, WO 2005/030941, WO 2005/095632, WO 2005/095617, WO 2005/095619, WO 2005/095618, WO 2005/123927, WO 2006/018319, WO 2006/103107, WO 2006/108702, WO 2007/009823, WO 2000/22140, WO 2006/063862, WO 2006/072603, WO 2002/034923, EP 06090134.5, EP 06090228.5, EP 06090227.7, EP 07090007.1, EP 07090009.7, WO 2001/14569, WO 2002/79410, WO 2003/33540, WO 2004/078983, WO 2001/19975, WO 95/26407, WO 96/34968, WO 98/20145, WO 99/12950, WO 99/66050, WO 99/53072, US 6,734,341, WO 2000/11192, WO 98/22604, WO 98/32326, WO 2001/98509, WO 2001/98509, WO 2005/002359, US 5,824,790, US 6,013,861, WO 94/004693, WO 94/009144, WO 94/11520, WO 95/35026 bzw. WO 97/20936 beschrieben.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, wie dies in EP 0663956, WO 96/001904, WO 96/021023, WO 98/039460 und WO 99/024593 beschrieben ist, Pflanzen, die alpha-1,4-Glucane produzieren, wie dies in WO 95/031553, US 2002/031826, US 6,284,479, US 5,712,107, WO 97/047806, WO 97/047807, WO 97/047808 und WO 2000/14249 beschrieben ist, Pflanzen, die alpha-1,6-verzweigte alpha-1,4-Glucane produzieren, wie dies in WO 2000/73422 beschrieben ist, und Pflanzen, die Alternan produzieren, wie dies in WO 2000/047727, EP 06077301.7, US 5,908,975 und EP 0728213 beschrieben ist.
3) Transgene Pflanzen, die Hyaluronan produzieren, wie dies zum Beispiel in WO 06/032538, WO 2007/039314, WO 2007/039315, WO 2007/039316, JP 2006/304779 und WO 2005/012529 beschrieben ist.

Pflanzen oder Pflanzensorten (die nach Methoden der pflanzlichen Biotechnologie, wie der Gentechnik, erhalten wurden), die ebenfalls mit den Verbindungen der allgemeinen Formel (I) behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten, wie dies in WO 98/000549 beschrieben ist,
b) Pflanzen wie Baumwollpflanzen, die eine veränderte Form von rsw2- oder rsw3-homologen Nukleinsäuren enthalten, wie dies in WO 2004/053219 beschrieben ist;
c) Pflanzen wie Baumwollpflanzen mit einer erhöhten Expression der Saccharosephosphatsynthase, wie dies in WO 2001/017333 beschrieben ist;
d) Pflanzen wie Baumwollpflanzen mit einer erhöhten Expression der Saccharosesynthase, wie dies in WO 02/45485 beschrieben ist;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase, wie dies in WO 2005/017157 beschrieben ist;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen, wie dies in WO 2006/136351 beschrieben ist.

Pflanzen oder Pflanzensorten (die nach Methoden der pflanzlichen Biotechnologie, wie der Gentechnik, erhalten wurden), die ebenfalls mit den Verbindungen der allgemeinen Formel (I) behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produzieren, wie dies zum Beispiel in US 5,969,169, US 5,840,946 oder US 6,323,392 oder US 6,063, 947 beschrieben ist;
b) Pflanzen wie Rapspflanzen, die Öl mit einem niedrigen Linolensäuregehalt produzieren, wie dies in US 6,270828, US 6,169,190 oder US 5,965,755 beschrieben ist.
c) Pflanzen wie Rapspflanzen, die Öl mit einem niedrigen gesättigten Fettsäuregehalt produzieren, wie dies z. B. in US 5,434,283 beschrieben ist.

Besonders nützliche transgene Pflanzen, die mit den Verbindungen der allgemeinen Formel (I) behandelt werden können, sind Pflanzen, die Transformations-Events, oder eine Kombination von Transformations-Events, enthalten und die zum Beispiel in den Dateien von verschiedenen nationalen oder regionalen Behörden angeführt sind.

Besonders nützliche transgene Pflanzen, die mit den Verbindungen der allgemeinen Formel (I) behandelt werden können, sind beispielhaft Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Die erfindungsgemäß zu verwendenden Verbindungen der Formel (I) können in übliche Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen. Im Rahmen der vorliegenden Erfindung ist es insbesondere bevorzugt, wenn die Verbindungen der allgemeinen Formel (I) in der Form einer Sprühformulieruing verwendet werden.

Die vorliegende Erfindung betrifft daher darüber hinaus auch eine Sprühformulierung zur Steigerung der Widerstandsfähigkeit von Pflanzen gegenüber abiotischem Stress. Im Folgenden wird eine Sprühformulierung näher beschrieben:

Die Formulierungen zur Sprühapplikation werden in bekannter Weise hergestellt, z.B. durch Vermischen der erfindungsgemäß zu verwendenden Verbindungen der allgmeinen Formel (I) mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Weitere übliche Zusatzstoffe, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser, können gegebenenfalls auch verwendet werden. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Als Netzmittel, die in den erfindungsgemäß verwendbaren Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutylnaphthalin-Sulfonate.

Als Dispergiermittel und/oder Emulgatoren, die in den erfindungsgemäß verwendbaren Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid-Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose. Als Gibberelline, die in den erfindungsgemäß verwendbaren Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Weitere Additive können Duftstoffe, mineralische oder vegetabilische gegebenenfalls modifizierte Öle, Wachse und Nährstoffe (auch Spurennährstoffe), wie Salze von Eisen, Mangan, Bor, Kupfer, Kobalt, Molybdän und Zink sein. Weiterhin enthalten sein können Stabilisatoren wie Kältestabilisatoren, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-%, vorzugsweise zwischen 0,5 und 90 %, der Verbindung der allgemeinen Formel (I).

Die Verbindungen der allgemeinen Formel (I) können in handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Ferner lässt sich die beschriebene positive Wirkung der Verbindungen der Formel (I) auf die pflanzeneigenen Abwehrkräfte durch eine zusätzliche Behandlung mit insektziden, fungiziden oder bakteriziden Wirkstoffen unterstützen.

Bevorzugte Zeitpunkte für die Applikation der erfindungsgemäß zu verwendenden Verbindungen der allgemeinen Formel (I) oder deren Salze zur Steigerung der Resistenz gegenüber abiotischem Stress sind Boden-, Stamm- und/oder Blattbehandlungen mit den zugelassenen Aufwandmengen.

Erfindunsgemäß wurde darüber hinaus gefunden, dass die Anwendung einer oder mehrerer Verbindungen der allgemeinen Formel (I) in Kombination mit mindestens einem Düngemittel wie weiter unten stehend definiert auf Pflanzen oder in deren Umgebung möglich ist.

Düngemittel, die erfindungsgemäß zusammen mit den oben näher erläuterten Verbindungen der allgemeinen Formel (I) verwendet werden können, sind im Allgemeinen organische und anorganische Stickstoff-haltige Verbindungen wie beispielsweise Harnstoffe, Harnstoff-Formaldehyd-Kondensationsprodukte, Aminosäuren, Ammoniumsalze und -nitrate, Kaliumsalze (bevorzugt Chloride, Sulfate, Nitrate), Phosphorsäuresalze und/oder Salze von Phosphoriger Säure (bevorzugt Kaliumsalze und Ammoniumsalze). Insbesondere zu nennen sind in diesem Zusammenhang die NPK-Dünger, d.h. Düngemittel, die Stickstoff, Phosphor und Kalium enthalten, Kalkammonsalpeter, d.h. Düngemittel, die noch Calcium enthalten, Ammonsulfatsalpeter (Allgemeine Formel (NH₄)₂SO₄ NH₄NO₃), Ammonphosphat und Ammonsulfat. Diese Düngemittel sind dem Fachmann allgemein bekannt, siehe auch beispielsweise Ullmann's Encyclopedia of Industrial Chemistry, 5. Edition, Vol. A 10, Seiten 323 bis 431, Verlagsgesellschaft, Weinheim, 1987.

Die Düngemittel können auch Salze aus Mikronährstoffen (bevorzugt Calcium, Schwefel, Bor, Mangan, Magnesium, Eisen, Bor, Kupfer, Zink, Molybdän und Kobalt) und Phytohormonen (z. B. Vitamin B1 und Indol-(III)essigsäure) oder Gemische davon enthalten. Erfindungsgemäß eingesetzte Düngemittel können auch weitere Salze wie Monoammoniumphosphat (MAP), Diammoniumphosphat (DAP), Kaliumsulfat, Kaliumchlorid, Magnesiumsulfat enthalten. Geeignete Mengen für die sekundären Nährstoffe oder Spurenelemente sind Mengen von 0,5 bis 5 Gew.-%, bezogen auf das gesamte Düngemittel. Weitere mögliche Inhaltsstoffe sind Pflanzenschutzmittel, Insektizide oder Fungizide, Wachstumsregulatoren oder Gemische davon. Hierzu folgen weiter unten weitergehende Ausführungen.

Die Düngemittel können beispielsweise in Form von Pulvern, Granulaten, Prills oder Kompaktaten eingesetzt werden. Die Düngemittel können jedoch auch in flüssiger Form, gelöst in einem wässrigen Medium, eingesetzt werden. In diesem Fall kann auch verdünnter wässriger Ammoniak als Stickstoffdüngemittel eingesetzt werden. Weitere mögliche Inhaltsstoffe für Düngemittel sind beispielsweise in Ullmann's Encyclopedia of Industrial Chemistry, 5. Auflage, 1987, Band A 10, Seiten 363 bis 401, DE-A 41 28 828, DE-A 19 05 834 und DE-A 196 31 764 beschrieben. Die allgemeine Zusammensetzung der Düngemittel, bei welchen es sich im Rahmen der vorliegenden Erfindung um Einzelnährstoff- und/oder Mehrnährstoffdünger handeln kann, beispielsweise aus Stickstoff, Kalium oder Phosphor, kann innerhalb eines breiten Bereichs variieren. Im Allgemeinen ist ein Gehalt von 1 bis 30 Gew.-% Stickstoff (bevorzugt 5 bis 20 Gew.-%), von 1 bis 20 Gew.-% Kalium (bevorzugt 3 bis 15 Gew.-%) und ein Gehalt von 1 bis 20 Gew.-% Phosphor (bevorzugt 3 bis 10 Gew.-%) vorteilhaft. Der Gehalt von Mikroelementen ist üblicherweise im ppm-Bereich, bevorzugt im Bereich von von 1 bis 1000 ppm.

Im Rahmen der vorliegenden Erfindung können das Düngemittel sowie eine oder mehrere Verbindungen der allgemeinen Formel (I) zeitgleich verabreicht werden. Es ist jedoch auch möglich, zunächst das Düngemittel und dann eine oder mehrere Verbindungen der allgemeinen Formel (I) oder zunächst eine oder mehrere Verbindungen der allgemeinen Formel (I) und dann das Düngemittel anzuwenden. Bei nicht zeitgleicher Anwendung einer oder mehrerer Verbindungen der allgemeinen Formel (I) und des Düngemittels erfolgt im Rahmen der vorliegenden Erfindung jedoch die Anwendung in funktionellem Zusammenhang, insbesondere innerhalb eines Zeitraums von im Allgemeinen 24 Stunden, bevorzugt 18 Stunden, besonders bevorzugt 12 Stunden, speziell 6 Stunden, noch spezieller 4 Stunden, noch weiter spezieller innerhalb 2 Stunden. In ganz besonderen Ausführungsformen der vorliegenden Erfindung erfolgt die Anwendung einer oder mehrerer Verbindungen der Formel (I) und des Düngemittels in einem zeitlichen Rahmen von weniger als 1 Stunden, vorzugsweise weniger als 30 Minuten, besonders bevorzugt weniger als 15 Minuten.

Die Erfindung soll durch die nachfolgenden biologischen Beispiele veranschaulicht werden, ohne sie jedoch darauf einzuschränken.

### Biologische Beispiele:

### In vivo:

Samen von mono- bzw. dikotylen Kulturpflanzen wurden in Plastiktöpfen in sandigem Lehmboden ausgesät, mit Erde oder Sand abgedeckt und im Gewächshaus unter guten Wachstumsbedingungen angezogen. Die Behandlung der Versuchspflanzen erfolgte im frühen Laubblattstadium (BBCH10 - BBCH13). Zur Gewährleistung einer uniformen Wasserversorgung vor Stressbeginn wurden die bepflanzten Töpfe vor Substanzapplikation durch Anstaubewässerung mit Wasser versorgt.

Die in Form von benetzbaren Pulvern (WP) formulierten erfindungsgemäßen Verbindungen wurden als wässrige Suspension mit einer Wasseraufwandmenge von umgerechnet 600 l/ha unter Zusatz von 0,2% Netzmittel (z.B. Agrotin) auf die grünen Pflanzenteile gesprüht. Unmittelbar nach Substanzapplikation erfolgte die Stressbehandlung der Pflanzen.

Der Trockenstress wurde durch langsames Abtrocknen unter folgenden Bedingungen induziert:
"Tag": 14 Stunden beleuchtet bei ∼ 26-30°C
"Nacht": 10 Stunden ohne Beleuchtung bei ∼ 18-20°C.

Die Dauer der jeweiligen Stressphasen richtete sich hauptsächlich nach dem Zustand der gestressten Kontrollpflanzen. Sie wurde (durch Wiederbewässerung und Transfer in ein Gewächshaus mit guten Wachstumsbedingungen) beendet, sobald irreversible Schäden an den gestressten Kontrollpflanzen zu beobachten waren.

Nach Beendigung der Stressphase folgte eine ca. 4-7 tägige Erholungsphase, während der die Pflanzen abermals unter guten Wachstumsbedingungen im Gewächshaus gehalten wurden. Die Dauer der Erholungsphase richtete sich hauptsächlich danach, wann die Versuchspflanzen einen Zustand erreicht hatten, der eine visuelle Bonitur potenzieller Effekte ermöglicht, und war daher variabel.

Wenn dieser Zeitpunkt erreicht war, wurde das Erscheinungsbild der mit Testsubstanzen behandelten Pflanzen im Vergleich zu den gestressten Kontrollpflanzen nach folgenden Kategorien erfasst:

| | |
|---|---|
| 0 | kein positiver Effekt |
| 10 | leicht positiver Effekt |
| 20 | deutlich positiver Effekt |
| 30 | stark positiver Effekt |

Bei den in unten stehenden Tabellen A1 und A2 angegebenen Werten handelt es sich um Ergebnisse aus mindestens zwei Replikaten

Wirkungen ausgewählter Verbindungen der allgemeinen Formel (I) unter Trockenstress:

**Tabelle A-1**

| No. | Substanz | Dosierung | Einheit | Effekt (BRSNS) |
|---|---|---|---|---|
| 1 | A1-26 | 2,5 | g/ha | 20 |
| 2 | A2-45 | 25 | g/ha | 20 |
| 3 | A4-292 | 25 | g/ha | 20 |
| 4 | G1-159 | 250 | g/ha | 10-20 |

**Tabelle A-2**

| No. | Substanz | Dosierung | Einheit | Effekt(TRZAS) |
|---|---|---|---|---|
| 1 | A1-1 | 250 | g/ha | 10-30 |
| 2 | A2-56 | 250 | g/ha | 10-20 |
| 3 | A3-153 | 25 | g/ha | 20 |
| 4 | G1-159 | 250 | g/ha | 20 |

In den zuvor genannten Tabellen bedeuten:
- BRSNS: = Brassica napus
- TRZAS: = Triticum aestivum

### In vitro:

Effekte des Phytohormons Abscisinsäure (ABA) auf das Verhalten von Pflanzen unter abiotischem Stress und der Wirkmechanismus von ABA sind in der Literatur beschrieben (vgl. Abrams et al, WO97/23441, Cutler, Park et al. Science, 2009, 324, 1068; Grill et al. Science, 2009, 324, 1064; Tanokura et al. Biophysics, 2011, 7, 123; Schroeder et al. Plant J. 2010, 61, 290). Daher kann man mit Hilfe eines geeigneten in vitro-Testsystems eine Korrelation zwischen der Wirkung von ABA und der Stressantwort einer Pflanze unter abiotischem Stress ableiten. Unter Wassermangel (Trockenstress) bilden Pflanzen das Phytohormon Abscisinsäure (ABA). Dieses bindet mit einem Co-Regulator (Regulatory Component of ABA-Receptor = RCAR nach Grill et al. Science, 2009, 324, 1064 oder PYR/PYL nach Cutler et al. Science, 2009, 324, 1068) an eine Phosphatase (z.B. ABI1, eine Typ 2C Proteinphosphatase, auch als PP2C abgekürzt) und hemmt diese in ihrer Aktivität. In der Folge wird eine "downstream" Kinase (z.B. SnRK2) nicht mehr dephosphoryliert. Diese somit aktive Kinase schaltet über Phosphorylierung von Transkriptionsfaktoren (z.B. AREB/ABF, vgl. Yoshida et al., Plant J. 2010, 61, 672) ein genetisches Schutzprogramm zur Erhöhung der Trockenstresstoleranz an.
In dem im Folgenden beschriebenen Assay wird die Hemmung der Phosphatase ABI1 über den Co-Regulator RCAR11/PYR1 aus *Arabidopsis thaliana* genutzt. Für die Aktivitätsbestimmung wurde die Dephosphorylierung von 4-Methylumbelliferylphosphat (MUP) bei 460nm gemessen. Der in vitro Assay wurde in Greiner 384-well PS-Mikroplatten F-well durchgeführt unter Verwendung von zwei Kontrollen, a) Dimethylsulfoxid (DMSO) 0.5% (f.c.) sowie b) 5 µM (f.c.) Abscisinsäure (ABA). Der hier beschriebene Assay wurde im Allgemeinen mit Substratkonzentrationen der entsprechenden chemischen Testsubstanzen in einem Konzentrationsbereich von 0.1 µM bis 100 µM in einer Lösung aus DMSO und Wasser durchgeführt. Die so erhaltene Substanzlösung wurde gegebenenfalls mit Esterase aus Schweineleber (EC 3.1.1.1) 3 h lang bei Raumtemperatur gerührt und 30 Min lang bei 4000rpm zentrifugiert. In jede Kavität der Mikroplatte wurde ein Gesamtvolumen von 45 µL gegeben, das sich wie folgt zusammensetzte:
1) 5µL Substanzlösung, d.h. a) DMSO 5% oder b) Abscisinsäurelösung oder c) die entsprechende Beispielverbindung der allgemeinen Formel (I) gelöst in 5% DMSO.
2) 20µL Enzympuffermix, der sich aus a) 40 Vol% Enzympuffer (10 mL enthalten zu gleichen Volumenanteilen 500 mM Tris-HCl pH8, 500 mM NaCl, 3.33 mM MnCl₂, 40 mM Dithiothreitol (DTT)), b) 4 Vol% ABI1-Verdünnung (Proteinstammlösung wurde so verdünnt, daß nach Zugabe eine Endkonzentration im Assay von 0.15 µg ABI1/well entsteht), c) 4 Vol% RCAR11-Verdünnung (Enzymstock wurde so verdünnt, daß bei Zugabe der Verdünnung in den Enzympuffermix eine Endkonzentration im Assay von 0.30 µg Enzym/well entsteht), d) 5 Vol% Tween20 (1%), e) 47 Vol% H₂O bi-dest zusammensetzt.
3) 20µL Substratmix, der sich aus a) 10 Vol% 500 mM Tris-HCl pH8, b) 10 Vol% 500 mM NaCl, c) 10 Vol% 3.33 mM MnCl₂, d) 5 Vol% 25 mM MUP, 5 Vol% Tween20 (1%), 60 Vol% H₂O bi-dest zusammensetzt

Enzympuffermix und Substratmix wurden 5 Minuten vor der Zugabe angesetzt und auf eine Temperatur von 35 °C erwärmt. Nach vollständigem Pipettieren aller Lösungen und vollständiger Durchmischung wurde die Platte 20 Minuten lang bei 35 °C inkubiert. Abschließend erfolgte eine relative Fluoreszenzmessung bei 35 °C mit einem Mikroplatten-Lesegerät "POLARstar Optima" der Firma BMG Labtech unter Verwendung eines Anregungsfilters 340/10 nm und einem Emmissionsfilter von 460 nm. Die Wirkstärke der Verbindungen der allgemeinen Formel (I) wird in der nachfolgenden Tabelle unter Verwendung von Abscisinsäure als Vergleichssubstanz nach folgender Einteilung angegeben: ++++ (≥ 90 % Inhibition), +++ (< 90 %, ≥ 70% Inhibition), ++ (< 70 %, ≥ 50% Inhibition), + (< 50 %, ≥ 30% Inhibition).

Effekte ausgewählter Verbindungen der allgemeinen Formel (I) im oben beschriebenen in vitro-Assay bei einer Konzentration von 5mM der betreffenden Substanz der allgemeinen Formel (I) in einer Lösung aus DMSO und Wasser::

**Tabelle A-1**

| No. | Substanz | ABI1 Inhibition |
|---|---|---|
| 1 | A1-178 | ++ |
| 2 | A2-152 | +++ |
| 3 | A2-165 | +++ |
| 4 | A2-178 | +++ |
| 5 | A2-181 | ++ |
| 6 | A3-152 | ++++ |
| 7 | A3-158 | ++ |
| 8 | A3-165 | +++ |
| 9 | A3-178 | ++++ |
| 10 | A3-181 | +++ |
| 11 | A4-178 | ++ |
| 12 | B1-178 | +++ |
| 13 | C2-152 | +++ |
| 14 | C2-165 | ++ |
| 15 | C3-45 | ++ |
| 16 | C3-152 | ++ |
| 17 | C3-165 | +++ |
| 18 | E7-152 | ++ |
| 19 | G1-165 | ++ |
| 20 | G1-172 | +++ |
| 21 | G1-178 | ++ |
| 22 | G1-184 | ++ |
| 23 | 11-152 | +++ |
| 24 | 11-165 | ++ |
| 25 | Abscisinsäure | ++++ |

Ähnliche Ergebnisse konnten auch noch mit weiteren Verbindungen der allgemeinen Formel (I) auch bei Applikation auf andere Pflanzenarten erzielt werden.

## Patentansprüche

1. Verwendung substituierter Dihydrooxindolylsulfonamide oder deren Salzen 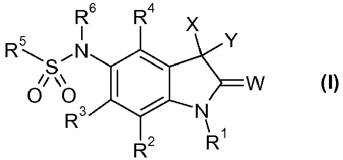 zur Erhöhung der Stresstoleranz in Pflanzen gegenüber abiotischem Stress und/oder zur Erhöhung des Pflanzenertrags,
wobei
R¹ für Wasserstoff, (C₁-C₁₀)-Alkyl, (C₃-C₈)-Cycloalkyl, (C₁-C₁₀)-Haloalkyl, (C₃-C₈)-Halocycloalkyl, (C₂-C₈)-Alkenyl, (C₂-C₈)-Haloalkenyl, (C₁-C₈)-Alkoxy-(C₁-C₈)-haloalkyl, (C₂-C₈)-Alkinyl, Aryl, Aryl-(C₁-C₈)-alkyl, Heteroaryl, Heteroaryl-(C₁-C₈)-alkyl, (C₃-C₈)-Cycloalkyl-(C₁-C₈)-alkyl, (C₂-C₈)-Haloalkinyl, Heterocyclyl, Heterocyclyl-(C₁-C₈)-alkyl, (C₁-C₈)-Alkoxy-(C₁-C₈)-alkyl, (C₁-C₈)-Alkylcarbonyl-(C₁-C₈)-alkyl, Hydroxycarbonyl-(C₁-C₈)-alkyl, (C₁-C₈)-Alkoxycarbonyl-(C₁-C₈)-alkyl, (C₂-C₈)-Alkenyloxycarbonyl-(C₁-C₈)-alkyl, (C₂-C₈)-Alkinyloxycarbonyl-(C₁-C₈)-alkyl, Aryl-(C₁-C₈)-alkoxycarbonyl-(C₁-C₈)-alkyl, (C₃-C₈)-Cycloalkoxycarbonyl-(C₁-C₈)-alkyl, (C₃-C₈)-Cycloalkyl-(C₁-C₈)-alkoxycarbonyl-(C₁-C₈)-alkyl, Aminocarbonyl-(C₁-C₈)-alkyl, (C₁-C₈)-Alkylaminocarbonyl-(C₁-C₈)-alkyl, (C₃-C₈)-Cycloalkylaminocarbonyl-(C₁-C₈)-alkyl, Aryl-(C₁-C₈)-alkylaminocarbonyl-(C₁-C₈)-alkyl, Heteroaryl-(C₁-C₈)-alkylaminocarbonyl-(C₁-C₈)-alkyl, (C₁-C₈)-Alkylthio-(C₁-C₈)-alkyl, (C₃-C₈)-Cycloalkylthio-(C₁-C₈)-alkyl, Arylthio-(C₁-C₈)-alkyl, Heterocyclylthio-(C₁-C₈)-alkyl, Heteroarylthio-(C₁-C₈)-alkyl, Aryl-(C₁-C₈)-alkylthio-(C₁-C₈)-alkyl, (C₁-C₈)-Alkylsulfinyl-(C₁-C₈)-alkyl, (C₁-C₈)-Alkylsulfonyl-(C₁-C₈)-alkyl, Arylsulfinyl-(C₁-C₈)-alkyl, Arylsulfonyl-(C₁-C₈)-alkyl, (C₃-C₈)-Cycloalkylsulfinyl-(C₁-C₈)-alkyl, (C₃-C₈)-Cycloalkylsulfonyl-(C₁-C₈)-alkyl, (C₁-C₈)-Alkoxy-(C₁-C₈)-alkoxy-(C₁-C₈)-alkyl, (C₁-C₈)-Alkylcarbonyl, (C₁-C₈)-Haloalkylcarbonyl, (C₃-C₈)-Cycloalkylcarbonyl, (C₁-C₈)-Alkoxycarbonyl, Aryl-(C₁-C₈)-alkoxycarbonyl, Arylcarbonyl, Heteroarylcarbonyl, Heterocyclylcarbonyl, Aryl-(C₁-C₈)-alkylcarbonyl, (C₁-C₈)-Alkylaminocarbonyl, (C₃-C₈)-Cycloalkylaminocarbonyl, Arylaminocarbonyl, Aryl-(C₁-C₈)-alkylaminocarbonylHeteroarylaminocarbonyl, Heterocyclylaminocarbonyl, Heteroaryl-(C₁-C₈)-alkylaminocarbonyl, Heterocyclyl-(C₁-C₈)-alkylaminocarbonyl, (C₁-C₈)-Alkylsulfonyl, (C₃-C₈)-Cycloalkylsulfonyl, Arylsulfonyl, Aryl-(C₁-C₈)-alkylsulfonyl, Heteroarylsulfonyl, Heterocyclylsulfonyl, Cyano-(C₁-C₈)-alkyl, (C₄-C₈)-Cycloalkenyl-(C₁-C₈)-alkyl, Nitro-(C₁-C₈)-alkyl, Halo-(C₁-C₈)-alkoxy-(C₁-C₈)-alkyl, Bis-[(C₁-C₈)-Alkyl]aminocarbonyl, (C₃-C₈)-Cycloalkyl-[(C₁-C₈)-Alkyl]aminocarbonyl, Aryl-[(C₁-C₈)-Alkyl]aminocarbonyl, Aryl-(C₁-C₈)-alkyl-[(C₁-C₈)-Alkyl]aminocarbonyl, (C₂-C₈)-Alkenylaminocarbonyl, (C₂-C₈)-Alkinylaminocarbonyl, (C₁-C₈)-Alkylaminosulfonyl, Bis-[(C₁-C₈)-Alkyl]aminosulfonyl, Heterocyclylsulfinyl-(C₁-C₈)-alkyl, Heteroarylsulfinyl-(C₁-C₈)-alkyl, Aryl-(C₁-C₈)-alkylsulfinyl-(C₁-C₈)-alkyl, Heterocyclylsulfonyl-(C₁-C₈)-alkyl, Heteroarylsulfonyl-(C₁-C₈)-alkyl, Aryl-(C₁-C₈)-alkylsulfonyl-(C₁-C₈)-alkyl, Bis-[(C₁-C₈)-Alkyl]aminocarbonyl-(C₁-C₈)-alkyl, (C₃-C₈)-Cycloalkyl-[(C₁-C₈)-Alkyl]aminocarbonyl-(C₁-C₈)-alkyl, Aryl-[(C₁-C₈)-Alkyl]aminocarbonyl-(C₁-C₈)-alkyl, Aryl-(C₁-C₈)-alkyl-[(C₁-C₈)-Alkyl]aminocarbonyl-(C₁-C₈)-alkyl, (C₂-C₈)-Alkenylaminocarbonyl-(C₁-C₈)-alkyl, (C₂-C₈)-Alkinylaminocarbonyl-(C₁-C₈)-alkyl, (C₁-C₈)-Alkylamino, Bis-[(C₁-C₈)-alkyl]amino, (C₃-C₈)-Cycloalkyl[(C₁-C₈)-alkyl]amino steht,
R², R³, R⁴ unabhängig voneinander für Wasserstoff, Halogen, (C₁-C₈)-Alkoxy, (C₁-C₈)-Alkyl, (C₁-C₈)-Haloalkyl, (C₁-C₈)-Haloalkoxy, (C₁-C₈)-Alkylthio, (C₁-C₈)-Haloalkylthio, Aryl, Aryl-(C₁-C₈)-alkyl, Heteroaryl, Heteroaryl-(C₁-C₈)-alkyl, Heterocyclyl, Heterocyclyl-(C₁-C₈)-alkyl, (C₃-C₈)-Cycloalkyl, Nitro, Amino, Hydroxy, (C₁-C₈)-Alkylamino, Bis-[(C₁-C₈)-alkyl]amino, Hydrothio, (C₁-C₈)-Alkylcarbonylamino, (C₃-C₈)-Cycloalkylcarbonylamino, Arylcarbonylamino, Heteroarylcarbonylamino, Heterocyclylcarbonylamino, Formyl, Hydroxyiminomethyl, (C₁-C₈)-Alkoxyiminomethyl, (C₃-C₈)-Cycloalkoxyiminomethyl, Aryloxyiminomethyl, (C₃-C₈)-Cycloalkyl-(C₁-C₈)-alkoxyiminomethyl, Thiocyanato, Isothiocyanato, Aryloxy, Heteroaryloxy, (C₃-C₈)-Cycloalkoxy, (C₃-C₈)-Cycloalkyl-(C₁-C₈)-alkoxy, Aryl-(C₁-C₈)-alkoxy, (C₂-C₈)-Alkinyl, (C₂-C₈)-Alkenyl, Aryl-(C₁-C₈)-alkinyl, Tris-[(C₁-C₈)-alkyl]silyl-(C₂-C₈)-alkinyl, Bis-[(C₁-C₈)-alkyl](aryl)silyl-(C₂-C₈)-alkinyl, Bis-aryl[(C₁-C₈)-alkyl]silyl-(C₂-C₈)-alkinyl, (C₃-C₈)-Cycloalkyl-(C₂-C₈)-alkinyl, Aryl-(C₂-C₈)-alkenyl, Heteroaryl-(C₂-C₈)-alkenyl, (C₃-C₈)-Cycloalkyl-(C₂-C₈)-alkenyl, (C₃-C₈)-Cycloalkyl-(C₂-C₈)-alkyl, (C₂-C₈)-Haloalkinyl, (C₂-C₈)-Haloalkenyl, (C₄-C₈)-Cycloalkenyl, (C₁-C₈)-Alkoxy-(C₁-C₈)-alkoxy-(C₁-C₈)-alkyl, (C₁-C₈)-Alkylsulfonyl, Arylsulfonyl, Heteroarylsulfonyl, (C₁-C₈)-Alkylsulfonylamino, Arylsulfonylamino, Aryl-(C₁-C₈)-alkylsulfonylamino, Heteroarylsulfonylamino, Heteroaryl-(C₁-C₈)-alkylsulfonylamino, Bis-[(C₁-C₈)-Alkyl]aminosulfonyl, (C₄-C₈)-Cycloalkenyl-(C₁-C₈)-alkyl, (C₁-C₈)-Alkylsulfinyl, Arylsulfinyl, Heteroarylsulfinyl stehen,
R⁵ für Amino, (C₁-C₈)-Alkyl, (C₃-C₈)-Cycloalkyl, (C₃-C₈)-Cycloalkyl-(C₁-C₈)-alkyl, (C₁-C₈)-Haloalkyl, (C₃-C₈)-Halocycloalkyl, (C₄-C₈)-Cycloalkenyl, Aryl, Heteroaryl, Heterocyclyl, Aryl-(C₁-C₈)-alkyl, Heteroaryl-(C₁-C₈)-alkyl, Heterocyclyl-(C₁-C₈)-alkyl, (C₁-C₈)-Alkoxycarbonyl-(C₁-C₈)-alkyl, Aryl-(C₁-C₈)-alkoxycarbonyl-(C₁-C₈)-alkyl, (C₃-C₈)-Cycloalkoxycarbonyl-(C₁-C₈)-alkyl, (C₃-C₈)-Cycloalkyl-(C₁-C₈)-alkoxycarbonyl-(C₁-C₈)-alkyl, Heteroaryl-(C₁-C₈)-alkoxycarbonyl-(C₁-C₈)-alkyl, Aminocarbonyl-(C₁-C₈)-alkyl, (C₁-C₈)-Alkylaminocarbonyl-(C₁-C₈)-alkyl, (C₃-C₈)-Cycloalkylaminocarbonyl-(C₁-C₈)-alkyl, Aryl-(C₁-C₈)-alkylaminocarbonyl-(C₁-C₈)-alkyl, (C₁-C₈)-Alkylamino, Arylamino, (C₃-C₈)-Cycloalkylamino, Aryl-(C₁-C₈)-alkylamino, Heteroaryl-(C₁-C₈)-alkylamino, Heteroarylamino, Heterocyclylamino, Aryloxy-(C₁-C₈)-alkyl, (C₁-C₈)-Alkoxy-(C₁-C₈)-alkyl, Heteroaryloxy-(C₁-C₈)-alkyl, (C₂-C₈)-Alkenyl, (C₂-C₈)-Alkinyl, (C₂-C₈)-Alkenylamino, (C₂-C₈)-Alkinylamino, Bis-[(C₁-C₈)-alkyl]amino, Aryloxy, Bis-[(C₁-C₈)-Alkyl]amino, Aryl-(C₂-C₈)-alkenyl, Heteroaryl-(C₂-C₈)-alkenyl, Heterocyclyl-(C₂-C₈)-alkenyl, Aryloxycarbonyl-(C₁-C₈)-alkyl, Heteroaryloxycarbonyl-(C₁-C₈)-alkyl,
steht, Bis[(C₁-C₈)-alkyl]aminocarbonyl-(C₁-C₈)-alkyl, (C₁-C₈)-Alkylthio-(C₁-C₈)-alkyl, Cyano-(C₁-C₈)-alkyl, (C₁-C₈)-Alkoxy-(C₁-C₈)-alkoxy-(C₁-C₈)-alkyl
R⁶ für Wasserstoff, (C₁-C₈)-Alkyl, (C₃-C₈)-Cycloalkyl, Cyano-(C₁-C₈)-alkyl, (C₃-C₈)-Cycloalkyl-(C₁-C₈)-alkyl, (C₁-C₈)-Alkylsulfonyl, Arylsulfonyl, Heteroarylsulfonyl, (C₃-C₈)-Cycloalkylsulfonyl, Heterocyclylsulfonyl, Aryl-(C₁-C₈)-alkylsulfonyl, (C₁-C₈)-Alkylcarbonyl, Arylcarbonyl, Heteroarylcarbonyl, (C₃-C₈)-Cycloalkylcarbonyl, Heterocyclylcarbonyl, (C₁-C₈)-Alkoxycarbonyl, Aryl-(C₁-C₈)-alkoxycarbonyl, (C₁-C₈)-Haloalkylcarbonyl, (C₂-C₈)-Alkenyl, (C₂-C₈)-Alkinyl, (C₁-C₈)-Haloalkyl, Halo-(C₂-C₈)-alkinyl, Halo-(C₂-C₈)-alkenyl, (C₁-C₈)-Alkoxy-(C₁-C₈)-alkyl steht,
W für Sauerstoff, Schwefel steht und
X, Y unabhängig voneinander für Wasserstoff, (C₁-C₈)-Alkyl, Halogen, (C₂-C₈)-Alkenyl, (C₂-C₈)-Alkinyl, (C₁-C₈)-Haloalkyl, Hydroxy-(C₁-C₈)-alkyl, Cyano-(C₁-C₈)-alkyl, Aryl, Heteroaryl, (C₃-C₈)-Cycloalkyl, (C₄-C₈)-Cycloalkenyl, Heterocyclyl, Cyano, Nitro, Hydroxy, (C₁-C₈)-Alkoxy, (C₁-C₈)-Alkylthio, (C₁-C₈)-Alkoxy-(C₁-C₈)-alkyl, (C₁-C₈)-Alkylthio-(C₁-C₈)-alkyl, Aryloxy, Aryl-(C₁-C₈)-alkoxy, (C₁-C₈)-Haloalkoxy, (C₁-C₈)-Haloalkylthio, (C₁-C₈)-Alkylamino, Bis-[(C₁-C₈)-Alkyl]amino, (C₁-C₈)-Alkoxy-(C₁-C₈)-alkoxy, Amino-(C₁-C₈)-alkyl, (C₁-C₈)-Alkylamino-(C₁-C₈)-alkyl, (C₃-C₈)-Cycloalkylamino-(C₁-C₈)-alkyl, Aryl-(C₁-C₈)-alkylamino-(C₁-C₈)-alkyl, Heteroaryl-(C₁-C₈)-alkylamino-(C₁-C₈)-alkyl, Heterocyclyl-(C₁-C₈)-alkylamino-(C₁-C₈)-alkyl, Heterocyclylamino-(C₁-C₈)-alkyl, Heteroarylamino-(C₁-C₈)-alkyl, (C₁-C₈)-Alkoxycarbonylamino-(C₁-C₈)-alkyl, Arylamino-(C₁-C₈)-alkyl, Aryl-(C₁-C₈)-alkoxycarbonylamino-(C₁-C₈)-alkyl, (C₃-C₈)-Cycloalkoxycarbonylamino-(C₁-C₈)-alkyl, (C₃-C₈)-Cycloalkyl-(C₁-C₈)-alkoxycarbonylamino-(C₁-C₈)-alkyl, Heteroaryl-(C₁-C₈)-alkoxycarbonylamino-(C₁-C₈)-alkyl, (C₁-C₈)-Alkylcarbonylamino-(C₁-C₈)-alkyl, (C₃-C₈)-Cycloalkylcarbonylamino-(C₁-C₈)-alkyl, Arylcarbonylamino-(C₁-C₈)-alkyl, Heteroarylcarbonylamino-(C₁-C₈)-alkyl, Heterocyclylcarbonylamino-(C₁-C₈)-alkyl, (C₂-C₈)-Alkenyloxycarbonylamino-(C₁-C₈)-alkyl, Aryl-(C₂-C₈)-Alkenylamino-(C₁-C₈)-alkyl, Arylsulfonyl-(C₁-C₈)-alkyl, Heteroarylsulfonyl-(C₁-C₈)-alkyl, (C₁-C₈)-Alkylsulfonyl-(C₁-C₈)-alkyl, (C₃-C₈)-Cycloalkylsulfonyl-(C₁-C₈)-alkyl, Arylsulfinyl-(C₁-C₈)-alkyl, Heteroarylsulfinyl-(C₁-C₈)-alkyl, (C₁-C₈)-Alkylsulfinyl-(C₁-C₈)-alkyl, (C₃-C₈)-Cycloalkylsulfinyl-(C₁-C₈)-alkyl, Bis[(C₁-C₈)-alkyl]amino-(C₁-C₈)-alkyl stehen oder
X und Y mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 7-gliedrigen monocyclischen oder bicyclischen Ring bilden.

2. Verwendung gemäß Anspruch 1, wobei in Formel (I)
R¹ Wasserstoff, (C₁-C₈)-Alkyl, (C₃-C₆)-Cycloalkyl, (C₁-C₈)-Haloalkyl, (C₃-C₆)-Halocycloalkyl, (C₂-C₆)-Alkenyl, (C₂-C₆)-Haloalkenyl, (C₁-C₆)-Alkoxy-(C₁-C₆)-haloalkyl, (C₂-C₆)-Alkinyl, Aryl, Aryl-(C₁-C₆)-alkyl, Heteroaryl, Heteroaryl-(C₁-C₆)-alkyl, (C₃-C₆)-Cycloalkyl-(C₁-C₆)-alkyl, (C₂-C₆)-Haloalkinyl, Heterocyclyl, Heterocyclyl-(C₁-C₆)-alkyl, (C₁-C₆)-Alkoxy-(C₁-C₆)-alkyl, (C₁-C₆)-Alkylcarbonyl-(C₁-C₆)-alkyl, Hydroxycarbonyl-(C₁-C₆)-alkyl, (C₁-C₆)-Alkoxycarbonyl-(C₁-C₆)-alkyl, (C₂-C₆)-Alkenyloxycarbonyl-(C₁-C₆)-alkyl, (C₂-C₆)-Alkinyloxycarbonyl-(C₁-C₆)-alkyl, Aryl-(C₁-C₆)-alkoxycarbonyl-(C₁-C₆)-alkyl, (C₃-C₆)-Cycloalkoxycarbonyl-(C₁-C₆)-alkyl, (C₃-C₆)-Cycloalkyl-(C₁-C₆)-alkoxycarbonyl-(C₁-C₆)-alkyl, Aminocarbonyl-(C₁-C₆)-alkyl, (C₁-C₆)-Alkylaminocarbonyl-(C₁-C₆)-alkyl, (C₃-C₆)-Cycloalkylaminocarbonyl-(C₁-C₆)-alkyl, Aryl-(C₁-C₆)-alkylaminocarbonyl-(C₁-C₆)-alkyl, Heteroaryl-(C₁-C₆)-alkylaminocarbonyl-(C₁-C₆)-alkyl, (C₁-C₆)-Alkylthio-(C₁-C₆)-alkyl, (C₃-C₆)-Cycloalkylthio-(C₁-C₆)-alkyl, Arylthio-(C₁-C₆)-alkyl, Heterocyclylthio-(C₁-C₆)-alkyl, Heteroarylthio-(C₁-C₆)-alkyl, Aryl-(C₁-C₆)-alkylthio-(C₁-C₆)-alkyl, (C₁-C₆)-Alkylsulfinyl-(C₁-C₆)-alkyl, (C₁-C₆)-Alkylsulfonyl-(C₁-C₆)-alkyl, Arylsulfinyl-(C₁-C₆)-alkyl, Arylsulfonyl-(C₁-C₆)-alkyl, (C₃-C₆)-Cycloalkylsulfinyl-(C₁-C₆)-alkyl, (C₃-C₆)-Cycloalkylsulfonyl-(C₁-C₆)-alkyl, (C₁-C₆)-Alkoxy-(C₁-C₆)-alkoxy-(C₁-C₆)-alkyl, (C₁-C₆)-Alkylcarbonyl, (C₁-C₆)-Haloalkylcarbonyl, (C₃-C₆)-Cycloalkylcarbonyl, (C₁-C₆)-Alkoxycarbonyl, Aryl-(C₁-C₆)-alkoxycarbonyl, Arylcarbonyl, Heteroarylcarbonyl, Heterocyclylcarbonyl, Aryl-(C₁-C₆)-alkylcarbonyl, (C₁-C₆)-Alkylaminocarbonyl, (C₃-C₆)-Cycloalkylaminocarbonyl, Arylaminocarbonyl, Aryl-(C₁-C₆)-alkylaminocarbonyl, Heteroarylaminocarbonyl, Heterocyclylaminocarbonyl, Heteroaryl-(C₁-C₆)-alkylaminocarbonyl, Heterocyclyl-(C₁-C₆)-alkylaminocarbonyl, (C₁-C₆)-Alkylsulfonyl, (C₃-C₆)-Cycloalkylsulfonyl, Arylsulfonyl, Aryl-(C₁-C₆)-alkylsulfonyl, Heteroarylsulfonyl, Heterocyclylsulfonyl, Cyano-(C₁-C₆)-alkyl, (C₄-C₆)-Cycloalkenyl-(C₁-C₆)-alkyl, Nitro-(C₁-C₆)-alkyl, Halo-(C₁-C₆)-alkoxy-(C₁-C₆)-alkyl, Bis-[(C₁-C₆)-Alkyl]aminocarbonyl, (C₃-C₆)-Cycloalkyl-[(C₁-C₆)-Alkyl]aminocarbonyl, Aryl-[(C₁-C₆)-Alkyl]aminocarbonyl, Aryl-(C₁-C₆)-alkyl-[(C₁-C₆)-Alkyl]aminocarbonyl, (C₂-C₆)-Alkenylaminocarbonyl, (C₂-C₆)-Alkinylaminocarbonyl, (C₁-C₆)-Alkylaminosulfonyl, Bis-[(C₁-C₆)-Alkyl]aminosulfonyl, Heterocyclylsulfinyl-(C₁-C₆)-alkyl, Heteroarylsulfinyl-(C₁-C₆)-alkyl, Aryl-(C₁-C₆)-alkylsulfinyl-(C₁-C₆)-alkyl, Heterocyclylsulfonyl-(C₁-C₆)-alkyl, Heteroarylsulfonyl-(C₁-C₆)-alkyl, Aryl-(C₁-C₆)-alkylsulfonyl-(C₁-C₆)-alkyl, Bis-[(C₁-C₆)-Alkyl]aminocarbonyl-(C₁-C₆)-alkyl, (C₃-C₆)-Cycloalkyl-[(C₁-C₆)-Alkyl]aminocarbonyl-(C₁-C₆)-alkyl, Aryl-[(C₁-C₆)-Alkyl]aminocarbonyl-(C₁-C₆)-alkyl, Aryl-(C₁-C₆)-alkyl-[(C₁-C₆)-Alkyl]aminocarbonyl-(C₁-C₆)-alkyl, (C₂-C₆)-Alkenylaminocarbonyl-(C₁-C₆)-alkyl, (C₂-C₆)-Alkinylaminocarbonyl-(C₁-C₆)-alkyl, Bis-[(C₁-C₆)-alkyl]amino, (C₃-C₆)-Cycloalkyl[(C₁-C₆)-alkyl]amino steht,
R², R³, R⁴ unabhängig voneinander für Wasserstoff, Halogen, (C₁-C₆)-Alkoxy, (C₁-C₆)-Alkyl, (C₁-C₆)-Haloalkyl, (C₁-C₆)-Haloalkoxy, (C₁-C₆)-Alkylthio, (C₁-C₆)-Haloalkylthio, Aryl, Aryl-(C₁-C₆)-alkyl, Heteroaryl, Heteroaryl-(C₁-C₆)-alkyl, Heterocyclyl, Heterocyclyl-(C₁-C₆)-alkyl, (C₃-C₆)-Cycloalkyl, Nitro, Amino, Hydroxy, (C₁-C₆)-Alkylamino, Bis-[(C₁-C₆)-alkyl]amino, Hydrothio, (C₁-C₆)-Alkylcarbonylamino, (C₃-C₆)-Cycloalkylcarbonylamino, Arylcarbonylamino, Heteroarylcarbonylamino, Heterocyclylcarbonylamino, Formyl, Hydroxyiminomethyl, (C₁-C₆)-Alkoxyiminomethyl, (C₃-C₆)-Cycloalkoxyiminomethyl, Aryloxyiminomethyl, (C₃-C₆)-Cycloalkyl-(C₁-C₆)-alkoxyiminomethyl, Thiocyanato, Isothiocyanato, Aryloxy, Heteroaryloxy, (C₃-C₆)-Cycloalkoxy, (C₃-C₆)-Cycloalkyl-(C₁-C₆)-alkoxy, Aryl-(C₁-C₆)-alkoxy, (C₂-C₆)-Alkinyl, (C₂-C₆)-Alkenyl, Aryl-(C₁-C₆)-alkinyl, Tris-[(C₁-C₆)-alkyl]silyl-(C₂-C₆)-alkinyl, Bis-[(C₁-C₆)-alkyl](aryl)silyl-(C₂-C₆)-alkinyl, Bis-aryl[(C₁-C₆)-alkyl]silyl-(C₂-C₆)-alkinyl, (C₃-C₆)-Cycloalkyl-(C₂-C₆)-alkinyl, Aryl-(C₂-C₆)-alkenyl, Heteroaryl-(C₂-C₆)-alkenyl, (C₃-C₆)-Cycloalkyl-(C₂-C₆)-alkenyl, (C₃-C₆)-Cycloalkyl-(C₂-C₆)-alkyl, (C₂-C₆)-Haloalkinyl, (C₂-C₆)-Haloalkenyl, (C₄-C₆)-Cycloalkenyl, (C₁-C₆)-Alkoxy-(C₁-C₆)-alkoxy-(C₁-C₆)-alkyl, (C₁-C₆)-Alkylsulfonyl, Arylsulfonyl, Heteroarylsulfonyl, (C₁-C₆)-Alkylsulfonylamino, Arylsulfonylamino, Aryl-(C₁-C₆)-alkylsulfonylamino, Heteroarylsulfonylamino, Heteroaryl-(C₁-C₆)-alkylsulfonylamino, Bis-[(C₁-C₆)-Alkyl]aminosulfonyl stehen,
R⁵ für Amino, (C₁-C₆)-Alkyl, (C₃-C₆)-Cycloalkyl, (C₃-C₆)-Cycloalkyl-(C₁-C₆)-alkyl, (C₁-C₆)-Haloalkyl, (C₃-C₆)-Halocycloalkyl, (C₄-C₆)-Cycloalkenyl, Aryl, Heteroaryl, Heterocyclyl, Aryl-(C₁-C₆)-alkyl, Heteroaryl-(C₁-C₆)-alkyl, Heterocyclyl-(C₁-C₆)-alkyl, (C₁-C₆)-Alkoxycarbonyl-(C₁-C₆)-alkyl, Aryl-(C₁-C₆)-alkoxycarbonyl-(C₁-C₆)-alkyl, (C₃-C₆)-Cycloalkoxycarbonyl-(C₁-C₆)-alkyl, (C₃-C₆)-Cycloalkyl-(C₁-C₆)-alkoxycarbonyl-(C₁-C₆)-alkyl, Heteroaryl-(C₁-C₆)-alkoxycarbonyl-(C₁-C₆)-alkyl, Aminocarbonyl-(C₁-C₆)-alkyl, (C₁-C₆)-Alkylaminocarbonyl-(C₁-C₆)-alkyl, (C₃-C₆)-Cycloalkylaminocarbonyl-(C₁-C₆)-alkyl, Aryl-(C₁-C₆)-alkylaminocarbonyl-(C₁-C₆)-alkyl, (C₁-C₆)-Alkylamino, Arylamino, (C₃-C₆)-Cycloalkylamino, Aryl-(C₁-C₆)-alkylamino, Heteroaryl-(C₁-C₆)-alkylamino, Heteroarylamino, Heterocyclylamino, Aryloxy-(C₁-C₆)-alkyl, (C₁-C₆)-Alkoxy-(C₁-C₆)-alkyl, Heteroaryloxy-(C₁-C₆)-alkyl, (C₂-C₆)-Alkenyl, (C₂-C₆)-Alkinyl, (C₂-C₆)-Alkenylamino, (C₂-C₆)-Alkinylamino, Bis-[(C₁-C₆)-alkyl]amino, Aryloxy, Bis-[(C₁-C₇)-Alkyl]amino, Aryl-(C₂-C₇)-alkenyl, Heteroaryl-(C₂-C₇)-alkenyl, Heterocyclyl-(C₂-C₇)-alkenyl steht,
R⁶ für Wasserstoff, (C₁-C₆)-Alkyl, (C₃-C₆)-Cycloalkyl, Cyano-(C₁-C₆)-alkyl, (C₃-C₆)-Cycloalkyl-(C₁-C₆)-alkyl, (C₁-C₆)-Alkylsulfonyl, Arylsulfonyl, Heteroarylsulfonyl, (C₃-C₆)-Cycloalkylsulfonyl, Heterocyclylsulfonyl, Aryl-(C₁-C₆)-alkylsulfonyl, (C₁-C₆)-Alkylcarbonyl, Arylcarbonyl, Heteroarylcarbonyl, (C₃-C₆)-Cycloalkylcarbonyl, Heterocyclylcarbonyl, (C₁-C₆)-Alkoxycarbonyl, Aryl-(C₁-C₆)-alkoxycarbonyl, (C₁-C₆)-Haloalkylcarbonyl, (C₂-C₆)-Alkenyl, (C₂-C₆)-Alkinyl, (C₁-C₆)-Haloalkyl, Halo-(C₂-C₆)-alkinyl, Halo-(C₂-C₆)-alkenyl, (C₁-C₆)-Alkoxy-(C₁-C₆)-alkyl steht
W für Sauerstoff, Schwefel steht und
X, Y unabhängig voneinander für Wasserstoff, (C₁-C₆)-Alkyl, Fluor, Chlor, (C₂-C₆)-Alkenyl, (C₁-C₆)-Haloalkyl, (C₃-C₆)-Cycloalkyl, (C₄-C₆)-Cycloalkenyl, Heterocyclyl, (C₁-C₆)-Alkoxy, (C₁-C₆)-Alkylthio, (C₁-C₆)-Alkoxy-(C₁-C₆)-alkyl, (C₁-C₆)-Alkylthio-(C₁-C₆)-alkyl, (C₁-C₆)-Haloalkoxy, (C₁-C₆)-Haloalkylthio, (C₁-C₆)-Alkoxy-(C₁-C₆)-alkoxy, Amino-(C₁-C₆)-alkyl, (C₁-C₆)-Alkylamino-(C₁-C₆)-alkyl, (C₃-C₆)-Cycloalkylamino-(C₁-C₆)-alkyl, Aryl-(C₁-C₆)-alkylamino-(C₁-C₆)-alkyl, Heteroaryl-(C₁-C₆)-alkylamino-(C₁-C₆)-alkyl, Heterocyclyl-(C₁-C₆)-alkylamino-(C₁-C₆)-alkyl, Heterocyclylamino-(C₁-C₆)-alkyl, Heteroarylamino-(C₁-C₆)-alkyl, (C₁-C₆)-Alkoxycarbonylamino-(C₁-C₆)-alkyl, Arylamino-(C₁-C₆)-alkyl, Aryl-(C₁-C₆)-alkoxycarbonylamino-(C₁-C₆)-alkyl, (C₃-C₆)-Cycloalkoxycarbonylamino-(C₁-C₆)-alkyl, (C₃-C₆)-Cycloalkyl-(C₁-C₆)-alkoxycarbonylamino-(C₁-C₆)-alkyl, Heteroaryl-(C₁-C₆)-alkoxycarbonylamino-(C₁-C₆)-alkyl, (C₁-C₆)-Alkylcarbonylamino-(C₁-C₆)-alkyl, (C₃-C₆)-Cycloalkylcarbonylamino-(C₁-C₆)-alkyl, Arylcarbonylamino-(C₁-C₆)-alkyl, Heteroarylcarbonylamino-(C₁-C₆)-alkyl, Heterocyclylcarbonylamino-(C₁-C₆)-alkyl, (C₂-C₆)-Alkenyloxycarbonylamino-(C₁-C₆)-alkyl, Aryl-(C₂-C₆)-Alkenylamino-(C₁-C₆)-alkyl, Arylsulfonyl-(C₁-C₆)-alkyl, Heteroarylsulfonyl-(C₁-C₆)-alkyl, (C₁-C₆)-Alkylsulfonyl-(C₁-C₆)-alkyl, (C₃-C₆)-Cycloalkylsulfonyl-(C₁-C₆)-alkyl, Arylsulfinyl-(C₁-C₆)-alkyl, Heteroarylsulfinyl-(C₁-C₆)-alkyl, (C₁-C₆)-Alkylsulfinyl-(C₁-C₆)-alkyl, (C₃-C₆)-Cycloalkylsulfinyl-(C₁-C₆)-alkyl, Bis[(C₁-C₆)-alkyl]amino-(C₁-C₆)-alkyl stehen oder
X und Y mit dem Kohlenstoffatom, an das sie gebunden sind, einen vollständig gesättigten oder teilgesättigten, gegebenenfalls durch O (Sauerstoff), S (Schwefel), N-H, (C₁-C₆)-Alkyl-N, (C₁-C₆)-Alkoxy-N, (C₁-C₆)-Alkoxycarbonyl-N, Aryl-(C₁-C₆)-alkoxycarbonyl-N unterbrochenen und gegebenenfalls weiter substituierten 3 bis 7-gliedrigen monocyclischen oder bicyclischen Ring bilden, wobei nicht mehr als zwei gleiche oder verschiedene Heteroatome aus der Gruppe O, S, N nebeneinander stehen.

3. Verwendung gemäß Anspruch 1, wobei die Verbindungen der allgemeinen Formel (I) durch die Formeln (la) bis (Iz) sowie (Iab) beschrieben werden 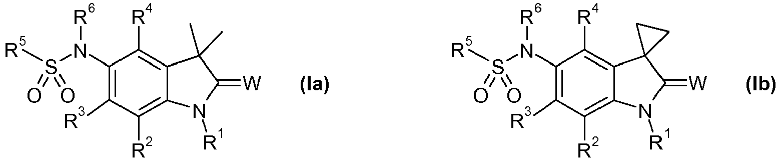 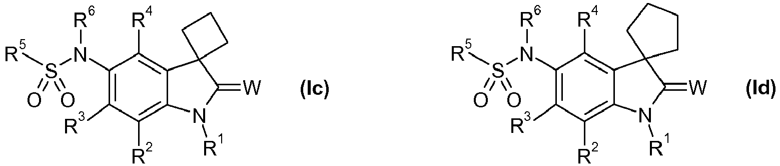 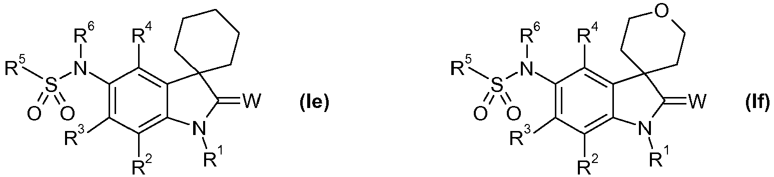 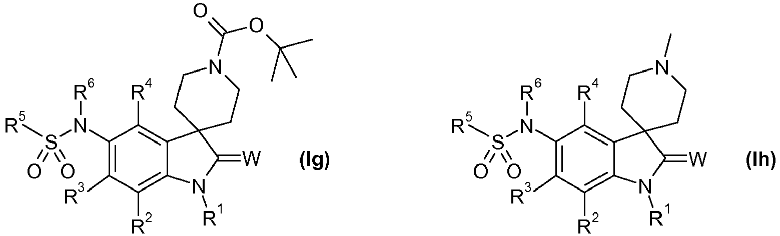 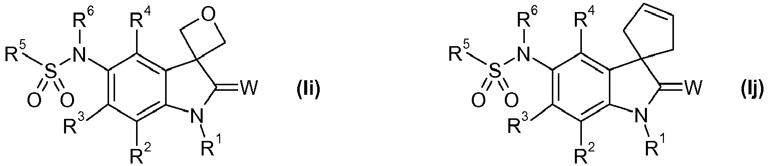 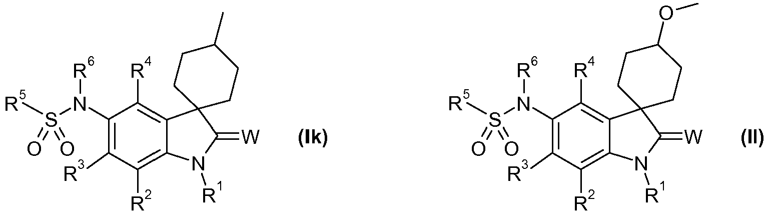 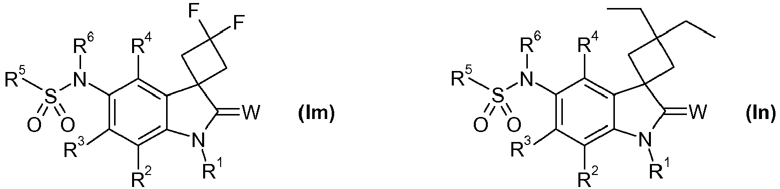 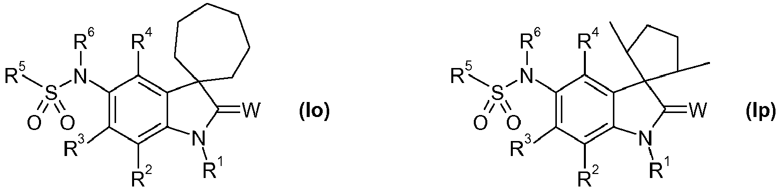 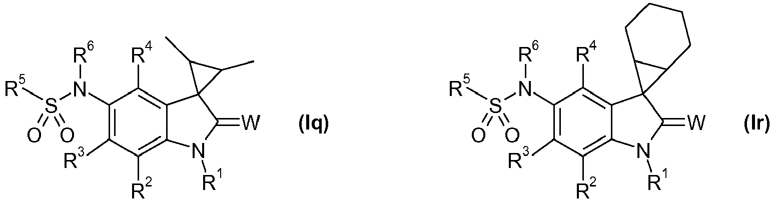 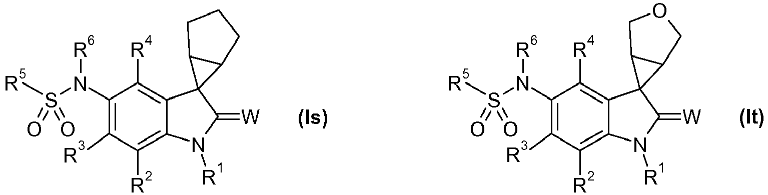 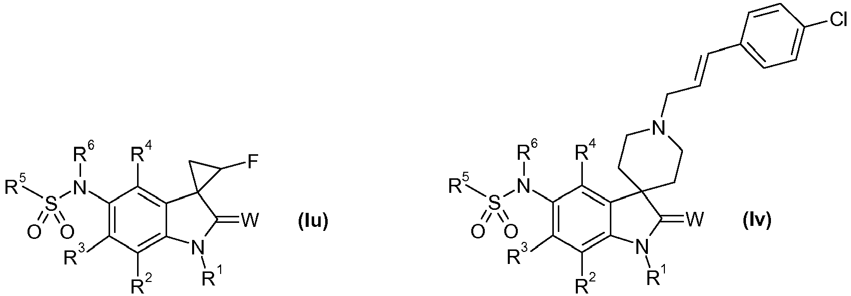 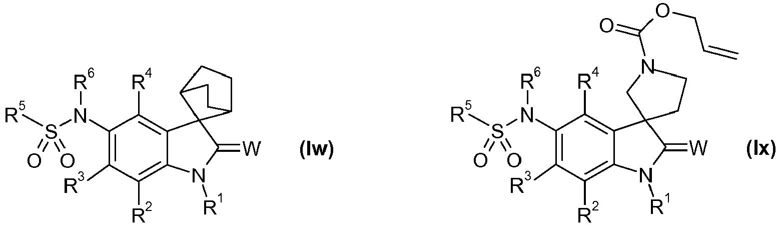 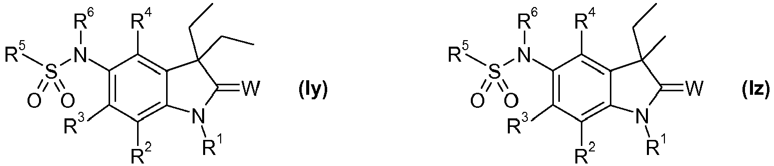 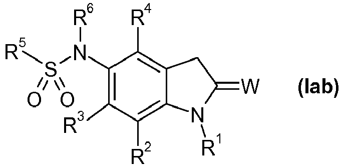 worin
R¹ Wasserstoff, (C₁-C₆)-Alkyl, (C₃-C₆)-Cycloalkyl, (C₁-C₆)-Haloalkyl, (C₃-C₆)-Halocycloalkyl, (C₂-C₆)-Alkenyl, (C₂-C₆)-Haloalkenyl, (C₁-C₅)-Alkoxy-(C₁-C₅)-haloalkyl, (C₂-C₅)-Alkinyl, Aryl, Aryl-(C₁-C₅)-alkyl, Heteroaryl, Heteroaryl-(C₁-C₅)-alkyl, (C₃-C₆)-Cycloalkyl-(C₁-C₅)-alkyl, (C₂-C₅)-Haloalkinyl, Heterocyclyl, Heterocyclyl-(C₁-C₅)-alkyl, (C₁-C₅)-Alkoxy-(C₁-C₅)-alkyl, (C₁-C₅)-Alkylcarbonyl-(C₁-C₅)-alkyl, Hydroxycarbonyl-(C₁-C₅)-alkyl, (C₁-C₅)-Alkoxycarbonyl-(C₁-C₅)-alkyl, (C₂-C₅)-Alkenyloxycarbonyl-(C₁-C₅)-alkyl, (C₂-C₅)-Alkinyloxycarbonyl-(C₁-C₅)-alkyl, Aryl-(C₁-C₅)-alkoxycarbonyl-(C₁-C₅)-alkyl, (C₃-C₆)-Cycloalkoxycarbonyl-(C₁-C₅)-alkyl, (C₃-C₆)-Cycloalkyl-(C₁-C₅)-alkoxycarbonyl-(C₁-C₅)-alkyl, Aminocarbonyl-(C₁-C₅)-alkyl, (C₁-C₅)-Alkylaminocarbonyl-(C₁-C₅)-alkyl, (C₃-C₆)-Cycloalkylaminocarbonyl-(C₁-C₅)-alkyl, Aryl-(C₁-C₅)-alkylaminocarbonyl-(C₁-C₅)-alkyl, Heteroaryl-(C₁-C₅)-alkylaminocarbonyl-(C₁-C₅)-alkyl, (C₁-C₅)-Alkoxy-(C₁-C₅)-alkoxy-(C₁-C₅)-alkyl, (C₁-C₅)-Alkylcarbonyl, (C₁-C₅)-Haloalkylcarbonyl, (C₃-C₆)-Cycloalkylcarbonyl, (C₁-C₅)-Alkoxycarbonyl, Aryl-(C₁-C₅)-alkoxycarbonyl, Arylcarbonyl, Heteroarylcarbonyl, Heterocyclylcarbonyl, Aryl-(C₁-C₆)-alkylcarbonyl, (C₁-C₆)-Alkylaminocarbonyl, (C₃-C₆)-Cycloalkylaminocarbonyl, Arylaminocarbonyl, Aryl-(C₁-C₆)-alkylaminocarbonyl, Heteroarylaminocarbonyl, Heterocyclylaminocarbonyl, Heteroaryl-(C₁-C₆)-alkylaminocarbonyl, Heterocyclyl-(C₁-C₆)-alkylaminocarbonyl, (C₁-C₆)-Alkylsulfonyl, (C₃-C₆)-Cycloalkylsulfonyl, Arylsulfonyl, Aryl-(C₁-C₆)-alkylsulfonyl, Heteroarylsulfonyl, Heterocyclylsulfonyl, Cyano-(C₁-C₅)-alkyl, Bis-[(C₁-C₅)-alkyl]amino, (C₃-C₆)-Cycloalkyl[(C₁-C₅)-akyl]amino steht,
R², R³, R⁴ unabhängig voneinander für Wasserstoff, Halogen, (C₁-C₅)-Alkoxy, (C₁-C₅)-Alkyl, (C₁-C₅)-Haloalkyl, (C₁-C₅)-Haloalkoxy, (C₁-C₅)-Alkylthio, (C₁-C₅)-Haloalkylthio, Aryl, Aryl-(C₁-C₅)-alkyl, Heteroaryl, Heteroaryl-(C₁-C₅)-alkyl, Heterocyclyl, Heterocyclyl-(C₁-C₅)-alkyl, (C₃-C₆)-Cycloalkyl, Nitro, Amino, Hydroxy, (C₁-C₅)-Alkylamino, Bis-[(C₁-C₅)-alkyl]amino, Hydrothio, (C₁-C₅)-Alkylcarbonylamino, (C₃-C₆)-Cycloalkylcarbonylamino, Arylcarbonylamino, Heteroarylcarbonylamino, Heterocyclylcarbonylamino, Formyl, Hydroxyiminomethyl, (C₁-C₅)-Alkoxyiminomethyl, (C₃-C₆)-Cycloalkoxyiminomethyl, Aryloxyiminomethyl, (C₃-C₆)-Cycloalkyl-(C₁-C₅)-alkoxyiminomethyl, Thiocyanato, Isothiocyanato, Aryloxy, Heteroaryloxy, (C₃-C₆)-Cycloalkoxy, (C₃-C₆)-Cycloalkyl-(C₁-C₅)-alkoxy, Aryl-(C₁-C₅)-alkoxy, (C₂-C₅)-Alkinyl, (C₂-C₅)-Alkenyl, Aryl-(C₁-C₅)-alkinyl, Tris-[(C₁-C₅)-alkyl]silyl-(C₂-C₅)-alkinyl, Bis-[(C₁-C₅)-alkyl](aryl)silyl-(C₂-C₅)-alkinyl, Bis-aryl[(C₁-C₅)-alkyl]silyl-(C₂-C₅)-alkinyl, (C₃-C₆)-Cycloalkyl-(C₂-C₅)-alkinyl, Aryl-(C₂-C₅)-alkenyl, Heteroaryl-(C₂-C₅)-alkenyl, (C₃-C₆)-Cycloalkyl-(C₂-C₅)-alkenyl, (C₂-C₅)-Haloalkinyl, (C₂-C₅)-Haloalkenyl, (C₄-C₅)-Cycloalkenyl, (C₁-C₅)-Alkoxy-(C₁-C₅)-alkoxy-(C₁-C₅)-alkyl, (C₁-C₅)-Alkylsulfonyl, Arylsulfonyl, Heteroarylsulfonyl, (C₁-C₅)-Alkylsulfonylamino, Arylsulfonylamino, Aryl-(C₁-C₅)-alkylsulfonylamino, Heteroarylsulfonylamino, Heteroaryl-(C₁-C₅)-alkylsulfonylamino, Bis-[(C₁-C₅)-Alkyl]aminosulfonyl stehen,
R⁵ für Amino, (C₁-C₅)-Alkyl, (C₃-C₆)-Cycloalkyl, (C₃-C₆)-Cycloalkyl-(C₁-C₅)-alkyl, (C₁-C₅)-Haloalkyl, (C₃-C₆)-Halocycloalkyl, (C₄-C₆)-Cycloalkenyl, Aryl, Heteroaryl, Heterocyclyl, Aryl-(C₁-C₅)-alkyl, Heteroaryl-(C₁-C₅)-alkyl, Heterocyclyl-(C₁-C₅)-alkyl, (C₁-C₅)-Alkoxycarbonyl-(C₁-C₅)-alkyl, Aryl-(C₁-C₅)-alkoxycarbonyl-(C₁-C₅)-alkyl, (C₃-C₆)-Cycloalkoxycarbonyl-(C₁-C₅)-alkyl, (C₃-C₆)-Cycloalkyl-(C₁-C₅)-alkoxycarbonyl-(C₁-C₅)-alkyl, Heteroaryl-(C₁-C₅)-alkoxycarbonyl-(C₁-C₅)-alkyl, Aminocarbonyl-(C₁-C₅)-alkyl, (C₁-C₅)-Alkylaminocarbonyl-(C₁-C₅)-alkyl, (C₃-C₆)-Cycloalkylaminocarbonyl-(C₁-C₅)-alkyl, Aryl-(C₁-C₅)-alkylaminocarbonyl-(C₁-C₅)-alkyl, (C₁-C₅)-Alkylamino, Arylamino, (C₃-C₆)-Cycloalkylamino, Aryl-(C₁-C₅)-alkylamino, Heteroaryl-(C₁-C₅)-alkylamino, Heteroarylamino, Heterocyclylamino, Aryloxy-(C₁-C₅)-alkyl, (C₁-C₅)-Alkoxy-(C₁-C₅)-alkyl, Heteroaryloxy-(C₁-C₅)-alkyl, (C₂-C₅)-Alkenyl, (C₂-C₅)-Alkinyl, (C₂-C₅)-Alkenylamino, (C₂-C₅)-Alkinylamino, Bis-[(C₁-C₅)-alkyl]amino, Aryloxy, (C₃-C₆)-Cycloalkyl-(C₂-C₅)-alkyl, Bis-[(C₁-C₅)-Alkyl]amino, Aryl-(C₂-C₅)-alkenyl, Heteroaryl-(C₂-C₅)-alkenyl, Heterocyclyl-(C₂-C₅)-alkenyl steht,
R⁶ für Wasserstoff, (C₁-C₅)-Alkyl, (C₃-C₆)-Cycloalkyl, Cyano-(C₁-C₅)-alkyl, (C₃-C₆)-Cycloalkyl-(C₁-C₅)-alkyl, (C₁-C₅)-Alkylsulfonyl, Arylsulfonyl, Heteroarylsulfonyl, (C₃-C₆)-Cycloalkylsulfonyl, Heterocyclylsulfonyl, Aryl-(C₁-C₅)-alkylsulfonyl, (C₁-C₅)-Alkylcarbonyl, Arylcarbonyl, Heteroarylcarbonyl, (C₃-C₆)-Cycloalkylcarbonyl, Heterocyclylcarbonyl, (C₁-C₅)-Alkoxycarbonyl, Aryl-(C₁-C₅)-alkoxycarbonyl, (C₁-C₅)-Haloalkylcarbonyl, (C₂-C₅)-Alkenyl, (C₂-C₅)-Alkinyl, (C₁-C₅)-Haloalkyl, Halo-(C₂-C₅)-alkinyl, Halo-(C₂-C₅)-alkenyl, (C₁-C₅)-Alkoxy-(C₁-C₅)-alkyl steht und
W für Sauerstoff oder Schwefel steht.

4. Behandlung von Pflanzen, umfassend die Applikation einer zur Steigerung der Widerstandsfähigkeit von Pflanzen gegenüber abiotischen Stressfaktoren wirksamen, nicht-toxischen Menge einer oder mehrerer der Verbindungen der Formel (I), oder jeweils deren Salze gemäß einem der Ansprüche 1 bis 3.

5. Behandlung gemäß Anspruch 4, wobei die abiotischen Stressbedingungen einer oder mehrerer Bedingungen ausgewählt aus der Gruppe bestehend aus Dürre, Kältestress, Hitzestress, Trockenstress, osmotischem Stress, Staunässe, erhöhtem Bodensalzgehalt, erhöhtem Ausgesetztsein an Mineralien, Ozonbedingungen, Starklichtbedingungen, beschränkter Verfügbarkeit von Stickstoffnährstoffen und beschränkter Verfügbarkeit von Phosphornährstoffen entsprechen.

6. Verwendung einer oder mehrerer Verbindungen der Formel (I), oder jeweils deren Salze gemäß einem der Ansprüche 1 bis 3 in der Sprühapplikation auf Pflanzen und Pflanzenteilen in Kombinationen mit einem oder mehrer Wirkstoffen ausgewählt aud der Gruppe der Insektizide, Lockstoffe, Akarizide, Fungizide, Nematizide, Herbizide, wachstumsregulatorische Stoffe, Safener, die Pflanzenreife beeinflussende Stoffe und Bakterizide.

7. Verwendung einer oder mehrerer Verbindungen der Formel (I), oder jeweils deren Salze gemäß einem der Ansprüche 1 bis 3 in der Sprühapplikation auf Pflanzen und Pflanzenteilen in Kombinationen mit Düngemitteln.

8. Verwendung einer oder mehrerer Verbindungen der Formel (I), oder jeweils deren Salze gemäß einem der Ansprüche 1 bis 3 zur Applikation auf gentechnisch veränderten Sorten, deren Saatgut, oder auf Anbauflächen auf denen diese Sorten wachsen.

9. Verwendung von Sprühlösungen, die eine oder mehrere der Verbindungen der Formel (I), oder jeweils deren Salze gemäß einem der Ansprüche 1 bis 3 enthalten, zur Steigerung der Widerstandsfähigkeit von Pflanzen gegenüber abiotischen Stressfaktoren.

10. Verfahren zur Erhöhung der Stresstoleranz bei Pflanzen ausgewählt aus der Gruppe der Nutzpflanzen, Zierpflanzen, Rasenarten, oder Bäumen, **dadurch gekennzeichnet, dass** die Applikation einer ausreichenden, nicht-toxischen Menge einer oder mehrere Verbindungen der Formel (I), oder jeweils deren Salze gemäß einem der Ansprüche 1 bis 3 auf die Fläche, wo die entsprechende Wirkung gewünscht wird, umfassend die Anwendung auf die Pflanzen, deren Saatgut oder auf die Fläche auf der die Pflanzen wachsen, erfolgt.

11. Verfahren gemäß Anspruch 10, wobei die Widerstandsfähigkeit der so behandelten Pflanzen gegenüber abiotischem Stress gegenüber nicht behandelten Pflanzen unter ansonsten gleichen physiologischen Bedingungen um mindestens 3% erhöht ist.

12. Substituierte Dihydrooxindolylsulfonamide gemäß der Formeln (Ib) bis (If), (Ii) bis (Iu) und (Iw) oder deren Salze 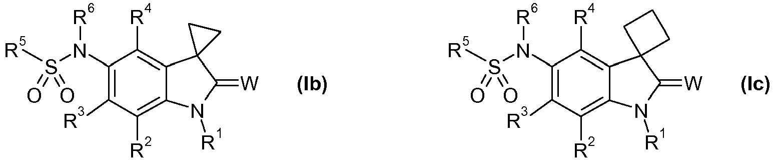 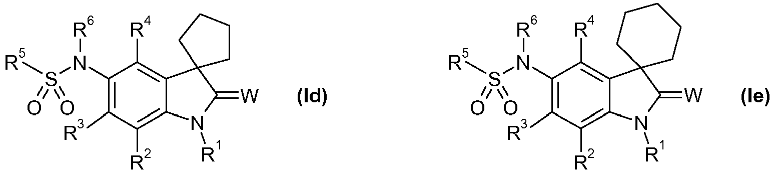 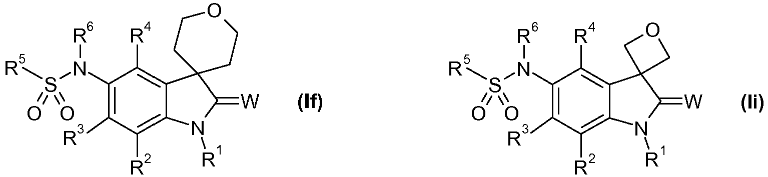 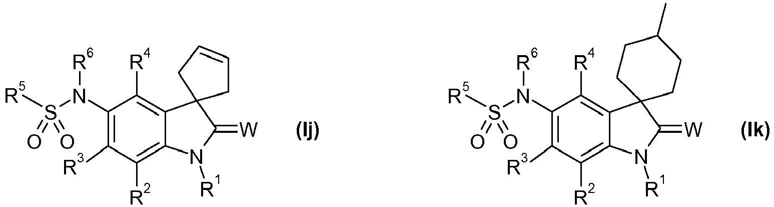 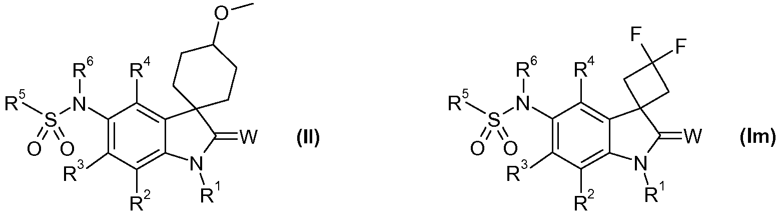 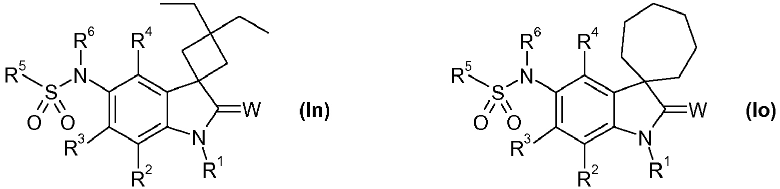 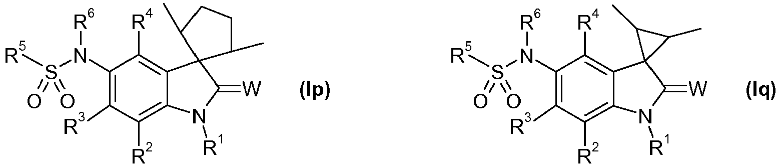 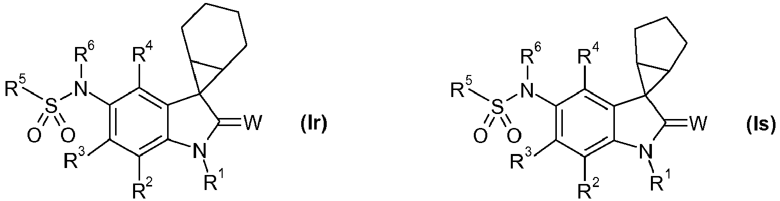 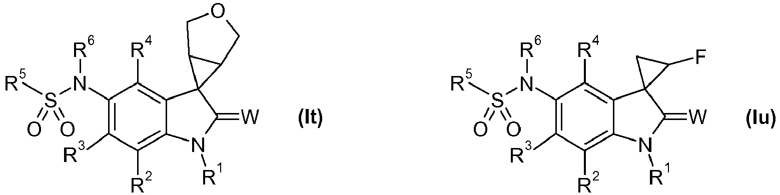 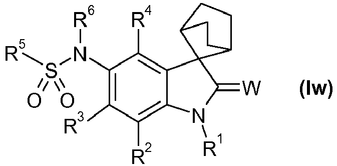 worin
R¹ für Wasserstoff, (C₁-C₆)-Alkyl, (C₃-C₆)-Cycloalkyl, (C₁-C₆)-Haloalkyl, (C₃-C₆)-Halocycloalkyl, (C₂-C₆)-Alkenyl, (C₂-C₆)-Haloalkenyl, (C₁-C₅)-Alkoxy-(C₁-C₅)-haloalkyl, (C₂-C₆)-Alkinyl, Aryl-(C₁-C₅)-alkyl, Heteroaryl-(C₁-C₅)-alkyl, (C₃-C₆)-Cycloalkyl-(C₁-C₅)-alkyl, (C₂-C₅)-Haloalkinyl, Heterocyclyl, Heterocyclyl-(C₁-C₅)-alkyl, (C₁-C₅)-Alkoxy-(C₁-C₅)-alkyl, (C₁-C₅)-Alkylcarbonyl-(C₁-C₅)-alkyl, Hydroxycarbonyl-(C₁-C₅)-alkyl, (C₁-C₅)-Alkoxycarbonyl-(C₁-C₅)-alkyl, (C₂-C₅)-Alkenyloxycarbonyl-(C₁-C₅)-alkyl, (C₂-C₅)-Alkinyloxycarbonyl-(C₁-C₅)-alkyl, Aryl-(C₁-C₅)-alkoxycarbonyl-(C₁-C₅)-alkyl, (C₃-C₆)-Cycloalkoxycarbonyl-(C₁-C₅)-alkyl, (C₃-C₆)-Cycloalkyl-(C₁-C₅)-alkoxycarbonyl-(C₁-C₅)-alkyl, Aminocarbonyl-(C₁-C₅)-alkyl, (C₁-C₅)-Alkylaminocarbonyl-(C₁-C₅)-alkyl, (C₃-C₆)-Cycloalkylaminocarbonyl-(C₁-C₅)-alkyl, Aryl-(C₁-C₅)-alkylaminocarbonyl-(C₁-C₅)-alkyl, Heteroaryl-(C₁-C₅)-alkylaminocarbonyl-(C₁-C₅)-alkyl, (C₁-C₅)-Alkylthio-(C₁-C₅)-alkyl, (C₃-C₆)-Cycloalkylthio-(C₁-C₅)-alkyl, Arylthio-(C₁-C₅)-alkyl, Heterocyclylthio-(C₁-C₅)-alkyl, Heteroarylthio-(C₁-C₅)-alkyl, Aryl-(C₁-C₅)-alkylthio-(C₁-C₅)-alkyl, (C₁-C₅)-Alkylsulfinyl-(C₁-C₅)-alkyl, (C₁-C₅)-Alkylsulfonyl-(C₁-C₅)-alkyl, Arylsulfinyl-(C₁-C₅)-alkyl, Arylsulfonyl-(C₁-C₅)-alkyl, (C₃-C₆)-Cycloalkylsulfinyl-(C₁-C₅)-alkyl, (C₃-C₆)-Cycloalkylsulfonyl-(C₁-C₅)-alkyl, (C₁-C₅)-Alkoxy-(C₁-C₅)-alkoxy-(C₁-C₅)-alkyl, (C₁-C₅)-Alkylcarbonyl, (C₃-C₆)-Cycloalkylcarbonyl, (C₁-C₅)-Alkoxycarbonyl, Aryl-(C₁-C₅)-alkoxycarbonyl, Arylcarbonyl, Heteroarylcarbonyl, Heterocyclylcarbonyl, Aryl-(C₁-C₅)-alkylcarbonyl, (C₁-C₅)-Alkylaminocarbonyl, (C₃-C₆)-Cycloalkylaminocarbonyl, Arylaminocarbonyl, Aryl-(C₁-C₅)-alkylaminocarbonyl, (C₁-C₅)-Alkylsulfonyl, (C₃-C₆)-Cycloalkylsulfonyl, Arylsulfonyl, Heteroarylsulfonyl, Heterocyclylsulfonyl, Cyano-(C₁-C₅)-alkyl, Bis-[(C₁-C₅)-alkyl]amino, (C₃-C₆)-Cycloalkyl[(C₁-C₅)-alkyl]amino steht,
R², R³, R⁴ unabhängig voneinander für Wasserstoff, Fluor, Chlor, Brom, Iod, (C₁-C₅)-Alkoxy, (C₁-C₅)-Alkyl, (C₁-C₅)-Haloalkyl, (C₁-C₅)-Haloalkoxy, (C₁-C₅)-Alkylthio, (C₁-C₅)-Haloalkylthio, Aryl, Heteroaryl, Heterocyclyl, (C₃-C₆)-Cycloalkyl stehen,
R⁵ für Amino, (C₁-C₅)-Alkyl, (C₃-C₆)-Cycloalkyl, (C₃-C₆)-Cycloalkyl-(C₁-C₅)-alkyl, (C₁-C₅)-Haloalkyl, (C₃-C₆)-Halocycloalkyl, (C₄-C₆)-Cycloalkenyl, gegebenenfalls substituiertes Phenyl, Heteroaryl, Heterocyclyl, Aryl-(C₁-C₅)-alkyl, Heteroaryl-(C₁-C₅)-alkyl, Heterocyclyl-(C₁-C₅)-alkyl, (C₁-C₅)-Alkoxycarbonyl-(C₁-C₅)-alkyl, Aryl-(C₁-C₅)-alkoxycarbonyl-(C₁-C₅)-alkyl, (C₃-C₆)-Cycloalkoxycarbonyl-(C₁-C₆)-alkyl, (C₃-C₆)-Cycloalkyl-(C₁-C₅)-alkoxycarbonyl-(C₁-C₅)-alkyl, Heteroaryl-(C₁-C₅)-alkoxycarbonyl-(C₁-C₅)-alkyl, Aminocarbonyl-(C₁-C₅)-alkyl, (C₁-C₅)-Alkylaminocarbonyl-(C₁-C₅)-alkyl, (C₃-C₆)-Cycloalkylaminocarbonyl-(C₁-C₅)-alkyl, Aryl-(C₁-C₅)-alkylaminocarbonyl-(C₁-C₅)-alkyl, (C₁-C₅)-Alkylamino, Bis-[(C₁-C₅)-alkyl]amino, Arylamino, (C₃-C₆)-Cycloalkylamino, Aryl-(C₁-C₅)-alkylamino, Heteroaryl-(C₁-C₅)-alkylamino, Heteroarylamino, Heterocyclylamino, (C₂-C₅)-Alkenylamino, (C₂-C₅)-Alkinylamino, Aryloxy-(C₁-C₅)-alkyl, Heteroaryloxy-(C₁-C₅)-alkyl, (C₁-C₅)-Alkoxy-(C₁-C₅)-alkyl, (C₂-C₆)-Alkenyl, (C₂-C₆)-Alkinyl, Cyano-(C₁-C₅)-alkyl, Aryloxy, Aryl-(C₂-C₅)-alkenyl, Heteroaryl-(C₂-C₅)-alkenyl, Heterocyclyl-(C₂-C₅)-alkenyl steht,
R⁶ für Wasserstoff, (C₁-C₅)-Alkyl, (C₃-C₆)-Cycloalkyl, Cyano-(C₁-C₅)-alkyl, (C₃-C₆)-Cycloalkyl-(C₁-C₅)-alkyl, (C₁-C₅)-Alkylsulfonyl, Arylsulfonyl, Aryl-(C₁-C₅)-alkylsulfonyl, Heteroarylsulfonyl, (C₃-C₆)-Cycloalkylsulfonyl, Heterocyclylsulfonyl, (C₁-C₅)-Alkylcarbonyl, Arylcarbonyl, Heteroarylcarbonyl, (C₃-C₆)-Cycloalkylcarbonyl, Heterocyclylcarbonyl, (C₁-C₅)-Alkoxycarbonyl, Aryl-(C₁-C₅)-alkoxycarbonyl, (C₁-C₅)-Haloalkylcarbonyl, (C₂-C₅)-Alkenyl, (C₂-C₅)-Alkinyl, (C₁-C₅)-Haloalkyl, Halo-(C₂-C₅)-alkinyl, Halo-(C₂-C₅)-alkenyl, (C₁-C₅)-Alkoxy-(C₁-C₅)-alkyl steht und
W für Sauerstoff oder Schwefel, bevorzugt Sauerstoff, steht.

13. Substituierte Dihydrooxindolylsulfonamide der Formeln (Ib) bis (Ie), (Ij) bis (Is) (Iu) und (Iw) oder deren Salze gemäß Anspruch 12, worin
R¹ für Wasserstoff, Methyl, Ethyl, n-Propyl, 1-Methylethyl, n-Butyl, 1-Me-thylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropyl, Cyclobutyl, Cyclopentyl, Cyclohexyl, Spiro[2.2]pent-1-yl, Spiro[2.3]hex-1-yl, Spiro[2.3]hex-4-yl, 3-Spiro[2.3]hex-5-yl, Spiro[3.3]hept-1-yl, Spiro[3.3]hept-2-yl, Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, Bicyclo[2.2.1]hept-2-yl, Bicyclo[2.2.2]octan-2-yl, Bicyclo[3.2.1]octan-2-yl, Bicyclo[3.2.2]nonan-2-yl, Adamantan-1-yl, Adamantan-2-yl, 1-Methylcyclopropyl, 2-Methylcyclopropyl, 2,2-Dimethylcyclopropyl, 2,3-Dimethylcyclopropyl, 1,1'-Bi(cyclopropyl)-1-yl, 1,1'-Bi(cyclopropyl)-2-yl, 2'-Methyl-1,1'-bi(cyclopropyl)-2-yl, 1-Cyanopropyl, 2-Cyanopropyl, 1-Methylcyclobutyl, 2-Methylcyclobutyl, 3-Methylcyclobutyl, 1-Cyanocyclobutyl, 2-Cyanocyclobutyl, 3-Cyanocyclobutyl, 1-Allylcyclopropyl, 1-Vinylcyclobutyl, 1-Vinylcyclopropyl, 1-Ethylcyclopropyl, 2-Ethylcyclopropyl, 1-Ethylcyclobutyl, 2-Ethylcyclobutyl, 3-Ethylcyclobutyl, 4-Methylcyclohexyl, 4-Methoxycyclohexyl, 4-Ethoxycyclohexyl, 4-n-Propyloxycyclohexyl, 4-Hydroxycyclohexyl, 4-Methoxycyclobutyl, 1-Cyclopropylcyclobutyl, 1-Prop-2-enylcyclobutyl, 2-Ethyl-3-Methylcyclobutyl,1-Propylcyclopropyl, 1-Methyl-2-propylcyclopropyl, 2-Propylcyclopropyl, 1-Propylcyclobutyl, 2-Propylcyclobutyl, 3-Propylcyclobutyl, 1-iso-Propylcyclobutyl, 1-iso-Propylcyclopropyl, 2-iso-Propylcyclopropyl, 3-iso-Propylcyclobutyl, 2-Dimethylaminocyclobutyl, 3-Dimethylaminocyclobutyl, 1-Butylcyclobutyl, 2-Butylcyclobutyl, 1-Butylcyclopropyl, 3-Butylcyclobutyl, 2-Butylcyclopropyl, 1-iso-Butylcyclobutyl, 3-tert-Butylcyclobutyl, 3,3-Diethylcyclobutyl, 2,2-Diethylcyclopropyl, 2-Methylidencyclopropyl, 1-Methoxymethylcyclopropyl, 1-iso-Butylcyclopropyl,2,2-Difluorethyl, 2,2,2-Trifluorethyl, 3,3,3-Trifluorpropyl, Ethenyl, 1-Propenyl, 2-Propenyl, 1-Methyl-ethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, Ethinyl, 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl, 1-Ethyl-1-methyl-2-propinyl, (C₃-C₆)-Halocycloalkyl, (C₂-C₅)-Haloalkenyl, (C₁-C₅)-Alkoxy-(C₁-C₅)-haloalkyl, Benzyl, p-Chlorbenzyl, p-Methoxybenzyl, p-Trifluormethylbenzyl, p-Methylbenzyl, p-Fluorbenzyl, p-Brombenzyl, p-lodbenzyl, p-Methylthiobenzyl, p-Trifluormethoxybenzyl, p-Nitrobenzyl, p-Trifluormethylthiobenzyl, m-Chlorbenzyl, m-Methoxybenzyl, m-Trifluormethylbenzyl, m-Methylbenzyl, m-Fluorbenzyl, m-Brombenzyl, m-lodbenzyl, m-Methylthiobenzyl, m-Trifluormethoxybenzyl, m-Nitrobenzyl, m-Trifluormethylthiobenzyl, o-Chlorbenzyl, o-Methoxybenzyl, o-Trifluormethylbenzyl, o-Methylbenzyl, o-Fluorbenzyl, o-Brombenzyl, o-lodbenzyl, o-Methylthiobenzyl, o-Trifluormethoxybenzyl, o-Nitrobenzyl, o-Trifluormethylthiobenzyl, p-Methoxycarbonylbenzyl, p-Ethoxycarbonylbenzyl, m-Methoxycarbonylbenzyl, m-Ethoxycarbonylbenzyl, 2,4-Dichlorbenzyl, 3,5-Dichlorbenzyl, 2,4-Difluorbenzyl, 3,5-Difluorbenzyl, 3,4-Dichlorbenzyl, 3,4-Difluorbenzyl, 2,5-Dichlorbenzyl, Phenylethyl, p-Chlorphenylethyl, p-Methoxyphenylethyl, p-Trifluormethylphenylethyl, p-Fluorphenylethyl, p-Trifluormethoxyphenylethyl, p-Trifluormethylthiophenylethyl, p-Methylphenylethyl, p-Nitrophenylethyl, p-Methoxycarbonylphenylethyl, p-Ethoxycarbonylphenylethyl, m-Chlorphenylethyl, m-Methoxyphenylethyl, m-Trifluormethylphenylethyl, m-Fluorphenylethyl, m-Trifluormethoxyphenylethyl, m-Trifluormethylthiophenylethyl, m-Methylphenylethyl, m-Nitrophenylethyl, m-Methoxycarbonylphenylethyl, m-Ethoxycarbonylphenylethyl, o-Chlorphenylethyl, o-Methoxyphenylethyl, o-Trifluormethylphenylethyl, o-Fluorphenylethyl, o-Trifluormethoxyphenylethyl, o-Trifluormethylthiophenylethyl, o-Methylphenylethyl, o-Nitrophenylethyl, o-Methoxycarbonylphenylethyl, o-Ethoxycarbonylphenylethyl, Heteroaryl-(C₁-C₅)-alkyl, (C₁-C₅)-Haloalkenyl, Heterocyclyl, Heterocyclyl-(C₁-C₅)-alkyl, (C₁-C₅)-Alkoxy-(C₁-C₅)-alkyl, (C₁-C₅)-Alkylcarbonyl-(C₁-C₅)-alkyl, Hydroxycarbonyl-(C₁-C₅)-alkyl, (C₁-C₅)-Alkoxycarbonyl-(C₁-C₅)-alkyl, (C₂-C₅)-Alkenyloxycarbonyl-(C₁-C₅)-alkyl, (C₂-C₅)-Alkinyloxycarbonyl-(C₁-C₅)-alkyl, Aryl-(C₁-C₅)-alkoxycarbonyl-(C₁-C₅)-alkyl, (C₃-C₆)-Cycloalkoxycarbonyl-(C₁-C₅)-alkyl, (C₃-C₆)-Cycloalkyl-(C₁-C₅)-alkoxycarbonyl-(C₁-C₅)-alkyl, Aminocarbonyl-(C₁-C₅)-alkyl, (C₁-C₅)-Alkylaminocarbonyl-(C₁-C₅)-alkyl, (C₃-C₆)-Cycloalkylaminocarbonyl-(C₁-C₅)-alkyl, Aryl-(C₁-C₅)-alkylaminocarbonyl-(C₁-C₅)-alkyl, Heteroaryl-(C₁-C₅)-alkylaminocarbonyl-(C₁-C₅)-alkyl, (C₁-C₅)-Alkylthio-(C₁-C₅)-alkyl, (C₃-C₆)-Cycloalkylthio-(C₁-C₅)-alkyl, Arylthio-(C₁-C₅)-alkyl, Heterocyclylthio-(C₁-C₅)-alkyl, Heteroarylthio-(C₁-C₈)-alkyl, Aryl-(C₁-C₅)-alkylthio-(C₁-C₅)-alkyl, (C₁-C₅)-Alkylsulfinyl-(C₁-C₅)-alkyl, (C₁-C₅)-Alkylsulfonyl-(C₁-C₅)-alkyl, Arylsulfinyl-(C₁-C₅)-alkyl, Arylsulfonyl-(C₁-C₅)-alkyl, (C₃-C₆)-Cycloalkylsulfinyl-(C₁-C₅)-alkyl, (C₃-C₆)-Cycloalkylsulfonyl-(C₁-C₅)-alkyl, (C₁-C₅)-Alkoxy-(C₁-C₅)-alkoxy-(C₁-C₅)-alkyl, (C₁-C₅)-Alkylcarbonyl, (C₃-C₆)-Cycloalkylcarbonyl, (C₁-C₅)-Alkoxycarbonyl, Aryl-(C₁-C₅)-alkoxycarbonyl, Arylcarbonyl, Heteroarylcarbonyl, Heterocyclylcarbonyl, Aryl-(C₁-C₅)-alkylcarbonyl, (C₁-C₅)-Alkylaminocarbonyl, (C₃-C₆)-Cycloalkylaminocarbonyl, Arylaminocarbonyl, Aryl-(C₁-C₅)-alkylaminocarbonyl, (C₁-C₅)-Alkylsulfonyl, (C₃-C₆)-Cycloalkylsulfonyl, Arylsulfonyl, Heteroarylsulfonyl, Heterocyclylsulfonyl, Bis-[(C₁-C₅)-alkyl]amino, (C₃-C₆)-Cycloalkyl[(C₁-C₅)-alkyl]amino steht,
R², R³, R⁴ unabhängig voneinander für Wasserstoff, Fluor, Chlor, Brom, Iod, Methoxy, Ethoxy, n-Propyloxy, iso-Propyloxy, Methyl, Ethyl, iso-Propyl, Trifluormethyl, Difluormethyl, Pentafluorethyl, Trifluormethoxy, Difluormethoxy,2,2-Difluorethoxy, 3,3,3-Trifluorethoxy, Methylthio, Ethylthio, Trifluormethylthio, gegebenenfalls substituiertes Phenyl, Heteroaryl, Heterocyclyl, Cyclopropyl, Cyclobutyl, Cyclopentyl, Cyclohexyl stehen,
R⁵ für Amino, Methyl, Ethyl, n-Propyl, 1-Methylethyl, n-Butyl, 1-Me-thylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropyl, Cyclobutyl, Cyclopentyl, Cyclohexyl, Spiro[2.2]pent-1-yl, Spiro[2.3]hex-1-yl, Spiro[2.3]hex-4-yl, 3-Spiro[2.3]hex-5-yl, Cyclopropylmethyl, Cyclobutylmethyl, Cyclopentylmethyl, Cyclohexylmethyl, Trifluormethyl, Difluormethyl, 2,2-Difluorethyl, 2,2,2-Trifluorethyl, 3,3,3-Trifluorpropyl, Pentafluorethyl, Heptafluor-n-Propyl, Heptafluor-iso-propyl, Nonafluor-n-butyl, (C₃-C₆)-Halocycloalkyl, (C₄-C₆)-Cycloalkenyl, gegebenenfalls substituiertes Phenyl, Heteroaryl, Heterocyclyl, Aryl-(C₁-C₅)-alkyl, Heteroaryl-(C₁-C₅)-alkyl, Heterocyclyl-(C₁-C₅)-alkyl, (C₁-C₅)-Alkoxycarbonyl-(C₁-C₅)-alkyl, Aryl-(C₁-C₅)-alkoxycarbonyl-(C₁-C₅)-alkyl, (C₁-C₆)-Cycloalkoxycarbonyl-(C₁-C₅)-alkyl, (C₃-C₆)-Cycloalkyl-(C₁-C₅)-alkoxycarbonyl-(C₁-C₅)-alkyl, Heteroaryl-(C₁-C₅)-alkoxycarbonyl-(C₁-C₅)-alkyl, Aminocarbonyl-(C₁-C₅)-alkyl, (C₁-C₅)-Alkylaminocarbonyl-(C₁-C₅)-alkyl, (C₃-C₆)-Cycloalkylaminocarbonyl-(C₁-C₅)-alkyl, Aryl-(C₁-C₅)-alkylaminocarbonyl-(C₁-C₅)-alkyl, (C₁-C₅)-Alkylamino, Arylamino, (C₃-C₆)-Cycloalkylamino, Aryl-(C₁-C₅)-alkylamino, Heteroaryl-(C₁-C₅)-alkylamino, Heteroarylamino, Heterocyclylamino, (C₂-C₅)-Alkenylamino, (C₂-C₅)-Alkinylamino, Aryloxy-(C₁-C₅)-alkyl, Heteroaryloxy-(C₁-C₅)-alkyl, (C₁-C₅)-Alkoxy-(C₁-C₅)-alkyl, Phenylethenyl, p-Chlorphenylethenyl, p-Methylphenylethenyl, p-Methoxyphenylethenyl, p-Trifluormethylphenylethenyl, p-Fluorphenylethenyl, p-Cyanophenylethenyl, p-Trifluormethoxyphenylethenyl, p-Nitrophenylethenyl, p-Bromphenylethenyl, p-Iodphenylethenyl, m-Chlorphenylethenyl, m-Methylphenylethenyl, m-Methoxyphenylethenyl, m-Trifluormethylphenylethenyl, m-Fluorphenylethenyl, m-Cyanophenylethenyl, m-Trifluormethoxyphenylethenyl, m-Nitrophenylethenyl, m-Bromphenylethenyl, m-Iodphenylethenyl, p-Methoxycarbonylphenylethenyl, m-Methoxycarbonylphenylethenyl, o-Methoxycarbonylphenylethenyl, p-Ethoxycarbonylphenylethenyl, m-Ethoxycarbonylphenylethenyl, o-Ethoxycarbonylphenylethenyl, Ethenyl, 1-Propenyl, 2-Propenyl, 1-Methyl-ethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl, 1-Ethyl-1-methyl-2-propinyl, Cyanoethyl, Cyanomethyl, Cyano-n-propyl, Cyano-n-Butyl, Aryloxy, Bis-[(C₁-C₅)-Alkyl]amino, Aryl-(C₂-C₅)-alkenyl, Heteroaryl-(C₂-C₅)-alkenyl, Heterocyclyl-(C₂-C₅)-alkenyl steht,
R⁶ für Wasserstoff, Methyl, Ethyl, n-Propyl, 1-Methylethyl, n-Butyl, 1-Me-thylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropyl, Cyclobutyl, Cyclopentyl, Cyclohexyl, Cyclopropylmethyl, Cyclopropylethyl, Cyclobutylmethyl, Cyclopentylmethyl, Cyclohexylmethyl, 2,2-Difluorethyl, 2,2,2-Trifluorethyl, 3,3,3-Trifluorpropyl, Cyanomethyl, Cyanoethyl, Cyano-n-propyl, Cyclopropyl,carbonyl, Cyclobutylcarbonyl, Cyclopentylcarbonyl, Cyclohexylcarbonyl, Methoxycarbonyl, (C₁-C₅)-Alkylsulfonyl, Arylsulfonyl, Aryl-(C₁-C₅)-alkylsulfonyl, Heteroarylsulfonyl, (C₃-C₆)-Cycloalkylsulfonyl, Heterocyclylsulfonyl, (C₁-C₅)-Alkylcarbonyl, Arylcarbonyl, Heteroarylcarbonyl, Heterocyclylcarbonyl, (C₁-C₅)-Alkoxycarbonyl, Aryl-(C₁-C₅)-alkoxycarbonyl, (C₁-C₅)-Haloalkylcarbonyl, (C₂-C₅)-Alkenyl, (C₂-C₅)-Alkinyl, Halo-(C₂-C₅)-alkinyl, Halo-(C₂-C₅)-alkenyl, (C₁-C₅)-Alkoxy-(C₁-C₅)-alkyl steht und
W für Sauerstoff oder Schwefel steht.

14. Substituierte Dihydrooxindolylsulfonamide der Formeln (Ib) bis (Ie), (Ij) bis (Is) (Iu) und (Iw) oder deren Salze gemäß Anspruch 12,
worin
R¹ für Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropyl, Cyclobutyl, Cyclopentyl, Cyclohexyl, Spiro[2.2]pent-1-yl, Spiro[2.3]hex-1-yl, Spiro[2.3]hex-4-yl, 3-Spiro[2.3]hex-5-yl, Spiro[3.3]hept-1-yl, Spiro[3.3]hept-2-yl, Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, Bicyclo[2.2.1]hept-2-yl, Bicyclo[2.2.2]octan-2-yl, Bicyclo[3.2.1]octan-2-yl, Bicyclo[3.2.2]nonan-2-yl, Adamantan-1-yl, Adamantan-2-yl, 1-Methylcyclopropyl, 2-Methylcyclopropyl, 2,2-Dimethylcyclopropyl, 2,3-Dimethylcyclopropyl, 1,1'-Bi(cyclopropyl)-1-yl, 1,1'-Bi(cyclopropyl)-2-yl, 2'-Methyl-1,1'-bi(cyclopropyl)-2-yl, 1-Cyanopropyl, 2-Cyanopropyl, 1-Methylcyclobutyl, 2-Methylcyclobutyl, 3-Methylcyclobutyl, 1-Cyanocyclobutyl, 2-Cyanocyclobutyl, 3-Cyanocyclobutyl, 1-Allylcyclopropyl, 1-Vinylcyclobutyl, 1-Vinylcyclopropyl, 1-Ethylcyclopropyl, 2-Ethylcyclopropyl, 1-Ethylcyclobutyl, 2-Ethylcyclobutyl, 3-Ethylcyclobutyl, 4-Methylcyclohexyl, 4-Methoxycyclohexyl, 4-Ethoxycyclohexyl, 4-n-Propyloxycyclohexyl, 4-Hydroxycyclohexyl, 4-Methoxycyclobutyl, 1-Cyclopropylcyclobutyl, 1-Prop-2-enylcyclobutyl, 2-Ethyl-3-Methylcyclobutyl,1-Propylcyclopropyl, 1-Methyl-2-propylcyclopropyl, 2-Propylcyclopropyl, 1-Propylcyclobutyl, 2-Propylcyclobutyl, 3-Propylcyclobutyl, 1-iso-Propylcyclobutyl, 1-iso-Propylcyclopropyl, 2-iso-Propylcyclopropyl, 3-iso-Propylcyclobutyl, 2-Dimethylaminocyclobutyl, 3-Dimethylaminocyclobutyl, 1-Butylcyclobutyl, 2-Butylcyclobutyl, 1-Butylcyclopropyl, 3-Butylcyclobutyl, 2-Butylcyclopropyl, 1-iso-Butylcyclobutyl, 3-tert-Butylcyclobutyl, 3,3-Diethylcyclobutyl, 2,2-Diethylcyclopropyl, 2-Methylidencyclopropyl, 1-Methoxymethylcyclopropyl, 1-iso-Butylcyclopropyl,2,2-Difluorethyl, 2,2,2-Trifluorethyl, 3,3,3-Trifluorpropyl, Ethenyl, 1-Propenyl, 2-Propenyl, 1-Methyl-ethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, Ethinyl, 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl, 1-Ethyl-1-methyl-2-propinyl, (C₃-C₆)-Halocycloalkyl, (C₂-C₅)-Haloalkenyl, (C₁-C₅)-Alkoxy-(C₁-C₅)-haloalkyl, Benzyl, p-Chlorbenzyl, p-Methoxybenzyl, p-Trifluormethylbenzyl, p-Methylbenzyl, p-Fluorbenzyl, p-Brombenzyl, p-lodbenzyl, p-Methylthiobenzyl, p-Trifluormethoxybenzyl, p-Nitrobenzyl, p-Trifluormethylthiobenzyl, m-Chlorbenzyl, m-Methoxybenzyl, m-Trifluormethylbenzyl, m-Methylbenzyl, m-Fluorbenzyl, m-Brombenzyl, m-Iodbenzyl, m-Methylthiobenzyl, m-Trifluormethoxybenzyl, m-Nitrobenzyl, m-Trifluormethylthiobenzyl, o-Chlorbenzyl, o-Methoxybenzyl, o-Trifluormethylbenzyl, o-Methylbenzyl, o-Fluorbenzyl, o-Brombenzyl, o-lodbenzyl, o-Methylthiobenzyl, o-Trifluormethoxybenzyl, o-Nitrobenzyl, o-Trifluormethylthiobenzyl, p-Methoxycarbonylbenzyl, p-Ethoxycarbonylbenzyl, m-Methoxycarbonylbenzyl, m-Ethoxycarbonylbenzyl, 2,4-Dichlorbenzyl, 3,5-Dichlorbenzyl, 2,4-Difluorbenzyl, 3,5-Difluorbenzyl, 3,4-Dichlorbenzyl, 3,4-Difluorbenzyl, 2,5-Dichlorbenzyl, Phenylethyl, p-Chlorphenylethyl, p-Methoxyphenylethyl, p-Trifluormethylphenylethyl, p-Fluorphenylethyl, p-Trifluormethoxyphenylethyl, p-Trifluormethylthiophenylethyl, p-Methylphenylethyl, p-Nitrophenylethyl, p-Methoxycarbonylphenylethyl, p-Ethoxycarbonylphenylethyl, m-Chlorphenylethyl, m-Methoxyphenylethyl, m-Trifluormethylphenylethyl, m-Fluorphenylethyl, m-Trifluormethoxyphenylethyl, m-Trifluormethylthiophenylethyl, m-Methylphenylethyl, m-Nitrophenylethyl, m-Methoxycarbonylphenylethyl, m-Ethoxycarbonylphenylethyl, o-Chlorphenylethyl, o-Methoxyphenylethyl, o-Trifluormethylphenylethyl, o-Fluorphenylethyl, o-Trifluormethoxyphenylethyl, o-Trifluormethylthiophenylethyl, o-Methylphenylethyl, o-Nitrophenylethyl, o-Methoxycarbonylphenylethyl, o-Ethoxycarbonylphenylethyl, Heteroaryl-(C₁-C₅)-alkyl, (C₁-C₅)-Haloalkenyl, Heterocyclyl, Heterocyclyl-(C₁-C₅)-alkyl, (C₁-C₅)-Alkoxy-(C₁-C₅)-alkyl, (C₁-C₅)-Alkylcarbonyl-(C₁-C₅)-alkyl, Hydroxycarbonyl-(C₁-C₅)-alkyl, (C₁-C₅)-Alkoxycarbonyl-(C₁-C₅)-alkyl, (C₂-C₅)-Alkenyloxycarbonyl-(C₁-C₅)-alkyl, (C₂-C₅)-Alkinyloxycarbonyl-(C₁-C₅)-alkyl, Aryl-(C₁-C₅)-alkoxycarbonyl-(C₁-C₅)-alkyl, (C₃-C₆)-Cycloalkoxycarbonyl-(C₁-C₅)-alkyl, (C₃-C₆)-Cycloalkyl-(C₁-C₅)-alkoxycarbonyl-(C₁-C₅)-alkyl, Aminocarbonyl-(C₁-C₅)-alkyl, (C₁-C₅)-Alkylaminocarbonyl-(C₁-C₅)-alkyl, (C₃-C₆)-Cycloalkylaminocarbonyl-(C₁-C₅)-alkyl, Aryl-(C₁-C₅)-alkylaminocarbonyl-(C₁-C₅)-alkyl, Heteroaryl-(C₁-C₅)-alkylaminocarbonyl-(C₁-C₅)-alkyl, (C₁-C₅)-Alkylthio-(C₁-C₅)-alkyl, (C₃-C₆)-Cycloalkylthio-(C₁-C₅)-alkyl, Arylthio-(C₁-C₅)-alkyl, Heterocyclylthio-(C₁-C₅)-alkyl, Heteroarylthio-(C₁-C₅)-alkyl, Aryl-(C₁-C₅)-alkylthio-(C₁-C₅)-alkyl, (C₁-C₅)-Alkylsulfinyl-(C₁-C₅)-alkyl, (C₁-C₅)-Alkylsulfonyl-(C₁-C₅)-alkyl, Arylsulfinyl-(C₁-C₅)-alkyl, Arylsulfonyl-(C₁-C₅)-alkyl, (C₃-C₆)-Cycloalkylsulfinyl-(C₁-C₅)-alkyl, (C₃-C₆)-Cycloalkylsulfonyl-(C₁-C₅)-alkyl, (C₁-C₅)-Alkoxy-(C₁-C₅)-alkoxy-(C₁-C₅)-alkyl, (C₁-C₅)-Alkylcarbonyl, (C₃-C₆)-Cycloalkylcarbonyl, (C₁-C₅)-Alkoxycarbonyl, Aryl-(C₁-C₅)-alkoxycarbonyl, Arylcarbonyl, Heteroarylcarbonyl, Heterocyclylcarbonyl, Aryl-(C₁-C₅)-alkylcarbonyl, (C₁-C₅)-Alkylaminocarbonyl, (C₃-C₆)-Cycloalkylaminocarbonyl, Arylaminocarbonyl, Aryl-(C₁-C₅)-alkylaminocarbonyl, (C₁-C₅)-Alkylsulfonyl, (C₃-C₆)-Cycloalkylsulfonyl, Arylsulfonyl, Heteroarylsulfonyl, Heterocyclylsulfonyl, Bis-[(C₁-C₅)-alkyl]amino, (C₃-C₆)-Cycloalkyl[(C₁-C₅)-alkyl]amino steht,
R², R³, R⁴ unabhängig voneinander für Wasserstoff, Fluor, Chlor, Brom, Iod, Methoxy, Ethoxy, n-Propyloxy, iso-Propyloxy, Methyl, Ethyl, iso-Propyl, Trifluormethyl, Difluormethyl, Pentafluorethyl, Trifluormethoxy, Difluormethoxy,2,2-Difluorethoxy, 3,3,3-Trifluorethoxy, Methylthio, Ethylthio, Trifluormethylthio, gegebenenfalls substituiertes Phenyl, Heteroaryl, Heterocyclyl, Cyclopropyl, Cyclobutyl, Cyclopentyl, Cyclohexyl stehen,
R⁵ für gegebenenfalls substituiertes Phenyl, Heteroaryl, Heterocyclyl, Aryl-(C₁-C₅)-alkyl, Heteroaryl-(C₁-C₅)-alkyl, Heterocyclyl-(C₁-C₅)-alkyl, (C₁-C₅)-Alkoxycarbonyl-(C₁-C₅)-alkyl, Aryl-(C₁-C₅)-alkoxycarbonyl-(C₁-C₅)-alkyl, (C₁-C₆)-Cycloalkoxycarbonyl-(C₁-C₅)-alkyl, (C₃-C₆)-Cycloalkyl-(C₁-C₅)-alkoxycarbonyl-(C₁-C₅)-alkyl, Heteroaryl-(C₁-C₅)-alkoxycarbonyl-(C₁-C₅)-alkyl, Aminocarbonyl-(C₁-C₅)-alkyl, (C₁-C₅)-Alkylaminocarbonyl-(C₁-C₅)-alkyl, (C₃-C₆)-Cycloalkylaminocarbonyl-(C₁-C₅)-alkyl, Aryl-(C₁-C₅)-alkylaminocarbonyl-(C₁-C₅)-alkyl, Aryloxy-(C₁-C₅)-alkyl, Heteroaryloxy-(C₁-C₅)-alkyl, Phenylethenyl, p-Chlorphenylethenyl, p-Methylphenylethenyl, p-Methoxyphenylethenyl, p-Trifluormethylphenylethenyl, p-Fluorphenylethenyl, p-Cyanophenylethenyl, p-Trifluormethoxyphenylethenyl, p-Nitrophenylethenyl, p-Bromphenylethenyl, p-lodphenylethenyl, m-Chlorphenylethenyl, m-Methylphenylethenyl, m-Methoxyphenylethenyl, m-Trifluormethylphenylethenyl, m-Fluorphenylethenyl, m-Cyanophenylethenyl, m-Trifluormethoxyphenylethenyl, m-Nitrophenylethenyl, m-Bromphenylethenyl, m-lodphenylethenyl, p-Methoxycarbonylphenylethenyl, m-Methoxycarbonylphenylethenyl, o-Methoxycarbonylphenylethenyl, p-Ethoxycarbonylphenylethenyl, m-Ethoxycarbonylphenylethenyl, o-Ethoxycarbonylphenylethenyl, Heteroaryl-(C₂-C₅)-alkenyl, Heterocyclyl-(C₂-C₅)-alkenyl steht,
R⁶ für Wasserstoff, Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropyl, Cyclobutyl, Cyclopentyl, Cyclohexyl, Cyclopropylmethyl, Cyclopropylethyl, Cyclobutylmethyl, Cyclopentylmethyl, Cyclohexylmethyl, 2,2-Difluorethyl, 2,2,2-Trifluorethyl, 3,3,3-Trifluorpropyl, Cyanomethyl, Cyanoethyl, Cyano-n-propyl, Cyclopropyl,carbonyl, Cyclobutylcarbonyl, Cyclopentylcarbonyl, Cyclohexylcarbonyl, Methoxycarbonyl, (C₁-C₅)-Alkylsulfonyl, Arylsulfonyl, Aryl-(C₁-C₅)-alkylsulfonyl, Heteroarylsulfonyl, (C₃-C₆)-Cycloalkylsulfonyl, Heterocyclylsulfonyl, (C₁-C₅)-Alkylcarbonyl, Arylcarbonyl, Heteroarylcarbonyl, Heterocyclylcarbonyl, (C₁-C₅)-Alkoxycarbonyl, Aryl-(C₁-C₅)-alkoxycarbonyl, (C₁-C₅)-Haloalkylcarbonyl, (C₂-C₅)-Alkenyl, (C₂-C₅)-Alkinyl, Halo-(C₂-C₅)-alkinyl, Halo-(C₂-C₅)-alkenyl, (C₁-C₅)-Alkoxy-(C₁-C₅)-alkyl steht und
W für Sauerstoff oder Schwefel steht.

15. Sprühlösung zur Behandlung von Pflanzen, enthaltend eine zur Steigerung der Widerstandsfähigkeit von Pflanzen gegenüber abiotischen Stressfaktoren wirksame Menge einer oder mehrerer der substituierten Dihydrooxindolylsulfonamide gemäß einem der Ansprüche 12, 13 oder 14.

## Claims

1. Use of substituted dihydrooxindolylsulfonamides or salts thereof 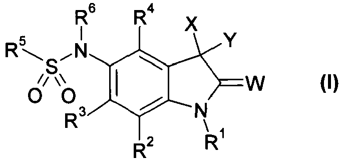 for increasing stress tolerance in plants to abiotic stress, and/or for increasing plant yield,
wherein
R¹ represents hydrogen, (C₁-C₁₀)-alkyl, (C₃-C₈)-cycloalkyl, (C₁-C₁₀)-haloalkyl, (C₃-C₈)-halocycloalkyl, (C₂-C₈)-alkenyl, (C₂-C₈)-haloalkenyl, (C₁-C₈) -alkoxy- (C₁-C₈)-haloalkyl, (C₂-C₈)-alkynyl, aryl, aryl-(C₁-C₈)-alkyl, heteroaryl, heteroaryl-(C₁-C₈)-alkyl, (C₃-C₈)-cycloalkyl-(C₁-C₈)-alkyl, (C₂-C₈)-haloalkynyl, heterocyclyl, heterocyclyl-(C₁-C₈)-alkyl, (C₁-C₈)-alkoxy-(C₁-C₈)-alkyl, (C₁-C₈)-alkylcarbonyl-(C₁-C₈)-alkyl, hydroxycarbonyl-(C₁-C₈)-alkyl, (C₁-C₈) -alkoxycarbonyl- (C₁-C₈)-alkyl, (C₂-C₈)-alkenyloxycarbonyl-(C₁-C₈)-alkyl, (C₂-C₈)-alkynyloxycarbonyl-(C₁-C₈)-alkyl, aryl-(C₁-C₈)-alkoxycarbonyl-(C₁-C₈)-alkyl, (C₃-C₈)-cycloalkoxycarbonyl-(C₁-C₈)-alkyl, (C₃-C₈)-cycloalkyl-(C₁-C₈)-alkoxycarbonyl-(C₁-C₈)-alkyl, aminocarbonyl-(C₁-C₈)-alkyl, (C₁-C₈)-alkylaminocarbonyl-(C₁-C₈)-alkyl, (C₃-C₈)-cycloalkylaminocarbonyl-(C₁-C₈)-alkyl, aryl-(C₁-C₈)-alkylaminocarbonyl-(C₁-C₈)-alkyl, heteroaryl-(C₁-C₈)-alkylaminocarbonyl-(C₁-C₈)-alkyl, (C₁-C₈)-alkylthio-(C₁-C₈)-alkyl, (C₃-C₈)-cycloalkylthio-(C₁-C₈)-alkyl, arylthio-(C₁-C₈)-alkyl, heterocyclylthio-(C₁-C₈)-alkyl, heteroarylthio-(C₁-C₈)-alkyl, aryl-(C₁-C₈)-alkylthio-(C₁-C₈)-alkyl, (C₁-C₈)-alkylsulfinyl-(C₁-C₈)-alkyl, (C₁-C₈)-alkylsulfonyl-(C₁-C₈)-alkyl, arylsulfinyl-(C₁-C₈)-alkyl, arylsulfonyl-(C₁-C₈)-alkyl, (C₃-C₈)-cycloalkylsulfinyl-(C₁-C₈)-alkyl, (C₃-C₈)-cycloalkylsulfonyl-(C₁-C₈)-alkyl, (C₁-C₈)-alkoxy-(C₁-C₈)-alkoxy-(C₁-C₈)-alkyl, (C₁-C₈)-alkylcarbonyl, (C₁-C₈)-haloalkylcarbonyl, (C₃-C₈)-cycloalkylcarbonyl, (C₁-C₈)-alkoxycarbonyl, aryl-(C₁-C₈)-alkoxycarbonyl, arylcarbonyl, heteroarylcarbonyl, heterocyclylcarbonyl, aryl-(C₁-C₈)-alkylcarbonyl, (C₁-C₈)-alkylaminocarbonyl, (C₃-C₈)-cycloalkylaminocarbonyl, arylaminocarbonyl, aryl-(C₁-C₈)-alkylaminocarbonylheteroarylaminocarbonyl, heterocyclylaminocarbonyl, heteroaryl-(C₁-C₈)-alkylaminocarbonyl, heterocyclyl-(C₁-C₈)-alkylaminocarbonyl, (C₁-C₈)-alkylsulfonyl, (C₃-C₈)-cycloalkylsulfonyl, arylsulfonyl, aryl-(C₁-C₈)-alkylsulfonyl, heteroarylsulfonyl, heterocyclylsulfonyl, cyano-(C₁-C₈)-alkyl, (C₄-C₈)-cycloalkenyl-(C₁-C₈)-alkyl, nitro-(C₁-C₈) -alkyl, halo-(C₁-C₈)-alkoxy-(C₁-C₈) -alkyl, bis-[(C₁-C₈)-alkyl]aminocarbonyl, (C₃-C₈)-cycloalkyl-[(C₁-C₈)-alkyl]aminocarbonyl, aryl-[(C₁-C₈)-alkyl]aminocarbonyl, aryl-(C₁-C₈)-alkyl-[(C₁-C₈)-alkyl]aminocarbonyl, (C₂-C₈)-alkenylaminocarbonyl, (C₂-C₈)-alkynylaminocarbonyl, (C₁-C₈)-alkylaminosulfonyl, bis-[(C₁-C₈)-alkyl]aminosulfonyl, heterocyclylsulfinyl-(C₁-C₈)-alkyl, heteroarylsulfinyl-(C₁-C₈)-alkyl, aryl-(C₁-C₈)-alkylsulfinyl-(C₁-C₈)-alkyl, heterocyclylsulfonyl-(C₁-C₈)-alkyl, heteroarylsulfonyl-(C₁-C₈)-alkyl, aryl-(C₁-C₈)-alkylsulfonyl-(C₁-C₈)-alkyl, bis-[(C₁-C₈)-alkyl] aminocarbonyl-(C₁-C₈)-alkyl, (C₃-C₈)-cycloalkyl-[(C₁-C₈)-alkyl]aminocarbonyl-(C₁-C₈)-alkyl, aryl-[(C₁-C₈)-alkyl]aminocarbonyl-(C₁-C₈)-alkyl, aryl-(C₁-C₈)-alkyl-[(C₁-C₈)-alkyl]aminocarbonyl-(C₁-C₈)-alkyl, (C₂-C₈)-alkenylaminocarbonyl-(C₁-C₈)-alkyl, (C₂-C₈)-alkynylaminocarbonyl-(C₁-C₈)-alkyl, (C₁-C₈)-alkylamino, bis-[(C₁-C₈)-alkyl]amino, (C₃-C₈)-cycloalkyl[(C₁-C₈)-alkyl]amino,
R², R³, R⁴ independently of one another represent hydrogen, halogen, (C₁-C₈)-alkoxy, (C₁-C₈)-alkyl, (C₁-C₈)-haloalkyl, (C₁-C₈)-haloalkoxy, (C₁-C₈)-alkylthio, (C₁-C₈)-haloalkylthio, aryl, aryl-(C₁-C₈)-alkyl, heteroaryl, heteroaryl-(C₁-C₈)-alkyl, heterocyclyl, heterocyclyl-(C₁-C₈)-alkyl, (C₃-C₈)-cycloalkyl, nitro, amino, hydroxy, (C₁-C₈)-alkylamino, bis-[(C₁-C₈)-alkyl]amino, hydrothio, (C₁-C₈)-alkylcarbonylamino, (C₃-C₈)-cycloalkylcarbonylamino, arylcarbonylamino, heteroarylcarbonylamino, heterocyclylcarbonylamino, formyl, hydroxyiminomethyl, (C₁-C₈)-alkoxyiminomethyl, (C₃-C₈)-cycloalkoxyiminomethyl, aryloxyiminomethyl, (C₃-C₈)-cycloalkyl-(C₁-C₈)-alkoxyiminomethyl, thiocyanato, isothiocyanato, aryloxy, heteroaryloxy, (C₃-C₈)-cycloalkoxy, (C₃-C₈)-cycloalkyl-(C₁-C₈)-alkoxy, aryl-(C₁-C₈)-alkoxy, (C₂-C₈)-alkynyl, (C₂-C₈)-alkenyl, aryl-(C₁-C₈)-alkynyl, tris-[(C₁-C₈)-alkyl]silyl-(C₂-C₈)-alkynyl, bis- [(C₁-C₈)-alkyl](aryl)silyl-(C₂-C₈)-alkynyl, bis-aryl[(C₁-C₈)-alkyl]silyl-(C₂-C₈)-alkynyl, (C₃-C₈)-cycloalkyl-(C₂-C₈)-alkynyl, aryl-(C₂-C₈)-alkenyl, heteroaryl-(C₂-C₈)-alkenyl, (C₃-C₈)-cycloalkyl-(C₂-C₈)-alkenyl, (C₃-C₈)-cycloalkyl-(C₂-C₈)-alkyl, (C₂-C₈)-haloalkynyl, (C₂-C₈)-haloalkenyl, (C₄-C₈)-cycloalkenyl, (C₁-C₈)-alkoxy-(C₁-C₈)-alkoxy-(C₁-C₈)-alkyl, (C₁-C₈)-alkylsulfonyl, arylsulfonyl, heteroarylsulfonyl, (C₁-C₈)-alkylsulfonylamino, arylsulfonylamino, aryl-(C₁-C₈)-alkylsulfonylamino, heteroarylsulfonylamino, heteroaryl-(C₁-C₈)-alkylsulfonylamino, bis-[(C₁-C₈)-alkyl]aminosulfonyl, (C₄-C₈-cycloalkenyl-(C₁-C₈)-alkyl, (C₁-C₈)-alkylsulfinyl, arylsulfinyl, heteroarylsulfinyl,
R⁵ represents amino, (C₁-C₈) -alkyl, (C₃-C₈)-cycloalkyl, (C₃-C₈)-cycloalkyl-(C₁-C₈)-alkyl, (C₁-C₈) -haloalkyl, (C₃-C₈)-halocycloalkyl, (C₄-C₈)-cycloalkenyl, aryl, heteroaryl, heterocyclyl, aryl-(C₁-C₈)-alkyl, heteroaryl-(C₁-C₈)-alkyl, heterocyclyl-(C₁-C₈)-alkyl, (C₁-C₈)-alkoxycarbonyl-(C₁-C₈)-alkyl, aryl-(C₁-C₈)-alkoxycarbonyl-(C₁-C₈)-alkyl, (C₃-C₈)-cycloalkoxycarbonyl-(C₁-C₈) -alkyl, (C₃-C₈)-cycloalkyl-(C₁-C₈)-alkoxycarbonyl-(C₁-C₈)-alkyl, heteroaryl-(C₁-C₈)-alkoxycarbonyl-(C₁-C₈)-alkyl, aminocarbonyl-(C₁-C₈)-alkyl, (C₁-C₈)-alkylaminocarbonyl-(C₁-C₈) -alkyl, (C₃-C₈)-cycloalkylaminocarbonyl-(C₁-C₈)-alkyl, aryl-(C₁-C₈)-alkylaminocarbonyl-(C₁-C₈)-alkyl, (C₁-C₈)-alkylamino, arylamino, (C₃-C₈)-cycloalkylamino, aryl-(C₁-C₈)-alkylamino, heteroaryl-(C₁-C₈)-alkylamino, heteroarylamino, heterocyclylamino, aryloxy-(C₁-C₈)-alkyl, (C₁-C₈)-alkoxy-(C₁-C₈)-alkyl, heteroaryloxy- (C₁-C₈) -alkyl, (C₂-C₈) -alkenyl, (C₂-C₈)-alkynyl, (C₂-C₈) -alkenylamino, (C₂-C₈)-alkynylamino, bis-[(C₁-C₈)-alkyl]amino, aryloxy, bis-[(C₁-C₈)-alkyl]amino, aryl-(C₂-C₈)-alkenyl, heteroaryl-(C₂-C₈)-alkenyl, heterocyclyl-(C₂-C₈)-alkenyl, aryloxycarbonyl-(C₁-C₈)-alkyl, heteroaryloxycarbonyl-(C₁-C₈)-alkyl, bis [(C₁-C₈)-alkyl]aminocarbonyl-(C₁-C₈)-alkyl, (C₁-C₈)-alkylthio-(C₁-C₈)-alkyl, cyano-(C₁-C₈)-alkyl, (C₁-C₈)-alkoxy-(C₁-C₈) -alkoxy-(C₁-C₈) -alkyl,
R⁶ represents hydrogen, (C₁-C₈)-alkyl, (C₃-C₈)-cycloalkyl, cyano-(C₁-C₈) -alkyl, (C₃-C₈)-cycloalkyl-(C₁-C₈)-alkyl, (C₁-C₈)-alkylsulfonyl, arylsulfonyl, heteroarylsulfonyl, (C₃-C₈)-cycloalkylsulfonyl, heterocyclylsulfonyl, aryl-(C₁-C₈)-alkylsulfonyl, (C₁-C₈)-alkylcarbonyl, arylcarbonyl, heteroarylcarbonyl, (C₃-C₈)-cycloalkylcarbonyl, heterocyclylcarbonyl, (C₁-C₈)-alkoxycarbonyl, aryl-(C₁-C₈)-alkoxycarbonyl, (C₁-C₈)-haloalkylcarbonyl, (C₂-C₈)-alkenyl, (C₂-C₈) -alkynyl, (C₁-C₈)-haloalkyl, halo-(C₂-C₈)-alkynyl, halo-(C₂-C₈)-alkenyl, (C₁-C₈)-alkoxy-(C₁-C₈)-alkyl,
W represents oxygen, sulfur and
X, Y independently of one another represent hydrogen, (C₁-C₈)-alkyl, halogen, (C₂-C₈)-alkenyl, (C₂-C₈)-alkynyl, (C₁-C₈)-haloalkyl, hydroxy-(C₁-C₈) -alkyl, cyano-(C₁-C₈) -alkyl, aryl, heteroaryl, (C₃-C₈)-cycloalkyl, (C₄-C₈)-cycloalkenyl, heterocyclyl, cyano, nitro, hydroxy, (C₁-C₈)-alkoxy, (C₁-C₈)-alkylthio, (C₁-C₈)-alkoxy-(C₁-C₈)-alkyl, (C₁-C₈)-alkylthio-(C₁-C₈)-alkyl, aryloxy, aryl-(C₁-C₈)-alkoxy, (C₁-C₈)-haloalkoxy, (C₁-C₈)-haloalkylthio, (C₁-C₈)-alkylamino, bis-[(C₁-C₈)-alkyl]amino, (C₁-C₈)-alkoxy-(C₁-C₈)-alkoxy, amino-(C₁-C₈)-alkyl, (C₁-C₈)-alkylamino-(C₁-C₈)-alkyl, (C₃-C₈)-cycloalkylamino-(C₁-C₈)-alkyl, aryl-(C₁-C₈)-alkylamino-(C₁-C₈)-alkyl, heteroaryl-(C₁-C₈)-alkylamino-(C₁-C₈)-alkyl, heterocyclyl-(C₁-C₈)-alkylamino-(C₁-C₈)-alkyl, heterocyclylamino-(C₁-C₈)-alkyl, heteroarylamino-(C₁-C₈)-alkyl, (C₁-C₈)-alkoxycarbonylamino-(C₁-C₈)-alkyl, arylamino-(C₁-C₈)-alkyl, aryl-(C₁-C₈)-alkoxycarbonylamino-(C₁-C₈)-alkyl, (C₃-C₈)-cycloalkoxycarbonylamino-(C₁-C₈)-alkyl, (C₃-C₈)-cycloalkyl-(C₁-C₈)-alkoxycarbonylamino-(C₁-C₈)-alkyl, heteroaryl-(C₁-C₈)-alkoxycarbonylamino-(C₁-C₈)-alkyl, (C₁-C₈)-alkylcarbonylamino-(C₁-C₈)-alkyl, (C₃-C₈)-cycloalkylcarbonylamino-(C₁-C₈)-alkyl, arylcarbonylamino-(C₁-C₈)-alkyl, heteroarylcarbonylamino-(C₁-C₈)-alkyl, heterocyclylcarbonylamino-(C₁-C₈)-alkyl, (C₂-C₈)-alkenyloxycarbonylamino-(C₁-C₈)-alkyl, aryl-(C₂-C₈)-alkenylamino-(C₁-C₈)-alkyl, arylsulfonyl-(C₁-C₈)-alkyl, heteroarylsulfonyl-(C₁-C₈)-alkyl, (C₁-C₈)-alkylsulfonyl- (C₁-C₈) -alkyl, (C₃-C₈)-cycloalkylsulfonyl-(C₁-C₈)-alkyl, arylsulfinyl-(C₁-C₈)-alkyl, heteroarylsulfinyl-(C₁-C₈)-alkyl, (C₁-C₈)-alkylsulfinyl-(C₁-C₈)-alkyl, (C₃-C₈)-cycloalkylsulfinyl-(C₁-C₈)-alkyl, bis [(C₁-C₈)-alkyl]amino-(C₁-C₈)-alkyl or
X and Y with the carbon atom to which they are attached form a fully saturated or partially saturated 3- to 7-membered monocyclic or bicyclic ring which is optionally interrupted by heteroatoms and optionally substituted further.

2. Use according to Claim 1, where, in formula (I),
R¹ represents hydrogen, (C₁-C₈)-alkyl, (C₃-C₆)-cycloalkyl, (C₁-C₈) -haloalkyl, (C₃-C₆)-halocycloalkyl, (C₂-C₆)-alkenyl, (C₂-C₆)-haloalkenyl, (C₁-C₆)-alkoxy-(C₁-C₆)-haloalkyl, (C₂-C₆)-alkynyl, aryl, aryl-(C₁-C₆)-alkyl, heteroaryl, heteroaryl-(C₁-C₆)-alkyl, (C₃-C₆)-cycloalkyl-(C₁-C₆)-alkyl, (C₂-C₆)-haloalkynyl, heterocyclyl, heterocyclyl-(C₁-C₆)-alkyl, (C₁-C₆)-alkoxy-(C₁-C₆)-alkyl, (C₁-C₆)-alkylcarbonyl-(C₁-C₆)-alkyl, hydroxycarbonyl-(C₁-C₆)-alkyl, (C₁-C₆)-alkoxycarbonyl-(C₁-C₆)-alkyl, (C₂-C₆)-alkenyloxycarbonyl-(C₁-C₆)-alkyl, (C₂-C₆)-alkynyloxycarbonyl-(C₁-C₆)-alkyl, aryl-(C₁-C₆)-alkoxycarbonyl-(C₁-C₆)-alkyl, (C₃-C₆)-cycloalkoxycarbonyl-(C₁-C₆)-alkyl, (C₃-C₆)-cycloalkyl-(C₁-C₆)-alkoxycarbonyl-(C₁-C₆)-alkyl, aminocarbonyl-(C₁-C₆)-alkyl, (C₁-C₆)-alkylaminocarbonyl-(C₁-C₆)-alkyl, (C₃-C₆)-cycloalkylaminocarbonyl-(C₁-C₆)-alkyl, aryl-(C₁-C₆)-alkylaminocarbonyl-(C₁-C₆)-alkyl, heteroaryl-(C₁-C₆)-alkylaminocarbonyl-(C₁-C₆)-alkyl, (C₁-C₆)-alkylthio-(C₁-C₆)-alkyl, (C₃-C₆)-cycloalkylthio-(C₁-C₆)-alkyl, arylthio-(C₁-C₆)-alkyl, heterocyclylthio-(C₁-C₆)-alkyl, heteroarylthio-(C₁-C₆)-alkyl, aryl-(C₁-C₆)-alkylthio-(C₁-C₈)-alkyl, (C₁-C₆)-alkylsulfinyl-(C₁-C₆)-alkyl, (C₁-C₆)-alkylsulfonyl-(C₁-C₆)-alkyl, arylsulfinyl-(C₁-C₆)-alkyl, arylsulfonyl-(C₁-C₆)-alkyl, (C₃-C₆)-cycloalkylsulfinyl-(C₁-C₆)-alkyl, (C₃-C₆)-cycloalkylsulfonyl-(C₁-C₆)-alkyl, (C₁-C₆)-alkoxy-(C₁-C₆)-alkoxy-(C₁-C₆)-alkyl, (C₁-C₆)-alkylcarbonyl, (C₁-C₆)-haloalkylcarbonyl, (C₃-C₆)-cycloalkylcarbonyl, (C₁-C₆)-alkoxycarbonyl, aryl-(C₁-C₆)-alkoxycarbonyl, arylcarbonyl, heteroarylcarbonyl, heterocyclylcarbonyl, aryl-(C₁-C₆)-alkylcarbonyl, (C₁-C₆)-alkylaminocarbonyl, (C₃-C₆)-cycloalkylaminocarbonyl, arylaminocarbonyl, aryl-(C₁-C₆)-alkylaminocarbonyl, heteroarylaminocarbonyl, heterocyclylaminocarbonyl, heteroaryl-(C₁-C₆)-alkylaminocarbonyl, heterocyclyl-(C₁-C₆)-alkylaminocarbonyl, (C₁-C₆)-alkylsulfonyl, (C₃-C₆)-cycloalkylsulfonyl, arylsulfonyl, aryl-(C₁-C₆)-alkylsulfonyl, heteroarylsulfonyl, heterocyclylsulfonyl, cyano-(C₁-C₆)-alkyl, (C₄-C₆)-cycloalkenyl-(C₁-C₆)-alkyl, nitro-(C₁-C₆)-alkyl, halo-(C₁-C₆)-alkoxy-(C₁-C₆)-alkyl, bis-[(C₁-C₆)-alkyl]aminocarbonyl, (C₃-C₆)-cycloalkyl-[(C₁-C₆)-alkyl]aminocarbonyl, aryl-[(C₁-C₆)-alkyl]aminocarbonyl, aryl-(C₁-C₆)-alkyl-[(C₁-C₆)-alkyl]aminocarbonyl, (C₂-C₆)-alkenylaminocarbonyl, (C₂-C₆)-alkynylaminocarbonyl, (C₁-C₆)-alkylaminosulfonyl, bis-[(C₁-C₆)-alkyl]aminosulfonyl, heterocyclylsulfinyl-(C₁-C₆)-alkyl, heteroarylsulfinyl-(C₁-C₈)-alkyl, aryl-(C₁-C₆)-alkylsulfinyl-(C₁-C₆)-alkyl, heterocyclylsulfonyl-(C₁-C₆)-alkyl, heteroarylsulfonyl-(C₁-C₆)-alkyl, aryl-(C₁-C₆)-alkylsulfonyl-(C₁-C₆)-alkyl, bis-[(C₁-C₆)-alkyl] aminocarbonyl-(C₁-C₆)-alkyl, (C₃-C₆)-cycloalkyl-[(C₁-C₆)-alkyl]aminocarbonyl-(C₁-C₆)-alkyl, aryl-[(C₁-C₆)-alkyl]aminocarbonyl-(C₁-C₆)-alkyl, aryl-(C₁-C₆)-alkyl-[(C₁-C₆)-alkyl]aminocarbonyl-(C₁-C₆)-alkyl, (C₂-C₆)-alkenylaminocarbonyl-(C₁-C₆)-alkyl, (C₂-C₆)-alkinylaminocarbonyl-(C₁-C₆)-alkyl, bis-[(C₁-C₆)-alkyl]amino, (C₃-C₆)-cycloalkyl[(C₁-C₆)-alkyl]amino,
R², R³, R⁴ independently of one another represent hydrogen, halogen, (C₁-C₆)-alkoxy, (C₁-C₆)-alkyl, (C₁-C₆)-haloalkyl, (C₁-C₆)-haloalkoxy, (C₁-C₆)-alkylthio, (C₁-C₆)-haloalkylthio, aryl, aryl-(C₁-C₆)-alkyl, heteroaryl, heteroaryl-(C₁-C₆)-alkyl, heterocyclyl, heterocyclyl-(C₁-C₆)-alkyl, (C₃-C₆)-cycloalkyl, nitro, amino, hydroxy, (C₁-C₆)-alkylamino, bis- (C₁-C₆)-alkyl]amino, hydrothio, (C₁-C₆)-alkylcarbonylamino, (C₃-C₆)-cycloalkylcarbonylamino, arylcarbonylamino, heteroarylcarbonylamino, heterocyclylcarbonylamino, formyl, hydroxyiminomethyl, (C₁-C₆)-alkoxyiminomethyl, (C₃-C₆)-cycloalkoxyiminomethyl, aryloxyiminomethyl, (C₃-C₆)-cycloalkyl-(C₁-C₆)-alkoxyiminomethyl, thiocyanato, isothiocyanato, aryloxy, heteroaryloxy, (C₃-C₆)-cycloalkoxy, (C₃-C₆)-cycloalkyl-(C₁-C₆)-alkoxy, aryl-(C₁-C₆)-alkoxy, (C₂-C₆)-alkynyl, (C₂-C₆)-alkenyl, aryl-(C₁-C₆)-alkynyl, tris-[(C₁-C₆)-alkyl]silyl-(C₂-C₆)-alkynyl, bis-[(C₁-C₆)-alkyl](aryl)silyl-(C₂-C₆)-alkynyl, bis-aryl [(C₁-C₆)-alkyl]silyl-(C₂-C₆)-alkynyl, (C₃-C₆)-cycloalkyl-(C₂-C₆)-alkynyl, aryl-(C₂-C₆)-alkenyl, heteroaryl-(C₂-C₆)-alkenyl, (C₃-C₆)-cycloalkyl-(C₂-C₆)-alkenyl, (C₃-C₆)-cycloalkyl-(C₂-C₆)-alkyl, (C₂-C₆)-haloalkynyl, (C₂-C₆)-haloalkenyl, (C₄-C₆) -cycloalkenyl, (C₁-C₆)-alkoxy-(C₁-C₆)-alkoxy-(C₁-C₆)-alkyl, (C₁-C₆)-alkylsulfonyl, arylsulfonyl, heteroarylsulfonyl, (C₁-C₆)-alkylsulfonylamino, arylsulfonylamino, aryl-(C₁-C₆)-alkylsulfonylamino, heteroarylsulfonylamino, heteroaryl-(C₁-C₆)-alkylsulfonylamino, bis-[(C₁-C₆)-alkyl]aminosulfonyl,
R⁵ represents amino, (C₁-C₆)-alkyl, (C₃-C₆)-cycloalkyl, (C₃-C₆)-cycloalkyl-(C₁-C₆)-alkyl, (C₁-C₆)-haloalkyl, (C₃-C₆)-halocycloalkyl, (C₄-C₆)-cycloalkenyl, aryl, heteroaryl, heterocyclyl, aryl-(C₁-C₆)-alkyl, heteroaryl-(C₁-C₆)-alkyl, heterocyclyl-(C₁-C₆)-alkyl, (C₁-C₆)-alkoxycarbonyl-(C₁-C₆)-alkyl, aryl-(C₁-C₆)-alkoxycarbonyl-(C₁-C₆)-alkyl, (C₃-C₆)-cycloalkoxycarbonyl-(C₁-C₆)-alkyl, (C₃-C₆)-cycloalkyl-(C₁-C₆)-alkoxycarbonyl-(C₁-C₆)-alkyl, heteroaryl-(C₁-C₆)-alkoxycarbonyl-(C₁-C₆)-alkyl, aminocarbonyl-(C₁-C₆)-alkyl, (C₁-C₆)-alkylaminocarbonyl-(C₁-C₆)-alkyl, (C₃-C₆)-cycloalkylaminocarbonyl-(C₁-C₆)-alkyl, aryl-(C₁-C₆)-alkylaminocarbonyl-(C₁-C₆)-alkyl, (C₁-C₆)-alkylamino, arylamino, (C₃-C₆)-cycloalkylamino, aryl-(C₁-C₆)-alkylamino, heteroaryl-(C₁-C₆)-alkylamino, heteroarylamino, heterocyclylamino, aryloxy-(C₁-C₆)-alkyl, (C₁-C₆)-alkoxy-(C₁-C₆)-alkyl, heteroaryloxy-(C₁-C₆)-alkyl, (C₂-C₆)-alkenyl, (C₂-C₆)-alkynyl, (C₂-C₆)-alkenylamino, (C₂-C₆)-alkynylamino, bis-[(C₁-C₆)-alkyl]amino, aryloxy, bis-[(C₁-C₇)-alkyl]amino, aryl-(C₂-C₇)-alkenyl, heteroaryl-(C₂-C₇)-alkenyl, heterocyclyl-(C₂-C₇)-alkenyl,
R⁶ represents hydrogen, (C₁-C₆)-alkyl, (C₃-C₆)-cycloalkyl, cyano-(C₁-C₆)-alkyl, (C₃-C₆)-cycloalkyl-(C₁-C₆)-alkyl, (C₁-C₆)-alkylsulfonyl, arylsulfonyl, heteroarylsulfonyl, (C₃-C₆)-cycloalkylsulfonyl, heterocyclylsulfonyl, aryl-(C₁-C₆-alkylsulfonyl, (C₁-C₆)-alkylcarbonyl, arylcarbonyl, heteroarylcarbonyl, (C₃-C₆)-cycloalkylcarbonyl, heterocyclylcarbonyl, (C₁-C₆)-alkoxycarbonyl, aryl-(C₁-C₆)-alkoxycarbonyl, (C₁-C₆)-haloalkylcarbonyl, (C₂-C₆)-alkenyl, (C₂-C₆)-alkynyl, (C₁-C₆)-haloalkyl, halo-(C₂-C₆)-alkynyl, halo-(C₂-C₆)-alkenyl, (C₁-C₆)-alkoxy-(C₁-C₆)-alkyl,
W represents oxygen, sulfur and
X, Y independently of one another represent hydrogen, (C₁-C₆)-alkyl, fluorine, chlorine, (C₂-C₆)-alkenyl, (C₁-C₆)-haloalkyl, (C₃-C₆)-cycloalkyl, (C₄-C₆)-cycloalkenyl, heterocyclyl, (C₁-C₆)-alkoxy, (C₁-C₆)-alkylthio, (C₁-C₆)-alkoxy- (C₁-C₆)-alkyl, (C₁-C₆)-alkylthio-(C₁-C₆)-alkyl, (C₁-C₆)-haloalkoxy, (C₁-C₆)-haloalkylthio, (C₁-C₆)-alkoxy-(C₁-C₆)-alkoxy, amino-(C₁-C₆)-alkyl, (C₁-C₆)-alkylamino-(C₁-C₆)-alkyl, (C₃-C₆)-cycloalkylamino-(C₁-C₆)-alkyl, aryl-(C₁-C₆)-alkylamino-(C₁-C₆)-alkyl, heteroaryl-(C₁-C₆)-alkylamino-(C₁-C₆)-alkyl, heterocyclyl-(C₁-C₆)-alkylamino-(C₁-C₆)-alkyl, heterocyclylamino-(C₁-C₆)-alkyl, heteroarylamino-(C₁-C₆)-alkyl, (C₁-C₆)-alkoxycarbonylamino-(C₁-C₆)-alkyl, arylamino-(C₁-C₆)-alkyl, aryl-(C₁-C₆)-alkoxycarbonylamino-(C₁-C₆)-alkyl, (C₃-C₆)-cycloalkoxycarbonylamino-(C₁-C₆)-alkyl, (C₃-C₆)-Cycloalkyl-(C₁-C₆)-alkoxycarbonylamino-(C₁-C₆)-alkyl, heteroaryl-(C₁-C₆)-alkoxycarbonylamino-(C₁-C₆)-alkyl, (C₁-C₆)-alkylcarbonylamino-(C₁-C₆)-alkyl, (C₃-C₆)-cycloalkylcarbonylamino-(C₁-C₆)-alkyl, arylcarbonylamino-(C₁-C₆)-alkyl, heteroarylcarbonylamino-(C₁-C₆)-alkyl, heterocyclylcarbonylamino-(C₁-C₆)-alkyl, (C₂-C₆)-alkenyloxycarbonylamino-(C₁-C₆)-alkyl, aryl-(C₂-C₆)-alkenylamino-(C₁-C₆)-alkyl, arylsulfonyl-(C₁-C₆)-alkyl, heteroarylsulfonyl-(C₁-C₆)-alkyl, (C₁-C₆)-alkylsulfonyl-(C₁-C₆)-alkyl, (C₃-C₆)-cycloalkylsulfonyl-(C₁-C₆)-alkyl, arylsulfinyl-(C₁-C₆)-alkyl, heteroarylsulfinyl-(C₁-C₆)-alkyl, (C₁-C₆)-alkylsulfinyl-(C₁-C₆)-alkyl, (C₃-C₆)-cycloalkylsulfinyl-(C₁-C₆)-alkyl, bis [(C₁-C₆)-alkyl]amino-(C₁-C₆)-alkyl or
X and Y with the carbon atom to which they are attached form a fully saturated or partially saturated 3- to 7-membered monocyclic or bicyclic ring which is optionally interrupted by O (oxygen), S (sulfur), N-H, (C₁-C₆)-alkyl-N, (C₁-C₆)-alkoxy-N, (C₁-C₆)-alkoxycarbonyl-N, aryl-(C₁-C₆)-alkoxycarbonyl-N and optionally substituted futher, where not more than two identical or different heteroatoms from the group consisting of O, S, N are adjacent to one another.

3. Use according to Claim 1 where the compounds of the general formula (I) are described by formulae (Ia) to (Iz) and (Iab) 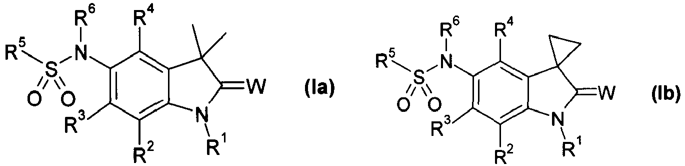 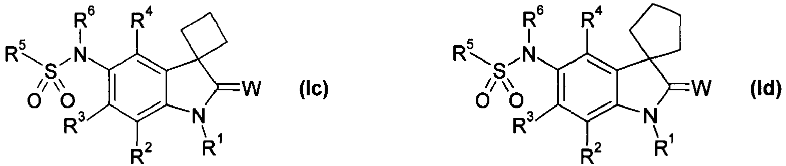 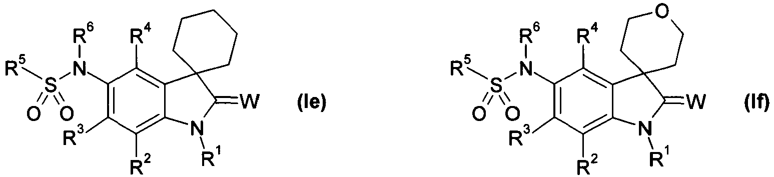 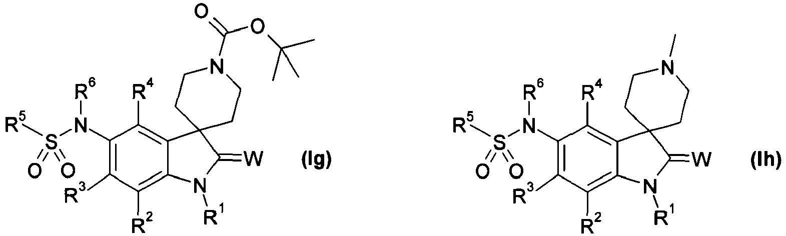 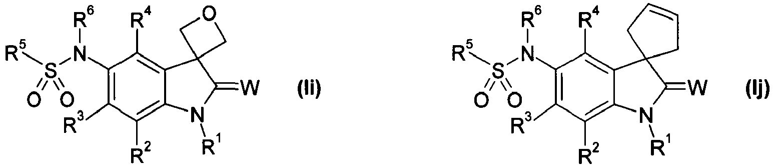 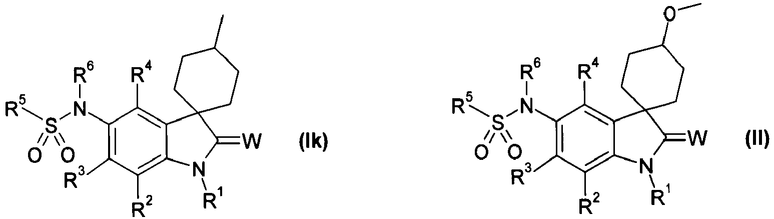 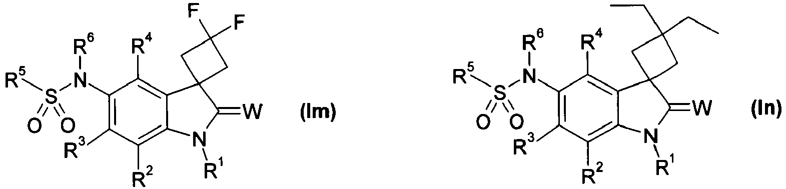 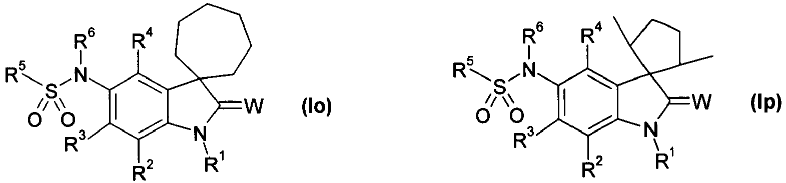 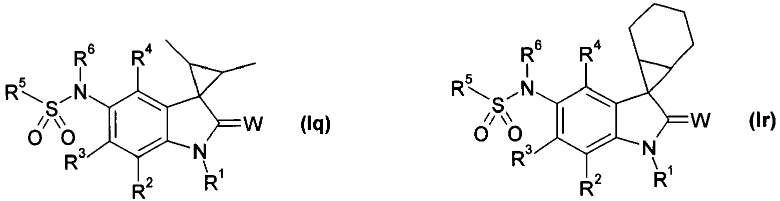 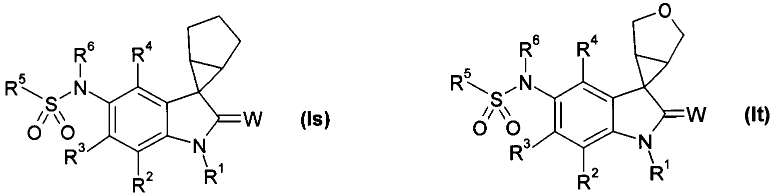 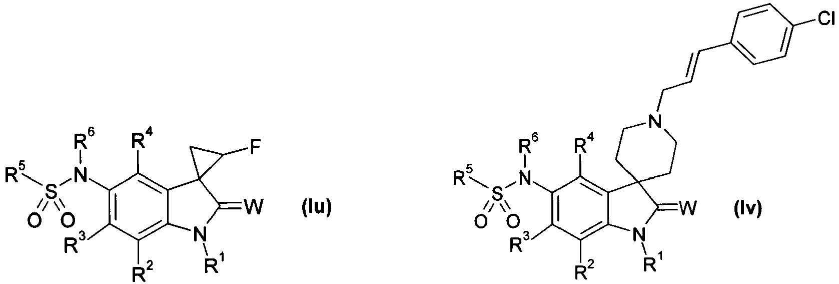 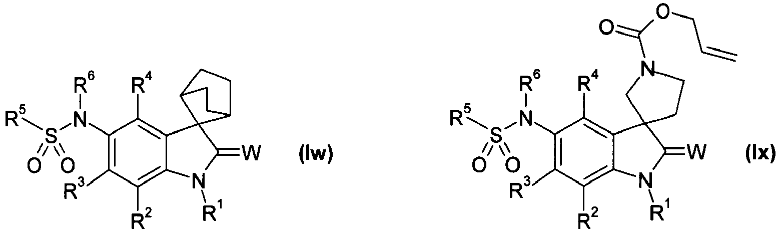 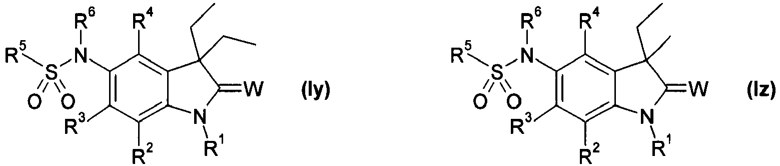 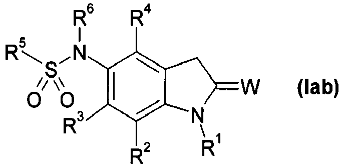 in which
R¹ represents hydrogen, (C₁-C₆)-alkyl, (C₃-C₆)-cycloalkyl, (C₁-C₆)-haloalkyl, (C₃-C₆)-halocycloalkyl, (C₂-C₆)-alkenyl, (C₂-C₆)-haloalkenyl, (C₁-C₅) -alkoxy-(C₁-C₅)-haloalkyl, (C₂-C₅)-alkynyl, aryl, aryl- (C₁-C₅) -alkyl, heteroaryl, heteroaryl-(C₁-C₅)-alkyl, (C₃-C₆)-cycloalkyl-(C₁-C₅)-alkyl, (C₂-C₅)-haloalkynyl, heterocyclyl, heterocyclyl-(C₁-C₅)-alkyl, (C₁-C₅)-alkoxy-(C₁-C₅)-alkyl, (C₁-C₅)-alkylcarbonyl-(C₁-C₅)-alkyl, hydroxycarbonyl-(C₁-C₆)-alkyl, (C₁-C₅)-alkoxycarbonyl-(C₁-C₅)-alkyl, (C₂-C₅)-alkenyloxycarbonyl-(C₁-C₅)-alkyl, (C₂-C₅)-alkynyloxycarbonyl-(C₁-C₅)-alkyl, aryl-(C₁-C₅)-alkoxycarbonyl-(C₁-C₅)-alkyl, (C₃-C₆)-cycloalkoxycarbonyl-(C₁-C₅)-alkyl, (C₃-C₆)-cycloalkyl-(C₁-C₅)-alkoxycarbonyl-(C₁-C₅)-alkyl, aminocarbonyl-(C₁-C₆)-alkyl, (C₁-C₅)-alkylaminocarbonyl-(C₁-C₅)-alkyl, (C₃-C₆)-cycloalkylaminocarbonyl-(C₁-C₆)-alkyl, aryl-(C₁-C₅)-alkylaminocarbonyl-(C₁-C₆)-alkyl, heteroaryl-(C₁-C₅)-alkylaminocarbonyl-(C₁-C₅)-alkyl, (C₁-C₅)-alkoxy-(C₁-C₅)-alkoxy-(C₁-C₅)-alkyl, (C₁-C₅)-alkylcarbonyl, (C₁-C₆)-haloalkylcarbonyl, (C₃-C₆)-cycloalkylcarbonyl, (C₁-C₅)-alkoxycarbonyl, aryl-(C₁-C₅)-alkoxycarbonyl, arylcarbonyl, heteroarylcarbonyl, heterocyclylcarbonyl, aryl-(C₁-C₆)-alkylcarbonyl, (C₁-C₆)-alkylaminocarbonyl, (C₃-C₆)-cycloalkylaminocarbonyl, arylaminocarbonyl, aryl-(C₁-C₆)-alkylaminocarbonyl, heteroarylaminocarbonyl, heterocyclylaminocarbonyl, heteroaryl-(C₁-C₆)-alkylaminocarbonyl, heterocyclyl-(C₁-C₆)-alkylaminocarbonyl, (C₁-C₆)-alkylsulfonyl, (C₃-C₆)-cycloalkylsulfonyl, arylsulfonyl, aryl-(C₁-C₆)-alkylsulfonyl, heteroarylsulfonyl, heterocyclylsulfonyl, cyano-(C₁-C₅)-alkyl, bis-[(C₁-C₅)-alkyl]amino, (C₃-C₆)-cycloalkyl[(C₁-C₅)-alkyl]amino,
R², R³, R⁴ independently of one another represent hydrogen, halogen, (C₁-C₅)-alkoxy, (C₁-C₅)-alkyl, (C₁-C₅)-haloalkyl, (C₁-C₅) -haloalkoxy, (C₁-C₅) -alkylthio, (C₁-C₅) -haloalkylthio, aryl, aryl-(C₁-C₅)-alkyl, heteroaryl, heteroaryl-(C₁-C₅)-alkyl, heterocyclyl, heterocyclyl-(C₁-C₆)-alkyl, (C₃-C₆)-cycloalkyl, nitro, amino, hydroxy, (C₁-C₅) -alkylamino, bis- [(C₁-C₅)-alkyl]amino, hydrothio, (C₁-C₅)-alkylcarbonylamino, (C₃-C₆)-cycloalkylcarbonylamino, arylcarbonylamino, heteroarylcarbonylamino, heterocyclylcarbonylamino, formyl, hydroxyiminomethyl, (C₁-C₅)-alkoxyiminomethyl, (C₃-C₆)-cycloalkoxyiminomethyl, aryloxyiminomethyl, (C₃-C₆)-cycloalkyl-(C₁-C₅)-alkoxyiminomethyl, thiocyanato, isothiocyanato, aryloxy, heteroaryloxy, (C₃-C₆)-cycloalkoxy, (C₃-C₆)-cycloalkyl-(C₁-C₅)-alkoxy, aryl-(C₁-C₅) -alkoxy, (C₂-C₅) -alkynyl, (C₂-C₅)-alkenyl, aryl-(C₁-C₅)-alkynyl, tris-[(C₁-C₅)-alkyl]silyl-(C₂-C₅)-alkynyl, bis-[(C₁-C₅)-alkyl] (aryl) silyl- (C₂-C₅) -alkynyl, bis-aryl[(C₁-C₅)-alkyl]silyl-(C₂-C₅)-alkynyl, (C₃-C₆)-cycloalkyl-(C₂-C₅)-alkynyl, aryl-(C₂-C₅)-alkenyl, heteroaryl-(C₂-C₅)-alkenyl, (C₃-C₆)-cycloalkyl-(C₂-C₅)-alkenyl, (C₂-C₅)-haloalkynyl, (C₂-C₅)-haloalkenyl, (C₄-C₅)-cycloalkenyl, (C₁-C₅)-alkoxy-(C₁-C₅)-alkoxy-(C₁-C₆)-alkyl, (C₁-C₅)-alkylsulfonyl, arylsulfonyl, heteroarylsulfonyl, (C₁-C₅)-alkylsulfonylamino, arylsulfonylamino, aryl-(C₁-C₅)-alkylsulfonylamino, heteroarylsulfonylamino, heteroaryl-(C₁-C₅)-alkylsulfonylamino, bis-[(C₁-C₅)-alkyl]aminosulfonyl,
R⁵ represents amino, (C₁-C₅)-alkyl, (C₃-C₆)-cycloalkyl, (C₃-C₆)-cycloalkyl-(C₁-C₅)-alkyl, (C₁-C₅)-haloalkyl, (C₃-C₆)-halocycloalkyl, (C₄-C₆)-cycloalkenyl, aryl, heteroaryl, heterocyclyl, aryl-(C₁-C₅)-alkyl, heteroaryl-(C₁-C₅)-alkyl, heterocyclyl-(C₁-C₅)-alkyl, (C₁-C₅)-alkoxycarbonyl-(C₁-C₅)-alkyl, aryl-(C₁-C₅)-alkoxycarbonyl-(C₁-C₅)-alkyl, (C₃-C₆)-cycloalkoxycarbonyl-(C₁-C₅)-alkyl,(C₃-C₆)-cycloalkyl-(C₁-C₅)-alkoxycarbonyl-(C₁-C₅)-alkyl, heteroaryl-(C₁-C₅)-alkoxycarbonyl-(C₁-C₅)-alkyl, aminocarbonyl-(C₁-C₅)-alkyl, (C₁-C₅) -alkylaminocarbonyl- (C₁-C₅)-alkyl, (C₃-C₆)-cycloalkylaminocarbonyl-(C₁-C₅)-alkyl, aryl-(C₁-C₅)-alkylaminocarbonyl-(C₁-C₅)-alkyl, (C₁-C₅)-alkylamino, arylamino, (C₃-C₆)-cycloalkylamino, aryl-(C₁-C₅)-alkylamino, heteroaryl-(C₁-C₅)-alkylamino, heteroarylamino, heterocyclylamino, aryloxy-(C₁-C₅)-alkyl, (C₁-C₅)-alkoxy- (C₁-C₅)-alkyl, heteroaryloxy- (C₁-C₅)-alkyl, (C₂-C₅)-alkenyl, (C₂-C₅)-alkynyl, (C₂-C₅)-alkenylamino, (C₂-C₅)-alkynylamino, bis-[(C₁-C₅)-alkyl]amino, aryloxy, (C₃-C₆) -cycloalkyl-(C₂-C₅)-alkyl, bis-[(C₁-C₅)-alkyl]amino, aryl-(C₂-C₅)-alkenyl, heteroaryl-(C₂-C₅)-alkenyl, heterocyclyl-(C₂-C₅)-alkenyl,
R⁶ represents hydrogen, (C₁-C₅)-alkyl, (C₃-C₆)-cycloalkyl, cyano-(C₁-C₅)-alkyl, (C₃-C₆)-cycloalkyl-(C₁-C₅)-alkyl, (C₁-C₅)-alkylsulfonyl, arylsulfonyl, heteroarylsulfonyl, (C₃-C₆)-cycloalkylsulfonyl, heterocyclylsulfonyl, aryl-(C₁-C₅)alkylsulfonyl, (C₁-C₅)-alkylcarbonyl, arylcarbonyl, heteroarylcarbonyl, (C₃-C₆)-cycloalkylcarbonyl, heterocyclylcarbonyl, (C₁-C₅)-alkoxycarbonyl, aryl-(C₁-C₅)-alkoxycarbonyl, (C₁-C₅)-haloalkylcarbonyl, (C₂-C₅)-alkenyl, (C₂-C₅)-alkynyl, (C₁-C₅)-haloalkyl, halo-(C₂-C₅)-alkynyl, halo-(C₂-C₅)-alkenyl, (C₁-C₅)-alkoxy-(C₁-C₅)-alkyl and
W represents oxygen or sulfur.

4. Treatment for plants, comprising the application of a nontoxic amount, effective for increasing the resistance of plants to abiotic stress factors, of one or more of the compounds of the formula (I) or their respective salts according to any of Claims 1 to 3.

5. Treatment according to Claim 4, wherein the abiotic stress conditions are one or more conditions selected from the group consisting of aridity, cold stress, heat stress, drought stress, osmotic stress, waterlogging, elevated soil salinity, elevated exposure to minerals, ozone conditions, strong light conditions, limited availability of nitrogen nutrients and limited availability of phosphorus nutrients.

6. Use of one or more compounds of the formula (I) or their respective salts according to any of Claims 1 to 3 in spray application to plants and parts of plants in combinations with one or more active compounds selected from the group of the insecticides, attractants, acaricides, fungicides, nematicides, herbicides, growth regulators, safeners, substances which affect plant maturity and bactericides.

7. Use of one or more compounds of the formula (I) or their respective salts according to any of Claims 1 to 3 in spray application to plants and parts of plants in combinations with fertilizers.

8. Use of one or more compounds of the formula (I) or their respective salts according to any of Claims 1 to 3 for application to genetically modified cultivars, the seed thereof, or to cultivated areas on which these cultivars grow.

9. Use of spray solutions comprising one or more of the compounds of the formula (I) or their respective salts according to any of Claims 1 to 3 for enhancing the resistance of plants to abiotic stress factors.

10. Method for increasing stress tolerance in plants selected from the group of useful plants, ornamental plants, turfgrasses and trees, **characterized in that** a sufficient nontoxic amount of one or more compounds of the formula (I) or their respective salts according to any of Claims 1 to 3 is applied to the area where the corresponding effect is desired, comprising application to the plants, the seed thereof or to the area on which the plants grow.

11. Method according to Claim 10, wherein the resistance of the plants thus treated to abiotic stress is increased by at least 3% compared to untreated plants under otherwise identical physiological conditions.

12. Substituted dihydrooxindolylsulfonamide according to formulae (Ib) to (If), (Ii) to (Iu) or (Iw) or a salt thereof 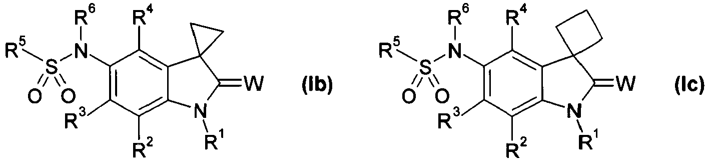 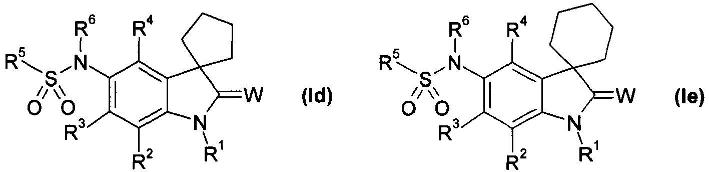 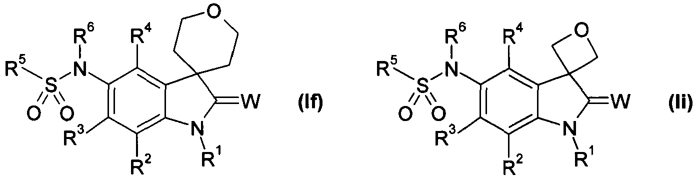 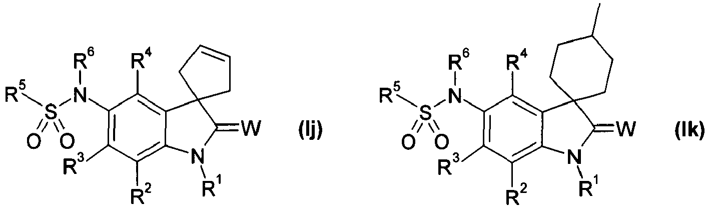 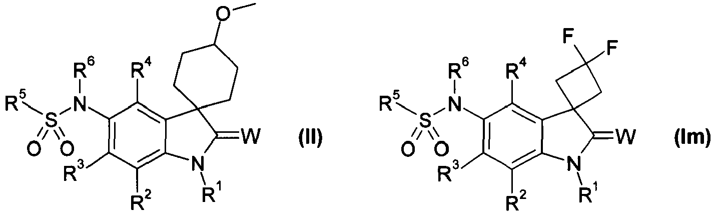 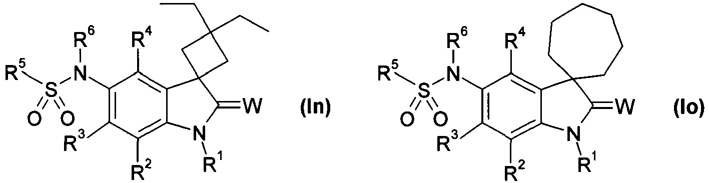 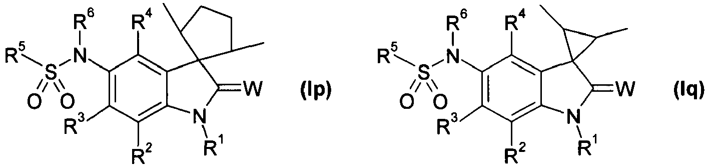 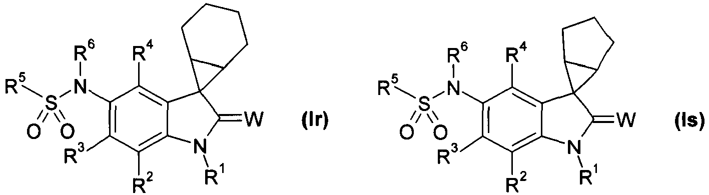 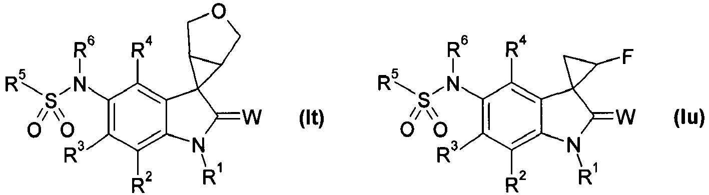 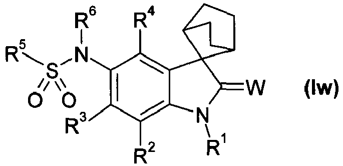 in which
R¹ represents hydrogen, (C₁-C₆) -alkyl, (C₃-C₆)-cycloalkyl, (C₁-C₆)-haloalkyl, (C₃-C₆)-halocycloalkyl, (C₂-C₆)-alkenyl, (C₂-C₆)-haloalkenyl, (C₁-C₅)-alkoxy- (C₁-C₅)-haloalkyl, (C₂-C₆)-alkynyl, aryl-(C₁-C₅) -alkyl, heteroaryl- (C₁-C₅)-alkyl, (C₃-C₆)-cycloalkyl-(C₁-C₅)-alkyl, (C₂-C₅)-haloalkynyl, heterocyclyl, heterocyclyl-(C₁-C₅)-alkyl, (C₁-C₅)-alkoxy-(C₁-C₅)-alkyl, (C₁-C₅)-alkylcarbonyl-(C₁-C₅)-alkyl, hydroxycarbonyl-(C₁-C₅)-alkyl, (C₁-C₅)-alkoxycarbonyl-(C₁-C₅)alkyl, (C₂-C₅) -alkenyloxycarbonyl- (C₁-C₅)-alkyl, (C₂-C₅)-alkynyloxycarbonyl-(C₁-C₅)-alkyl, aryl-(C₁-C₅)-alkoxycarbonyl-(C₁-C₅)-alkyl, (C₃-C₆)-cycloalkoxycarbonyl- (C₁-C₅)-alkyl, (C₃-C₆)-cycloalkyl-(C₁-C₅)-alkoxycarbonyl-(C₁-C₅)-alkyl, aminocarbonyl-(C₁-C₅)-alkyl, (C₁-C₅)-alkylaminocarbonyl-(C₁-C₅)-alkyl, (C₃-C₆)-cycloalkylaminocarbonyl-(C₁-C₅)-alkyl, aryl-(C₁-C₅)-alkylaminocarbonyl-(C₁-C₅)-alkyl, heteroaryl-(C₁-C₅)-alkylaminocarbonyl-(C₁-C₅)-alkyl, (C₁-C₅)-alkylthio-(C₁-C₅)-alkyl, (C₃-C₆-cycloalkylthio-(C₁-C₅)-alkyl, arylthio-(C₁-C₅)-alkyl, heterocyclylthio-(C₁-C₅)-alkyl, heteroarylthio-(C₁-C₅)-alkyl, aryl-(C₁-C₅)-alkylthio-(C₁-C₅)-alkyl, (C₁-C₅)-alkylsulfinyl- (C₁-C₅)-alkyl, (C₁-C₅)-alkylsulfonyl-(C₁-C₅)-alkyl, arylsulfinyl-(C₁-C₅)-alkyl, arylsulfonyl-(C₁-C₅)-alkyl, (C₃-C₆)-cycloalkylsulfinyl-(C₁-C₅)-alkyl, (C₃-C₆)-cycloalkylsulfonyl-(C₁-C₅)-alkyl, (C₁-C₅)-alkoxy-(C₁-C₅)-alkoxy-(C₁-C₅)-alkyl, (C₁-C₅)-alkylcarbonyl, (C₃-C₆)-cycloalkylcarbonyl, (C₁-C₅)-alkoxycarbonyl, aryl-(C₁-C₅)-alkoxycarbonyl, arylcarbonyl, heteroarylcarbonyl, heterocyclylcarbonyl, aryl-(C₁-C₅)-alkylcarbonyl, (C₁-C₅)-alkylaminocarbonyl, (C₃-C₆)-cycloalkylaminocarbonyl, arylaminocarbonyl, aryl-(C₁-C₅)-alkylaminocarbonyl, (C₁-C₅)-alkylsulfonyl, (C₃-C₆)-cycloalkylsulfonyl, arylsulfonyl, heteroarylsulfonyl, heterocyclylsulfonyl, cyano-(C₁-C₅)-alkyl, bis-[(C₁-C₅)-alkyl]amino, (C₃-C₆)-cycloalkyl[(C₁-C₅)-alkyl]amino,
R², R³, R⁴ independently of one another represent hydrogen, fluorine, chlorine, bromine, iodine, (C₁-C₅)-alkoxy, (C₁-C₅)-alkyl, (C₁-C₅)-haloalkyl, (C₁-C₅)-haloalkoxy, (C₁-C₅)-alkylthio, (C₁-C₅)-haloalkylthio, aryl, heteroaryl, heterocyclyl, (C₃-C₆)-cycloalkyl,
R⁵ represents amino, (C₁-C₅)-alkyl, (C₃-C₆)-cycloalkyl, (C₃-C₆) -cycloalkyl- (C₁-C₅)-alkyl, (C₁-C₅)-haloalkyl, (C₃-C₆) -halocycloalkyl, (C₄-C₆)-cycloalkenyl, optionally substituted phenyl, heteroaryl, heterocyclyl, aryl-(C₁-C₅)-alkyl, heteroaryl-(C₁-C₅)-alkyl, heterocyclyl-(C₁-C₅)-alkyl, (C₁-C₅)-alkoxycarbonyl-(C₁-C₅)-alkyl, aryl-(C₁-C₅) -alkoxycarbonyl- (C₁-C₅) -alkyl, (C₃-C₆)-cycloalkoxycarbonyl- (C₁-C₆) -alkyl, (C₃-C₆)-cycloalkyl-(C₁-C₅)-alkoxycarbonyl-(C₁-C₅)-alkyl, heteroaryl-(C₁-C₅) -alkoxycarbonyl-(C₁-C₅)-alkyl, aminocarbonyl-(C₁-C₅)-alkyl, (C₁-C₅)-alkylaminocarbonyl-(C₁-C₅)-alkyl, (C₃-C₆)-cycloalkylaminocarbonyl-(C₁-C₅)-alkyl, aryl-(C₁-C₅) -alkylaminocarbonyl-(C₁-C₅)-alkyl, (C₁-C₅)-alkylamino, bis-[(C₁-C₅)-alkyl]amino, arylamino, (C₃-C₆) -cycloalkylamino, aryl-(C₁-C₅)-alkylamino, heteroaryl-(C₁-C₅)-alkylamino, heteroarylamino, heterocyclylamino, (C₂-C₅)-alkenylamino, (C₂-C₅)-alkynylamino, aryloxy-(C₁-C₅)-alkyl, heteroaryloxy-(C₁-C₅)-alkyl, (C₁-C₅)-alkoxy-(C₁-C₅)-alkyl, (C₂-C₆)-alkenyl, (C₂-C₆) -alkynyl, cyano-(C₁-C₅)-alkyl, aryloxy, aryl-(C₂-C₅)-alkenyl, heteroaryl-(C₂-C₅)-alkenyl, heterocyclyl-(C₂-C₅)-alkenyl,
R⁶ represents hydrogen, (C₁-C₅)-alkyl, (C₃-C₆)-cycloalkyl, cyano- (C₁-C₅)-alkyl, (C₃-C₆)-cycloalkyl-(C₁-C₅)-alkyl, (C₁-C₅)-alkylsulfonyl, arylsulfonyl, aryl-(C₁-C₅)-alkylsulfonyl, heteroarylsulfonyl, (C₃-C₆)-cycloalkylsulfonyl, heterocyclylsulfonyl, (C₁-C₅)-alkylcarbonyl, arylcarbonyl, heteroarylcarbonyl, (C₃-C₆)-cycloalkylcarbonyl, heterocyclylcarbonyl, (C₁-C₅)-alkoxycarbonyl, aryl-(C₁-C₅)-alkoxycarbonyl, (C₁-C₅)-haloalkylcarbonyl, (C₂-C₅)-alkenyl, (C₂-C₅)-alkynyl, (C₁-C₅)-haloalkyl, halo-(C₂-C₅)-alkynyl, halo-(C₂-C₅)-alkenyl, (C₁-C₅)-alkoxy-(C₁-C₅)-alkyl and
W represents oxygen or sulfur, preferably oxygen.

13. Substituted dihydrooxindolylsulfonamide of formulae (Ib) to (Ie), (Ij) to (Is) (lu) and (Iw) or a salt thereof according to Claim 12 in which
R¹ represents hydrogen, methyl, ethyl, n-propyl, 1-methylethyl, n-butyl, 1-methylpropyl, 2-methylpropyl, 1,1-dimethylethyl, n-pentyl, 1-methylbutyl, 2-methylbutyl, 3-methylbutyl, 1,1-dimethylpropyl, 1,2-dimethylpropyl, 2,2-dimethylpropyl, 1-ethylpropyl, n-hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, cyclopropyl, cyclobutyl, cyclopentyl, cyclohexyl, spiro[2.2]pent-1-yl, spiro[2.3]hex-1-yl, spiro[2.3]hex-4-yl, 3-spiro[2.3]hex-5-yl, spiro[3.3]hept-1-yl, spiro[3.3]hept-2-yl, bicyclo[1.1.0]butan-1-yl, bicyclo[1.1.0]butan-2-yl, bicyclo[2.1.0]pentan-1-yl, bicyclo[1.1.1]pentan-1-yl, bicyclo[2.1.0]pentan-2-yl, bicyclo[2.1.0]pentan-5-yl, bicyclo[2.1.1]hexyl, bicyclo[2.2.1]hept-2-yl, bicyclo[2.2.2]octan-2-yl, bicyclo[3.2.1]octan-2-yl, bicyclo[3.2.2]nonan-2-yl, adamantan-1-yl, adamantan-2-yl, 1-methylcyclopropyl, 2-methylcyclopropyl, 2,2-dimethylcyclopropyl, 2,3-dimethylcyclopropyl, 1,1'-bi(cyclopropyl)-1-yl, 1,1'-bi(cyclopropyl)-2-yl, 2'-methyl-1,1'-bi(cyclopropyl)-2-yl, 1-cyanopropyl, 2-cyanopropyl, 1-methylcyclobutyl, 2-methylcyclobutyl, 3-methylcyclobutyl, 1-cyanocyclobutyl, 2-cyanocyclobutyl, 3-cyanocyclobutyl, 1-allylcyclopropyl, 1-vinylcyclobutyl, 1-vinylcyclopropyl, 1-ethylcyclopropyl, 2-ethylcyclopropyl, 1-ethylcyclobutyl, 2-ethylcyclobutyl, 3-ethylcyclobutyl, 4-methylcyclohexyl, 4-methoxycyclohexyl, 4-ethoxycyclohexyl, 4-n-propyloxycyclohexyl, 4-hydroxycyclohexyl, 4-methoxycyclobutyl, 1-cyclopropylcyclobutyl, 1-prop-2-enylcyclobutyl, 2-ethyl-3-methylcyclobutyl,1-propylcyclopropyl, 1-methyl-2-propylcyclopropyl, 2-propylcyclopropyl, 1-propylcyclobutyl, 2-propylcyclobutyl, 3-propylcyclobutyl, 1-isopropylcyclobutyl, 1-isopropylcyclopropyl, 2-isopropylcyclopropyl, 3-isopropylcyclobutyl, 2-dimethylaminocyclobutyl, 3-dimethylaminocyclobutyl, 1-butylcyclobutyl, 2-butylcyclobutyl, 1-butylcyclopropyl, 3-butylcyclobutyl, 2-butylcyclopropyl, 1-isobutylcyclobutyl, 3-tert-butylcyclobutyl, 3,3-diethylcyclobutyl, 2,2-diethylcyclopropyl, 2-methylidenecyclopropyl, 1-methoxymethylcyclopropyl, 1-isobutylcyclopropyl, 2,2-difluoroethyl, 2,2,2-trifluoroethyl, 3,3,3-trifluoropropyl, ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl, ethynyl, 2-propynyl, 2-butynyl, 3-butynyl, 1-methyl-2-propynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl, 1-ethyl-1-methyl-2-propynyl, (C₃-C₆)-halocycloalkyl, (C₂-C₅)-haloalkenyl, (C₁-C₅)-alkoxy-(C₁-C₅)-haloalkyl, benzyl, p-chlorobenzyl, p-methoxybenzyl, p-trifluoromethylbenzyl, p-methylbenzyl, p-fluorobenzyl, p-bromobenzyl, p-iodobenzyl, p-methylthiobenzyl, p-trifluoromethoxybenzyl, p-nitrobenzyl, p-trifluoromethylthiobenzyl, m-chlorobenzyl, m-methoxybenzyl, m-trifluoromethylbenzyl, m-methylbenzyl, m-fluorobenzyl, m-bromobenzyl, m-iodobenzyl, m-methylthiobenzyl, m-trifluoromethoxybenzyl, m-nitrobenzyl, m-trifluoromethylthiobenzyl, o-chlorobenzyl, o-methoxybenzyl, o-trifluoromethylbenzyl, o-methylbenzyl, o-fluorobenzyl, o-bromobenzyl, o-iodobenzyl, o-methylthiobenzyl, o-trifluoromethoxybenzyl, o-nitrobenzyl, o-trifluoromethylthiobenzyl, p-methoxycarbonylbenzyl, p-ethoxycarbonylbenzyl, m-methoxycarbonylbenzyl, m-ethoxycarbonylbenzyl, 2,4-dichlorobenzyl, 3,5-dichlorobenzyl, 2,4-difluorobenzyl, 3,5-difluorobenzyl, 3,4-dichlorobenzyl, 3,4-difluorobenzyl, 2,5-dichlorobenzyl, phenylethyl, p-chlorophenylethyl, p-methoxyphenylethyl, p-trifluoromethylphenylethyl, p-fluorophenylethyl, p-trifluoromethoxyphenylethyl, p-trifluoromethylthiophenylethyl, p-methylphenylethyl, p-nitrophenylethyl, p-methoxycarbonylphenylethyl, p-ethoxycarbonylphenylethyl, m-chlorophenylethyl, m-methoxyphenylethyl, m-trifluoromethylphenylethyl, m-fluorophenylethyl, m-trifluoromethoxyphenylethyl, m-trifluoromethylthiophenylethyl, m-methylphenylethyl, m-nitrophenylethyl, m-methoxycarbonylphenylethyl, m-ethoxycarbonylphenylethyl, o-chlorophenylethyl, o-methoxyphenylethyl, o-trifluoromethylphenylethyl, o-fluorophenylethyl, o-trifluoromethoxyphenylethyl, o-trifluoromethylthiophenylethyl, o-methylphenylethyl, o-nitrophenylethyl, o-methoxycarbonylphenylethyl, o-ethoxycarbonylphenylethyl, heteroaryl-(C₁-C₅)-alkyl, (C₁-C₅)-haloalkenyl, heterocyclyl, heterocyclyl- (C₁-C₅)-alkyl, (C₁-C₅)-alkoxy-(C₁-C₅) -alkyl, (C₁-C₅)-alkylcarbonyl-(C₁-C₅)-alkyl, hydroxycarbonyl-(C₁-C₅)-alkyl, (C₁-C₅)-alkoxycarbonyl-(C₁-C₅)-alkyl, (C₂-C₅)-alkenyloxycarbonyl-(C₁-C₅)-alkyl, (C₂-C₅)-alkynyloxycarbonyl-(C₁-C₅)-alkyl, aryl-(C₁-C₅)-alkoxycarbonyl-(C₁-C₅)-alkyl, (C₃-C₆)-cycloalkoxycarbonyl-(C₁-C₅)-alkyl, (C₃-C₆)-cycloalkyl-(C₁-C₅)-alkoxycarbonyl-(C₁-C₅)-alkyl, aminocarbonyl-(C₁-C₅)-alkyl, (C₁-C₅)-alkylaminocarbonyl-(C₁-C₅)-alkyl, (C₃-C₆)-cycloalkylaminocarbonyl-(C₁-C₅)-alkyl, aryl-(C₁-C₅)-alkylaminocarbonyl-(C₁-C₅)-alkyl, heteroaryl-(C₁-C₅)-alkylaminocarbonyl-(C₁-C₅)-alkyl, (C₁-C₅)-alkylthio-(C₁-C₅)-alkyl, (C₃-C₆)-cycloalkylthio-(C₁-C₅)-alkyl, arylthio- (C₁-C₅) -alkyl, heterocyclylthio- (C₁-C₅) -alkyl, heteroarylthio-(C₁-C₅)-alkyl, aryl-(C₁-C₅)-alkylthio-(C₁-C₅)-alkyl, (C₁-C₅)-alkylsulfinyl-(C₁-C₅)-alkyl, (C₁-C₅)-alkylsulfonyl-(C₁-C₅)-alkyl, arylsulfinyl-(C₁-C₅)-alkyl, arylsulfonyl-(C₁-C₅)-alkyl, (C₃-C₆)-cycloalkylsulfinyl-(C₁-C₅)-alkyl, (C₃-C₆)-cycloalkylsulfonyl-(C₁-C₅)-alkyl, (C₁-C₅)-alkoxy-(C₁-C₅)-alkoxy-(C₁-C₅)-alkyl, (C₁-C₅)-alkylcarbonyl, (C₃-C₆)-cycloalkylcarbonyl, (C₁-C₅)-alkoxycarbonyl, aryl-(C₁-C₅)-alkoxycarbonyl, arylcarbonyl, heteroarylcarbonyl, heterocyclylcarbonyl, aryl-(C₁-C₅)-alkylcarbonyl, (C₁-C₅)-alkylaminocarbonyl, (C₃-C₆)-cycloalkylaminocarbonyl, arylaminocarbonyl, aryl-(C₁-C₅)-alkylaminocarbonyl, (C₁-C₅)-alkylsulfonyl, (C₃-C₆)-cycloalkylsulfonyl, arylsulfonyl, heteroarylsulfonyl, heterocyclylsulfonyl, bis-[(C₁-C₅)-alkyl] amino, (C₃-C₆)-cycloalkyl[(C₁-C₅)-alkyl]amino,
R², R³, R⁴ independently of one another represent hydrogen, fluorine, chlorine, bromine, iodine, methoxy, ethoxy, n-propyloxy, isopropyloxy, methyl, ethyl, isopropyl, trifluoromethyl, difluoromethyl, pentafluoroethyl, trifluoromethoxy, difluoromethoxy, 2,2-difluoroethoxy, 3,3,3-trifluoroethoxy, methylthio, ethylthio, trifluoromethylthio, optionally substituted phenyl, heteroaryl, heterocyclyl, cyclopropyl, cyclobutyl, cyclopentyl, cyclohexyl,
R⁵ represents amino, methyl, ethyl, n-propyl, 1-methylethyl, n-butyl, 1-methylpropyl, 2-methylpropyl, 1,1-dimethylethyl, n-pentyl, 1-methylbutyl, 2-methylbutyl, 3-methylbutyl, 1,1-dimethylpropyl, 1,2-dimethylpropyl, 2,2-dimethylpropyl, 1-ethylpropyl, n-hexyl, 1-methylpentyl, 2-methylpentyl, 3-methylpentyl, 4-methylpentyl, 1,1-dimethylbutyl, 1,2-dimethylbutyl, 1,3-di-methylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, cyclopropyl, cyclobutyl, cyclopentyl, cyclohexyl, spiro[2.2]pent-1-yl, spiro[2.3]hex-1-yl, spiro[2.3]hex-4-yl, 3-spiro[2.3]hex-5-yl, cyclopropylmethyl, cyclobutylmethyl, cyclopentylmethyl, cyclohexylmethyl, trifluoromethyl, difluoromethyl, 2,2-difluoroethyl, 2,2,2-trifluoroethyl, 3,3,3-trifluoropropyl, pentafluoroethyl, heptafluoro-n-propyl, heptafluoroisopropyl, nonafluoro-n-butyl, (C₃-C₆)-halocycloalkyl, (C₄-C₆)-cycloalkenyl, optionally substituted phenyl, heteroaryl, heterocyclyl, aryl-(C₁-C₅)-alkyl, heteroaryl-(C₁-C₅)-alkyl, heterocyclyl-(C₁-C₅)-alkyl, (C₁-C₅)-alkoxycarbonyl-(C₁-C₅)-alkyl, aryl-(C₁-C₅)-alkoxycarbonyl-(C₁-C₅)-alkyl, (C₁-C₆)-cycloalkoxycarbonyl- (C₁-C₅)-alkyl, (C₃-C₆)-cycloalkyl-(C₁-C₅)-alkoxycarbonyl-(C₁-C₅)-alkyl, heteroaryl-(C₁-C₅)-alkoxycarbonyl-(C₁-C₅)-alkyl, aminocarbonyl-(C₁-C₅)-alkyl, (C₁-C₅) -alkylaminocarbonyl- (C₁-C₅)-alkyl, (C₃-C₆)-cycloalkylaminocarbonyl-(C₁-C₅)-alkyl, aryl-(C₁-C₅)-alkylaminocarbonyl-(C₁-C₅)-alkyl, (C₁-C₅)-alkylamino, arylamino, (C₃-C₆)-cycloalkylamino, aryl-(C₁-C₅)-alkylamino, heteroaryl-(C₁-C₅)-alkylamino, heteroarylamino, heterocyclylamino, (C₂-C₅)-alkenylamino, (C₂-C₅)-alkynylamino, aryloxy-(C₁-C₅)-alkyl, heteroaryloxy- (C₁-C₅)-alkyl, (C₁-C₅)-alkoxy-(C₁-C₅)-alkyl, phenylethenyl, p-chlorophenylethenyl, p-methylphenylethenyl, p-methoxyphenylethenyl, p-trifluoromethylphenylethenyl, p-fluorophenylethenyl, p-cyanophenylethenyl, p-trifluoromethoxyphenylethenyl, p-nitrophenylethenyl, p-bromophenylethenyl, p-iodophenylethenyl, m-chlorophenylethenyl, m-methylphenylethenyl, m-methoxyphenylethenyl, m-trifluoromethylphenylethenyl, m-fluorophenylethenyl, m-cyanophenylethenyl, m-trifluoromethoxyphenylethenyl, m-nitrophenylethenyl, m-bromophenylethenyl, m-iodophenylethenyl, p-methoxycarbonylphenylethenyl, m-methoxycarbonylphenylethenyl, o-methoxycarbonylphenylethenyl, p-ethoxycarbonylphenylethenyl, m-ethoxycarbonylphenylethenyl, o-ethoxycarbonylphenylethenyl, ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl, ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl, 1-ethyl-1-methyl-2-propynyl, cyanoethyl, cyanomethyl, cyano-n-propyl, cyano-n-butyl, aryloxy, bis-[(C₁-C₅)-alkyl]amino, aryl-(C₂-C₅)-alkenyl, heteroaryl-(C₂-C₅)-alkenyl, heterocyclyl-(C₂-C₅)-alkenyl,
R⁶ represents hydrogen, methyl, ethyl, n-propyl, 1-methylethyl, n-butyl, 1-methylpropyl, 2-methylpropyl, 1,1-dimethylethyl, n-pentyl, 1-methylbutyl, 2-methylbutyl, 3-methylbutyl, 1,1-dimethylpropyl, 1,2-dimethylpropyl, 2,2-dimethylpropyl, 1-ethylpropyl, n-hexyl, 1-methylpentyl, 2-methylpentyl, 3-methylpentyl, 4-methylpentyl, 1,1-dimethylbutyl, 1,2-dimethylbutyl, 1,3-di-methylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, cyclopropyl, cyclobutyl, cyclopentyl, cyclohexyl, cyclopropylmethyl, cyclopropylethyl, cyclobutylmethyl, cyclopentylmethyl, cyclohexylmethyl, 2,2-difluoroethyl, 2,2,2-trifluoroethyl, 3,3,3-trifluoropropyl, cyanomethyl, cyanoethyl, cyano-n-propyl, cyclopropylcarbonyl, cyclobutylcarbonyl, cyclopentylcarbonyl, cyclohexylcarbonyl, methoxycarbonyl, (C₁-C₅)-alkylsulfonyl, arylsulfonyl, aryl-(C₁-C₅)-alkylsulfonyl, heteroarylsulfonyl, (C₃-C₆)-cycloalkylsulfonyl, heterocyclylsulfonyl, (C₁-C₅)-alkylcarbonyl, arylcarbonyl, heteroarylcarbonyl, heterocyclylcarbonyl, (C₁-C₅)-alkoxycarbonyl, aryl-(C₁-C₅)-alkoxycarbonyl, (C₁-C₅)-haloalkylcarbonyl, (C₂-C₅)-alkenyl, (C₂-C₅)-alkynyl, halo-(C₂-C₅)-alkynyl, halo-(C₂-C₅)-alkenyl, (C₁-C₅)-alkoxy-(C₁-C₅)-alkyl and
W represents oxygen or sulfur.

14. Substituted dihydrooxindolylsulfonamide of formulae (Ib) to (Ie), (Ij) to (Is) (lu) and (Iw) or a salt thereof according to Claim 12 in which
R¹ represents methyl, ethyl, n-propyl, 1-methylethyl, n-butyl, 1-methylpropyl, 2-methylpropyl, 1,1-dimethylethyl, n-pentyl, 1-methylbutyl, 2-methylbutyl, 3-methylbutyl, 1,1-dimethylpropyl, 1,2-dimethylpropyl, 2,2-dimethylpropyl, 1-ethylpropyl, n-hexyl, 1-methylpentyl, 2-methylpentyl, 3-methylpentyl, 4-methylpentyl, 1,1-dimethylbutyl, 1,2-dimethylbutyl, 1,3-di-methylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, cyclopropyl, cyclobutyl, cyclopentyl, cyclohexyl, spiro[2.2]pent-1-yl, spiro[2.3]hex-1-yl, spiro[2.3]hex-4-yl, 3-spiro[2.3]hex-5-yl, spiro[3.3]hept-1-yl, spiro[3.3]hept-2-yl, bicyclo[1.1.0]butan-1-yl, bicyclo[1.1.0]butan-2-yl, bicyclo[2.1.0]pentan-1-yl, bicyclo[1.1.1]pentan-1-yl, bicyclo[2.1.0]pentan-2-yl, bicyclo[2.1.0]pentan-5-yl, bicyclo[2.1.1]hexyl, bicyclo[2.2.1]hept-2-yl, bicyclo[2.2.2]octan-2-yl, bicyclo[3.2.1]octan-2-yl, bicyclo[3.2.2]nonan-2-yl, adamantan-1-yl, adamantan-2-yl, 1-methylcyclopropyl, 2-methylcyclopropyl, 2,2-dimethylcyclopropyl, 2,3-dimethylcyclopropyl, 1,1'-bi(cyclopropyl)-1-yl, 1,1'-bi(cyclopropyl)-2-yl, 2'-methyl-1,1'-bi(cyclopropyl)-2-yl, 1-cyanopropyl, 2-cyanopropyl, 1-methylcyclobutyl, 2-methylcyclobutyl, 3-methylcyclobutyl, 1-cyanocyclobutyl, 2-cyanocyclobutyl, 3-cyanocyclobutyl, 1-allylcyclopropyl, 1-vinylcyclobutyl, 1-vinylcyclopropyl, 1-ethylcyclopropyl, 2-ethylcyclopropyl, 1-ethylcyclobutyl, 2-ethylcyclobutyl, 3-ethylcyclobutyl, 4-methylcyclohexyl, 4-methoxycyclohexyl, 4-ethoxycyclohexyl, 4-n-propyloxycyclohexyl, 4-hydroxycyclohexyl, 4-methoxycyclobutyl, 1-cyclopropylcyclobutyl, 1-prop-2-enylcyclobutyl, 2-ethyl-3-methylcyclobutyl,1-propylcyclopropyl, 1-methyl-2-propylcyclopropyl, 2-propylcyclopropyl, 1-propylcyclobutyl, 2-propylcyclobutyl, 3-propylcyclobutyl, 1-isopropylcyclobutyl, 1-isopropylcyclopropyl, 2-isopropylcyclopropyl, 3-isopropylcyclobutyl, 2-dimethylaminocyclobutyl, 3-dimethylaminocyclobutyl, 1-butylcyclobutyl, 2-butylcyclobutyl, 1-butylcyclopropyl, 3-butylcyclobutyl, 2-butylcyclopropyl, 1-isobutylcyclobutyl, 3-tert-butylcyclobutyl, 3,3-diethylcyclobutyl, 2,2-diethylcyclopropyl, 2-methylidenecyclopropyl, 1-methoxymethylcyclopropyl, 1-isobutylcyclopropyl, 2,2-difluoroethyl, 2,2,2-trifluoroethyl, 3,3,3-trifluoropropyl, ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl, ethynyl, 2-propynyl, 2-butynyl, 3-butynyl, 1-methyl-2-propynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl, 1-ethyl-1-methyl-2-propynyl, (C₃-C₆)-halocycloalkyl, (C₂-C₅)-haloalkenyl, (C₁-C₅)-alkoxy-(C₁-C₅)-haloalkyl, benzyl, p-chlorobenzyl, p-methoxybenzyl, p-trifluoromethylbenzyl, p-methylbenzyl, p-fluorobenzyl, p-bromobenzyl, p-iodobenzyl, p-methylthiobenzyl, p-trifluoromethoxybenzyl, p-nitrobenzyl, p-trifluoromethylthiobenzyl, m-chlorobenzyl, m-methoxybenzyl, m-trifluoromethylbenzyl, m-methylbenzyl, m-fluorobenzyl, m-bromobenzyl, m-iodobenzyl, m-methylthiobenzyl, m-trifluoromethoxybenzyl, m-nitrobenzyl, m-trifluoromethylthiobenzyl, o-chlorobenzyl, o-methoxybenzyl, o-trifluoromethylbenzyl, o-methylbenzyl, o-fluorobenzyl, o-bromobenzyl, o-iodobenzyl, o-methylthiobenzyl, o-trifluoromethoxybenzyl, o-nitrobenzyl, o-trifluoromethylthiobenzyl, p-methoxycarbonylbenzyl, p-ethoxycarbonylbenzyl, m-methoxycarbonylbenzyl, m-ethoxycarbonylbenzyl, 2,4-dichlorobenzyl, 3,5-dichlorobenzyl, 2,4-difluorobenzyl, 3,5-difluorobenzyl, 3,4-dichlorobenzyl, 3,4-difluorobenzyl, 2,5-dichlorobenzyl, phenylethyl, p-chlorophenylethyl, p-methoxyphenylethyl, p-trifluoromethylphenylethyl, p-fluorophenylethyl, p-trifluoromethoxyphenylethyl, p-trifluoromethylthiophenylethyl, p-methylphenylethyl, p-nitrophenylethyl, p-methoxycarbonylphenylethyl, p-ethoxycarbonylphenylethyl, m-chlorophenylethyl, m-methoxyphenylethyl, m-trifluoromethylphenylethyl, m-fluorophenylethyl, m-trifluoromethoxyphenylethyl, m-trifluoromethylthiophenylethyl, m-methylphenylethyl, m-nitrophenylethyl, m-methoxycarbonylphenylethyl, m-ethoxycarbonylphenylethyl, o-chlorophenylethyl, o-methoxyphenylethyl, o-trifluoromethylphenylethyl, o-fluorophenylethyl, o-trifluoromethoxyphenylethyl, o-trifluoromethylthiophenylethyl, o-methylphenylethyl, o-nitrophenylethyl, o-methoxycarbonylphenylethyl, o-ethoxycarbonylphenylethyl, heteroaryl-(C₁-C₅)-alkyl, (C₁-C₅)-haloalkenyl, heterocyclyl, heterocyclyl- (C₁-C₅)-alkyl, (C₁-C₅)-alkoxy- (C₁-C₅) -alkyl, (C₁-C₅)-alkylcarbonyl- (C₁-C₅)-alkyl, hydroxycarbonyl- (C₁-C₅)-alkyl, (C₁-C₅)-alkoxycarbonyl-(C₁-C₅)-alkyl, (C₂-C₅)-alkenyloxycarbonyl-(C₁-C₅)-alkyl, (C₂-C₅)-alkynyloxycarbonyl-(C₁-C₅)-alkyl, aryl- (C₁-C₅)-alkoxycarbonyl-(C₁-C₅)-alkyl, (C₃-C₆)-cycloalkoxycarbonyl- (C₁-C₅)-alkyl, (C₃-C₆)-cycloalkyl-(C₁-C₅)-alkoxycarbonyl-(C₁-C₅)-alkyl, aminocarbonyl-(C₁-C₅)-alkyl, (C₁-C₅)-alkylaminocarbonyl-(C₁-C₅)-alkyl, (C₃-C₆)-cycloalkylaminocarbonyl-(C₁-C₅)-alkyl, aryl-(C₁-C₅)-alkylaminocarbonyl-(C₁-C₅)-alkyl, heteroaryl-(C₁-C₅)-alkylaminocarbonyl-(C₁-C₅)-alkyl, (C₁-C₅)-alkylthio-(C₁-C₅)-alkyl, (C₃-C₆)-cycloalkylthio-(C₁-C₅)-alkyl, arylthio-(C₁-C₅) -alkyl, heterocyclylthio- (C₁-C₅)-alkyl, heteroarylthio- (C₁-C₅)-alkyl, aryl- (C₁-C₅)-alkylthio-(C₁-C₅)-alkyl, (C₁-C₅)-alkylsulfinyl-(C₁-C₅)-alkyl, (C₁-C₅)-alkylsulfonyl-(C₁-C₅)-alkyl, arylsulfinyl-(C₁-C₅)-alkyl, arylsulfonyl-(C₁-C₅)-alkyl, (C₃-C₆)-cycloalkylsulfinyl-(C₁-C₅)-alkyl, (C₃-C₆)-cycloalkylsulfonyl-(C₁-C₅)-alkyl, (C₁-C₅)-alkoxy-(C₁-C₅)-alkoxy-(C₁-C₅)-alkyl, (C₁-C₅)-alkylcarbonyl, (C₃-C₆)-cycloalkylcarbonyl, (C₁-C₅)-alkoxycarbonyl, aryl-(C₁-C₅)-alkoxycarbonyl, arylcarbonyl, heteroarylcarbonyl, heterocyclylcarbonyl, aryl-(C₁-C₅)-alkylcarbonyl, (C₁-C₅)-alkylaminocarbonyl, (C₃-C₆)-cycloalkylaminocarbonyl, arylaminocarbonyl, aryl-(C₁-C₅)-alkylaminocarbonyl, (C₁-C₅)-alkylsulfonyl, (C₃-C₆)-cycloalkylsulfonyl, arylsulfonyl, heteroarylsulfonyl, heterocyclylsulfonyl, bis-[(C₁-C₅)-alkyl]amino, (C₃-C₆)-cycloalkyl[(C₁-C₅)-alkyl]amino,
R², R³, R⁴ independently of one another represent hydrogen, fluorine, chlorine, bromine, iodine, methoxy, ethoxy, n-propyloxy, isopropyloxy, methyl, ethyl, isopropyl, trifluoromethyl, difluoromethyl, pentafluoroethyl, trifluoromethoxy, difluoromethoxy, 2,2-difluoroethoxy, 3,3,3-trifluoroethoxy, methylthio, ethylthio, trifluoromethylthio, optionally substituted phenyl, heteroaryl, heterocyclyl, cyclopropyl, cyclobutyl, cyclopentyl, cyclohexyl,
R⁵ represents optionally substituted phenyl, heteroaryl, heterocyclyl, aryl-(C₁-C₅)-alkyl, heteroaryl-(C₁-C₅)-alkyl, heterocyclyl-(C₁-C₅)-alkyl, (C₁-C₅)-alkoxycarbonyl-(C₁-C₅)-alkyl, aryl-(C₁-C₅)-alkoxycarbonyl-(C₁-C₅)-alkyl, (C₁-C₆)-cycloalkoxycarbonyl-(C₁-C₅)-alkyl, (C₃-C₆)-cycloalkyl-(C₁-C₅)-alkoxycarbonyl-(C₁-C₅)-alkyl, heteroaryl-(C₁-C₅)-alkoxycarbonyl-(C₁-C₅)-alkyl, aminocarbonyl-(C₁-C₅)-alkyl, (C₁-C₅)-alkylaminocarbonyl-(C₁-C₅)-alkyl, (C₃-C₆)-cycloalkylaminocarbonyl-(C₁-C₅)-alkyl, aryl-(C₁-C₅)-alkylaminocarbonyl-(C₁-C₅)-alkyl, aryloxy-(C₁-C₅)-alkyl, heteroaryloxy-(C₁-C₅)-alkyl, phenylethenyl, p-chlorophenylethenyl, p-methylphenylethenyl, p-methoxyphenylethenyl, p-trifluoromethylphenylethenyl, p-fluorophenylethenyl, p-cyanophenylethenyl, p-trifluoromethoxyphenylethenyl, p-nitrophenylethenyl, p-bromophenylethenyl, p-iodophenylethenyl, m-chlorophenylethenyl, m-methylphenylethenyl, m-methoxyphenylethenyl, m-trifluoromethylphenylethenyl, m-fluorophenylethenyl, m-cyanophenylethenyl, m-trifluoromethoxyphenylethenyl, m-nitrophenylethenyl, m-bromophenylethenyl, m-iodophenylethenyl, p-methoxycarbonylphenylethenyl, m-methoxycarbonylphenylethenyl, o-methoxycarbonylphenylethenyl, p-ethoxycarbonylphenylethenyl, m-ethoxycarbonylphenylethenyl, o-ethoxycarbonylphenylethenyl, heteroaryl-(C₂-C₅)-alkenyl, heterocyclyl-(C₂-C₅)-alkenyl,
R⁶ represents hydrogen, methyl, ethyl, n-propyl, 1-methylethyl, n-butyl, 1-methylpropyl, 2-methylpropyl, 1,1-dimethylethyl, n-pentyl, 1-methylbutyl, 2-methylbutyl, 3-methylbutyl, 1,1-dimethylpropyl, 1,2-dimethylpropyl, 2,2-dimethylpropyl, 1-ethylpropyl, n-hexyl, 1-methylpentyl, 2-methylpentyl, 3-methylpentyl, 4-methylpentyl, 1,1-dimethylbutyl, 1,2-dimethylbutyl, 1,3-di-methylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, cyclopropyl, cyclobutyl, cyclopentyl, cyclohexyl, cyclopropylmethyl, cyclopropylethyl, cyclobutylmethyl, cyclopentylmethyl, cyclohexylmethyl, 2,2-difluoroethyl, 2,2,2-trifluoroethyl, 3,3,3-trifluoropropyl, cyanomethyl, cyanoethyl, cyano-n-propyl, cyclopropylcarbonyl, cyclobutylcarbonyl, cyclopentylcarbonyl, cyclohexylcarbonyl, methoxycarbonyl, (C₁-C₅)-alkylsulfonyl, arylsulfonyl, aryl-(C₁-C₅)-alkylsulfonyl, heteroarylsulfonyl, (C₃-C₆)-cycloalkylsulfonyl, heterocyclylsulfonyl, (C₁-C₅)-alkylcarbonyl, arylcarbonyl, heteroarylcarbonyl, heterocyclylcarbonyl, (C₁-C₅)-alkoxycarbonyl, aryl-(C₁-C₅)-alkoxycarbonyl, (C₁-C₅)-haloalkylcarbonyl, (C₂-C₅)-alkenyl, (C₂-C₅)-alkynyl, halo-(C₂-C₅)-alkynyl, halo-(C₂-C₅)-alkenyl, (C₁-C₅)-alkoxy-(C₁-C₅)-alkyl and
W represents oxygen or sulfur.

15. Spray solution for treatment of plants, comprising an amount, effective for enhancing the resistance of plants to abiotic stress factors, of one or more of the substituted dihydrooxindolylsulfonamides according to any of Claims 12, 13 and 14.

## Revendications

1. Utilisation de dihydrooxindolylsulfonamides substitués ou leurs sels 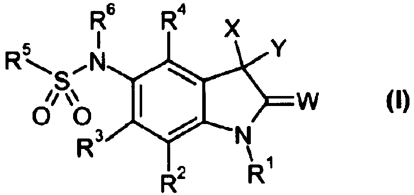 pour augmenter la tolérance au stress de plantes en ce qui concerne le stress abiotique et/ou pour augmenter le rendement de plantes,
dans lesquels
R¹ représente hydrogène, alkyle en (C₁-C₁₀), cycloalkyle en (C₃-C₈), haloalkyle en (C₁-C₁₀), halocycloalkyle en (C₃-C₈), alcényle en (C₂-C₈), haloalcényle en (C₂-C₈), alcoxy en (C₁-C₈)-haloalkyle en (C₁-C₈), alcynyle en (C₂-C₈), aryle, aryl-alkyle en (C₁-C₈), hétéroaryle, hétéroaryl-alkyle en (C₁-C₈), cycloalkyle en (C₃-C₈)-alkyle en (C₁-C₈), haloalcynyle en (C₂-C₈), hétérocyclyle, hétérocyclyl-alkyle en (C₁-C₈), alcoxy en (C₁-C₈)-alkyle en (C₁-C₈), alkylcarbonyle en (C₁-C₈)-alkyle en (C₁-C₈), hydroxycarbonyle-alkyle en (C₁-C₈), alcoxycarbonyle en (C₁-C₈)-alkyle en (C₁-C₈), alcényloxycarbonyle en (C₂-C₈)-alkyle en (C₁-C₈), alcynyloxycarbonyle en (C₂-C₈)-alkyle en (C₁-C₈), aryl-alcoxycarbonyle en (C₁-C₈)-alkyle en (C₁-C₈), cycloalcoxycarbonyle en (C₃-C₈)-alkyle en (C₁-C₈), cycloalkyle en (C₃-C₈) -alcoxycarbonyle en (C₁-C₈) -alkyle en (C₁-C₈), aminocarbonyl-alkyle en (C₁-C₈), alkylaminocarbonyle en (C₁-C₈)-alkyle en (C₁-C₈), cycloalkylaminocarbonyle en (C₃-C₈)-alkyle en (C₁-C₈), aryl-alkylaminocarbonyle en (C₁-C₈)-alkyle en (C₁-C₈), hétéroaryl-alkylaminocarbonyle en (C₁-C₈)-alkyle en (C₁-C₈), alkylthio en (C₁-C₈)-alkyle en (C₁-C₈), cycloalkylthio en (C₃-C₈)-alkyle en (C₁-C₈), arylthio-alkyle en (C₁-C₈), hétérocyclylthio-alkyle en (C₁-C₈), hétéroarylthio-alkyle en (C₁-C₈), aryl-alkylthio en (C₁-C₈)-alkyle en (C₁-C₈), alkylsulfinyle en (C₁-C₈)-alkyle en (C₁-C₈), alkylsulfonyle en (C₁-C₈)-alkyle en (C₁-C₈), arylsulfinyl-alkyle en (C₁-C₈), arylsulfonyl-alkyle en (C₁-C₈), cycloalkylsulfinyle en (C₃-C₈)-alkyle en (C₁-C₈), cycloalkylsulfonyle en (C₃-C₈)-alkyle en (C₁-C₈), alcoxy en (C₁-C₈)-alcoxy en (C₁-C₈)-alkyle en (C₁-C₈), alkylcarbonyle en (C₁-C₈), haloalkylcarbonyle en (C₁-C₈), cycloalkylcarbonyle en (C₃-C₈), alcoxycarbonyle en (C₁-C₈), aryl-alcoxycarbonyle en (C₁-C₈), arylcarbonyle, hétéroarylcarbonyle, hétérocyclylcarbonyle, aryl-alkylcarbonyle en (C₁-C₈), alkylaminocarbonyle en (C₁-C₈) cycloalkylaminocarbonyle en (C₃-C₈), arylaminocarbonyle, aryl-alkylaminocarbonyle en (C₁-C₈), hétéroarylaminocarbonyle, hétérocyclylaminocarbonyle, hétéroaryl-alkylaminocarbonyle en (C₁-C₈), hétérocyclyle-alkylaminocarbonyle en (C₁-C₈), alkylsulfonyle en (C₁-C₈), cycloalkylsulfonyle en (C₃-C₈), arylsulfonyle, aryl-alkylsulfonyle en (C₁-C₈), hétéroarylsulfonyle, hétérocyclylsulfonyle, cyano-alkyle en (C₁-C₈), cycloalcényle en (C₄-C₈)-alkyle en (C₁-C₈), nitro-alkyle en (C₁-C₈), halo-alcoxy en (C₁-C₈)-alkyle en (C₁-C₈), bis-[alkyle en (C₁-C₈)]aminocarbonyle, cycloalkyle en (C₃-C₈)-[alkyle en (C₁-C₈)]aminocarbonyle, aryl-[alkyle en (C₁-C₈)]aminocarbonyle, aryl-alkyle en (C₁-C₈)-[alkyle en (C₁-C₈)]aminocarbonyle, alcénylaminocarbonyle en (C₂-C₈), alcynylaminocarbonyle en (C₂-C₈), alkylaminosulfonyle en (C₁-C₈), bis-[alkyle en (C₁-C₈)]aminosulfonyle, hétérocyclylsulfinyl-alkyle en (C₁-C₈), hétéroarylsulfinyl-alkyle en (C₁-C₈), aryl-alkylsulfinyle en (C₁-C₈) -alkyle en (C₁-C₈), hétérocyclylsulfonyl-alkyle en (C₁-C₈), hétéroarylsulfonyl-alkyle en (C₁-C₈), aryl-alkylsulfonyle en (C₁-C₈)-alkyle en (C₁-C₈), bis-[alkyle en (C₁-C₈)]aminocarbonyl-alkyle en (C₁-C₈), cycloalkyle en (C₃-C₈)-[alkyle en (C₁-C₈)]aminocarbonyl-alkyle en (C₁-C₈), aryl-[alkyle en (C₁-C₈)]aminocarbonyl-alkyle en (C₁-C₈), aryl-alkyle en (C₁-C₈)-[alkyle en (C₁-C₈)]aminocarbonyl-alkyle en (C₁-C₈), alcénylaminocarbonyle en (C₂-C₈)-alkyle en (C₁-C₈), alcynylaminocarbonyle en (C₂-C₈)-alkyle en (C₁-C₈), alkylamino en (C₁-C₈), bis-[alkyle en (C₁-C₈)]amino, cycloalkyle en (C₃-C₈)-[alkyle en (C₁-C₈)]amino,
R², R³, R⁴ représentent indépendamment les uns des autres hydrogène, halogène, alcoxy en (C₁-C₈), alkyle en (C₁-C₈), haloalkyle en (C₁-C₈), haloalcoxy en (C₁-C₈), alkylthio en (C₁-C₈), haloalkylthio en (C₁-C₈), aryle, aryl-alkyle en (C₁-C₈), hétéroaryle, hétéroaryl-alkyle en (C₁-C₈), hétérocyclyle, hétérocyclyl-alkyle en (C₁-C₈), cycloalkyle en (C₃-C₈), nitro, amino, hydroxy, alkylamino en (C₁-C₈), bis-[alkyle en (C₁-C₈)]amino, hydrothio, alkylcarbonylamino en (C₁-C₈), cycloalkylcarbonylamino en (C₃-C₈), arylcarbonylamino, hétéroarylcarbonylamino, hétérocyclylcarbonylamino, formyle, hydroxyiminométhyle, alcoxyiminométhyle en (C₁-C₈), cycloalcoxyiminométhyle en (C₃-C₈), aryloxyiminométhyle, cycloalkyle en (C₃-C₈)-alcoxyiminométhyle en (C₁-C₈), thiocyanato, isothiocyanato, aryloxy, hétéroaryloxy, cycloalcoxy en (C₃-C₈), cycloalkyle en (C₃-C₈)-alcoxy en (C₁-C₈), aryl-alcoxy en (C₁-C₈), alcynyle en (C₂-C₈), alcényle en (C₂-C₈), aryl-alcynyle en (C₁-C₈), tris-[alkyle en (C₁-C₈)]silyl-alcynyle en (C₂-C₈), bis-[alkyle en (C₁-C₈)](aryl)silyl-alcynyle en (C₂-C₈), bis-aryl[alkyle en (C₁-C₈)]silyl-alcynyle en (C₂-C₈), cycloalkyle en (C₃-C₈)-alcynyle en (C₂-C₈), aryl-alcényle en (C₂-C₈), hétéroaryl-alcényle en (C₂-C₈), cycloalkyle en (C₃-C₈)-alcényle en (C₂-C₈), cycloalkyle en (C₃-C₈)-alkyle en (C₂-C₈), haloalcynyle en (C₂-C₈), haloalcényle en (C₂-C₈), cycloalcényle en (C₄-C₈), alcoxy en (C₁-C₈)-alcoxy en (C₁-C₈)-alkyle en (C₁-C₈), alkylsulfonyle en (C₁-C₈), arylsulfonyle, hétéroarylsulfonyle, alkylsulfonylamino en (C₁-C₈), arylsulfonylamino, aryl-alkylsulfonylamino en (C₁-C₈), hétéroarylsulfonylamino, hétéroaryl-alkylsulfonylamino en (C₁-C₈), bis-[alkyle en (C₁-C₈)]aminosulfonyle, cycloalcényle en (C₄-C₈)-alkyle en (C₁-C₈), alkylsulfinyle en (C₁-C₈), arylsulfinyle, hétéroarylsulfinyle,
R⁵ représente amino, alkyle en (C₁-C₈), cycloalkyle en (C₃-C₈), cycloalkyle en (C₃-C₈)-alkyle en (C₁-C₈), haloalkyle en (C₁-C₈), halocycloalkyle en (C₃-C₈), cycloalcényle en (C₄-C₈), aryle, hétéroaryle, hétérocyclyle, aryl-alkyle en (C₁-C₈), hétéroaryl-alkyle en (C₁-C₈), hétérocyclyl-alkyle en (C₁-C₈), alcoxycarbonyle en (C₃-C₈)-alkyle en (C₁-C₈), aryl-alcoxycarbonyle en (C₁-C₈)-alkyle en (C₁-C₈), cycloalcoxycarbonyle en (C₃-C₈)-alkyle en (C₁-C₈), cycloalkyle en (C₃-C₈) -alcoxycarbonyle en (C₃-C₈) -alkyle en (C₁-C₈), hétéroaryl-alcoxycarbonyle en (C₁-C₈)-alkyle en (C₁-C₈), aminocarbonyl-alkyle en (C₁-C₈), alkylaminocarbonyle en (C₁-C₈)-alkyle en (C₁-C₈), cycloalkylaminocarbonyle en (C₃-C₈)-alkyle en (C₁-C₈), aryl-alkylaminocarbonyle en (C₁-C₈)-alkyle en (C₁-C₈), alkylamino en (C₁-C₈), arylamino, cycloalkylamino en (C₃-C₈), aryl-alkylamino en (C₁-C₈), hétéroaryl-alkylamino en (C₁-C₈), hétéroarylamino, hétérocyclylamino, aryloxy-alkyle en (C₁-C₈), alcoxy en (C₁-C₈)-alkyle en (C₁-C₈), hétéroaryloxy-alkyle en (C₁-C₈), alcényle en (C₂-C₈), alcynyle en (C₂-C₈), alcénylamino en (C₂-C₈), alcynylamino en (C₂-C₈), bis-[alkyle en (C₁-C₈)]amino, aryloxy, bis-[alkyle en (C₁-C₈)]amino, aryl-alcényle en (C₂-C₈), hétéroaryl-alcényle en (C₂-C₈), hétérocyclyl-alcényle en (C₂-C₈), aryloxycarbonyl-alkyle en (C₁-C₈), hétéroaryloxycarbonyl-alkyle en (C₁-C₈), bis[alkyle en (C₁-C₈)]aminocarbonyl-alkyle en (C₁-C₈), alkylthio en (C₁-C₈)-alkyle en (C₁-C₈), cyano-alkyle en (C₁-C₈), alcoxy en (C₁-C₈)-alcoxy en (C₁-C₈)-alkyle en (C₁-C₈),
R⁶ représente hydrogène, alkyle en (C₁-C₈), cycloalkyle en (C₃-C₈), cyano-alkyle en (C₁-C₈), cycloalkyle en (C₃-C₈)-alkyle en (C₁-C₈), alkylsulfonyle en (C₁-C₈), arylsulfonyle, hétéroarylsulfonyle, cycloalkylsulfonyle en (C₃-C₈), hétérocyclylsulfonyle, aryl-alkylsulfonyle en (C₁-C₈), alkylcarbonyle en (C₁-C₈), arylcarbonyle, hétéroarylcarbonyle, cycloalkylcarbonyle en (C₃-C₈), hétérocyclylcarbonyle, alcoxycarbonyle en (C₁-C₈), aryl-alcoxycarbonyle en (C₁-C₈), haloalkylcarbonyle en (C₁-C₈), alcényle en (C₂-C₈), alcynyle en (C₂-C₈), haloalkyle en (C₁-C₈), halo-alcynyle en (C₂-C₈), halo-alcényle en (C₂-C₈), alcoxy en (C₁-C₈)-alkyle en (C₁-C₈),
W représente oxygène, soufre, et
X, Y représentent indépendamment l'un de l'autre hydrogène, alkyle en (C₁-C₈), halogène, alcényle en (C₂-C₈), alcynyle en (C₂-C₈), haloalkyle en (C₁-C₈), hydroxy-alkyle en (C₁-C₈), cyano-alkyle en (C₁-C₈), aryle, hétéroaryle, cycloalkyle en (C₃-C₈), cycloalcényle en (C₄-C₈), hétérocyclyle, cyano, nitro, hydroxy, alcoxy en (C₁-C₈), alkylthio en (C₁-C₈), alcoxy en (C₁-C₈)-alkyle en (C₁-C₈), alkylthio en (C₁-C₈)-alkyle en (C₁-C₈), aryloxy, aryl-alcoxy en (C₁-C₈), haloalcoxy en (C₁-C₈), haloalkylthio en (C₁-C₈), alkylamino en (C₁-C₈), bis-[alkyle en (C₁-C₈)]amino, alcoxy en (C₁-C₈)-alcoxy en (C₁-C₈), amino-alkyle en (C₁-C₈), alkylamino en (C₁-C₈)-alkyle en (C₁-C₈), cycloalkylamino en (C₃-C₈)-alkyle en (C₁-C₈), aryl-alkylamino en (C₁-C₈)-alkyle en (C₁-C₈), hétéroaryl-alkylamino en (C₁-C₈)-alkyle en (C₁-C₈), hétérocyclyl-alkylamino en (C₁-C₈)-alkyle en (C₁-C₈), hétérocyclylamino-alkyle en (C₁-C₈), hétéroarylamino-alkyle en (C₁-C₈), alcoxycarbonylamino en (C₁-C₈)-alkyle en (C₁-C₈), arylamino-alkyle en (C₁-C₈), aryl-alcoxycarbonylamino en (C₁-C₈)-alkyle en (C₁-C₈), cycloalcoxycarbonylamino en (C₃-C₈)-alkyle en (C₁-C₈), cycloalkyle en (C₃-C₈)-alcoxycarbonylamino en (C₁-C₈)-alkyle en (C₁-C₈), hétéroaryl-alcoxycarbonylamino en (C₁-C₈)-alkyle en (C₁-C₈), alkylcarbonylamino en (C₁-C₈)-alkyle en (C₁-C₈), cycloalkylcarbonylamino en (C₃-C₈)-alkyle en (C₁-C₈), arylcarbonylamino-alkyle en (C₁-C₈), hétéroarylcarbonylamino-alkyle en (C₁-C₈), hétérocyclylcarbonylamino-alkyle en (C₁-C₈), alcényloxycarbonylamino en (C₂-C₈)-alkyle en (C₁-C₈), aryl-alcénylamino en (C₂-C₈)-alkyle en (C₁-C₈), arylsulfonyl-alkyle en (C₁-C₈), hétéroarylsulfonyl-alkyle en (C₁-C₈), alkylsulfonyle en (C₁-C₈)-alkyle en (C₁-C₈), cycloalkylsulfonyle en (C₃-C₈)-alkyle en (C₁-C₈), arylsulfinyl-alkyle en (C₁-C₈), hétéroarylsulfinyl-alkyle en (C₁-C₈), alkylsulfinyle en (C₁-C₈)-alkyle en (C₁-C₈), cycloalkylsulfinyle en (C₃-C₈)-alkyle en (C₁-C₈), bis [alkyle en (C₁-C₈)]amino-alkyle en (C₁-C₈) ou
X et Y forment avec l'atome de carbone auquel ils sont reliés un cycle monocyclique ou bicyclique entièrement saturé ou partiellement saturé, éventuellement interrompu par des hétéroatomes et éventuellement davantage substitué, de 3 à 7 chaînons.

2. Utilisation selon la revendication 1, dans laquelle, dans la formule (I),
R¹ représente hydrogène, alkyle en (C₁-C₈), cycloalkyle en (C₃-C₆), haloalkyle en (C₁-C₈), halocycloalkyle en (C₃-C₆), alcényle en (C₂-C₆), haloalcényle en (C₂-C₆), alcoxy en (C₁-C₆)-haloalkyle en (C₁-C₆), alcynyle en (C₂-C₆), aryle, aryl-alkyle en (C₁-C₆), hétéroaryle, hétéroaryl-alkyle en (C₁-C₆), cycloalkyle en (C₃-C₆)-alkyle en (C₁-C₆), haloalcynyle en (C₂-C₆), hétérocyclyle, hétérocyclyl-alkyle en (C₁-C₆), alcoxy en (C₁-C₆)-alkyle en (C₁-C₆), alkylcarbonyle en (C₁-C₆)-alkyle en (C₁-C₆), hydroxycarbonyle-alkyle en (C₁-C₆), alcoxycarbonyle en (C₁-C₆)-alkyle en (C₁-C₆), alcényloxycarbonyle en (C₂-C₆)-alkyle en (C₁-C₆), alcynyloxycarbonyle en (C₂-C₆)-alkyle en (C₁-C₆), aryl-alcoxycarbonyle en (C₁-C₆)-alkyle en (C₁-C₆), cycloalcoxycarbonyle en (C₃-C₆)-alkyle en (C₁-C₆), cycloalkyle en (C₃-C₆) -alcoxycarbonyle en (C₁-C₆) -alkyle en (C₁-C₆), aminocarbonyl-alkyle en (C₁-C₆), alkylaminocarbonyle en (C₁-C₆)-alkyle en (C₁-C₆), cycloalkylaminocarbonyle en (C₃-C₆)-alkyle en (C₁-C₆), aryl-alkylaminocarbonyle en (C₁-C₆)-alkyle en (C₁-C₆), hétéroaryl-alkylaminocarbonyle en (C₁-C₆)-alkyle en (C₁-C₆), alkylthio en (C₁-C₆)-alkyle en (C₁-C₆), cycloalkylthio en (C₃-C₆)-alkyle en (C₁-C₈), arylthio-alkyle en (C₁-C₆), hétérocyclylthio-alkyle en (C₁-C₈), hétéroarylthio-alkyle en (C₁-C₆), aryl-alkylthio en (C₁-C₆)-alkyle en (C₁-C₆), alkylsulfinyle en (C₁-C₆)-alkyle en (C₁-C₆), alkylsulfonyle en (C₁-C₆)-alkyle en (C₁-C₆), arylsulfinyl-alkyle en (C₁-C₆), arylsulfonyl-alkyle en (C₁-C₆), cycloalkylsulfinyle en (C₃-C₆)-alkyle en (C₁-C₆), cycloalkylsulfonyle en (C₃-C₆)-alkyle en (C₁-C₆), alcoxy en (C₁-C₆)-alcoxy en (C₁-C₆)-alkyle en (C₁-C₆), alkylcarbonyle en (C₁-C₆), haloalkylcarbonyle en (C₁-C₈), cycloalkylcarbonyle en (C₃-C₆), alcoxycarbonyle en (C₁-C₆), aryl-alcoxycarbonyle en (C₁-C₆), arylcarbonyle, hétéroarylcarbonyle, hétérocyclylcarbonyle, aryl-alkylcarbonyle en (C₁-C₆), alkylaminocarbonyle en (C₁-C₆), cycloalkylaminocarbonyle en (C₃-C₆), arylaminocarbonyle, aryl-alkylaminocarbonyle en (C₁-C₆), hétéroarylaminocarbonyle, hétérocyclylaminocarbonyle, hétéroaryl-alkylaminocarbonyle en (C₁-C₆), hétérocyclyle-alkylaminocarbonyle en (C₁-C₆), alkylsulfonyle en (C₁-C₆), cycloalkylsulfonyle en (C₃-C₆), arylsulfonyle, aryl-alkylsulfonyle en (C₁-C₆), hétéroarylsulfonyle, hétérocyclylsulfonyle, cyano-alkyle en (C₁-C₆), cycloalcényle en (C₄-C₆)-alkyle en (C₁-C₆), nitro-alkyle en (C₁-C₆), halo-alcoxy en (C₁-C₆)-alkyle en (C₁-C₆), bis-[alkyle en (C₁-C₆) ]aminocarbonyle, cycloalkyle en (C₃-C₆)-[alkyle en (C₁-C₆)]aminocarbonyle, aryl-[alkyle en (C₁-C₆)]aminocarbonyle, aryl-alkyle en (C₁-C₆)-[alkyle en (C₁-C₆)]aminocarbonyle, alcénylaminocarbonyle en (C₂-C₆), alcynylaminocarbonyle en (C₂-C₆), alkylaminosulfonyle en (C₁-C₆), bis-[alkyle en (C₁-C₆)]aminosulfonyle, hétérocyclylsulfinyl-alkyle en (C₁-C₆), hétéroarylsulfinyl-alkyle en (C₁-C₆), aryl-alkylsulfinyle en (C₁-C₆)-alkyle en (C₁-C₆), hétérocyclylsulfonyl-alkyle en (C₁-C₆), hétéroarylsulfonyl-alkyle en (C₁-C₆), aryl-alkylsulfonyle en (C₁-C₆)-alkyle en (C₁-C₆), bis-[alkyle en (C₁-C₆)]aminocarbonyl-alkyle en (C₁-C₆), cycloalkyle en (C₃-C₆)-[alkyle en (C₁-C₆) ]aminocarbonyl-alkyle en (C₁-C₆), aryl-[alkyle en (C₁-C₆)]aminocarbonyl-alkyle en (C₁-C₆), aryl-alkyle en (C₁-C₆)-[alkyle en (C₁-C₆)]aminocarbonyl-alkyle en (C₁-C₆), alcénylaminocarbonyle en (C₂-C₆)-alkyle en (C₁-C₆), alcynylaminocarbonyle en (C₂-C₆)-alkyle en (C₁-C₆), bis-[alkyle en (C₁-C₆)]amino, cycloalkyle en (C₃-C₆)-[alkyle en (C₁-C₆)]amino,
R², R³, R⁴ représentent indépendamment les uns des autres hydrogène, halogène, alcoxy en (C₁-C₆), alkyle en (C₁-C₆), haloalkyle en (C₁-C₆), haloalcoxy en (C₁-C₆), alkylthio en (C₁-C₆), haloalkylthio en (C₁-C₆), aryle, aryl-alkyle en (C₁-C₆), hétéroaryle, hétéroaryl-alkyle en (C₁-C₆), hétérocyclyle, hétérocyclyl-alkyle en (C₁-C₆), cycloalkyle en (C₃-C₆), nitro, amino, hydroxy, alkylamino en (C₁-C₆), bis-[alkyle en (C₁-C₆)]amino, hydrothio, alkylcarbonylamino en (C₁-C₆), cycloalkylcarbonylamino en (C₃-C₆), arylcarbonylamino, hétéroarylcarbonylamino, hétérocyclylcarbonylamino, formyle, hydroxyiminométhyle, alcoxyiminométhyle en (C₁-C₆), cycloalcoxyiminométhyle en (C₃-C₆), aryloxyiminométhyle, cycloalkyle en (C₃-C₆)-alcoxyiminométhyle en (C₁-C₆), thiocyanato, isothiocyanato, aryloxy, hétéroaryloxy, cycloalcoxy en (C₃-C₆), cycloalkyle en (C₃-C₆)-alcoxy en (C₁-C₆), aryl-alcoxy en (C₁-C₆), alcynyle en (C₂-C₆), alcényle en (C₂-C₆), aryl-alcynyle en (C₁-C₆), tris- [alkyle en (C₁-C₆) ]silyl-alcynyle en (C₂-C₆), bis-[alkyle en (C₁-C₆)](aryl)silyl-alcynyle en (C₂-C₆), bis-aryl [alkyle en (C₁-C₆)]silyl-alcynyle en (C₂-C₆), cycloalkyle en (C₃-C₆)-alcynyle en (C₂-C₆), aryl-alcényle en (C₂-C₆), hétéroaryl-alcényle en (C₂-C₆), cycloalkyle en (C₃-C₆)-alcényle en (C₂-C₆), cycloalkyle en (C₃-C₆)-alkyle en (C₂-C₆), haloalcynyle en (C₂-C₆), haloalcényle en (C₂-C₆), cycloalcényle en (C₄-C₆), alcoxy en (C₁-C₆)-alcoxy en (C₁-C₆)-alkyle en (C₁-C₆), alkylsulfonyle en (C₁-C₆), arylsulfonyle, hétéroarylsulfonyle, alkylsulfonylamino en (C₁-C₆), arylsulfonylamino, aryl-alkylsulfonylamino en (C₁-C₆), hétéroarylsulfonylamino, hétéroaryl-alkylsulfonylamino en (C₁-C₆), bis-[alkyle en (C₁-C₆)]aminosulfonyle,
R⁵ représente amino, alkyle en (C₁-C₆), cycloalkyle en (C₃-C₆), cycloalkyle en (C₃-C₆)-alkyle en (C₁-C₆), haloalkyle en (C₁-C₆), halocycloalkyle en (C₃-C₆), cycloalcényle en (C₄-C₆), aryle, hétéroaryle, hétérocyclyle, aryl-alkyle en (C₁-C₆), hétéroaryl-alkyle en (C₁-C₆), hétérocyclyl-alkyle en (C₁-C₆), alcoxycarbonyle en (C₁-C₆)-alkyle en (C₁-C₆), aryl-alcoxycarbonyle en (C₁-C₆)-alkyle en (C₁-C₆), cycloalcoxycarbonyle en (C₃-C₆)-alkyle en (C₁-C₆), cycloalkyle en (C₃-C₆) -alcoxycarbonyle en (C₁-C₆) -alkyle en (C₁-C₆), hétéroaryl-alcoxycarbonyle en (C₁-C₆)-alkyle en (C₁-C₆), aminocarbonyl-alkyle en (C₁-C₆), alkylaminocarbonyle en (C₁-C₆)-alkyle en (C₁-C₆), cycloalkylaminocarbonyle en (C₃-C₆)-alkyle en (C₁-C₆), aryl-alkylaminocarbonyle en (C₁-C₆)-alkyle en (C₁-C₆), alkylamino en (C₁-C₆), arylamino, cycloalkylamino en (C₃-C₆), aryl-alkylamino en (C₁-C₆), hétéroaryl-alkylamino en (C₁-C₆), hétéroarylamino, hétérocyclylamino, aryloxy-alkyle en (C₁-C₆), alcoxy en (C₁-C₆)-alkyle en (C₁-C₆), hétéroaryloxy-alkyle en (C₁-C₆), alcényle en (C₂-C₆), alcynyle en (C₂-C₆), alcénylamino en (C₂-C₆), alcynylamino en (C₂-C₆), bis-[alkyle en (C₁-C₆)]amino, aryloxy, bis-[alkyle en (C₁-C₇)]amino, aryl-alcényle en (C₂-C₇), hétéroaryl-alcényle en (C₂-C₇), hétérocyclyl-alcényle en (C₂-C₇),
R⁶ représente hydrogène, alkyle en (C₁-C₆), cycloalkyle en (C₃-C₆), cyano-alkyle en (C₁-C₆), cycloalkyle en (C₃-C₆)-alkyle en (C₁-C₆), alkylsulfonyle en (C₁-C₆), arylsulfonyle, hétéroarylsulfonyle, cycloalkylsulfonyle en (C₃-C₆), hétérocyclylsulfonyle, aryl-alkylsulfonyle en (C₁-C₆), alkylcarbonyle en (C₁-C₆), arylcarbonyle, hétéroarylcarbonyle, cycloalkylcarbonyle en (C₃-C₆), hétérocyclylcarbonyle, alcoxycarbonyle en (C₁-C₆), aryl-alcoxycarbonyle en (C₁-C₆), haloalkylcarbonyle en (C₁-C₆), alcényle en (C₂-C₆), alcynyle en (C₂-C₆), haloalkyle en (C₁-C₆), halo-alcynyle en (C₂-C₆), halo-alcényle en (C₂-C₆), alcoxy en (C₁-C₆)-alkyle en (C₁-C₆),
W représente oxygène, soufre, et
X, Y représentent indépendamment l'un de l'autre hydrogène, alkyle en (C₁-C₆), fluor, chlore, alcényle en (C₂-C₆), haloalkyle en (C₁-C₆), cycloalkyle en (C₃-C₆), cycloalcényle en (C₄-C₆), hétérocyclyle, alcoxy en (C₁-C₆), alkylthio en (C₁-C₆), alcoxy en (C₁-C₆)-alkyle en (C₁-C₆), alkylthio en (C₁-C₆)-alkyle en (C₁-C₆), haloalcoxy en (C₁-C₆), haloalkylthio en (C₁-C₆), alcoxy en (C₁-C₆)-alcoxy en (C₁-C₆), amino-alkyle en (C₁-C₆), alkylamino en (C₁-C₆)-alkyle en (C₁-C₆), cycloalkylamino en (C₃-C₆)-alkyle en (C₁-C₆), aryl-alkylamino en (C₁-C₆)-alkyle en (C₁-C₆), hétéroaryl-alkylamino en (C₁-C₆)-alkyle en (C₁-C₆), hétérocyclyl-alkylamino en (C₁-C₆)-alkyle en (C₁-C₆), hétérocyclylamino-alkyle en (C₁-C₆), hétéroarylamino-alkyle en (C₁-C₆), alcoxycarbonylamino en (C₁-C₆)-alkyle en (C₁-C₆), arylamino-alkyle en (C₁-C₆), aryl-alcoxycarbonylamino en (C₁-C₆)-alkyle en (C₁-C₆), cycloalcoxycarbonylamino en (C₃-C₆)-alkyle en (C₁-C₆), cycloalkyle en (C₃-C₆)-alcoxycarbonylamino en (C₁-C₆)-alkyle en (C₁-C₆), hétéroaryl-alcoxycarbonylamino en (C₁-C₆)-alkyle en (C₁-C₆), alkylcarbonylamino en (C₁-C₆)-alkyle en (C₁-C₆), cycloalkylcarbonylamino en (C₃-C₆)-alkyle en (C₁-C₆), arylcarbonylamino-alkyle en (C₁-C₆), hétéroarylcarbonylamino-alkyle en (C₁-C₆), hétérocyclylcarbonylamino-alkyle en (C₁-C₆),
alcényloxycarbonylamino en (C₂-C₆)-alkyle en (C₁-C₆), aryl-alcénylamino en (C₂-C₆)-alkyle en (C₁-C₆), arylsulfonyl-alkyle en (C₁-C₆), hétéroarylsulfonyl-alkyle en (C₁-C₆), alkylsulfonyle en (C₁-C₆)-alkyle en (C₁-C₆), cycloalkylsulfonyle en (C₃-C₆)-alkyle en (C₁-C₆), arylsulfinyl-alkyle en (C₁-C₆), hétéroarylsulfinyl-alkyle en (C₁-C₆), alkylsulfinyle en (C₁-C₆)-alkyle en (C₁-C₆), cycloalkylsulfinyle en (C₃-C₆)-alkyle en (C₁-C₆), bis [alkyle en (C₁-C₆)]amino-alkyle en (C₁-C₆) ou
X et Y forment avec l'atome de carbone auquel ils sont reliés un cycle monocyclique ou bicyclique entièrement saturé ou partiellement saturé, éventuellement interrompu par O (oxygène), S (soufre), N-H, alkyle en (C₁-C₆)-N, alcoxy en (C₁-C₆)-N, alcoxycarbonyle en (C₁-C₆) -N, aryle-alcoxycarbonyle en (C₁-C₆)-N et éventuellement davantage substitué, de 3 à 7 chaînons, pas plus de deux hétéroatomes identiques ou différents du groupe 0, S, N étant voisins.

3. Utilisation selon la revendication 1, dans laquelle les composés de formule générale (I) sont décrits par les formules (Ia) à (Iz), ainsi que (Iab) : 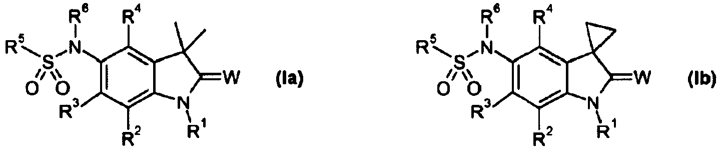 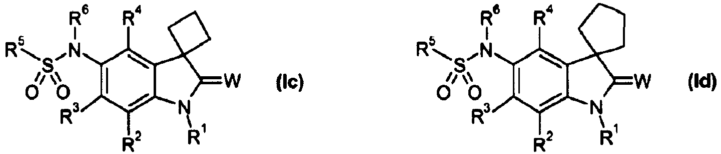 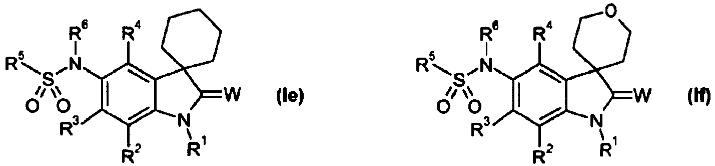 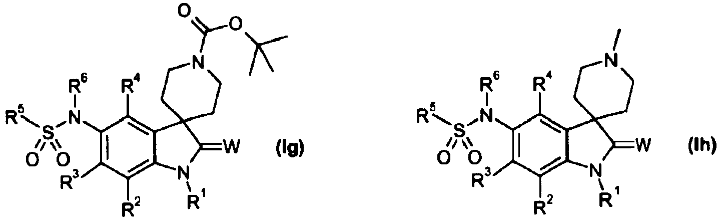 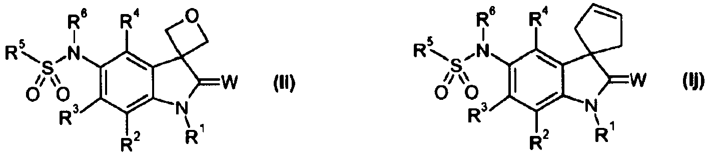 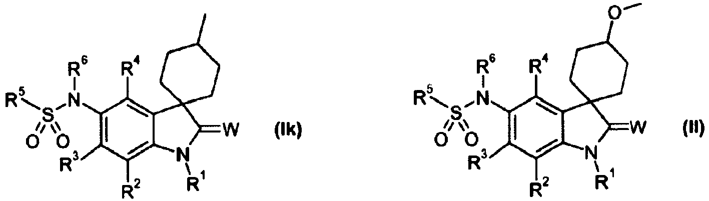 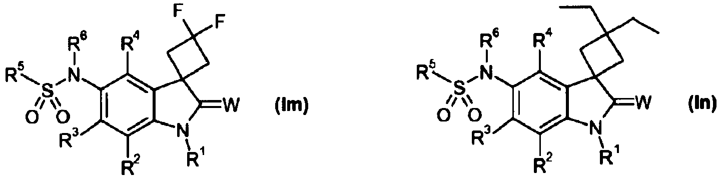 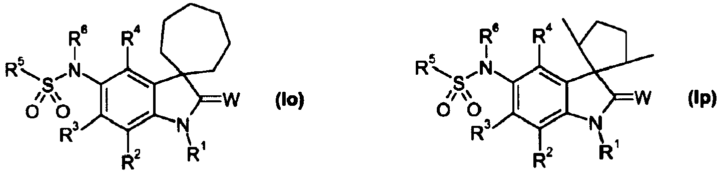 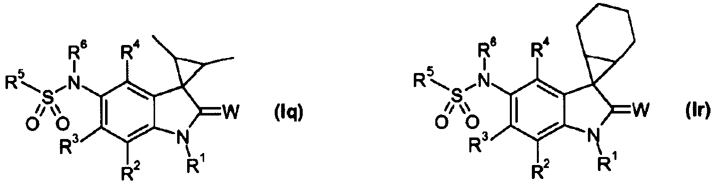 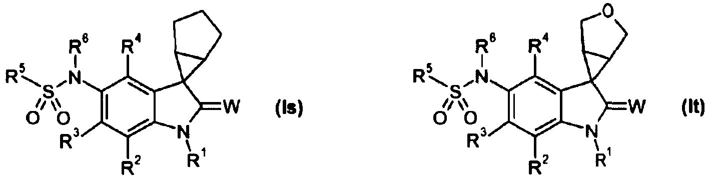 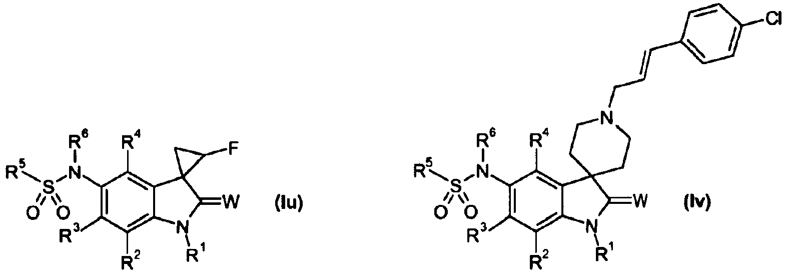 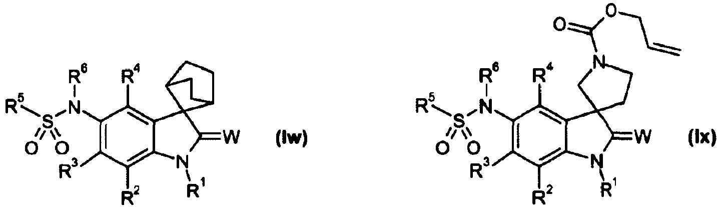 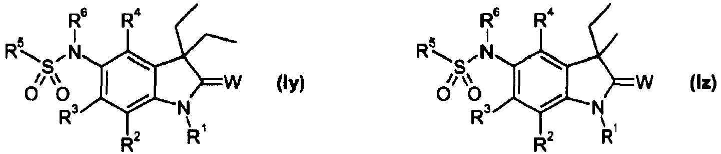 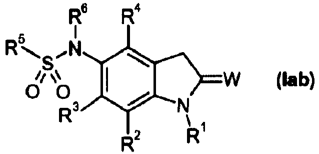 dans lesquelles
R¹ représente hydrogène, alkyle en (C₁-C₆), cycloalkyle en (C₃-C₆), haloalkyle en (C₁-C₆), halocycloalkyle en (C₃-C₆), alcényle en (C₂-C₆), haloalcényle en (C₂-C₆), alcoxy en (C₁-C₅)-haloalkyle en (C₁-C₅), alcynyle en (C₂-C₅), aryle, aryl-alkyle en (C₁-C₅), hétéroaryle, hétéroaryl-alkyle en (C₁-C₅), cycloalkyle en (C₃-C₆)-alkyle en (C₁-C₅), haloalcynyle en (C₂-C₅), hétérocyclyle, hétérocyclyl-alkyle en (C₁-C₅), alcoxy en (C₁-C₅)-alkyle en (C₁-C₅), alkylcarbonyle en (C₁-C₅)-alkyle en (C₁-C₅), hydroxycarbonyle-alkyle en (C₁-C₅), alcoxycarbonyle en (C₁-C₅)-alkyle en (C₁-C₅), alcényloxycarbonyle en (C₂-C₅)-alkyle en (C₁-C₅), alcynyloxycarbonyle en (C₂-C₅)-alkyle en (C₁-C₅), aryl-alcoxycarbonyle en (C₁-C₅)-alkyle en (C₁-C₅), cycloalcoxycarbonyle en (C₃-C₆)-alkyle en (C₁-C₅), cycloalkyle en (C₃-C₆) -alcoxycarbonyle en (C₁-C₅) -alkyle en (C₁-C₅), aminocarbonyl-alkyle en (C₁-C₅), alkylaminocarbonyle en (C₁-C₅)-alkyle en (C₁-C₅), cycloalkylaminocarbonyle en (C₃-C₆)-alkyle en (C₁-C₅), aryl-alkylaminocarbonyle en (C₁-C₅)-alkyle en (C₁-C₅), hétéroaryl-alkylaminocarbonyle en (C₁-C₅)-alkyle en (C₁-C₅), alcoxy en (C₁-C₅)-alcoxy en (C₁-C₅)-alkyle en (C₁-C₅), alkylcarbonyle en (C₁-C₅), haloalkylcarbonyle en (C₁-C₅), cycloalkylcarbonyle en (C₃-C₆), alcoxycarbonyle en (C₁-C₅), aryl-alcoxycarbonyle en (C₁-C₅), arylcarbonyle, hétéroarylcarbonyle, hétérocyclylcarbonyle, aryl-alkylcarbonyle en (C₁-C₆), alkylaminocarbonyle en (C₁-C₆), cycloalkylaminocarbonyle en (C₃-C₆), arylaminocarbonyle, aryl-alkylaminocarbonyle en (C₁-C₆), hétéroarylaminocarbonyle, hétérocyclylaminocarbonyle, hétéroaryl-alkylaminocarbonyle en (C₁-C₆), hétérocyclyl-alkylaminocarbonyle en (C₁-C₆), alkylsulfonyle en (C₁-C₆), cycloalkylsulfonyle en (C₃-C₆), arylsulfonyl, aryl-alkylsulfonyle en (C₁-C₆), hétéroarylsulfonyle, hétérocyclylsulfonyle, cyano-alkyle en (C₁-C₅), bis-[alkyle en (C₁-C₅)]amino, cycloalkyle en (C₃-C₆)[alkyle en (C₁-C₅)]amino,
R², R³, R⁴ représentent indépendamment les uns des autres hydrogène, halogène, alcoxy en (C₁-C₅), alkyle en (C₁-C₅), haloalkyle en (C₁-C₅), haloalcoxy en (C₁-C₅), alkylthio en (C₁-C₅), haloalkylthio en (C₁-C₅), aryle, aryl-alkyle en (C₁-C₅), hétéroaryle, hétéroaryl-alkyle en (C₁-C₅), hétérocyclyle, hétérocyclyl-alkyle en (C₁-C₅), cycloalkyle en (C₃-C₆), nitro, amino, hydroxy, alkylamino en (C₁-C₅), bis-[alkyle en (C₁-C₅)]amino, hydrothio, alkylcarbonylamino en (C₁-C₅), cycloalkylcarbonylamino en (C₃-C₆), arylcarbonylamino, hétéroarylcarbonylamino, hétérocyclylcarbonylamino, formyle, hydroxyiminométhyle, alcoxyiminométhyle en (C₁-C₅), cycloalcoxyiminométhyle en (C₃-C₆), aryloxyiminométhyle, cycloalkyle en (C₃-C₆)-alcoxyiminométhyle en (C₁-C₅), thiocyanato, isothiocyanato, aryloxy, hétéroaryloxy, cycloalcoxy en (C₃-C₆), cycloalkyle en (C₃-C₆)-alcoxy en (C₁-C₅), aryl-alcoxy en (C₁-C₅), alcynyle en (C₂-C₅), alcényle en (C₂-C₅), aryl-alcynyle en (C₁-C₅), tris-[alkyle en (C₁-C₅) ]silyl-alcynyle en (C₂-C₅), bis-[alkyle en (C₁-C₅)](aryl)silyl-alcynyle en (C₂-C₅), bis-aryl [alkyle en (C₁-C₅)]silyl-alcynyle en (C₂-C₅), cycloalkyle en (C₃-C₆)-alcynyle en (C₂-C₅), aryl-alcényle en (C₂-C₅), hétéroaryl-alcényle en (C₂-C₅), cycloalkyle en (C₃-C₆)-alcényle en (C₂-C₅), haloalcynyle en (C₂-C₅), haloalcényle en (C₂-C₅), cycloalcényle en (C₄-C₅), alcoxy en (C₁-C₅)-alcoxy en (C₁-C₅)-alkyle en (C₁-C₅), alkylsulfonyle en (C₁-C₅), arylsulfonyle, hétéroarylsulfonyle, alkylsulfonylamino en (C₁-C₅), arylsulfonylamino, aryl-alkylsulfonylamino en (C₁-C₅), hétéroarylsulfonylamino, hétéroaryl-alkylsulfonylamino en (C₁-C₅), bis-[alkyle en (C₁-C₅)]aminosulfonyle,
R⁵ représente amino, alkyle en (C₁-C₅), cycloalkyle en (C₃-C₆), cycloalkyle en (C₃-C₆)-alkyle en (C₁-C₅), haloalkyle en (C₁-C₅), halocycloalkyle en (C₃-C₆), cycloalcényle en (C₄-C₆), aryle, hétéroaryle, hétérocyclyle, aryl-alkyle en (C₁-C₅), hétéroaryl-alkyle en (C₁-C₅), hétérocyclyl-alkyle en (C₁-C₅), alcoxycarbonyle en (C₁-C₅)-alkyle en (C₁-C₅), aryl-alcoxycarbonyle en (C₁-C₅)-alkyle en (C₁-C₅), cycloalcoxycarbonyle en (C₃-C₆)-alkyle en (C₁-C₅), cycloalkyle en (C₃-C₆) -alcoxycarbonyle en (C₁-C₅) -alkyle en (C₁-C₅), hétéroaryl-alcoxycarbonyle en (C₁-C₅)-alkyle en (C₁-C₅), aminocarbonyl-alkyle en (C₁-C₅), alkylaminocarbonyle en (C₁-C₅)-alkyle en (C₁-C₅), cycloalkylaminocarbonyle en (C₃-C₆)-alkyle en (C₁-C₅), aryl-alkylaminocarbonyle en (C₁-C₅)-alkyle en (C₁-C₅), alkylamino en (C₁-C₅), arylamino, cycloalkylamino en (C₃-C₆), aryl-alkylamino en (C₁-C₅), hétéroaryl-alkylamino en (C₁-C₅), hétéroarylamino, hétérocyclylamino, aryloxy-alkyle en (C₁-C₅), alcoxy en (C₁-C₅)-alkyle en (C₁-C₅), hétéroaryloxy-alkyle en (C₁-C₅), alcényle en (C₂-C₅), alcynyle en (C₂-C₅), alcénylamino en (C₂-C₅), alcynylamino en (C₂-C₅), bis-[alkyle en (C₁-C₅)]amino, aryloxy, cycloalkyle en (C₃-C₆) -alkyle en (C₂-C₅), bis-[alkyle en (C₁-C₅)]amino, aryl-alcényle en (C₂-C₅), hétéroaryl-alcényle en (C₂-C₅), hétérocyclyl-alcényle en (C₂-C₅),
R⁶ représente hydrogène, alkyle en (C₁-C₅), cycloalkyle en (C₃-C₆), cyano-alkyle en (C₁-C₅), cycloalkyle en (C₃-C₆) -alkyle en (C₁-C₅), alkylsulfonyle en (C₁-C₅), arylsulfonyle, hétéroarylsulfonyle, cycloalkylsulfonyle en (C₃-C₆), hétérocyclylsulfonyle, aryl-alkylsulfonyle en (C₁-C₅), alkylcarbonyle en (C₁-C₅), arylcarbonyle, hétéroarylcarbonyle, cycloalkylcarbonyle en (C₃-C₆), hétérocyclylcarbonyle, alcoxycarbonyle en (C₁-C₅), aryl-alcoxycarbonyle en (C₁-C₅), haloalkylcarbonyle en (C₁-C₅), alcényle en (C₂-C₅), alcynyle en (C₂-C₅), haloalkyle en (C₁-C₅), halo-alcynyle en (C₂-C₅), halo-alcényle en (C₂-C₅), alcoxy en (C₁-C₅)-alkyle en (C₁-C₅) et
W représente oxygène ou soufre.

4. Traitement de plantes, comprenant l'application d'une quantité non toxique efficace pour augmenter la résistance de plantes aux facteurs de stress abiotique d'un ou de plusieurs des composés de formule (I) ou de leurs sels respectifs selon l'une quelconque des revendications 1 à 3.

5. Traitement selon la revendication 4, dans lequel les conditions de stress abiotique correspondent à une ou plusieurs conditions choisies dans le groupe constitué par la sécheresse, le stress dû au froid, le stress thermique, le stress hydrique, le stress osmotique, l'humidité de stagnation, la teneur en sels élevée du sol, l'exposition élevée à des minéraux, les conditions d'ozone, les conditions de lumière forte, la disponibilité limitée de nutriments azotés et la disponibilité limitée de nutriments phosphorés.

6. Utilisation d'un ou de plusieurs composés de formule (I) ou de leurs sels respectifs selon l'une quelconque des revendications 1 à 3 dans l'application par pulvérisation sur des plantes et des parties de plantes dans des combinaisons avec un ou plusieurs agents actifs choisis dans le groupe constitué par les insecticides, les appâts, les acaricides, les fongicides, les nématicides, les herbicides, les régulateurs de croissance, les agents protecteurs, les substances influençant la maturation des plantes et les bactéricides.

7. Utilisation d'un ou de plusieurs composés de formule (I) ou de leurs sels respectifs selon l'une quelconque des revendications 1 à 3 dans l'application par pulvérisation sur des plantes et des parties de plantes dans des combinaisons avec des engrais.

8. Utilisation d'un ou de plusieurs composés de formule (I) ou de leurs sels respectifs selon l'une quelconque des revendications 1 à 3 pour l'application sur des variétés modifiés génétiquement, leurs graines, ou sur des surfaces de culture sur lesquelles ces variétés poussent.

9. Utilisation de solutions à pulvériser, qui contiennent un ou de plusieurs des composés de formule (I) ou leurs sels respectifs selon l'une quelconque des revendications 1 à 3, pour augmenter la résistance de plantes aux facteurs de stress abiotique.

10. Procédé d'augmentation de la tolérance au stress de plantes choisies dans le groupe constitué par les plantes utiles, les plantes ornementales, les variétés de gazon ou les arbres, **caractérisé en ce que** l'application d'une quantité non toxique suffisante d'un ou de plusieurs composés de formule (I) ou de leurs sels respectifs selon l'une quelconque des revendications 1 à 3 sur la surface où l'effet en question est souhaité, comprenant l'utilisation sur les plantes, leurs graines ou sur la surface sur laquelle les plantes poussent, a lieu.

11. Procédé selon la revendication 10, dans lequel la résistance des plantes ainsi traitées au stress abiotique est augmentée d'au moins 3 % par rapport à des plantes non traitées dans des conditions physiologiques autrement identiques.

12. Dihydrooxindolylsulfonamides substitués selon les formules (Ib) à (If), (Ii) à (Iu) et (Iw) ou leurs sels 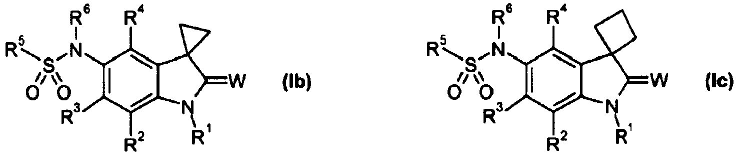 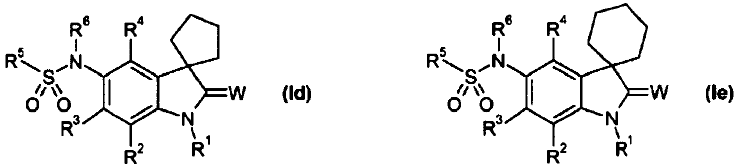 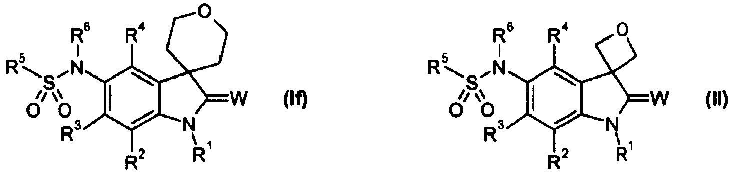 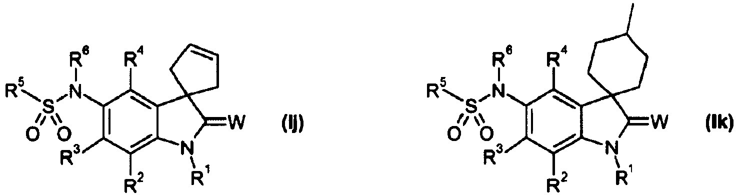 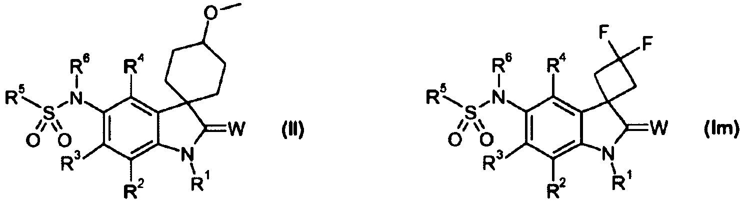 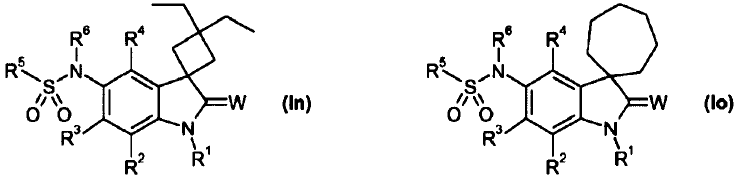 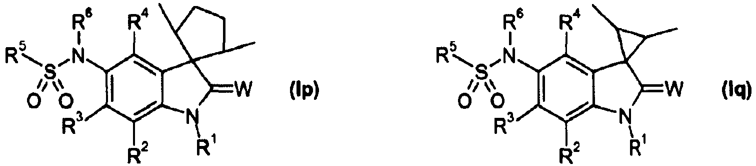 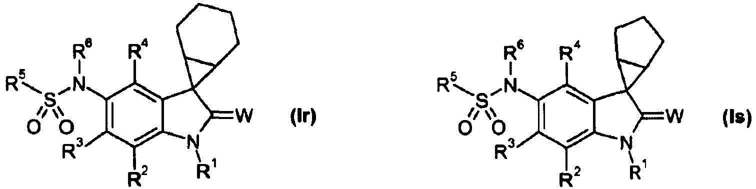 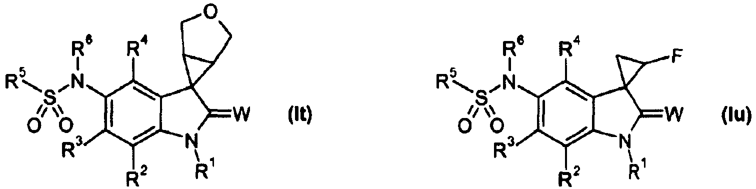 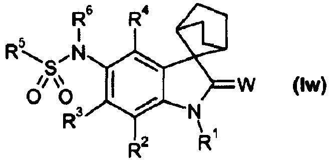 dans lesquelles
R¹ représente hydrogène, alkyle en (C₁-C₆), cycloalkyle en (C₃-C₆), haloalkyle en (C₁-C₆), halocycloalkyle en (C₃-C₆), alcényle en (C₂-C₆), haloalcényle en (C₂-C₆), alcoxy en (C₁-C₅)-haloalkyle en (C₁-C₅), alcynyle en (C₂-C₆), aryl-alkyle en (C₁-C₅), hétéroaryl-alkyle en (C₁-C₅), cycloalkyle en (C₃-C₆)-alkyle en (C₁-C₅), haloalcynyle en (C₂-C₅), hétérocyclyle, hétérocyclyl-alkyle en (C₁-C₅), alcoxy en (C₁-C₅)-alkyle en (C₁-C₅), alkylcarbonyle en (C₁-C₅)-alkyle en (C₁-C₅), hydroxycarbonyl-alkyle en (C₁-C₅), alcoxycarbonyle en (C₁-C₅)-alkyle en (C₁-C₅), alcényloxycarbonyle en (C₂-C₅)-alkyle en (C₁-C₅), alcynyloxycarbonyle en (C₂-C₅)-alkyle en (C₁-C₅), aryl-alcoxycarbonyle en (C₁-C₅)-alkyle en (C₁-C₅), cycloalcoxycarbonyle en (C₃-C₆)-alkyle en (C₁-C₅), cycloalkyle en (C₃-C₆) -alcoxycarbonyle en (C₁-C₅)-alkyle en (C₁-C₅), aminocarbonyl-alkyle en (C₁-C₅), alkylaminocarbonyle en (C₁-C₅) -alkyle en (C₁-C₅), cycloalkylaminocarbonyle en (C₃-C₆)-alkyle en (C₁-C₅), aryl-alkylaminocarbonyle en (C₁-C₅)-alkyle en (C₁-C₅), hétéroaryl-alkylaminocarbonyle en (C₁-C₅)-alkyle en (C₁-C₅), alkylthio en (C₁-C₅)-alkyle en (C₁-C₅), cycloalkylthio en (C₃-C₆)-alkyle en (C₁-C₅), arylthio-alkyle en (C₁-C₅), hétérocyclylthio-alkyle en (C₁-C₅), hétéroarylthio-alkyle en (C₁-C₅), aryl-alkylthio en (C₁-C₅)-alkyle en (C₁-C₅), alkylsulfinyle en (C₁-C₅)-alkyle en (C₁-C₅), alkylsulfonyle en (C₁-C₅)-alkyle en (C₁-C₅), arylsulfinyl-alkyle en (C₁-C₅), arylsulfonyl-alkyle en (C₁-C₅), cycloalkylsulfinyle en (C₃-C₆)-alkyle en (C₁-C₅), cycloalkylsulfonyle en (C₃-C₆)-alkyle en (C₁-C₅), alcoxy en (C₁-C₅)-alcoxy en (C₁-C₅)-alkyle en (C₁-C₅), alkylcarbonyle en (C₁-C₅), cycloalkylcarbonyle en (C₃-C₆), alcoxycarbonyle en (C₁-C₅), aryl-alcoxycarbonyle en (C₁-C₅), arylcarbonyle, hétéroarylcarbonyle, hétérocyclylcarbonyle, aryl-alkylcarbonyle en (C₁-C₅), alkylaminocarbonyle en (C₁-C₅), cycloalkylaminocarbonyle en (C₃-C₆), arylaminocarbonyle, aryl-alkylaminocarbonyle en (C₁-C₅), alkylsulfonyle en (C₁-C₅), cycloalkylsulfonyle en (C₃-C₆), arylsulfonyle, hétéroarylsulfonyle, hétérocyclylsulfonyle, cyano-alkyle en (C₁-C₅), bis-[alkyle en (C₁-C₅)]amino, cycloalkyle en (C₃-C₆) [alkyle en (C₁-C₅)]amino, R², R³, R⁴ représentent indépendamment les uns des autres hydrogène, fluor, chlore, brome, iode, alcoxy en (C₁-C₅), alkyle en (C₁-C₅), haloalkyle en (C₁-C₅), haloalcoxy en (C₁-C₅), alkylthio en (C₁-C₅), haloalkylthio en (C₁-C₅), aryle, hétéroaryle, hétérocyclyle, cycloalkyle en (C₃-C₆),
R⁵ représente amino, alkyle en (C₁-C₅), cycloalkyle en (C₃-C₆), cycloalkyle en (C₃-C₆)-alkyle en (C₁-C₅), haloalkyle en (C₁-C₅), halocycloalkyle en (C₃-C₆), cycloalcényle en (C₄-C₆), phényle éventuellement substitué, hétéroaryle, hétérocyclyle, aryl-alkyle en (C₁-C₅), hétéroaryl-alkyle en (C₁-C₅), hétérocyclyl-alkyle en (C₁-C₅), alcoxycarbonyle en (C₁-C₅)-alkyle en (C₁-C₅), aryl-alcoxycarbonyle en (C₁-C₅)-alkyle en (C₁-C₅), cycloalcoxycarbonyle en (C₃-C₆)-alkyle en (C₁-C₆), cycloalkyle en (C₃-C₆)-alcoxycarbonyle en (C₁-C₅)-alkyle en (C₁-C₅), hétéroaryl-alcoxycarbonyle en (C₁-C₅)-alkyle en (C₁-C₅), aminocarbonyl-alkyle en (C₁-C₅), alkylaminocarbonyle en (C₁-C₅)-alkyle en (C₁-C₅), cycloalkylaminocarbonyle en (C₃-C₆)-alkyle en (C₁-C₅), aryl-alkylaminocarbonyle en (C₁-C₅)-alkyle en (C₁-C₅), alkylamino en (C₁-C₅), bis-[alkyle en (C₁-C₅)]amino, arylamino, cycloalkylamino en (C₃-C₆), aryl-alkylamino en (C₁-C₅), hétéroaryl-alkylamino en (C₁-C₅), hétéroarylamino, hétérocyclylamino, alcénylamino en (C₂-C₅), alcynylamino en (C₂-C₅), aryloxy-alkyle en (C₁-C₅), hétéroaryloxy-alkyle en (C₁-C₅), alcoxy en (C₁-C₅)-alkyle en (C₁-C₅), alcényle en (C₂-C₆), alcynyle en (C₂-C₆), cyano-alkyle en (C₁-C₅), aryloxy, aryl-alcényle en (C₂-C₅), hétéroaryl-alcényle en (C₂-C₅), hétérocyclyl-alcényle en (C₂-C₅),
R⁶ représente hydrogène, alkyle en (C₁-C₅), cycloalkyle en (C₃-C₆), cyano-alkyle en (C₁-C₅), cycloalkyle en (C₃-C₆)-alkyle en (C₁-C₅), alkylsulfonyle en (C₁-C₅), arylsulfonyle, aryl-alkylsulfonyle en (C₁-C₅), hétéroarylsulfonyle, cycloalkylsulfonyle en (C₃-C₆), hétérocyclylsulfonyle, alkylcarbonyle en (C₁-C₅), arylcarbonyle, hétéroarylcarbonyle, cycloalkylcarbonyle en (C₃-C₆), hétérocyclylcarbonyle, alcoxycarbonyle en (C₁-C₅), aryl-alcoxycarbonyle en (C₁-C₅), haloalkylcarbonyle en (C₁-C₅), alcényle en (C₂-C₅), alcynyle en (C₂-C₅), haloalkyle en (C₁-C₅), halo-alcynyle en (C₂-C₅), halo-alcényle en (C₂-C₅), alcoxy en (C₁-C₅)-alkyle en (C₁-C₅), et
W représente oxygène ou soufre, de préférence oxygène.

13. Dihydrooxindolylsulfonamides substitués des formules (Ib) à (Ie), (Ij) à (Is), (Iu) et (Iw) ou leurs sels selon la revendication 2, dans lesquels
R¹ représente hydrogène, méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 1,1-diméthylpropyle, 1,2-diméthylpropyle, 2,2-diméthylpropyle, 1-éthylpropyle, n-hexyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,1,2-triméthylpropyle, 1,2,2-triméthylpropyle, 1-éthyl-1-méthylpropyle, 1-éthyl-2-méthylpropyle, cyclopropyle, cyclobutyle, cyclopentyle, cyclohexyle, spiro[2.2]pent-1-yle, spiro[2.3]hex-1-yle, spiro[2.3]hex-4-yle, 3-spiro[2.3]hex-5-yle, spiro[3.3]hept-1-yle, spiro[3.3]hept-2-yle, bicyclo[1.1.0]butan-1-yle, bicyclo[1.1,0]butan-2-yle, bicyclo[2.1.0]pentan-1-yle, bicyclo[1.1.1]pentan-1-yle, bicyclo[2.1.0]pentan-2-yle, bicyclo[2.1.0]pentan-5-yle, bicyclo[2.1.1]hexyle, bicyclo[2.2.1]hept-2-yle, bicyclo[2.2.2]octan-2-yle, bicyclo[3.2.1]octan-2-yle, bicyclo[3.2.2]nonan-2-yle, adamantan-1-yle, adamantan-2-yle, 1-méthylcyclopropyle, 2-méthylcyclopropyle, 2,2-diméthylcyclopropyle, 2,3-diméthylcyclopropyle, 1,1'-bi(cyclopropyl)-1-yle, 1,1'-bi(cyclopropyl)-2-yle, 2'-méthyl-1,1'-bi(cyclopropyl)-2-yle, 1-cyanopropyle, 2-cyanopropyle, 1-méthylcyclobutyle, 2-méthylcyclobutyle, 3-méthylcyclobutyle, 1-cyanocyclobutyle, 2-cyanocyclobutyle, 3-cyanocyclobutyle, 1-allylcyclopropyle, 1-vinylcyclobutyle, 1-vinylcyclopropyle, 1-éthylcyclopropyle, 2-éthylcyclopropyle, 1-éthylcyclobutyle, 2-éthylcyclobutyle, 3-éthylcyclobutyle, 4-méthylcyclohexyle, 4-méthoxycyclohexyle, 4-éthoxycyclohexyle, 4-n-propyloxycyclohexyle, 4-hydroxycyclohexyle, 4-méthoxycyclobutyle, 1-cyclopropylcyclobutyle, 1-prop-2-énylcyclobutyle, 2-éthyl-3-méthylcyclobutyle, 1-propylcyclopropyle, 1-méthyl-2-propylcyclopropyle, 2-propylcyclopropyle, 1-propylcyclobutyle, 2-propylcyclobutyle, 3-propylcyclobutyle, 1-iso-propylcyclobutyle, 1-iso-propylcyclopropyle, 2-iso-propylcyclopropyle, 3-iso-propylcyclobutyle, 2-diméthylaminocyclobutyle, 3-diméthylaminocyclobutyle, 1-butylcyclobutyle, 2-butylcyclobutyle, 1-butylcyclopropyle, 3-butylcyclobutyle, 2-butylcyclopropyle, 1-iso-butylcyclobutyle, 3-tert-butylcyclobutyle, 3,3-diéthylcyclobutyle, 2,2-diéthylcyclopropyle, 2-méthylidène-cyclopropyle, 1-méthoxyméthylcyclopropyle, 1-iso-butylcyclopropyle, 2,2-difluoréthyle, 2,2,2-trifluoréthyle, 3,3,3-trifluorpropyle, éthényle, 1-propényle, 2-propényle, 1-méthyl-éthényle, 1-butényle, 2-butényle, 3-butényle, 1-méthyl-1-propényle, 2-méthyl-1-propényle, 1-méthyl-2-propényle, 2-méthyl-2-propényle, 1-pentényle, 2-pentényle, 3-pentényle, 4-pentényle, 1-méthyl-1-butényle, 2-méthyl-1-butényle, 3-méthyl-1-butényle, 1-méthyl-2-butényle, 2-méthyl-2-butényle, 3-méthyl-2-butényle, 1-méthyl-3-butényle, 2-méthyl-3-butényle, 3-méthyl-3-butényle, 1,1-diméthyl-2-propényle, 1,2-diméthyl-1-propényle, 1,2-diméthyl-2-propényle, 1-éthyl-1-propényle, 1-éthyl-2-propényle, 1-hexényle, 2-hexényle, 3-hexényle, 4-hexényle, 5-hexényle, 1-méthyl-1-pentényle, 2-méthyl-1-pentényle, 3-méthyl-1-pentényle, 4-méthyl-1-pentényle, 1-méthyl-2-pentényle, 2-méthyl-2-pentényle, 3-méthyl-2-pentényle, 4-méthyl-2-pentényle, 1-méthyl-3-pentényle, 2-méthyl-3-pentényle, 3-méthyl-3-pentényle, 4-méthyl-3-pentényle, 1-méthyl-4-pentényle, 2-méthyl-4-pentényle, 3-méthyl-4-pentényle, 4-méthyl-4-pentényle, 1,1-diméthyl-2-butényle, 1,1-diméthyl-3-butényle, 1,2-diméthyl-1-butényle, 1,2-diméthyl-2-butényle, 1,2-diméthyl-3-butényle, 1,3-diméthyl-1-butényle, 1,3-diméthyl-2-butényle, 1,3-diméthyl-3-butényle, 2,2-diméthyl-3-butényle, 2,3-diméthyl-1-butényle, 2,3-diméthyl-2-butényle, 2,3-diméthyl-3-butényle, 3,3-diméthyl-1-butényle, 3,3-diméthyl-2-butényle, 1-éthyl-1-butényle, 1-éthyl-2-butényle, 1-éthyl-3-butényle, 2-éthyl-1-butényle, 2-éthyl-2-butényle, 2-éthyl-3-butényle, 1,1,2-triméthyl-2-propényle, 1-éthyl-1-méthyl-2-propényle, 1-éthyl-2-méthyl-1-propényle et 1-éthyl-2-méthyl-2-propényle, éthinyle, 2-propinyle, 2-butinyle, 3-butinyle, 1-méthyl-2-propinyle, 2-pentinyle, 3-pentinyle, 4-pentinyle, 1-méthyl-2-butinyle, 1-méthyl-3-butinyle, 2-méthyl-3-butinyle, 3-méthyl-1-butinyle, 1,1-diméthyl-2-propinyle, 1-éthyl-2-propinyle, 2-hexinyle, 3-hexinyle, 4-hexinyle, 5-hexinyle, 1-méthyl-2-pentinyle, 1-méthyl-3-pentinyle, 1-méthyl-4-pentinyle, 2-méthyl-3-pentinyle, 2-méthyl-4-pentinyle, 3-méthyl-1-pentinyle, 3-méthyl-4-pentinyle, 4-méthyl-1-pentinyle, 4-méthyl-2-pentinyle, 1,1-diméthyl-2-butinyle, 1,1-diméthyl-3-butinyle, 1,2-diméthyl-3-butinyle, 2,2-diméthyl-3-butinyle, 3,3-diméthyl-1-butinyle, 1-éthyl-2-butinyle, 1-éthyl-3-butinyle, 2-éthyl-3-butinyle, 1-éthyl-1-méthyl-2-propinyle, halocycloalkyle en (C₃-C₆), haloalcényle en (C₂-C₅), alcoxy en (C₁-C₅)-haloalkyle en (C₁-C₅), benzyle, p-chlorobenzyle, p-méthoxybenzyle, p-trifluorométhylbenzyle, p-méthylbenzyle, p-fluorobenzyle, p-bromobenzyle, p-iodobenzyle, p-méthylthiobenzyle, p-trifluorométhoxybenzyle, p-nitrobenzyle, p-trifluorométhylthiobenzyle, m-chlorobenzyle, m-méthoxybenzyle, m-trifluorométhylbenzyle, m-méthylbenzyle, m-fluorobenzyle, m-bromobenzyle, m-iodobenzyle, m-méthylthiobenzyle, m-trifluorométhoxybenzyle, m-nitrobenzyle, m-trifluorométhylthiobenzyle, o-chlorobenzyle, o-méthoxybenzyle, o-trifluorométhylbenzyle, o-méthylbenzyle, o-fluorobenzyle, o-bromobenzyle, o-iodobenzyle, o-méthylthiobenzyle, o-trifluorométhoxybenzyle, o-nitrobenzyle, o-trifluorométhylthiobenzyle, p-méthoxycarbonylbenzyle, p-éthoxycarbonylbenzyle, m-méthoxycarbonylbenzyle, m-éthoxycarbonylbenzyle, 2,4-dichlorobenzyle, 3,5-dichlorobenzyle, 2,4-difluorobenzyle, 3,5-difluorobenzyle, 3,4-dichlorobenzyle, 3,4-difluorobenzyle, 2,5-dichlorobenzyle, phényléthyle, p-chlorophényléthyle, p-méthoxyphényléthyle, p-trifluorométhylphényléthyle, p-fluorophényléthyle, p-trifluorométhoxyphényléthyle, p-trifluorométhylthiophényléthyle, p-méthylphényléthyle, p-nitrophényléthyle, p-méthoxycarbonylphényléthyle, p-éthoxycarbonylphényléthyle, m-chlorophényléthyle, m-méthoxyphényléthyle, m-trifluorométhylphényléthyle, m-fluorophényléthyle, m-trifluorométhoxyphényléthyle, m-trifluorométhylthiophényléthyle, m-méthylphényléthyle, m-nitrophényléthyle, m-méthoxycarbonylphényléthyle, méthoxycarbonylphényléthyle, o-chlorophényléthyle, o-méthoxyphényléthyle, o-trifluorométhylphényléthyle, o-fluorophényléthyle, o-trifluorométhoxyphényléthyle, o-trifluorométhylthiophényléthyle, o-méthylphényléthyle, o-nitrophényléthyle, o-méthoxycarbonylphényléthyle, o-éthoxycarbonylphényléthyle, hétéroaryl-alkyle en (C₁-C₅), haloalcényle en (C₁-C₅), hétérocyclyle, hétérocyclyl-alkyle en (C₁-C₅), alcoxy en (C₁-C₅)-alkyle en (C₁-C₅), alkylcarbonyle en (C₁-C₅)-alkyle en (C₁-C₅), hydroxycarbonyl-alkyle en (C₁-C₅), alcoxycarbonyle en (C₁-C₅)-alkyle en (C₁-C₅), alcényloxycarbonyle en (C₂-C₅)-alkyle en (C₁-C₅) , alcynyloxycarbonyle en (C₂-C₅)-alkyle en (C₁-C₅), aryl-alcoxycarbonyle en (C₁-C₅)-alkyle en (C₁-C₅), cycloalcoxycarbonyle en (C₃-C₆)-alkyle en (C₁-C₅), cycloalkyle en (C₃-C₆)-alcoxycarbonyle en (C₁-C₅)-alkyle en (C₁-C₅), aminocarbonyl-alkyle en (C₁-C₅), alkylaminocarbonyle en (C₁-C₅)-alkyle en (C₁-C₅), cycloalkylaminocarbonyle en (C₃-C₆)-alkyle en (C₁-C₅), aryl-alkylaminocarbonyle en (C₁-C₅)-alkyle en (C₁-C₅), hétéroaryl-alkylaminocarbonyle en (C₁-C₅)-alkyle en (C₁-C₅), alkylthio en (C₁-C₅)-alkyle en (C₁-C₅), cycloalkylthio en (C₃-C₆)-alkyle en (C₁-C₅), arylthio-alkyle en (C₁-C₅), hétérocyclylthio-alkyle en (C₁-C₅), hétéroarylthio-alkyle en (C₁-C₅), aryl-alkylthio en (C₁-C₅)-alkyle en (C₁-C₅), alkylsulfinyle en (C₁-C₅)-alkyle en (C₁-C₅), alkylsulfonyle en (C₁-C₅) -alkyle en (C₁-C₅), arylsulfinyl-alkyle en (C₁-C₅), arylsulfonyl-alkyle en (C₁-C₅), cycloalkylsulfinyle en (C₃-C₆)-alkyle en (C₁-C₅), cycloalkylsulfonyle en (C₃-C₆)-alkyle en (C₁-C₅), alcoxy en (C₁-C₅)-alcoxy en (C₁-C₅)-alkyle en (C₁-C₅), alkylcarbonyle en (C₁-C₅), cycloalkylcarbonyle en (C₃-C₆), alcoxycarbonyle en (C₁-C₅), aryl-alcoxycarbonyle en (C₁-C₅), arylcarbonyle, hétéroarylcarbonyle, hétérocyclylcarbonyle, aryl-alkylcarbonyle en (C₁-C₅), alkylaminocarbonyle en (C₁-C₅), cycloalkylaminocarbonyle en (C₃-C₆), arylaminocarbonyle, aryl-alkylaminocarbonyle en (C₁-C₅), alkylsulfonyle en (C₁-C₅), cycloalkylsulfonyle en (C₃-C₆), arylsulfonyle, hétéroarylsulfonyle, hétérocyclylsulfonyle, bis-[alkyle en (C₁-C₅)]amino, cycloalkyle en (C₃-C₆)[alkyle en (C₁-C₅)]amino,
R², R³, R⁴ représentent indépendamment les uns des autres hydrogène, fluor, chlore, brome, iode, méthoxy, éthoxy, n-propyloxy, iso-propyloxy, méthyle, éthyle, iso-propyle, trifluorométhyle, difluorométhyle, pentafluoroéthyle, trifluorométhoxy, difluorométhoxy, 2,2-difluoroéthoxy, 3,3,3-trifluoroéthoxy, méthylthio, éthylthio, trifluorométhylthio, phényle éventuellement substitué, hétéroaryle, hétérocyclyle, cyclopropyle, cyclobutyle, cyclopentyle, cyclohexyle,
R⁵ représente amino, méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 1,1-diméthylpropyle, 1,2-diméthylpropyle, 2,2-diméthylpropyle, 1-éthylpropyle, n-hexyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,1,2-triméthylpropyle, 1,2,2-triméthylpropyle, 1-éthyl-1-méthylpropyle, 1-éthyl-2-méthylpropyle, cyclopropyle, cyclobutyle, cyclopentyle, cyclohexyle, spiro[2.2]pent-1-yle, spiro[2.3]hex-1-yle, spiro[2.3]hex-4-yle, 3-spiro[2.3]hex-5-yle, cyclopropylméthyle, cyclobutylméthyle, cyclopentylméthyle, cyclohexylméthyle, trifluorométhyle, difluorométhyle, 2,2-difluoroéthyle, 2,2,2-trifluoroéthyle, 3,3,3-trifluoropropyle, pentafluoroéthyle, heptafluoro-n-propyle, heptafluoro-iso-propyle, nonafluoro-n-butyle, halocycloalkyle en (C₃-C₆), cycloalcényle en (C₄-C₆), phényle éventuellement substitué, hétéroaryle, hétérocyclyle, aryl-alkyle en (C₁-C₅), hétéroaryl-alkyle en (C₁-C₅), hétérocyclyl-alkyle en (C₁-C₅), alcoxycarbonyle en (C₁-C₅)-alkyle en (C₁-C₅), aryl-alcoxycarbonyle en (C₁-C₅)-alkyle en (C₁-C₅), cycloalcoxycarbonyle en (C₁-C₆)-alkyle en (C₁-C₅), cycloalkyle en (C₃-C₆)-alcoxycarbonyle en (C₁-C₅)-alkyle en (C₁-C₅), hétéroaryl-alcoxycarbonyle en (C₁-C₅)-alkyle en (C₁-C₅), aminocarbonyl-alkyle en (C₁-C₅), alkylaminocarbonyle en (C₁-C₅)-alkyle en (C₁-C₅), cycloalkylaminocarbonyle en (C₃-C₆)-alkyle en (C₁-C₅), aryl-alkylaminocarbonyle en (C₁-C₅)-alkyle en (C₁-C₅), alkylamino en (C₁-C₅), arylamino, cycloalkylamino en (C₃-C₆), aryl-alkylamino en (C₁-C₅), hétéroaryl-alkylamino en (C₁-C₅), hétéroarylamino, hétérocyclylamino, alcénylamino en (C₂-C₅), alcynylamino en (C₂-C₅), aryloxy-alkyle en (C₁-C₅), hétéroaryloxy-alkyle en (C₁-C₅), alcoxy en (C₁-C₅)-alkyle en (C₁-C₅), phényléthényle, p-chlorophényléthényle, p-méthylphényléthényle, p-méthoxyphényléthényle, p-trifluorométhylphényléthényle, p-fluorophényléthényle, p-cyanophényléthényle, p-trifluorométhoxyphényléthényle, p-nitrophényléthényle, p-bromophényléthényle, p-iodophényléthényle, m-chlorophényléthényle, m-méthylphényléthényle, m-méthoxyphényléthényle, m-trifluorométhylphényléthényle, m-fluorophényléthényle, m-cyanophényléthényle, m-trifluorométhoxyphényléthényle, m-nitrophényléthényle, m-bromophényléthényle, m-iodophényléthényle, p-méthoxycarbonylphényléthényle, m-méthoxycarbonylphényléthényle, o-méthoxycarbonylphényléthényle, p-éthoxycarbonylphényléthényle, m-éthoxycarbonylphényléthényle, o-éthoxycarbonylphényléthényle, éthényle, 1-propényle, 2-propényle, 1-méthyl-éthényle, 1-butényle, 2-butényle, 3-butényle, 1-méthyl-1-propényle, 2-méthyl-1-propényle, 1-méthyl-2-propényle, 2-méthyl-2-propényle, 1-pentényle, 2-pentényle, 3-pentényle, 4-pentényle, 1-méthyl-1-butényle, 2-méthyl-1-butényle, 3-méthyl-1-butényle, 1-méthyl-2-butényle, 2-méthyl-2-butényle, 3-méthyl-2-butényle, 1-méthyl-3-butényle, 2-méthyl-3-butényle, 3-méthyl-3-butényle, 1,1-diméthyl-2-propényle, 1,2-diméthyl-1-propényle, 1,2-diméthyl-2-propényle, 1-éthyl-1-propényle, 1-éthyl-2-propényle, 1-hexényle, 2-hexényle, 3-hexényle, 4-hexényle, 5-hexényle, 1-méthyl-1-pentényle, 2-méthyl-1-pentényle, 3-méthyl-1-pentényle, 4-méthyl-1-pentényle, 1-méthyl-2-pentényle, 2-méthyl-2-pentényle, 3-méthyl-2-pentényle, 4-méthyl-2-pentényle, 1-méthyl-3-pentényle, 2-méthyl-3-pentényle, 3-méthyl-3-pentényle, 4-méthyl-3-pentényle, 1-méthyl-4-pentényle, 2-méthyl-4-pentényle, 3-méthyl-4-pentényle, 4-méthyl-4-pentényle, 1,1-diméthyl-2-butényle, 1,1-diméthyl-3-butényle, 1,2-diméthyl-1-butényle, 1,2-diméthyl-2-butényle, 1,2-diméthyl-3-butényle, 1,3-diméthyl-1-butényle, 1,3-diméthyl-2-butényle, 1,3-diméthyl-3-butényle, 2,2-diméthyl-3-butényle, 2,3-diméthyl-1-butényle, 2,3-diméthyl-2-butényle, 2,3-diméthyl-3-butényle, 3,3-diméthyl-1-butényle, 3,3-diméthyl-2-butényle, 1-éthyl-1-butényle, 1-éthyl-2-butényle, 1-éthyl-3-butényle, 2-éthyl-1-butényle, 2-éthyl-2-butényle, 2-éthyl-3-butényle, 1,1,2-triméthyl-2-propényle, 1-éthyl-1-méthyl-2-propényle, 1-éthyl-2-méthyl-1-propényle et 1-éthyl-2-méthyl-2-propényle, éthinyle, 1-propinyle, 2-propinyle, 1-butinyle, 2-butinyle, 3-butinyle, 1-méthyl-2-propinyle, 1-pentinyle, 2-pentinyle, 3-pentinyle, 4-pentinyle, 1-méthyl-2-butinyle, 1-méthyl-3-butinyle, 2-méthyl-3-butinyle, 3-méthyl-1-butinyle, 1,1-diméthyl-2-propinyle, 1-éthyl-2-propinyle, 1-hexinyle, 2-hexinyle, 3-hexinyle, 4-hexinyle, 5-hexinyle, 1-méthyl-2-pentinyle, 1-méthyl-3-pentinyle, 1-méthyl-4-pentinyle, 2-méthyl-3-pentinyle, 2-méthyl-4-pentinyle, 3-méthyl-1-pentinyle, 3-méthyl-4-pentinyle, 4-méthyl-1-pentinyle, 4-méthyl-2-pentinyle, 1,1-diméthyl-2-butinyle, 1,1-diméthyl-3-butinyle, 1,2-diméthyl-3-butinyle, 2,2-diméthyl-3-butinyle, 3,3-diméthyl-1-butinyle, 1-éthyl-2-butinyle, 1-éthyl-3-butinyle, 2-éthyl-3-butinyle, 1-éthyl-1-méthyl-2-propinyle, cyanoéthyle, cyanométhyle, cyano-n-propyle, cyano-n-butyle, aryloxy, bis-[alkyle en (C₁-C₅)]amino, aryl-alcényle en (C₂-C₅), hétéroaryl-alcényle en (C₂-C₅), hétérocyclyl-alcényle en (C₂-C₅),
R⁶ représente hydrogène, méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 1,1-diméthylpropyle, 1,2-diméthylpropyle, 2,2-diméthylpropyle, 1-éthylpropyle, n-hexyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,1,2-triméthylpropyle, 1,2,2-triméthylpropyle, 1-éthyl-1-méthylpropyle, 1-éthyl-2-méthylpropyle, cyclopropyle, cyclobutyle, cyclopentyle, cyclohexyle, cyclopropylméthyle, cyclopropyléthyle, cyclobutylméthyle, cyclopentylméthyle, cyclohexylméthyle, 2,2-difluoréthyle, 2,2,2-trifluoréthyle, 3,3,3-trifluorpropyle, cyanométhyle, cyanoéthyle, cyano-n-propyle, cyclopropyle, carbonyle, cyclobutylcarbonyle, cyclopentylcarbonyle, cyclohexylcarbonyle, méthoxycarbonyle, alkylsulfonyle en (C₁-C₅), arylsulfonyle, aryl-alkylsulfonyle en (C₁-C₅), hétéroarylsulfonyle, cycloalkylsulfonyle en (C₃-C₆), hétérocyclylsulfonyle, alkylcarbonyle en (C₁-C₅), arylcarbonyle, hétéroarylcarbonyle, hétérocyclylcarbonyle, alcoxycarbonyle en (C₁-C₅), aryl-alcoxycarbonyle en (C₁-C₅), haloalkylcarbonyle en (C₁-C₅), alcényle en (C₂-C₅), alcynyle en (C₂-C₅), haloalcynyle en (C₂-C₅), halo-alcényle en (C₂-C₅), alcoxy en (C₁-C₅)-alkyle en (C₁-C₅), et
W représente oxygène ou soufre.

14. Dihydrooxindolylsulfonamides substitués des formules (Ib) à (Ie), (Ij) à (Is), (Iu) et (Iw) ou leurs sels selon la revendication 12, dans lesquels
R¹ représente méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 1,1-diméthylpropyle, 1,2-diméthylpropyle, 2,2-diméthylpropyle, 1-éthylpropyle, n-hexyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,1,2-triméthylpropyle, 1,2,2-triméthylpropyle, 1-éthyl-1-méthylpropyle, 1-éthyl-2-méthylpropyle, cyclopropyle, cyclobutyle, cyclopentyle, cyclohexyle, spiro[2.2]pent-1-yle, spiro[2.3]hex-1-yle, spiro[2.3]hex-4-yle, 3-spiro[2.3]hex-5-yle, spiro[3.3]hept-1-yle, spiro[3.3]hept-2-yle, bicyclo[1.1,0]butan-1-yle, bicyclo[1.1,0]butan-2-yle, bicyclo[2.1.0]pentan-1-yle, bicyclo[1.1.1]pentan-1-yle, bicyclo[2.1.0]pentan-2-yle, bicyclo[2.1.0]pentan-5-yle, bicyclo[2.1.1]hexyle, bicyclo[2.2.1]hept-2-yle, bicyclo[2.2.2]octan-2-yle, bicyclo[3.2.1]octan-2-yle, bicyclo[3.2.2]nonan-2-yle, adamantan-1-yle, adamantan-2-yle, 1-méthylcyclopropyle, 2-méthylcyclopropyle, 2,2-diméthylcyclopropyle, 2,3-diméthylcyclopropyle, 1,1'-bi(cyclopropyl)-1-yle, 1,1'-bi(cyclopropyl)-2-yle, 2'-méthyl-1,1'-bi(cyclopropyl)-2-yle, 1-cyanopropyle, 2-cyanopropyle, 1-méthylcyclobutyle, 2-méthylcyclobutyle, 3-méthylcyclobutyle, 1-cyanocyclobutyle, 2-cyanocyclobutyle, 3-cyanocyclobutyle, 1-allylcyclopropyle, 1-vinylcyclobutyle, 1-vinylcyclopropyle, 1-éthylcyclopropyle, 2-éthylcyclopropyle, 1-éthylcyclobutyle, 2-éthylcyclobutyle, 3-éthylcyclobutyle, 4-méthylcyclohexyle, 4-méthoxycyclohexyle, 4-éthoxycyclohexyle, 4-n-propyloxycyclohexyle, 4-hydroxycyclohexyle, 4-méthoxycyclobutyle, 1-cyclopropylcyclobutyle, 1-prop-2-énylcyclobutyle, 2-éthyl-3-méthylcyclobutyle, 1-propylcyclopropyle, 1-méthyl-2-propylcyclopropyle, 2-propylcyclopropyle, 1-propylcyclobutyle, 2-propylcyclobutyle, 3-propylcyclobutyle, 1-iso-propylcyclobutyle, 1-iso-propylcyclopropyle, 2-iso-propylcyclopropyle, 3-iso-propylcyclobutyle, 2-diméthylaminocyclobutyle, 3-diméthylaminocyclobutyle, 1-butylcyclobutyle, 2-butylcyclobutyle, 1-butylcyclopropyle, 3-butylcyclobutyle, 2-butylcyclopropyle, 1-iso-butylcyclobutyle, 3-tert-butylcyclobutyle, 3,3-diéthylcyclobutyle, 2,2-diéthylcyclopropyle, 2-méthylidène-cyclopropyle, 1-méthoxyméthylcyclopropyle, 1-iso-butylcyclopropyle, 2,2-difluoroéthyle, 2,2,2-trifluoroéthyle, 3,3,3-trifluoropropyle, éthényle, 1-propényle, 2-éthyl-3-butinyle, 2-méthyl-3-butinyle, 3-méthyl-1-butinyle, 1,1-diméthyl-2-propinyle, 1-éthyl-2-propinyle, 2-hexinyle, 3-hexinyle, 4-hexinyle, 5-hexinyle, 1-méthyl-2-pentinyle, 1-méthyl-3-pentinyle, 1-méthyl-4-pentinyle, 2-méthyl-3-pentinyle, 2-méthyl-4-pentinyle, 3-méthyl-1-pentinyle, 3-méthyl-4-pentinyle, 4-méthyl-1-pentinyle, 4-méthyl-2-pentinyle, 1,1-diméthyl-2-butinyle, 1,1-diméthyl-3-butinyle, 1,2-diméthyl-3-butinyle, 2,2-diméthyl-3-butinyle, 3,3-diméthyl-1-butinyle, 1-éthyl-2-butinyle, 1-éthyl-3-butinyle, 2-éthyl-3-butinyle, 1-éthyl-1-méthyl-2-propinyle, halocycloalkyle en (C₃-C₆), haloalcényle en (C₂-C₅), alcoxy en (C₁-C₅)-haloalkyle en (C₁-C₅), benzyle, p-chlorobenzyle, p-méthoxybenzyle, p-trifluorométhylbenzyle, p-méthylbenzyle, p-fluorobenzyle, p-bromobenzyle, p-iodobenzyle, p-méthylthiobenzyle, p-trifluorométhoxybenzyle, p-nitrobenzyle, p-trifluorométhylthiobenzyle, m-chlorobenzyle, m-méthoxybenzyle, m-trifluorométhylbenzyle, m-méthylbenzyle, m-fluorobenzyle, m-bromobenzyle, m-iodobenzyle, m-méthylthiobenzyle, m-trifluorométhoxybenzyle, m-nitrobenzyle, m-trifluorométhylthiobenzyle, o-chlorobenzyle, o-méthoxybenzyle, o-trifluorométhylbenzyle, o-méthylbenzyle, o-fluorobenzyle, o-bromobenzyle, o-iodobenzyle, o-méthylthiobenzyle, o-trifluorométhoxybenzyle, o-nitrobenzyle, o-trifluorométhylthiobenzyle, p-méthoxycarbonylbenzyle, p-éthoxycarbonylbenzyle, m-méthoxycarbonylbenzyle, m-éthoxycarbonylbenzyle, 2,4-dichlorobenzyle, 3,5-dichlorobenzyle, 2,4-difluorobenzyle, 3,5-difluorobenzyle, 3,4-dichlorobenzyle, 3,4-difluorobenzyle, 2,5-dichlorobenzyle, phényléthyle, p-chlorophényléthyle, p-méthoxyphényléthyle, p-trifluorométhylphényléthyle, p-fluorophényléthyle, p-trifluorométhoxyphényléthyle, p-trifluorométhylthiophényléthyle, p-méthylphényléthyle, p-nitrophényléthyle, p-méthoxycarbonylphényléthyle, p-éthoxycarbonylphényléthyle, m-chlorophényléthyle, m-méthoxyphényléthyle, m-trifluorométhylphényléthyle, m-fluorophényléthyle, m-trifluorométhoxyphényléthyle, m-trifluorométhylthiophényléthyle, m-méthylphényléthyle, m-nitrophényléthyle, m-méthoxycarbonylphényléthyle, méthoxycarbonylphényléthyle, o-chlorophényléthyle, o-méthoxyphényléthyle, o-trifluorométhylphényléthyle, o-fluorophényléthyle, o-trifluorométhoxyphényléthyle, o-trifluorométhylthiophényléthyle, o-méthylphényléthyle, o-nitrophényléthyle, o-méthoxycarbonylphényléthyle, o-éthoxycarbonylphényléthyle, hétéroaryl-alkyle en (C₁-C₅), haloalcényle en (C₁-C₅), héterocyclyle, hétérocyclyl-alkyle en (C₁-C₅), alcoxy en (C₁-C₅)-alkyle en (C₁-C₅), alkylcarbonyle en (C₁-C₅)-alkyle en (C₁-C₅), hydroxycarbonyl-alkyle en (C₁-C₅), alcoxycarbonyle en (C₁-C₅)-alkyle en (C₁-C₅), alcényloxycarbonyle en (C₂-C₅)-alkyle en (C₁-C₅), alcynyloxycarbonyle en (C₂-C₅)-alkyle en (C₁-C₅), aryl-alcoxycarbonyle en (C₁-C₅)-alkyle en (C₁-C₅), cycloalcoxycarbonyle en (C₃-C₆)-alkyle en (C₁-C₅), cycloalkyle en (C₃-C₆) -alcoxycarbonyle en (C₁-C₅)-alkyle en (C₁-C₅), aminocarbonyl-alkyle en (C₁-C₅), alkylaminocarbonyle en (C₁-C₅)-alkyle en (C₁-C₅), cycloalkylaminocarbonyle en (C₃-C₆)-alkyle en (C₁-C₅), aryl-alkylaminocarbonyle en (C₁-C₅)-alkyle en (C₁-C₅), hétéroaryl-alkylaminocarbonyle en (C₁-C₅)-alkyle en (C₁-C₅), alkylthio en (C₁-C₂)-alkyle en (C₁-C₅), cycloalkylthio en (C₃-C₆)-alkyle en (C₁-C₅), arylthio-alkyle en (C₁-C₅), hétérocyclylthio-alkyle en (C₁-C₅), hétéroarylthio-alkyle en (C₁-C₅), aryl-alkylthio en (C₁-C₅)-alkyle en (C₁-C₅), alkylsulfinyle en (C₁-C₅)-alkyle en (C₁-C₅), alkylsulfonyle en (C₁-C₅)-alkyle en (C₁-C₅), arylsulfinyl-alkyle en (C₁-C₅), arylsulfonyl-alkyle en (C₁-C₅), cycloalkylsulfinyle en (C₃-C₆-alkyle en (C₁-C₅), cycloalkylsulfonyle en (C₃-C₆)-alkyle en (C₁-C₅), alcoxy en (C₁-C₅-alcoxy en (C₁-C₅)-alkyle en (C₁-C₅), alkylcarbonyle en (C₁-C₅), cycloalkylcarbonyle en (C₃-C₆), alcoxycarbonyle en (C₁-C₅), aryl-alcoxycarbonyle en (C₁-C₅), arylcarbonyle, hétéroarylcarbonyle, hétérocyclylcarbonyle, aryl-alkylcarbonyle en (C₁-C₅), alkylaminocarbonyle en (C₁-C₅), cycloalkylaminocarbonyle en (C₃-C₆), arylaminocarbonyle, aryl-alkylaminocarbonyle en (C₁-C₅), alkylsulfonyle en (C₁-C₅), cycloalkylsulfonyle en (C₃-C₆), arylsulfonyle, hétéroarylsulfonyle, hétérocyclylsulfonyle, bis-[alkyle en (C₁-C₅)]amino, cycloalkyle en (C₃-C₆)[alkyle en (C₁-C₅)]amino,
R², R³, R⁴ représentent indépendamment les uns des autres hydrogène, fluor, chlore, brome, iode, méthoxy, éthoxy, n-propyloxy, iso-propyloxy, méthyle, éthyle, iso-propyle, trifluorométhyle, difluorométhyle, pentafluoroéthyle, trifluorométhoxy, difluorométhoxy,2,2-difluoroéthoxy, 3,3,3-trifluoroéthoxy, méthylthio, éthylthio, trifluorométhylthio, phényle éventuellement substitué, hétéroaryle, hétérocyclyle, cyclopropyle, cyclobutyle, cyclopentyle, cyclohexyle,
R⁵ représente phényle éventuellement substitué, hétéroaryle, hétérocyclyle, aryl-alkyle en (C₁-C₅), hétéroaryl-alkyle en (C₁-C₅), hétérocyclyl-alkyle en (C₁-C₅), alcoxycarbonyle en (C₁-C₅)-alkyle en (C₁-C₅), aryl-alcoxycarbonyle en (C₁-C₅)-alkyle en (C₁-C₅), cycloalcoxycarbonyle en (C₁-C₆)-alkyle en (C₁-C₅), cycloalkyle en (C₃-C₆) -alcoxycarbonyle en (C₁-C₅) -alkyle en (C₁-C₅), hétéroaryl-alcoxycarbonyle en (C₁-C₅)-alkyle en (C₁-C₅), aminocarbonyl-alkyle en (C₁-C₅), alkylaminocarbonyle en (C₁-C₅)-alkyle en (C₁-C₅), cycloalkylaminocarbonyle en (C₃-C₆)-alkyle en (C₁-C₅), aryl-alkylaminocarbonyle en (C₁-C₅)-alkyle en (C₁-C₅), aryloxy-alkyle en (C₁-C₅), hétéroaryloxy-alkyle en (C₁-C₅), phényléthényle, p-chlorophényléthényle, p-méthylphényléthényle, p-méthoxyphényléthényle, p-trifluorométhylphényléthényle, p-fluorophényléthényle, p-cyanophényléthényle, p-trifluorométhoxyphényléthényle, p-nitrophényléthényle, p-bromophényléthényle, p-iodophényléthényle, m-chlorophényléthényle, m-méthylphényléthényle, m-méthoxyphényléthényle, m-trifluorométhylphényléthényle, m-fluorophényléthényle, m-cyanophényléthényle, m-trifluorométhoxyphényléthényle, m-nitrophényléthényle, m-bromophényléthényle, m-iodophényléthényle, p-méthoxycarbonylphényléthényle, m-méthoxycarbonylphényléthényle, o-méthoxycarbonylphényléthényle, p-éthoxycarbonylphényléthényle, m-éthoxycarbonylphényléthényle, o-éthoxycarbonylphényléthényle, hétéroaryl-alcényle en (C₂-C₅), hétérocyclyl-alcényle en (C₂-C₅),
R⁶ représente hydrogène, méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 1,1-diméthylpropyle, 1,2-diméthylpropyle, 2,2-diméthylpropyle, 1-éthylpropyle, n-hexyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,1,2-triméthylpropyle, 1,2,2-triméthylpropyle, 1-éthyl-1-méthylpropyle, 1-éthyl-2-méthylpropyle, cyclopropyle, cyclobutyle, cyclopentyle, cyclohexyle, cyclopropylméthyle, cyclopropyléthyle, cyclobutylméthyle, cyclopentylméthyle, cyclohexylméthyle, 2,2-difluoréthyle, 2,2,2-trifluoréthyle, 3,3,3-trifluorpropyle, cyanométhyle, cyanoéthyle, cyano-n-propyle, cyclopropyle, carbonyle, cyclobutylcarbonyle, cyclopentylcarbonyle, cyclohexylcarbonyle, méthoxycarbonyle, alkylsulfonyle en (C₁-C₅), arylsulfonyle, aryl-alkylsulfonyle en (C₁-C₅), hétéroarylsulfonyle, cycloalkylsulfonyle en (C₃-C₆), hétérocyclylsulfonyle, alkylcarbonyle en (C₁-C₅), arylcarbonyle, hétéroarylcarbonyle, hétérocyclylcarbonyle, alcoxycarbonyle en (C₁-C₅), aryl-alcoxycarbonyle en (C₁-C₅), haloalkylcarbonyle en (C₁-C₅), alcényle en (C₂-C₅), alcynyle en (C₂-C₅), halo-alcynyle en (C₂-C₅), halo-alcényle en (C₂-C₅), alcoxy en (C₁-C₅)-alkyle en (C₁-C₅) et
W représente oxygène ou soufre.

15. Solution à pulvériser pour le traitement de plantes, contenant une quantité efficace pour augmenter la résistance de plantes aux facteurs de stress abiotique d'un ou de plusieurs dihydrooxindolylsulfonamides substitués selon l'une quelconque des revendications 12, 13 ou 14.
